# EUROPEAN PATENT APPLICATION

(11) **EP 1 865 062 A2**
(43) Date of publication of application: **12.12.2007**
(21) Application number: 07011369.1
(22) Date of filing: 17.08.2001
(51) Int. Cl.: C12N 15/31, C07K 14/20, C12N 15/62, C07K 19/00, C12N 15/63, A61K 48/00, A61K 39/02, G01N 33/53

(54) **Altered OspA of Borrelia burgdorferi**

(30) Priority: 18.08.2000 US 226484 P
(62) Divisional of application: 01964163.8
(71) Applicant: RESEARCH FOUNDATION OF STATE UNIVERSITY OF NEW YORK, New York 11794-3368 (US); Brookhaven Sciences Associates, LLC, Upton, New York 11973-5000 (US); UNIVERSITY OF ROCHESTER, Rochester, NY 14642 (US)
(72) Inventor: Luft, Benjamin J., East Setauket, NY 11733-3951 (US); Dunn, John J., Bellport, NY 11713-2417 (US); Lawson, Catherine I., Pisacataway, NJ 08854 (US); Koide, Shohei, Chicago, IL 60615 (US)
(74) Representative: Kirkham, Nicholas Andrew

(57) **Abstract**

Provided herein are OspA polypeptides from Lyme Disease-causing *Borrelia* having certain alteration(s). In one embodiment, the alteration(s) increase the conformational stability of the OspA polypeptide containing the alteration(s) while maintaining at least some of the antigenicity of the corresponding unaltered OspA polypeptide. In another embodiment, the altered OspA polypeptide has reduced cross-reactivity to hLFA-1, as compared to the corresponding unaltered OspA polypeptide.

## Description

### RELATED APPLICATIONS

This application claims the benefit of U.S. Provisional Application No. 60/226,484, filed August 18, 2000, the teachings of which are incorporated herein by reference in their entirety.

### GOVERNMENT SUPPORT

The invention was supported, in whole or in part, by Grant 2R01AI37256-05A1 from the National Institute of Allergy and Infectious Diseases. The Government has certain rights in the invention.

### BACKGROUND OF THE INVENTION

Lyme disease (Lyme borreliosis) is the most common tick-borne infectious disease in North America and Europe, and has been found in Russia, Japan, China and Australia. Lyme disease begins at the site of a tick bite, producing a primary infection with spread of the organism to secondary sites occurring during the course of infection. The causative bacterial agent of this disease is the spirochete *Borrelia burgdorferi,* which was first isolated and cultivated in 1982 (Burgdorferi, W.A. et al., Science 216: 1317-1319 (1982); Steere, A.R. et al., N. Engl. J. Med., 308:733-740 (1983)).

Three pathogenic genospecies of *Borrelia, B. burgdorferi sensu stricto (B. burgdorferi* or *B.b.s.s.*)*, B. afzelii* and *B. garinii* have been described (Baranton, G., et al., Int. J. Syst. Bacteriol., 42:378-383 (1992)). These are members of a species complex, *B. burgdorferi* sensu lato, which consists of at least 10 different genospecies (Piken, R.N. et al., J. Invest. Dermatol., 110:211-214 (1998); Postic, D. et al., Int. J. Syst. Bacteriol., 44:743-752 (1994); Valsangiacomo, C.T. et al., Int. J. Syst. Bacteriol., 47:1-10 (1997)). The three genospecies, *B. burgdorferi sensu stricto, B. afzelii* and *B. garinii,* are all thought to be pathogenic and all are found in Europe.

*B. burgdorferi* has an outer membrane whose major protein constituents are the outer surface proteins A and B (OspA and OspB). OspA is a basic lipoprotein of approximately 31 kd, which is encoded on a large linear plasmid along with OspB, a basic lipoprotein of approximately 34 kd (Szczepanski, A., and J.L. Benach, Microbiol. Rev., 55:21 (1991)). The immune response to these outer surface proteins tends to occur late in the disease, if at all (Craft, J. E. et al., J. Clin Invest. 78:934-939 (1986); Dattwyler, R.J. and B.J. Luft, Rheum. Clin. North Am., 15:727-734 (1989)). Furthermore, patients acutely and chronically infected with *B. burgdorferi* respond variably to the different antigens, including OspA, OspB, OspC, OspD, p39, p41 and p93.

Currently, Lyme Disease is treated with a range of antibiotics, e.g., tetracyclines, penicillin and cephalosporins. However, such treatment is not always successful in clearing the infection. Treatment is often delayed due to improper diagnosis with the deleterious effect that the infection proceeds to a chronic condition, where treatment with antibiotics is often not useful. One of the factors contributing to delayed treatment is the lack of effective diagnostic tools.

Vaccines against Lyme borreliosis have been attempted. However, a vaccine that consists of recombinant OspA may require frequent booster immunizations. An additional concern of OspA-based vaccines is the recent identification of a putative autoreactive OspA domain with a high degree of similarity to a region of human leukocyte function-associated antigen-1 (hLFA-1) (Gross, D.M. et al., Science, 281: 703-706 (1998)).

Therefore, it should be advantageous to develop modified OspA proteins having decreased cross-reactivity to hLFA-1 in order to reduce potential side effects of an OspA vaccine. Development of OspA proteins with decreased hLFA-1 cross-reactivity that maintain or have increased immunoreactivity to more than one member of the *Borrelia* complex would also be desirable. To be useful as vaccines, the conformations of these modified proteins must be sufficiently stable to retain certain OspA structural features that are required to elicit a protective immune response. OspA proteins with these features would allow for improvements in diagnosis and/or vaccination against all, or most, of the *Borrelia* that cause Lyme Disease.

Analysis of the immune status of OspA immunized individuals revealed that the overall quantitative response is not predictive of protection, but rather the reactivity with a specific epitope of the OspA lipoprotein directly correlates to protective immunity. The anti-OspA monoclonal antibody, LA-2 (Kramer *et al.,* 1990) defines an epitope of the lipoprotein that is apparently necessary for protective immunity after OspA vaccination. For instance, passive immunization of mice with this antibody leads to protection against infection with the spirochete (Schaible *et al.,* 1993). In addition, immunization of mice and canines with OspA resulting in significant titers of LA-2 equivalent serum antibody accurately predicts protection from tick transmission of infection (Golde, 1997). Insufficient levels of LA-2 equivalent antibody result in a lack of protection in the face of high serum antibody titers to OspA (Johnson *et al.,* 1995)*.*

### SUMMARY OF THE INVENTION

The present invention is drawn to altered forms of OspA from *Borrelia burgdorferi* that have increased conformational stability while maintaining at least some of the antigenicity of wild type OspA. In some embodiments, the altered OspA polypeptide has decreased cross-reactivity with hLFA-1, as compared to the corresponding unaltered OspA polypeptide. The altered OspA polypeptides can comprise almost all or only a portion of the native OspA polypeptide. In some embodiments, the altered OspA polypeptide can be part of a cocktail which includes one or more other proteins, such as, for example, other *Borrelia burgdorferi* polypeptides including OspA, OspB, OspC, OspD, p93 and p41. In other embodiments, the altered OspA polypeptide can be part of a chimeric protein, such as those described in U.S. Patent No. 6,248,562, the entire teachings of which are incorporated herein by reference.

In one embodiment, the altered OspA polypeptides of the present invention comprise an amino acid sequence of OspA protein from *Borrelia burgdorferi* from about residue 139 to about residue 273, wherein the sequence includes at least one alteration selected from the group consisting of: residue 139 changed to methionine, residue 160 changed to tyrosine, residue 189 changed to methionine and combinations thereof. In other embodiments, the altered OspA polypeptides of the present invention comprise an amino acid sequence of OspA protein from *Borrelia burgdorferi* from about residue 131 to about residue 273 or from about residue 17 to about residue 273. The OspA polypeptides of the present invention can comprise longer or shorter fragments of OspA protein. The numbering of the residues corresponds to the numbering of SEQ ID NO:7 (OspA from B31).

In another embodiment, the OspA polypeptides of the present invention comprise an amino acid sequence of OspA protein from *Borrelia burgdorferi* from about residue 160 to about residue 170, wherein the sequence includes at least two alterations selected from the group consisting of: residue 165 changed to phenylalanine, residue 166 changed to threonine and residue 170 changed to lysine. In yet another embodiment, the OspA polypeptides of the present invention comprise an amino acid sequence of OspA protein from a *sensu stricto* strain of *Borrelia burgdorferi* from about residue 160 to about residue 170, wherein the sequence includes at least one alteration selected from the group consisting of: residue 165 changed to phenylalanine, residue 166 changed to threonine, residue 170 changed to lysine and combinations thereof. In other embodiments, the altered OspA polypeptides of the present invention comprise an amino acid sequence of OspA protein from *Borrelia burgdorferi* from about residue 150 to about residue 180 or from about residue 17 to about residue 273. The OspA polypeptides of the present invention can comprise longer or shorter fragments of OspA protein. The numbering of the residues corresponds to the numbering of SEQ ID NO:7.

The polypeptides of the present invention include polypeptides selected from the group consisting of: SEQ ID NO:96, 98, 100, 102, 104, 106, 108, 110, 112, 114 and 116.

The present invention is also drawn to polynucleotides encoding the amino acid sequences described herein, such as polynucleotides encoding OspA polypeptides from *Borrelia burgdorferi* from about residue 131 to about residue 273, wherein the sequence encodes at least one alteration selected from the group consisting of: codon 139 encoding methionine, codon 160 encoding tyrosine, codon 189 encoding methionine and combinations thereof. The polynucleotide encoding OspA polypeptides of the present invention can encode longer or shorter fragments of OspA protein. The numbering of the residues corresponds to the numbering of SEQ ID NO:7.

In another embodiment, the polynucleotide encodes an amino acid sequence of an OspA polypeptide from *Borrelia burgdorferi* from about residue 160 to about residue 170, wherein the sequence encodes at least one alteration selected from the group consisting of: codon 165 encoding phenylalanine, codon 166 encoding threonine, codon 170 encoding lysine and combinations thereof. The polynucleotides which encode OspA polypeptides of the present invention can encode longer or shorter fragments of OspA protein. The numbering of the residues corresponds to the numbering of SEQ ID NO:7.

The polynucleotides of the present invention include a polynucleotide selected from the group consisting of: SEQ ID NO:95, 97, 99, 101, 103, 105, 107, 109, 111, 113 and 115.

The present invention is also drawn to a method of generating an altered *Borrelia burgdorferi* OspA polypeptide with increased conformational stability, as compared to the corresponding unaltered *Borrelia burgdorferi* OspA polypeptide. The method comprises selecting a polynucleotide encoding a *Borrelia burgdorferi* OspA polypeptide that includes at least one of residues 139, 160 and 189, wherein the numbering corresponds to the numbering of SEQ ID NO:7. The polynucleotide is altered such that at least one of the following alterations is present: residue 139 is changed to methionine, residue 160 is changed to tyrosine and residue 189 is changed to methionine or a combination thereof. In one embodiment, both the alteration at residue 160 and the alteration at 189 is made. In another embodiment, the alterations at all three residues are made. The altered polynucleotide is expressed, thereby generating an altered *Borrelia burgdorferi* OspA polypeptide with increased conformational stability, as compared to the corresponding unaltered *Borrelia burgdorferi* OspA polypeptide.

In another embodiment, the present invention is drawn to a method of generating an altered *Borrelia burgdorferi* OspA polypeptide with reduced cross-reactivity with an hLFA-1 molecule, as compared to the corresponding unaltered *Borrelia burgdorferi* OspA polypeptide. The method comprises selecting a polynucleotide encoding an OspA polypeptide from *Borrelia burgdorferi* that includes at least one of residues 165, 166 and 170, wherein the numbering corresponds to the numbering of SEQ ID NO:7. The polynucleotide is altered such that at least one alteration from the following list is present: residue 165 is changed to phenylalanine, residue 166 is changed to tyrosine and residue 170 is changed to lysine or combination thereof. The altered polynucleotide is expressed, thereby generating an altered *Borrelia burgdorferi* OspA polypeptide with reduced cross-reactivity with the hLFA-1 molecule, as compared to the corresponding unaltered *Borrelia burgdorferi* OspA polypeptide.

The present invention is also drawn to an expression vector which comprises an isolated DNA encoding an altered *Borrelia* OspA protein. The present invention also encompasses a host cell which comprises a recombinant nucleic acid that encodes an altered OspA protein as described herein.

The present invention is also drawn to a method of delivering the altered *Borrelia* OspA polypeptides described herein. In one embodiment, the method comprises administering the altered OspA polypeptide in a physiologically-acceptable carrier to an individual. As a result of the administration of the altered OspA protein, the individual develops at least some immune response to the protein. As an example, the individual generates a humoral immune response, wherein antibodies that recognize at least a portion of said polypeptide are produced by the individual. In a preferred embodiment, the individual generates an immunoprotective response, for example, by generating antibodies that recognize the LA-2 epitope.

The present invention is also drawn to a method of delivering a nucleic acid which encodes an altered OspA polypeptide described herein. In one embodiment, the method comprises administering the nucleic acid in a physiologically-acceptable carrier to an individual. As a result of the administration of the nucleic acid, the altered OspA polypeptide is at least transiently expressed and the individual develops at least some immune response, preferably an immunoprotective response to the altered OspA protein encoded by the nucleic acid. As an example, the individual generates a humoral immune response, wherein antibodies that recognize at least a portion of the altered OspA polypeptide produced from the nucleic acid are produced by the individual. In a preferred embodiment, the individual generates an immunoprotective response, for example, by generating antibodies that recognize the LA-2 epitope.

The invention also encompasses methods of using the proteins described herein in diagnostic assays. In one embodiment, the method can be used to detect the presence of OspA specific antibodies in a host sample of interest. The method comprises contacting a host sample of interest with the altered protein, under conditions wherein antibodies, if present in the host sample, bind to the altered protein, forming antigen-antibody complexes. The antigen-antibody complexes are then detected using standard methods known in the art.

The present invention is also drawn to a diagnostic kit comprising the altered polypeptides described herein. The kit comprises an altered *Borrelia burgdorferi* OspA protein as described herein. The kit also includes reagents for detecting antibody-antigen complexes that are formed between the OspA altered protein and antibodies that are present in the user-supplied host sample.

As a result of the present invention, OspA proteins, or fragments thereof, having either increased conformational stability while maintaining at least some antigenicity, or having reduced cross-reactivity to hLFA-1, are available for use in research, vaccines and/or diagnostic assays. Furthermore, as a result of the present invention, nucleic acids encoding OspA polypeptides having reduced cross-reactivity with hLFA-1 are available for research and vaccines. The altered OspA polypeptides of the present invention are expected to allow for improved vaccines having fewer side effects.

For a better understanding of the present invention together with other and further objects, reference is made to the following description, taken together with the accompanying drawings.

### BRIEF DESCRIPTION OF THE DRAWINGS

Fig. 1 summarizes peptides and antigenic domains localized by proteolytic and chemical fragmentation of OspA.

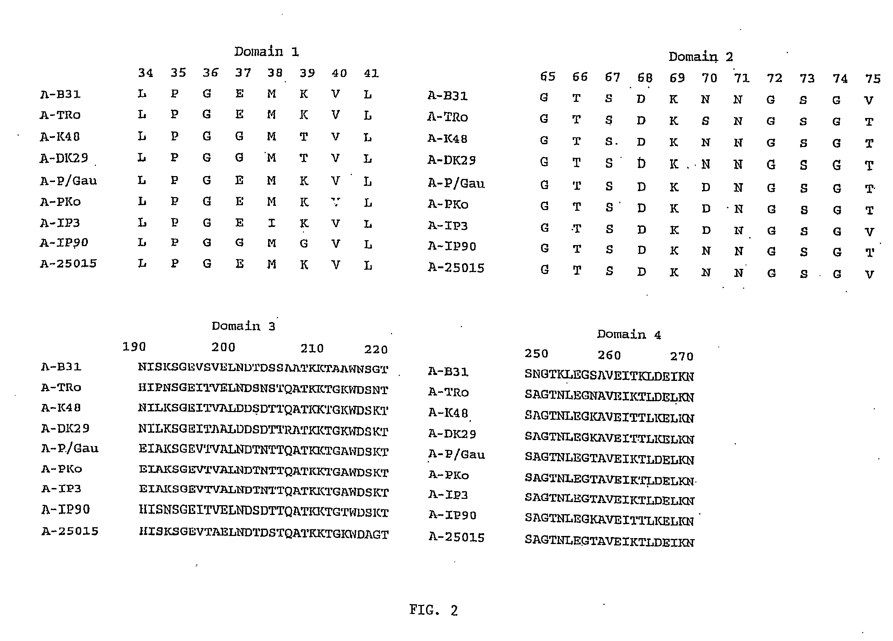

Fig. 2 is a comparison of the antigenic domains depicted in Fig. 1, for OspA in nine strains of *B. burgdorferi.*

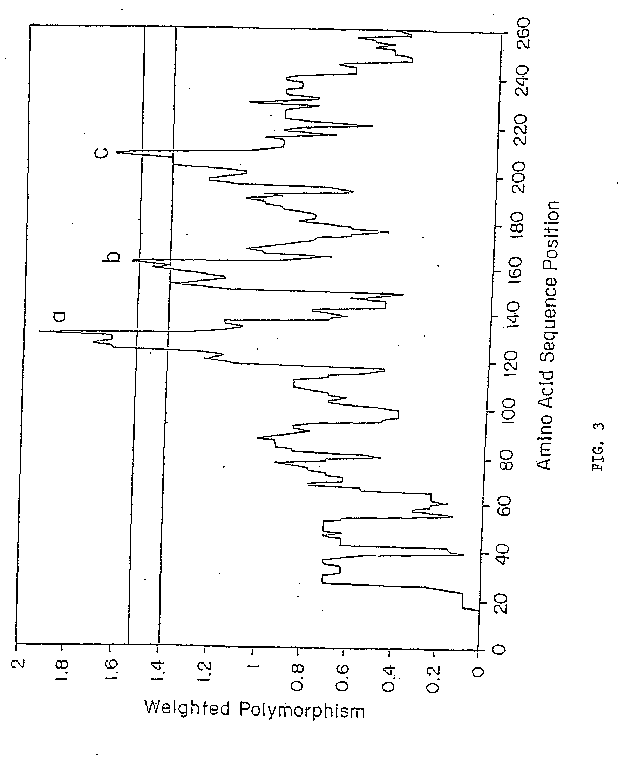

Fig. 3 is a graph depicting a plot of weighted polymorphism versus amino acid position among 14 OspA variants. The marked peaks are: a) amino acids 132-145; b) amino acids 163-177; c) amino acids 208-221. The lower dotted line at polymorphism value 1.395 demarcates statistically significant excesses of polymorphism at p = 0.05. The upper dotted line at 1.520 is the same, except that the first 29 amino acids at the monomorphic-terminus have been removed from the original analysis.

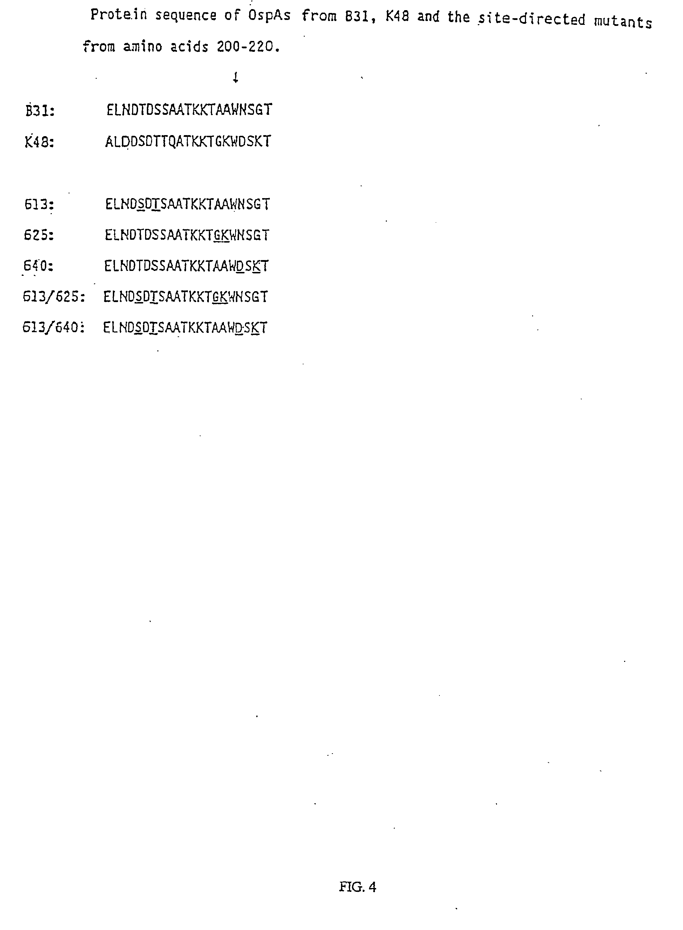

Fig. 4 depicts the amino acid alignment of residues 200 through 220 for OspAs from strains B31 and K48 as well as for the site-directed mutants 613, 625, 640, 613/625, and 613/640. Arrow indicates Trp216. Amino acid changes are underlined.

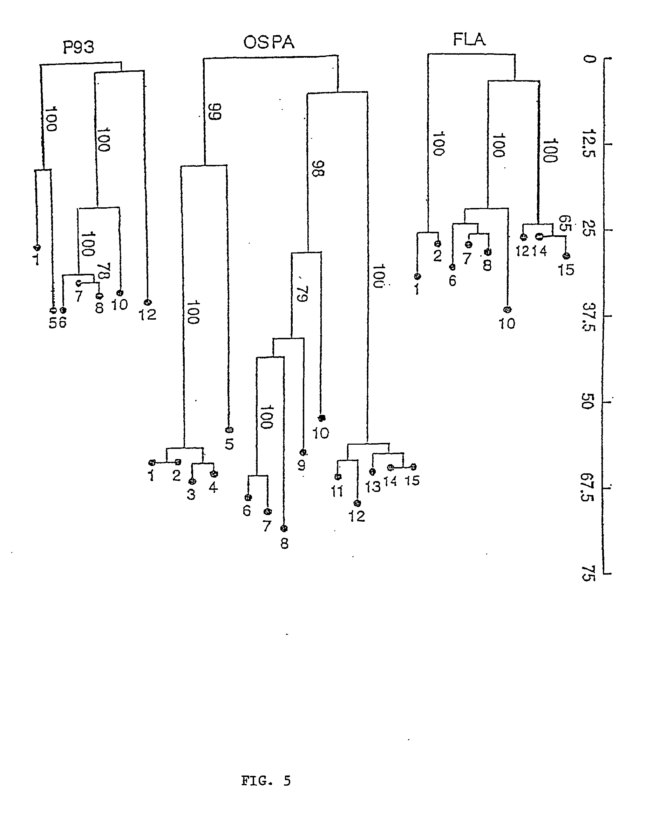

Fig. 5 depicts a phylogenetic tree for strains *of Borrelia* described in Table I. The strains are as follows: 1 = B31; 2 = Pka1; 3 = ZS7; 4 = N40; 5 = 25015; 6 = K48; 7 = DK29; 8 = PHei; 9 = Ip90; 10 = PTrob; 11 = ACAI; 12 = PGau; 13 = Ip3; 14 = PBo; 15 = Pko.

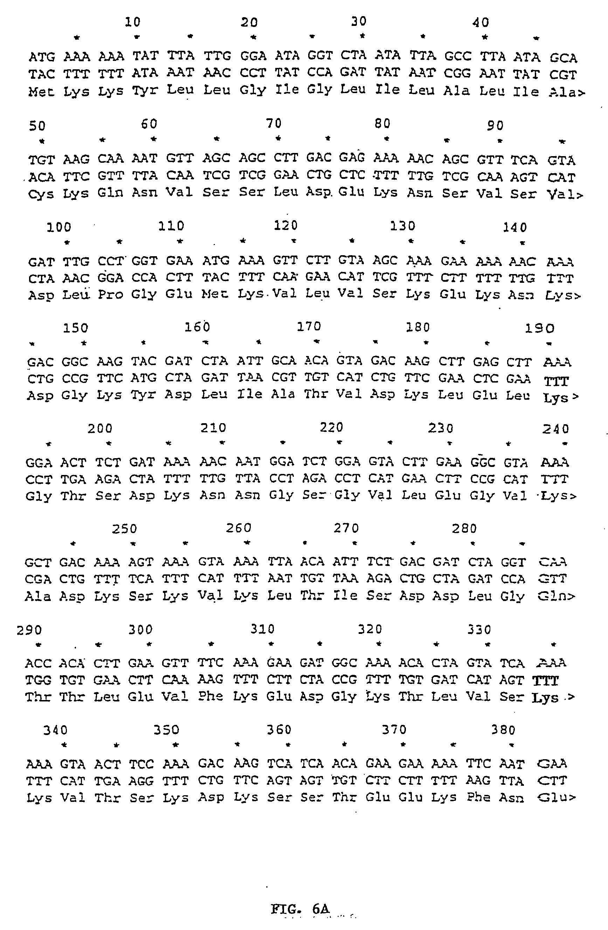

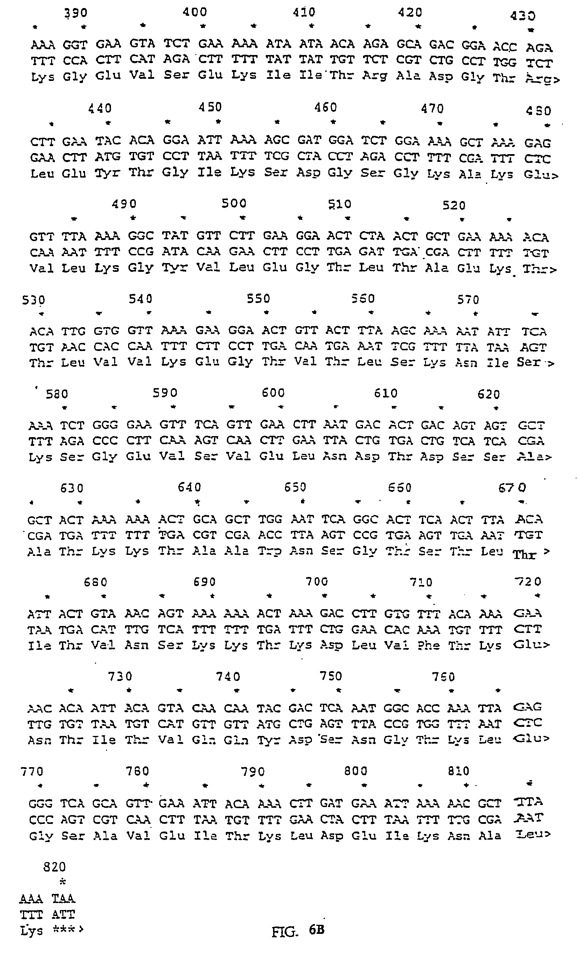

Figs. 6A and 6B depict the nucleic acid sequence of OspA-B31 (SEQ ID NO:6), and the encoded protein sequence (SEQ ID NO:7).

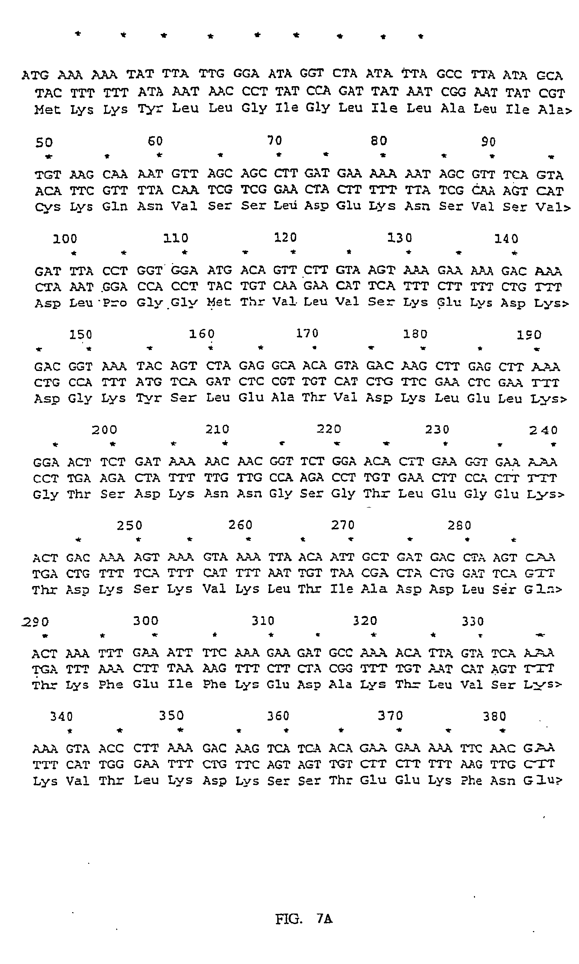

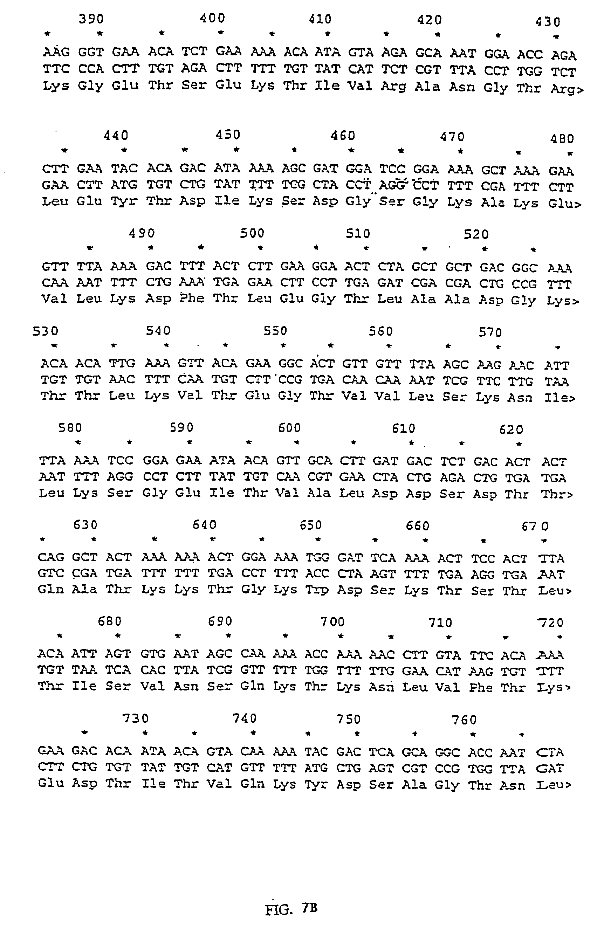

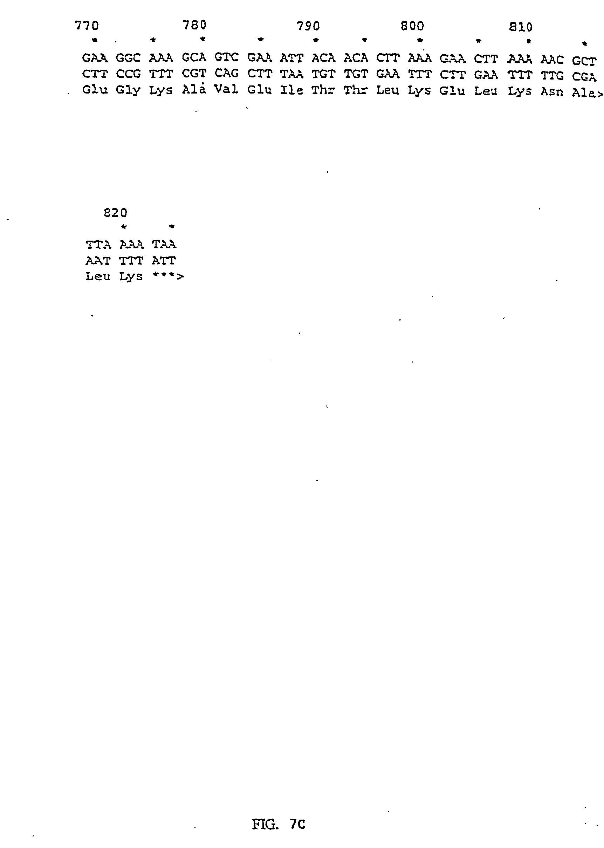

Figs. 7A, 7B and 7C depict the nucleic acid sequence of OspA-K48 (SEQ ID NO:8), and the encoded protein sequence (SEQ ID NO:9).

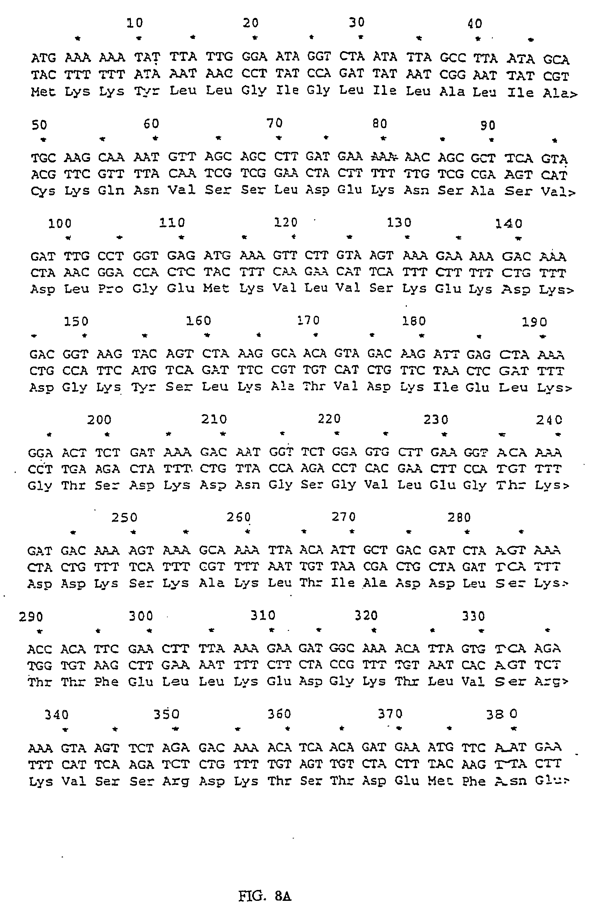

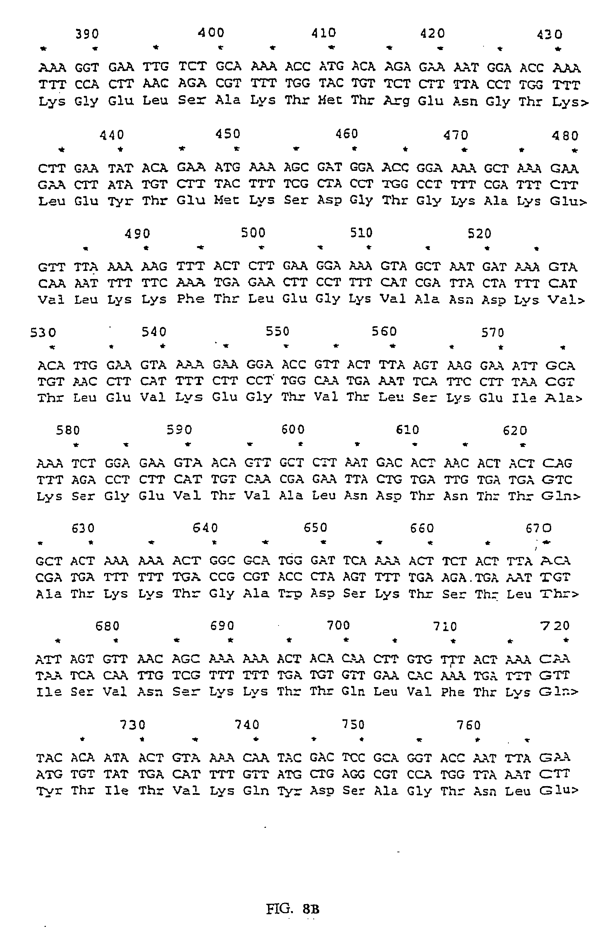

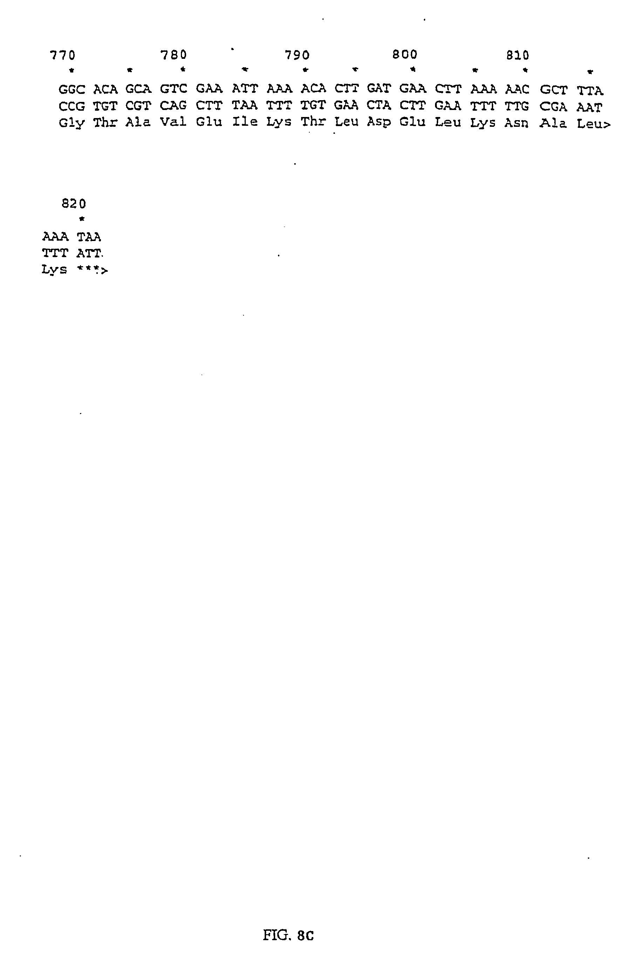

Figs. 8A, 8B and 8C depict the nucleic acid sequence of OspA-PGau (SEQ ID NO:10), and the encoded protein sequence (SEQ ID NO:11).

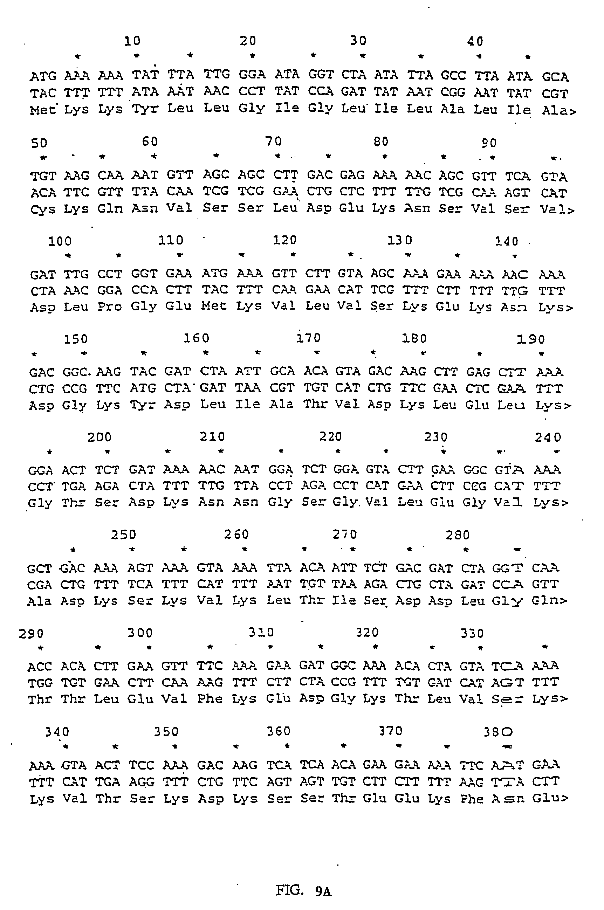

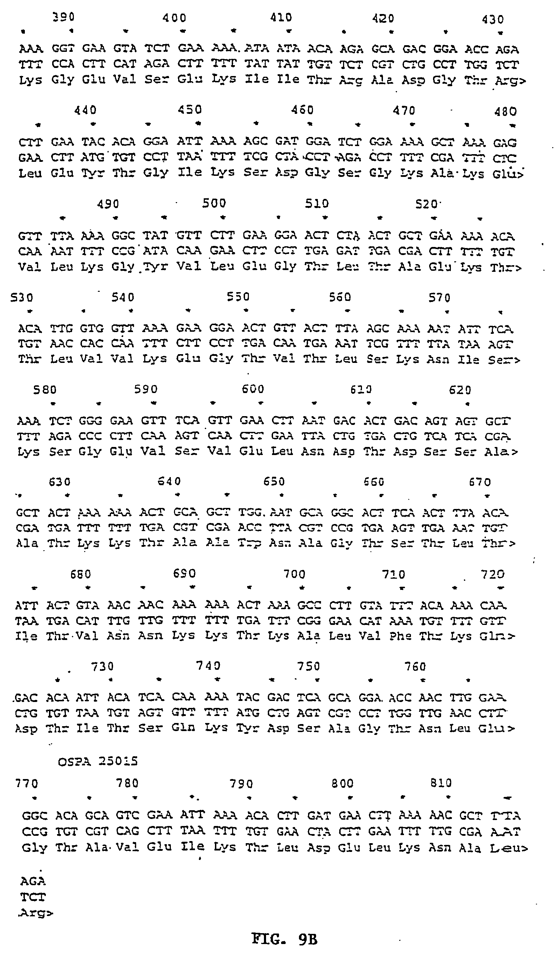

Figs. 9A and 9B depict the nucleic acid sequence of an OspA gene and its encoded protein sequence.

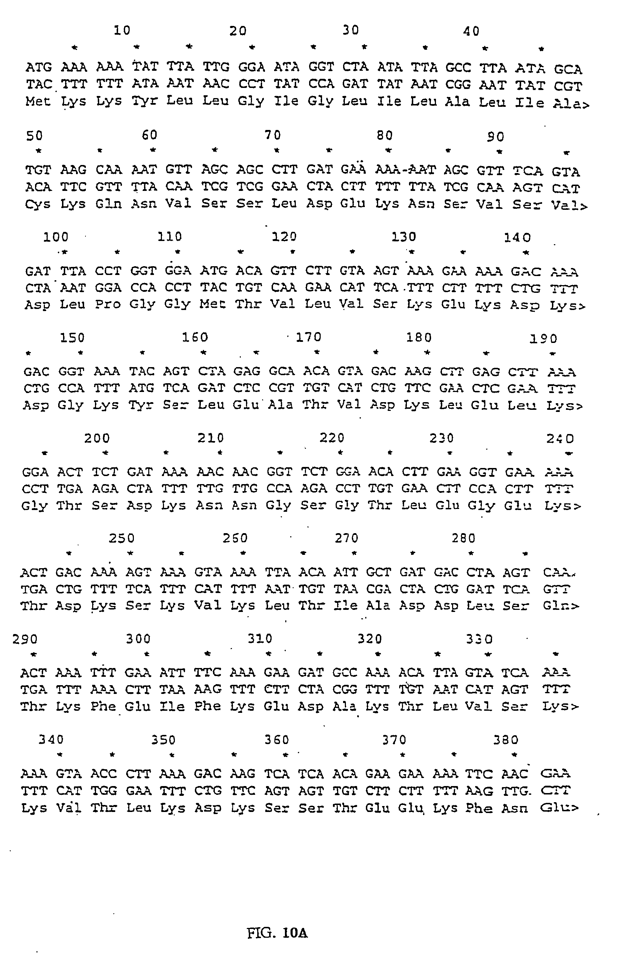

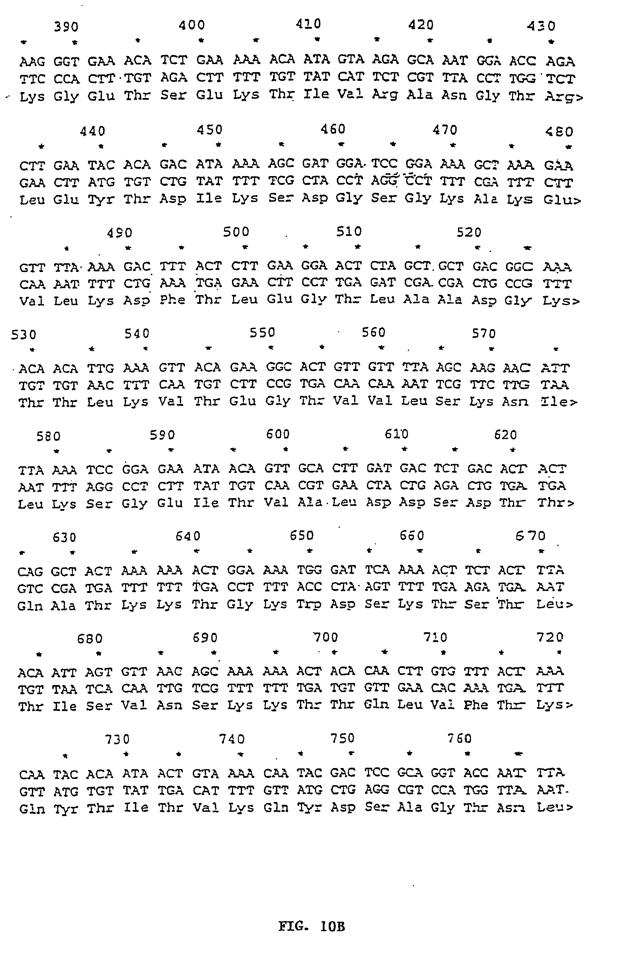

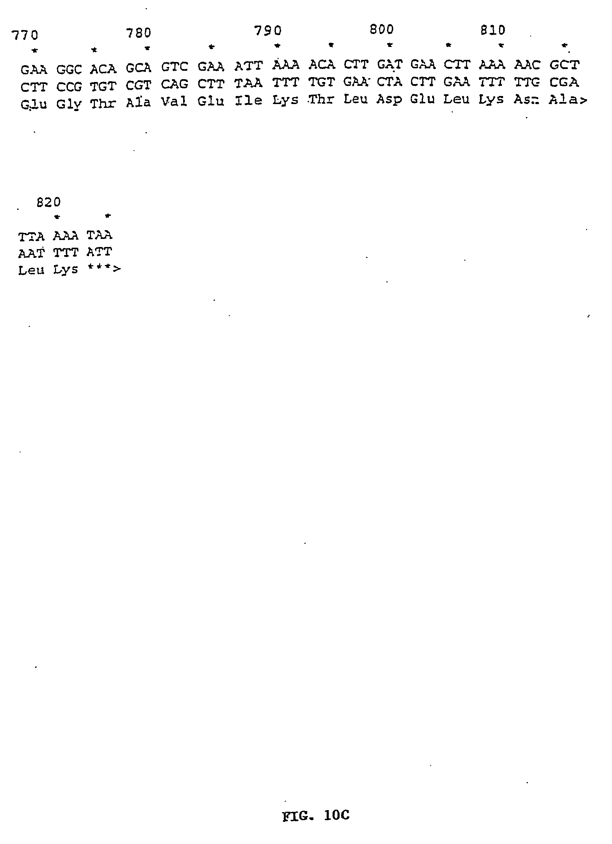

Figs. 10A, 10B and 10C depict the nucleic acid sequence of the OspA-K48/OspA-PGau chimer (SEQ ID NO:28) and the encoded chimeric protein sequence (SEQ ID NO:29).

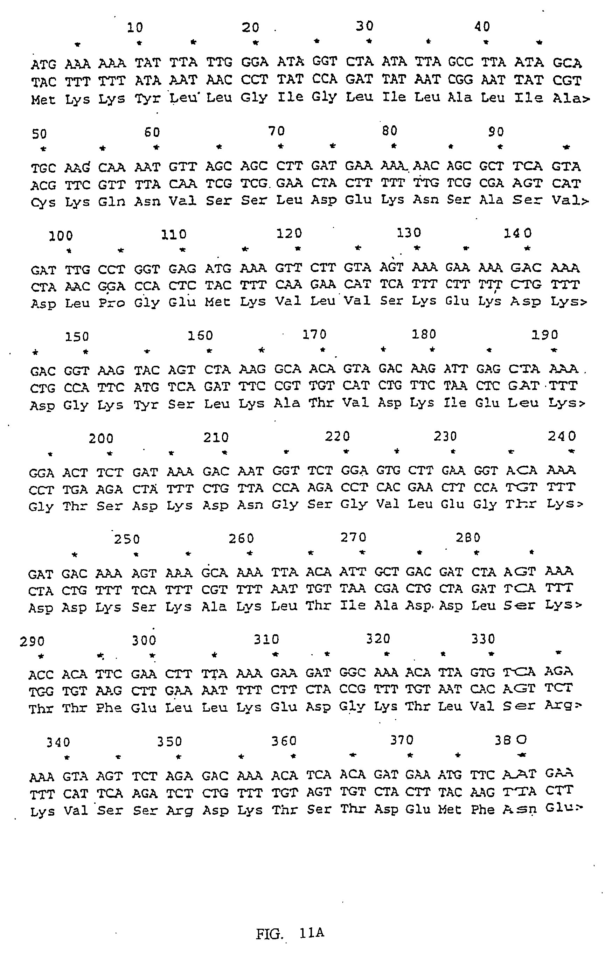

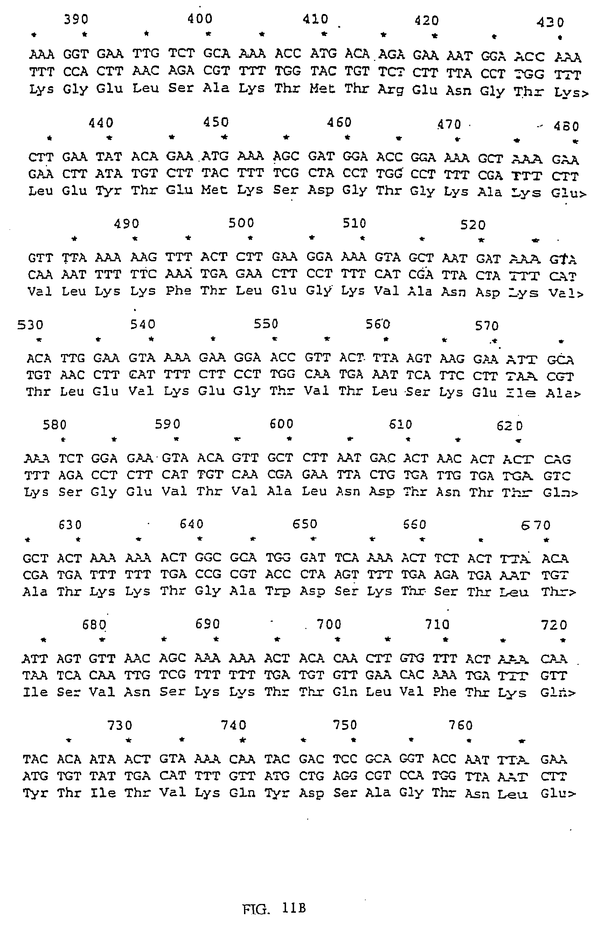

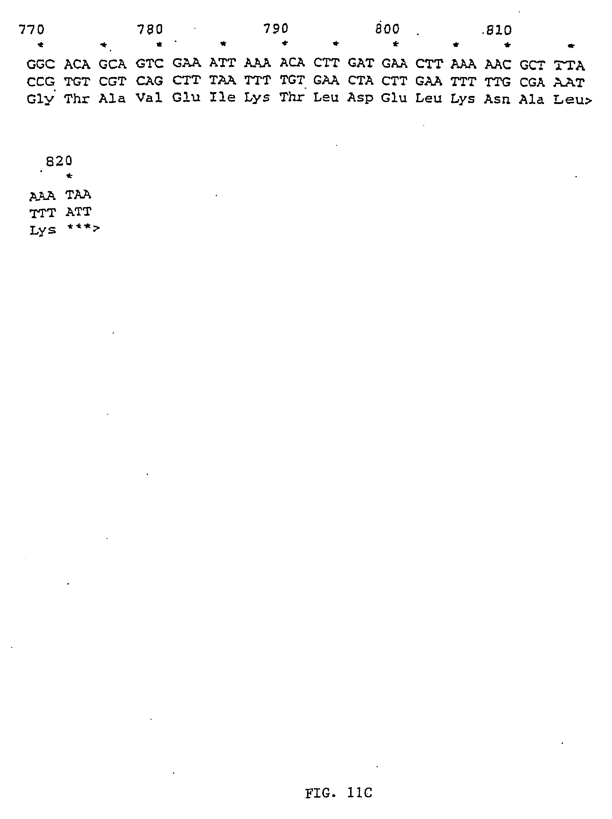

Figs. 11A, 11B and 11C depict the nucleic acid sequence of the OspA-B31/OspA-PGau chimer (SEQ ID N0:30) and the encoded chimeric protein sequence (SEQ ID N0:31).

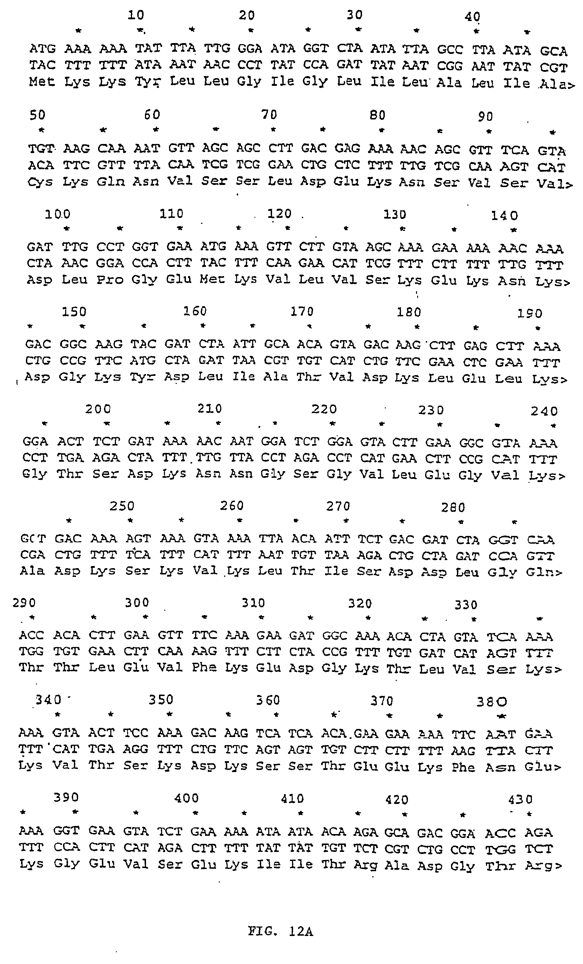

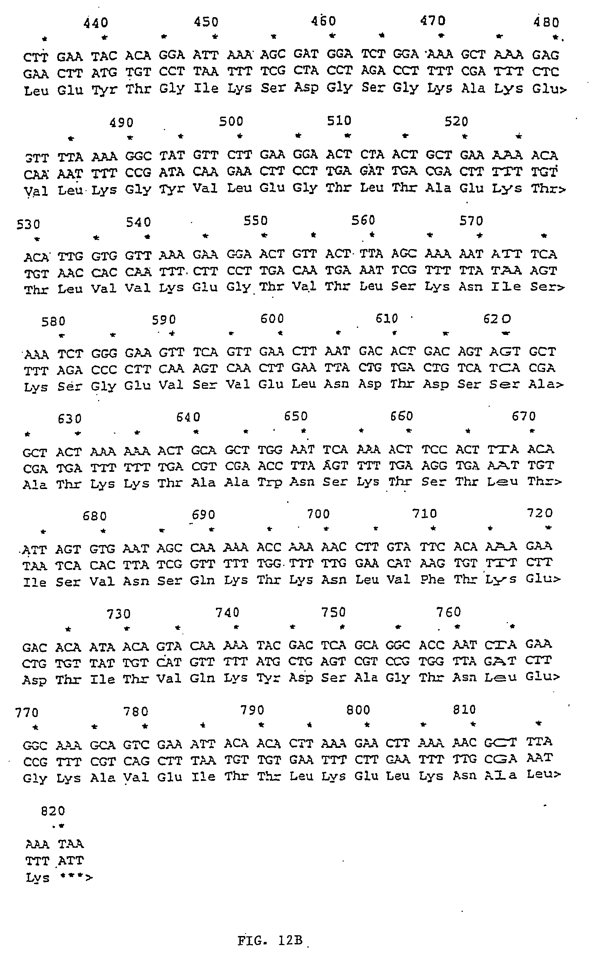

Figs. 12A and 12B depict the nucleic acid sequence of the OspA-B31/OspA-K48 chimer (SEQ ID NO:32) and the encoded chimeric protein sequence (SEQ ID N0:33).

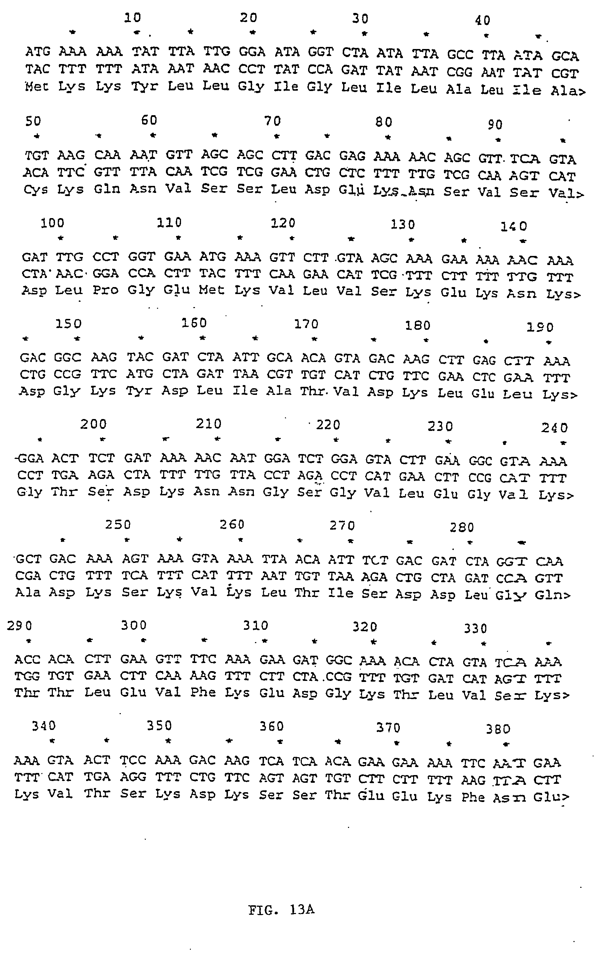

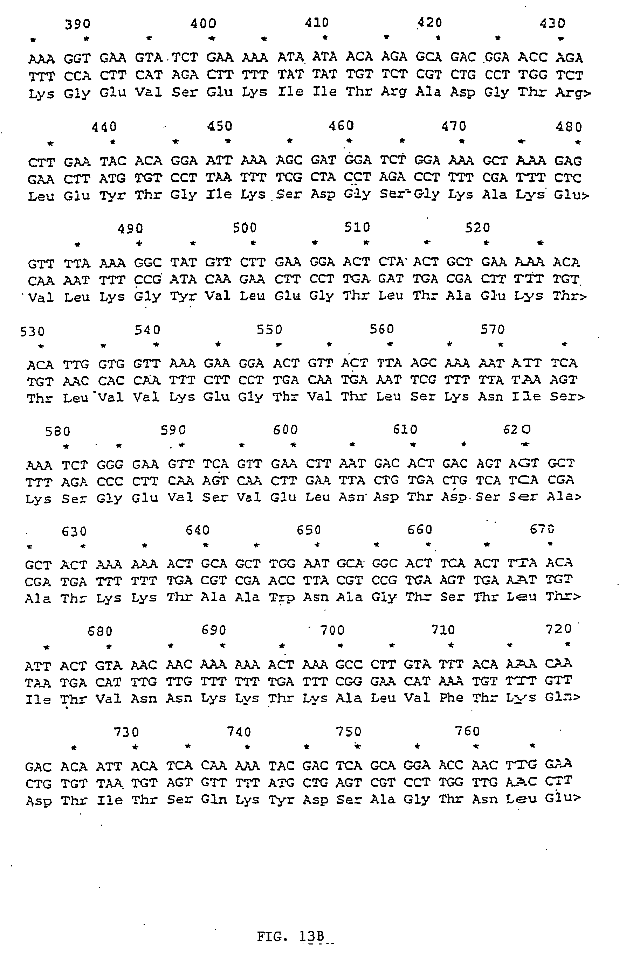

Figs. 13A, 13B and 13C depict the nucleic acid sequence of the OspA-B31/OspA-25015 chimer (SEQ ID NO:34) and the encoded chimeric protein sequence (SEQ ID N0:35).

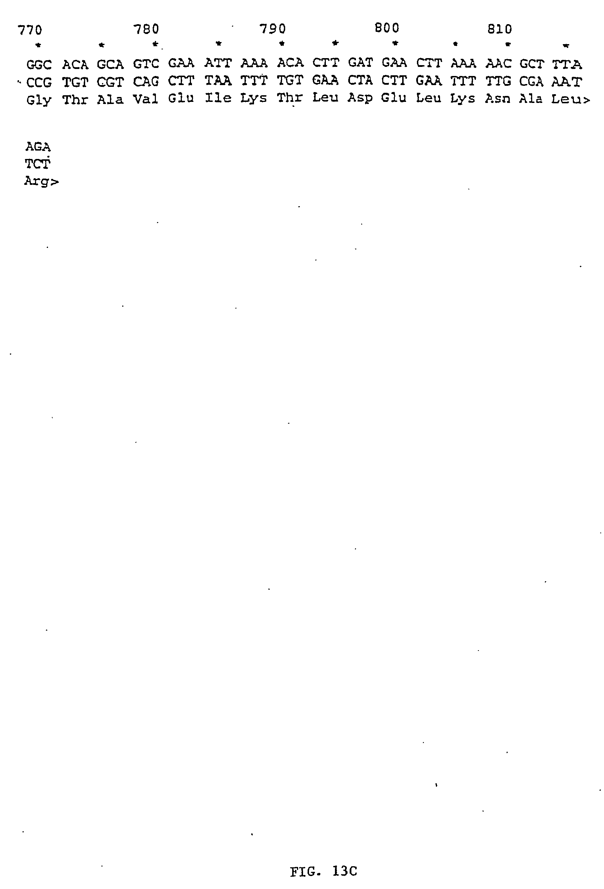

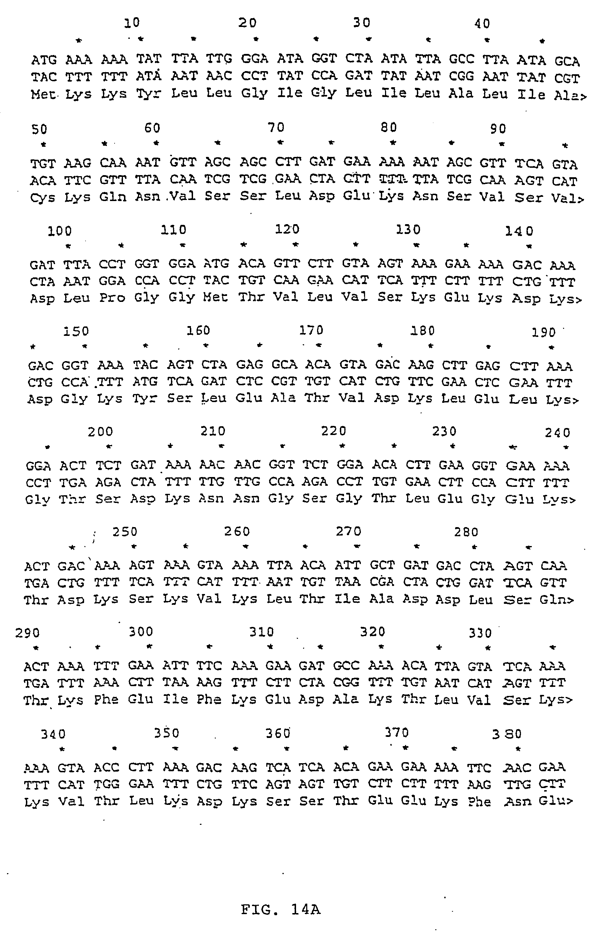

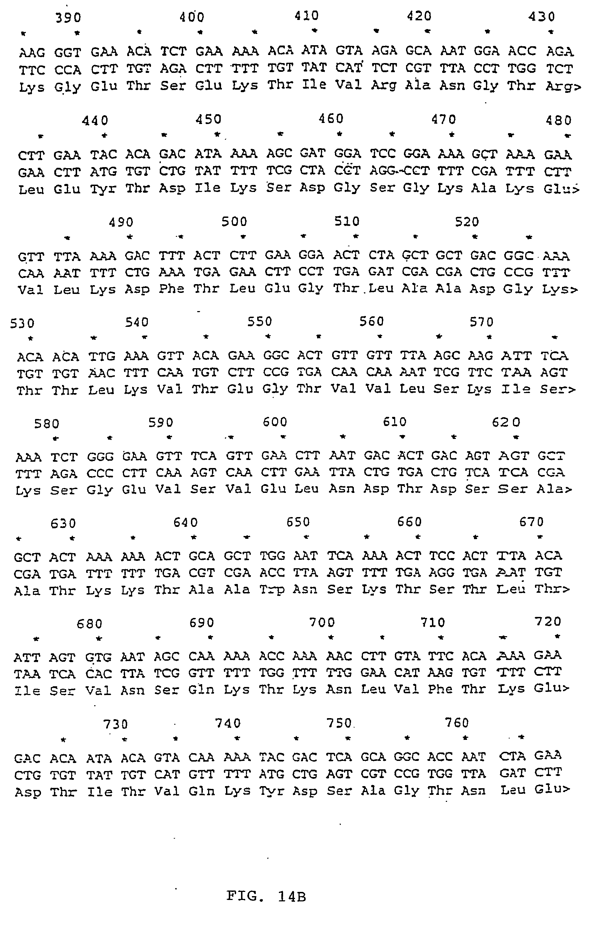

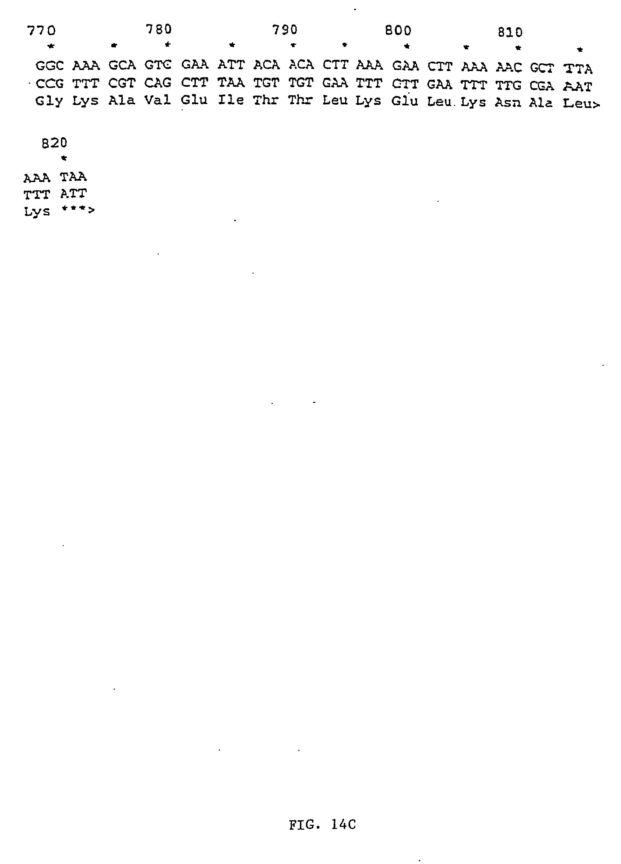

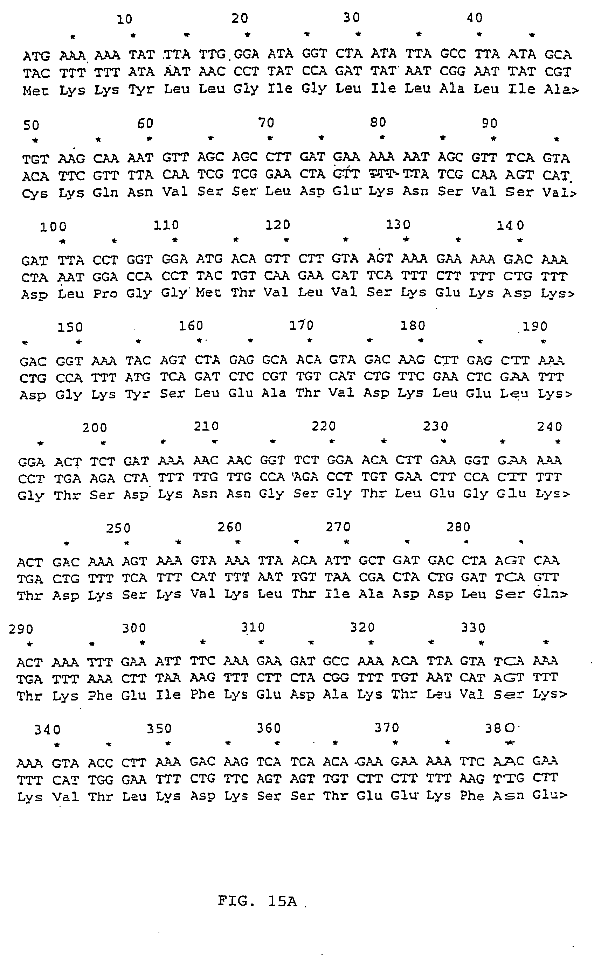

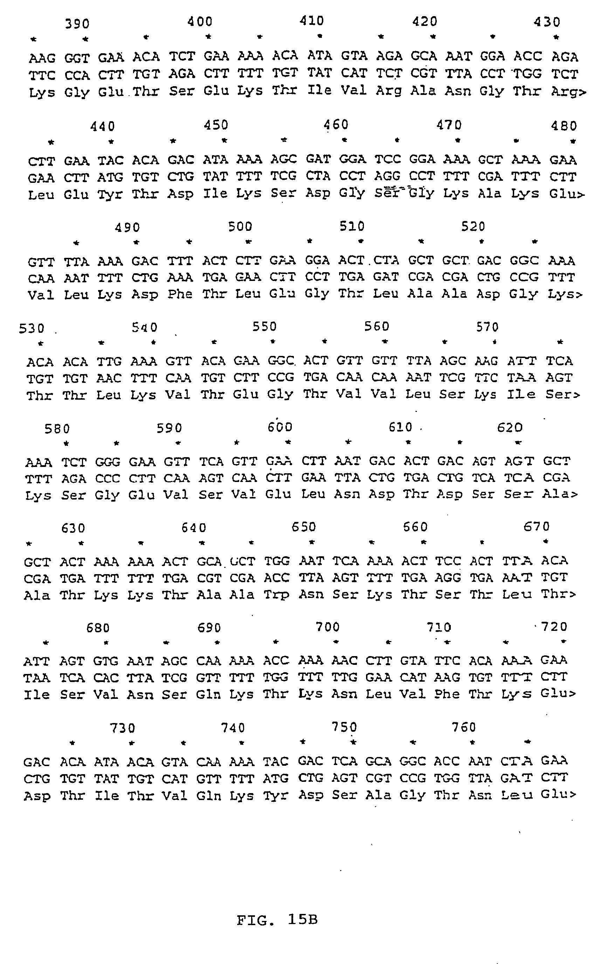

Figs. 14A, 14B and 14C depict the nucleic acid sequence of the OspA-K48/OspA-B31/OspA-K48 chimer (SEQ ID NO:36) and the encoded chimeric protein sequence (SEQ ID NO:37).

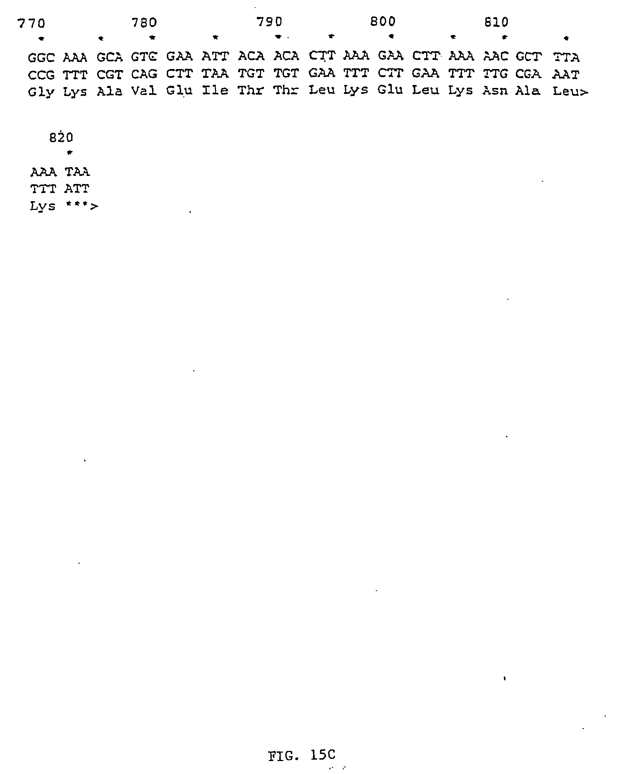

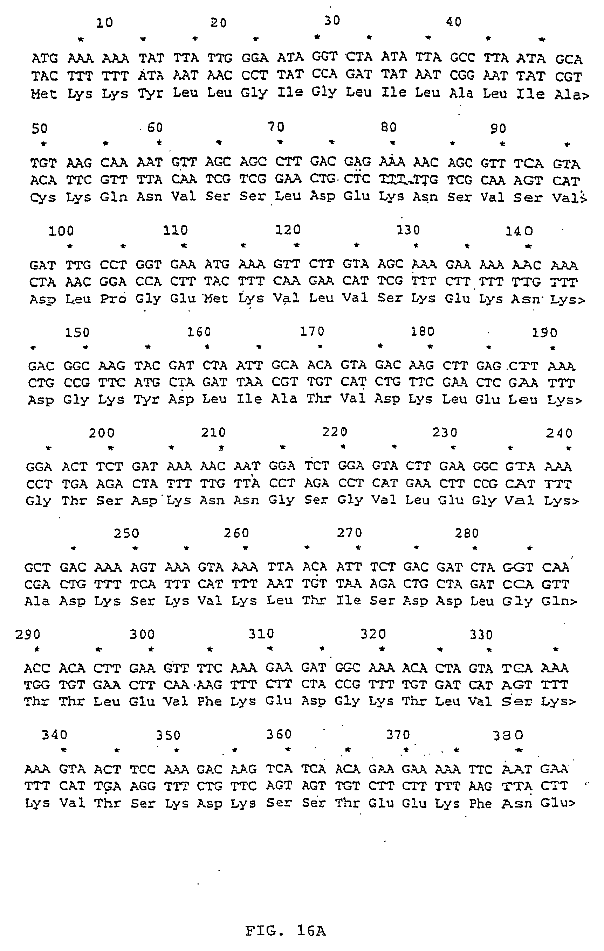

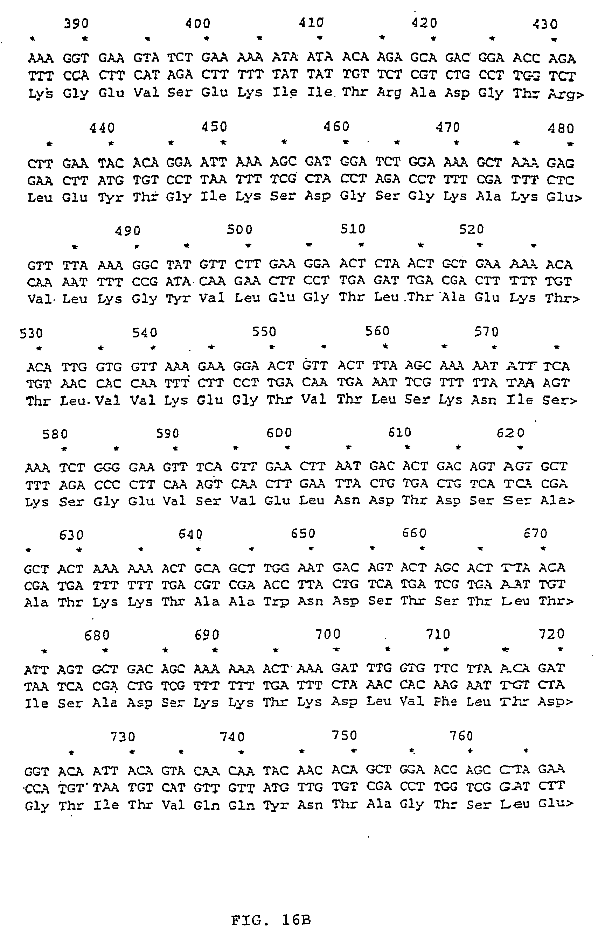

Figs. 15A, 15B and 15C depict the nucleic acid sequence of the OspA-B31/OspA-K48/OspA-B31/OspA-K48 chimer (SEQ ID NO:38) and the encoded chimeric protein sequence (SEQ ID NO:39).

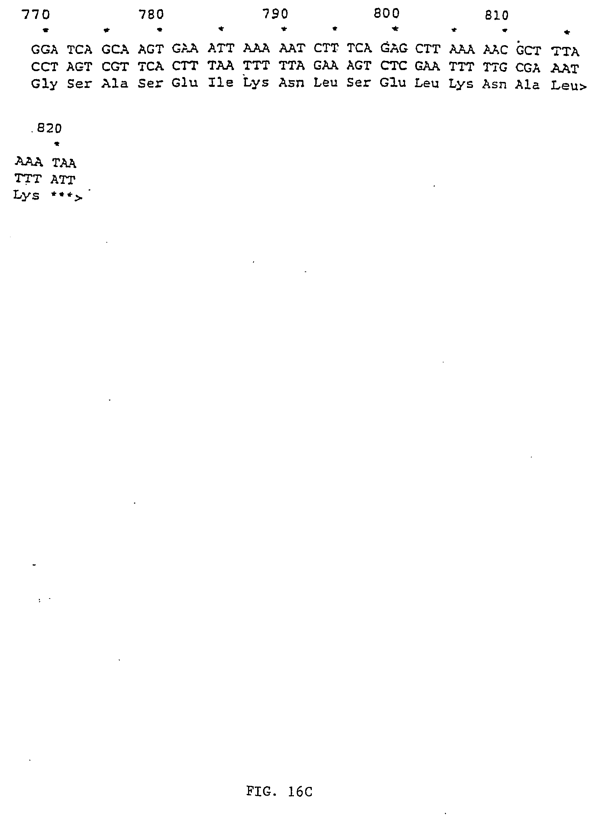

Figs. 16A, 16B and 16C depict the nucleic acid sequence of the OspA-B31/OspB-B31 chimer (SEQ ID NO:40) and the encoded chimeric protein sequence (SEQ ID NO:41).

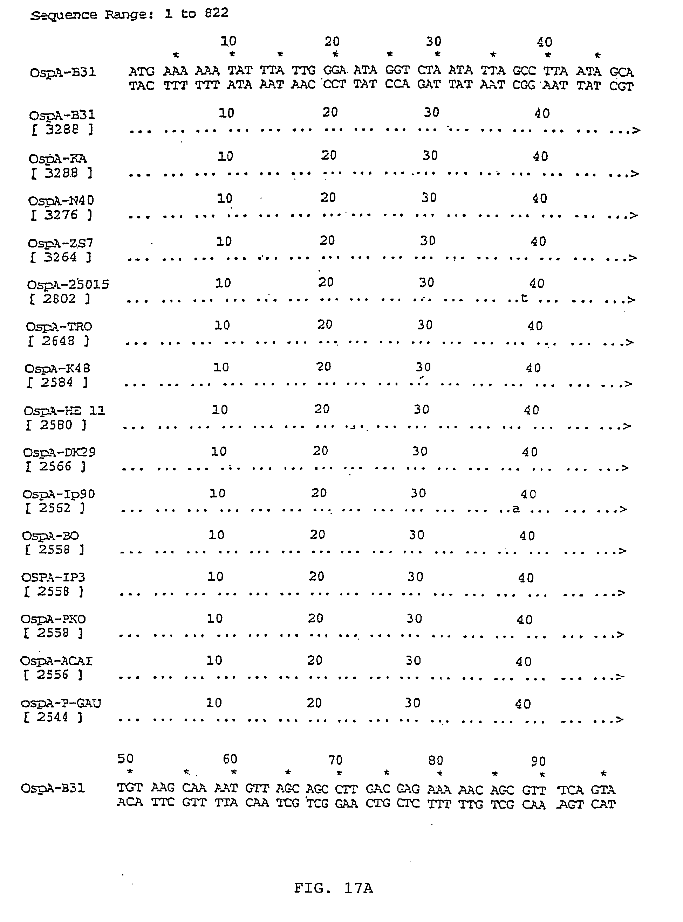

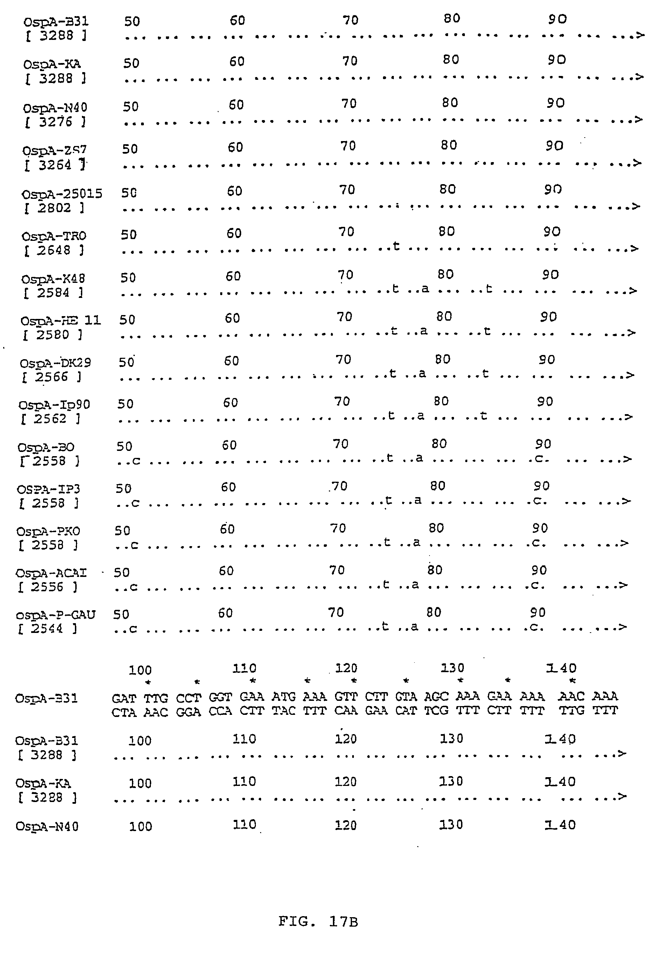

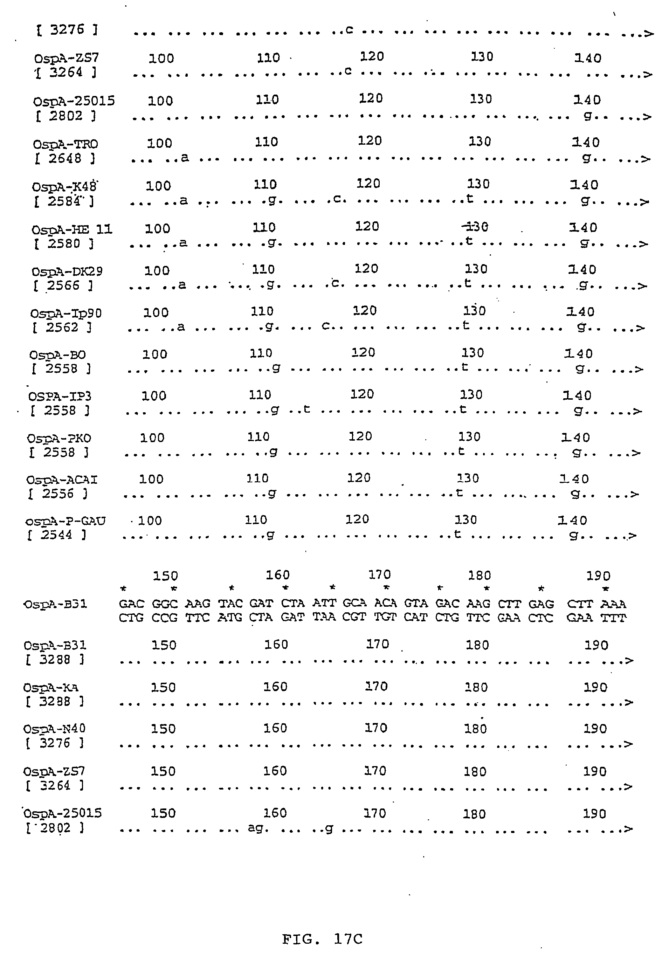

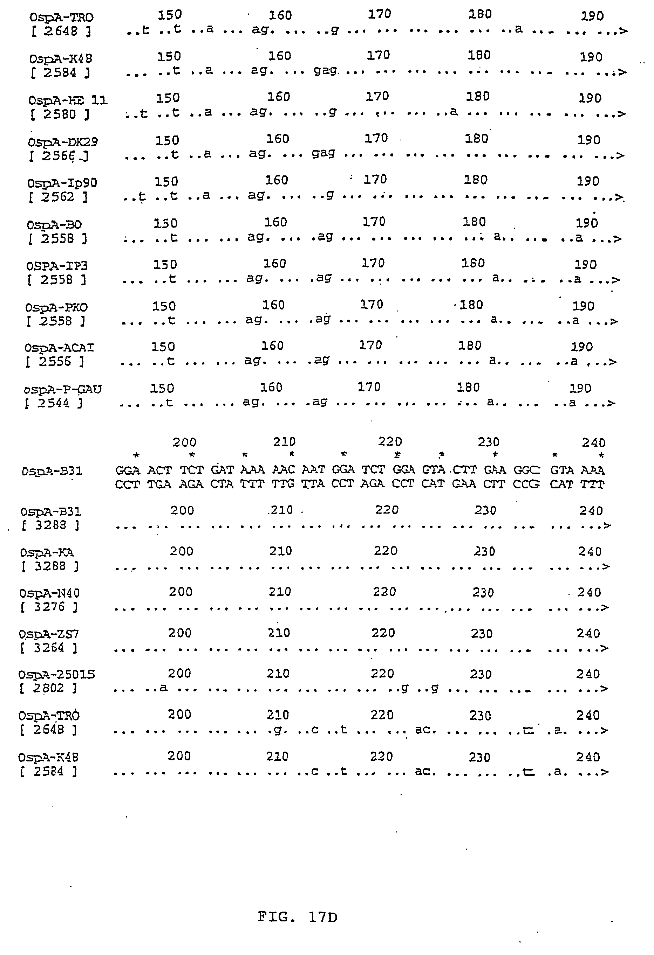

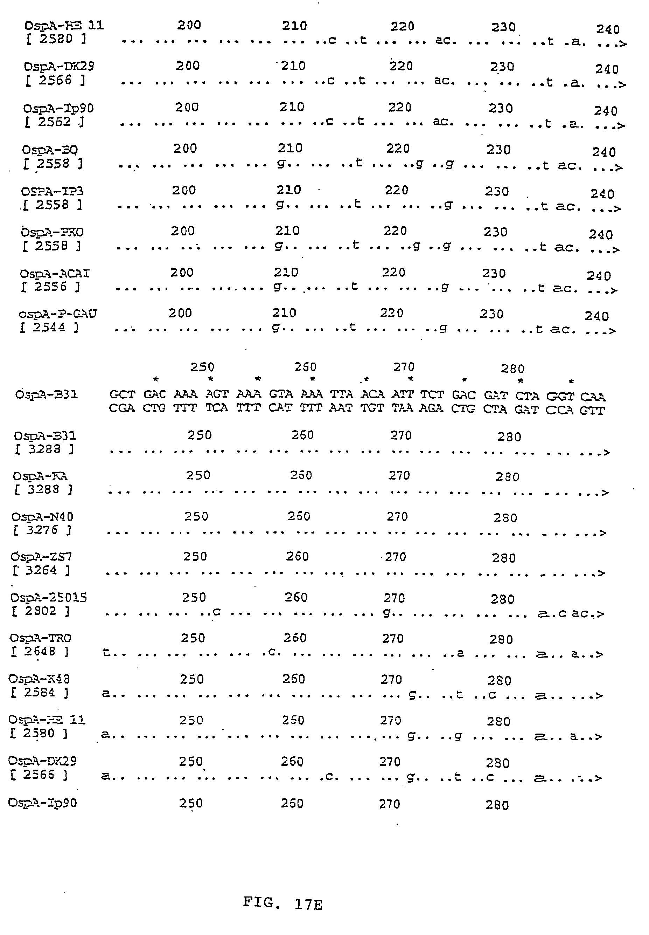

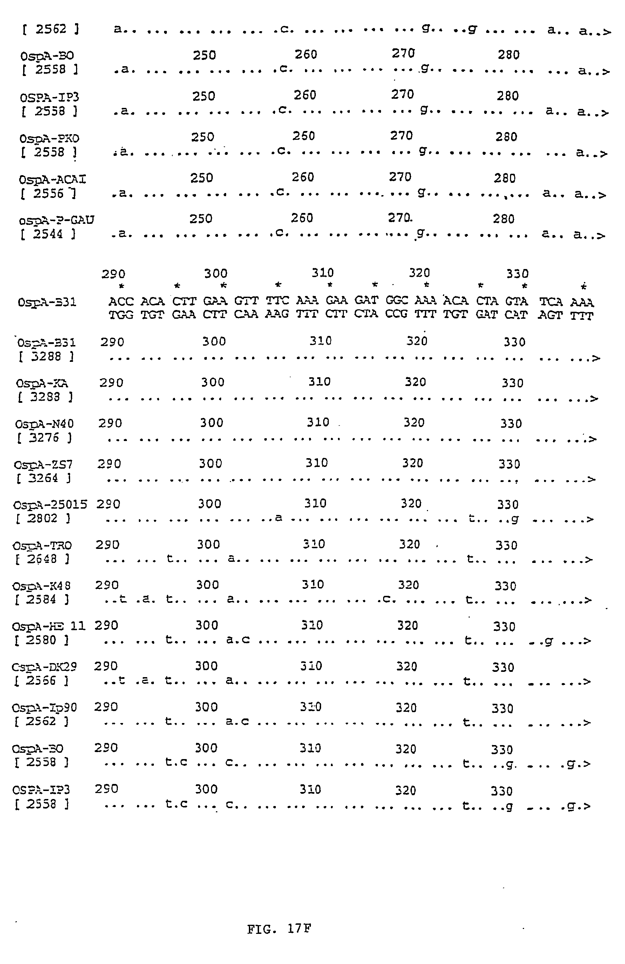

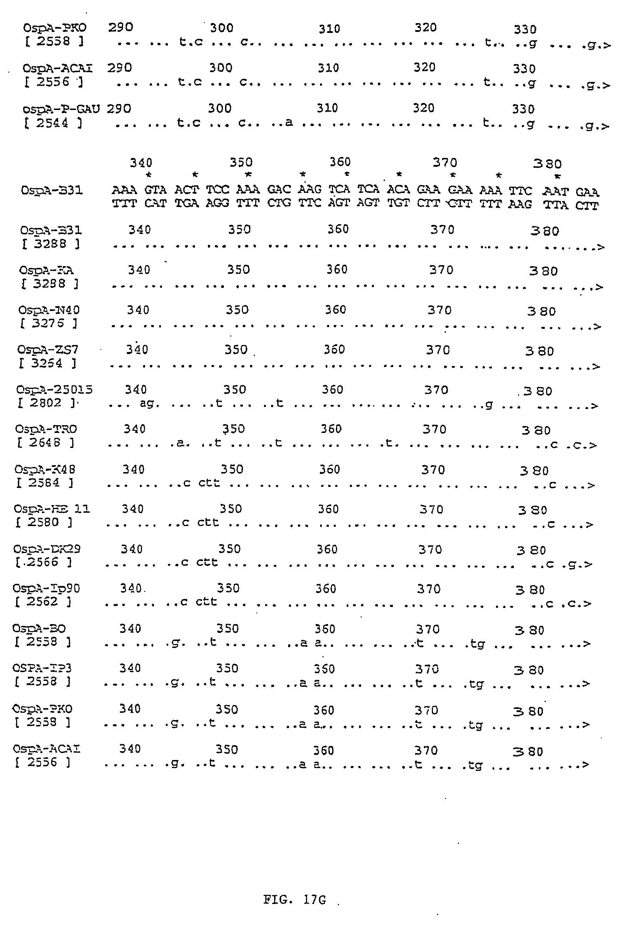

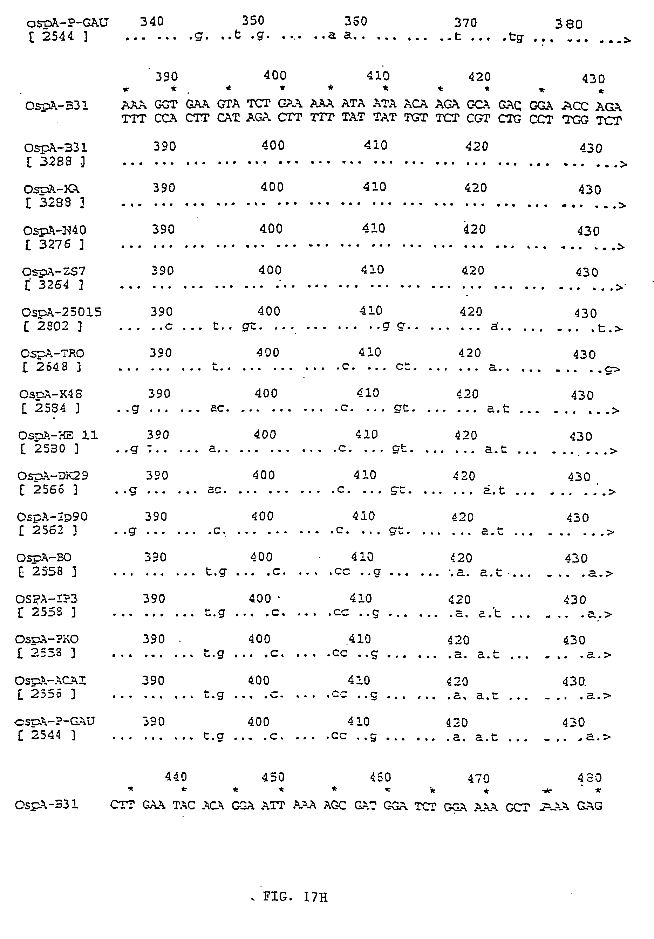

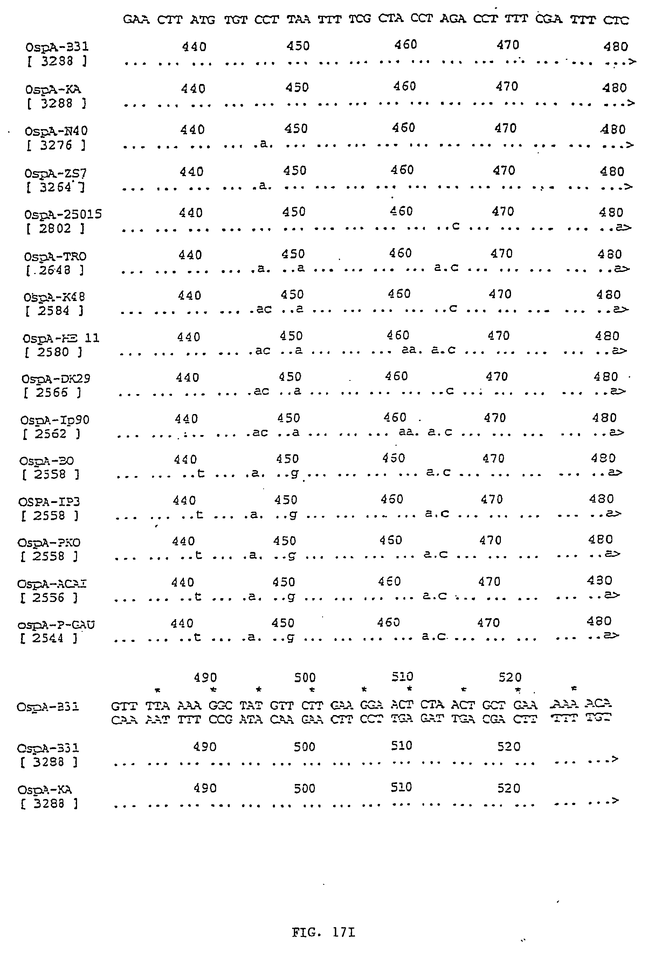

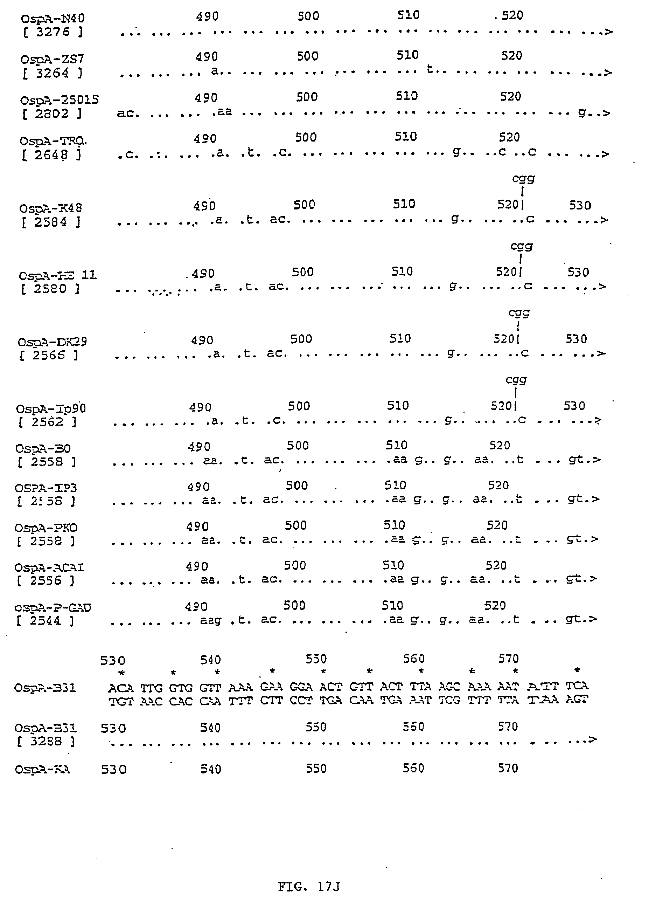

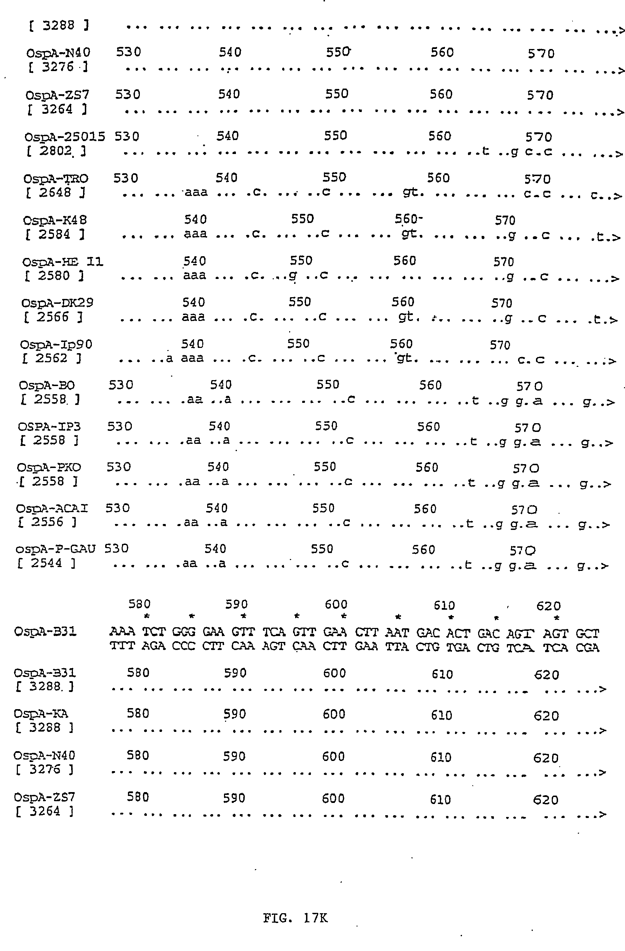

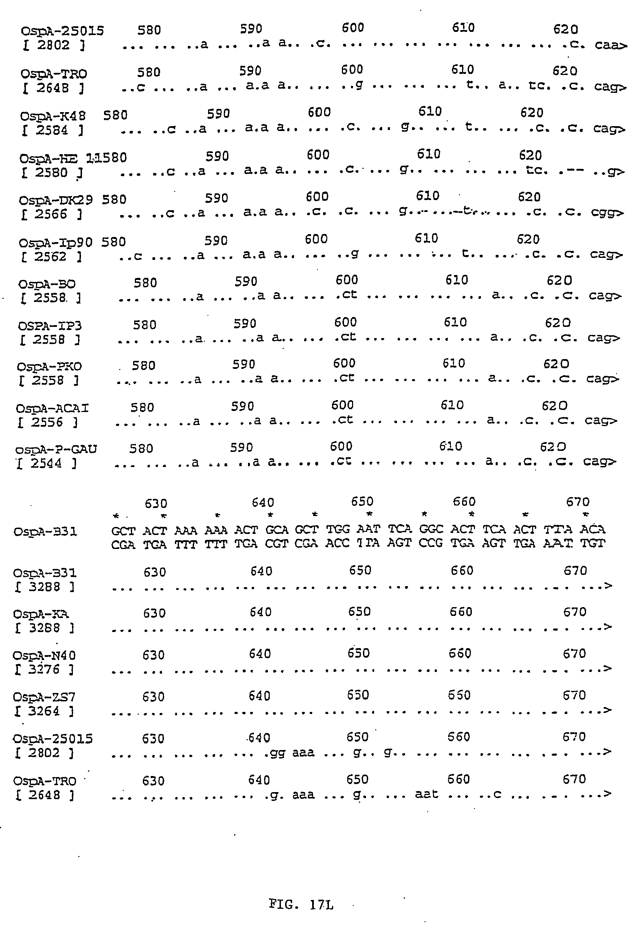

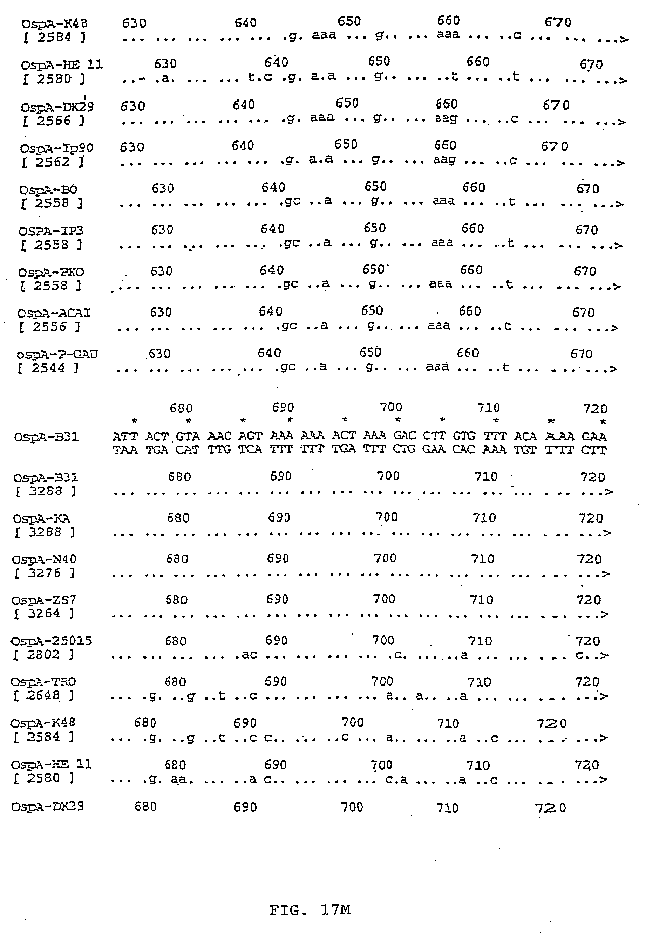

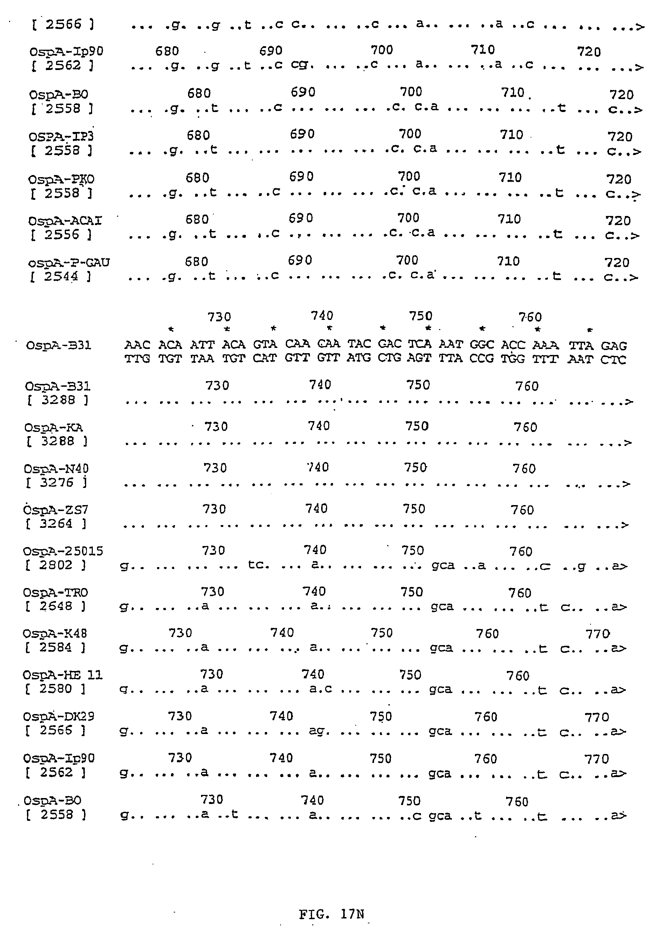

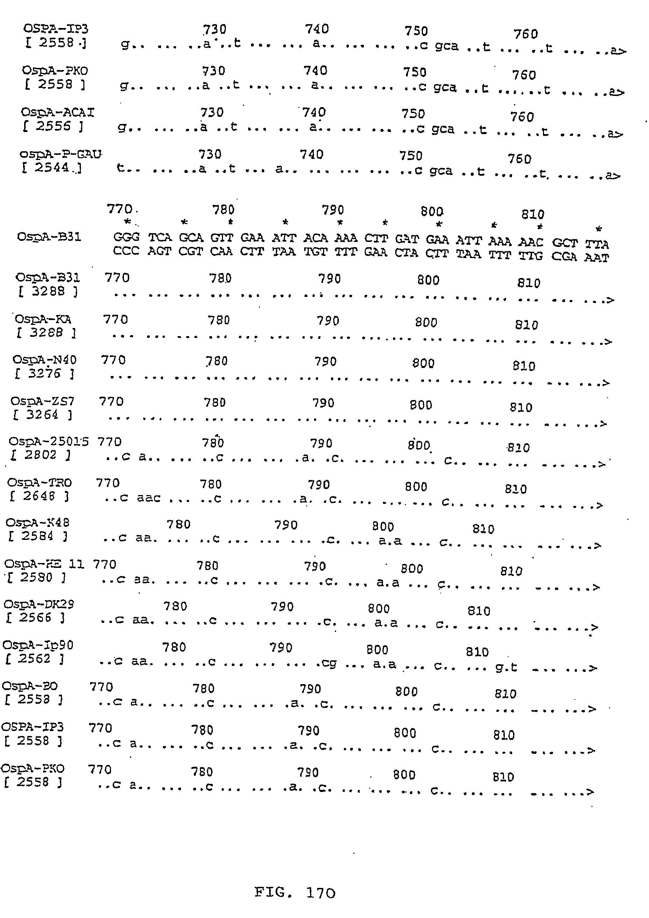

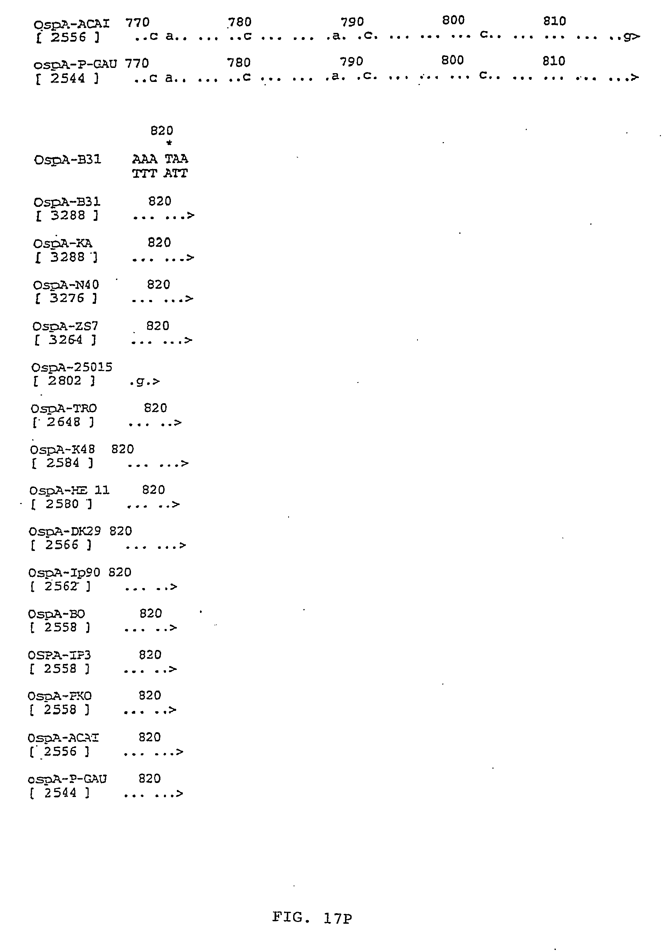

Figs. 17A, 17B, 17C, 17D, 17E, 17F, 17G, 17H, 17I, 17J, 17K, 17L, 17M, 17N, 170, and 17P, depict an alignment of the nucleic acid sequences for OspA-B31 (SEQ ID NO:6), OspA-pKal (SEQ ID NO:42), OspA-N40 (SEQ ID NO:43), OspA-ZS7 (SEQ ID NO:44), OspA-25015 (SEQ ID NO: 12), OspA-pTrob (SEQ ID NO:45), OspA-K48 (SEQ ID NO:8), OspA-Hei (SEQ ID NO:46), OspA-DK29 (SEQ ID NO:21), OspA-Ip90 (SEQ ID NO:22), OspA-pBo (SEQ ID NO:23), OspA-Ip3 (SEQ ID NO:24), OspA-Pko (SEQ ID NO:25), OspA-ACAI (SEQ ID NO:26), and OspA-PGau (SEQ ID NO: 10). Nucleic acids which are identical to those in the lead nucleic acid sequence (here, OspA-B31) are represented by a period (.); differing nucleic acids are shown in lower case letters.

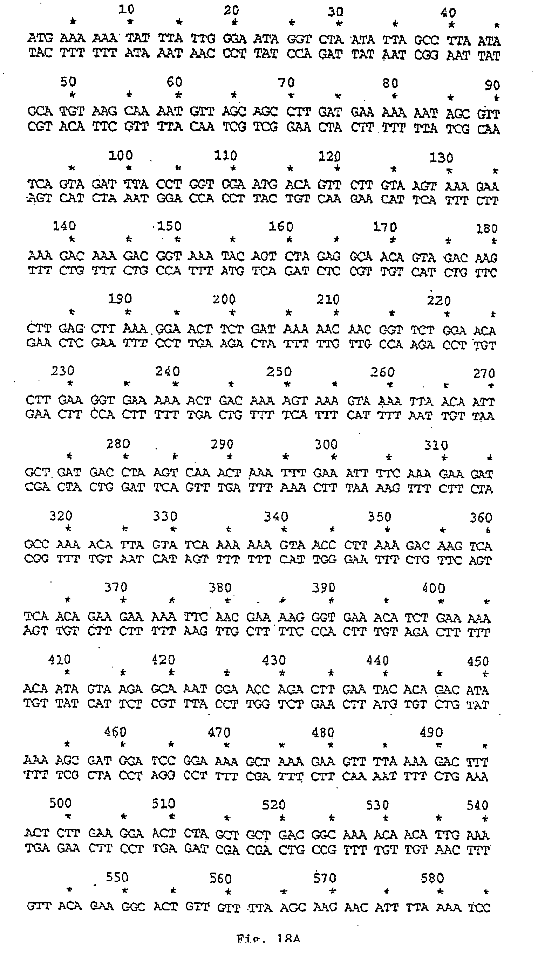

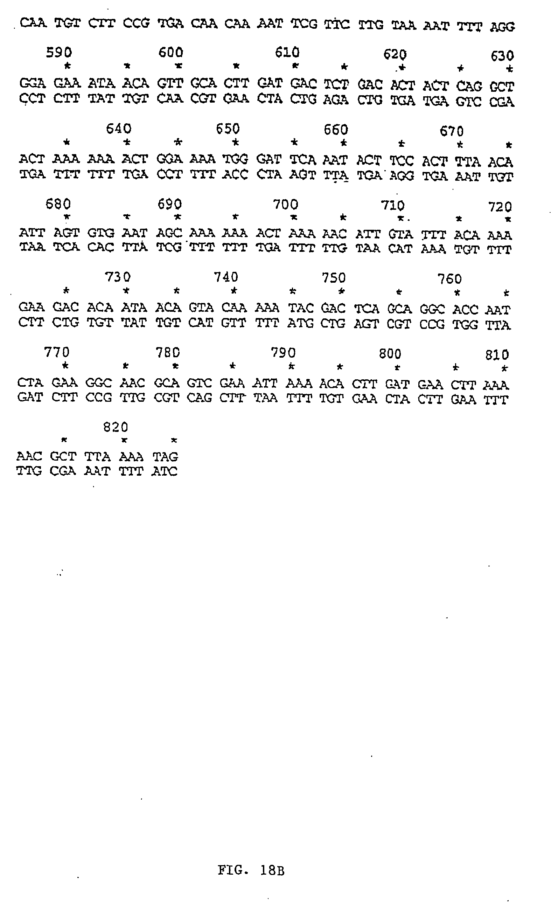

Figs. 18A and 18B depict the nucleic acid sequence of the OspA-Tro/OspA-Bo chimer (SEQ ID NO:47) and the encoded chimeric protein sequence (SEQ ID NO:48).

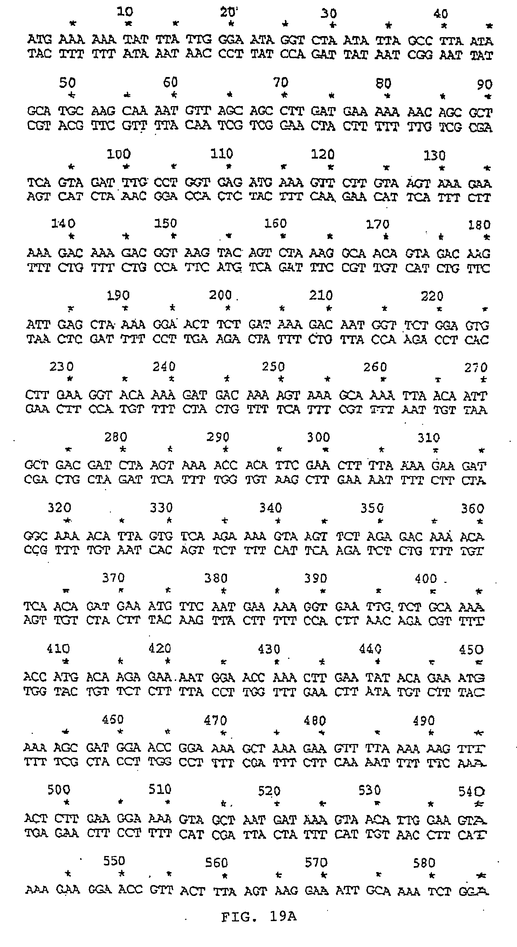

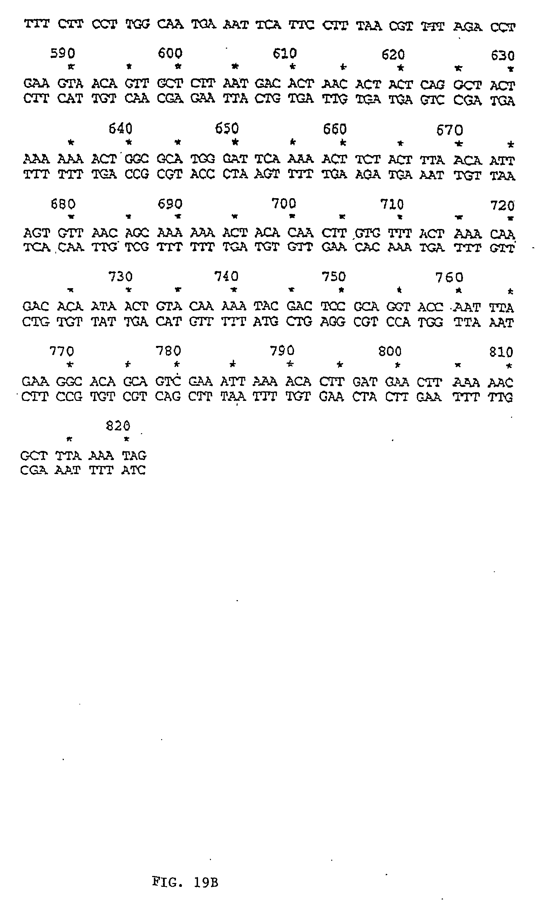

Figs. 19A and 19B depict the nucleic acid sequence of the OspA-PGau/OspA-Bo chimer (SEQ ID NO:49) and the encoded chimeric protein sequence (SEQ ID NO:50).

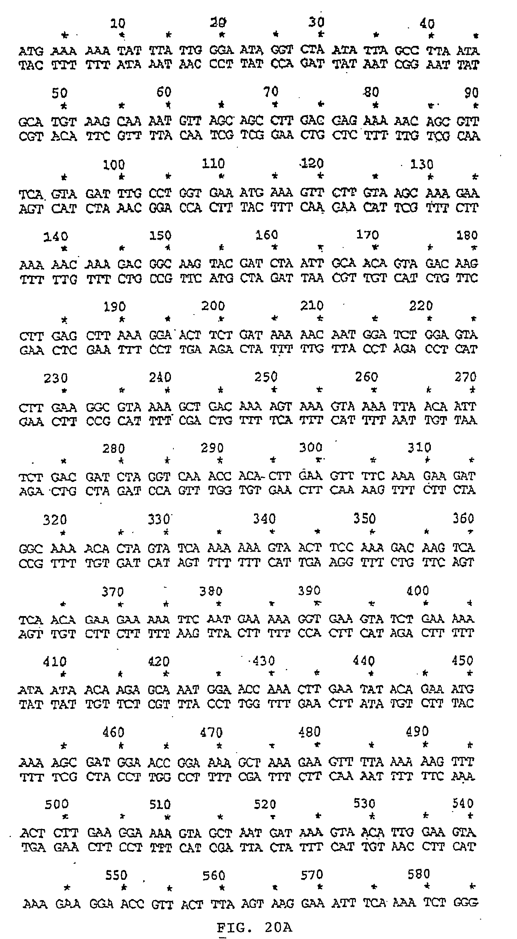

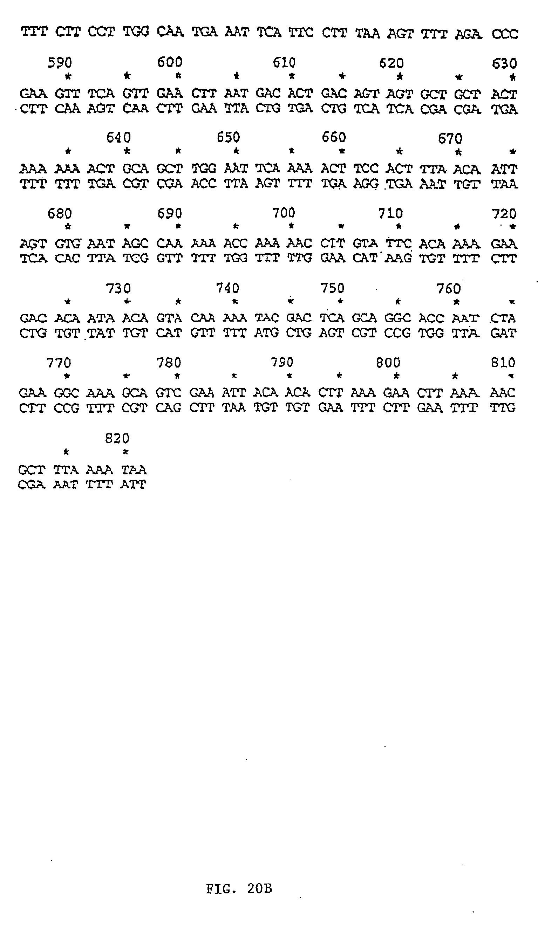

Figs. 20A and 20B depict the nucleic acid sequence of the OspA-B31/OspA-PGau/OspA-B31/OspA-K48 chimer (SEQ ID NO:53) and the encoded chimeric protein sequence (SEQ ID NO:54).

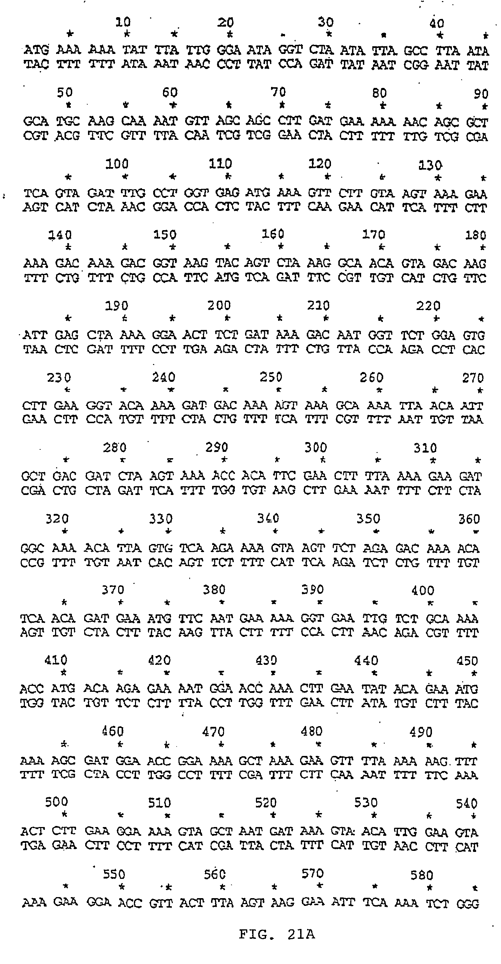

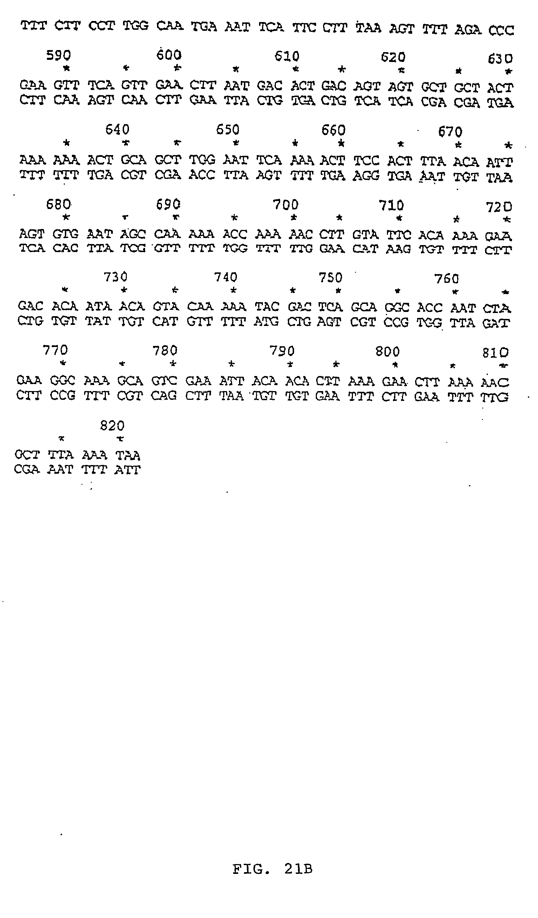

Fig. 21A and 21B depict the nucleic acid sequence of the OspA-PGau/OspA-B31/OspA-K48 chimer (SEQ ID NO:51) and the encoded chimeric protein sequence (SEQ ID NO:52).
Fig. 22 is a bar graph showing the reactivity (as measured by ELISA) of sera from mice immunized with the indicated *Borrelia* protein (OspA or OspC) or recombinant chimeric protein (OspC2-OspA)(X-axis) against the indicated OspA or OspC antigens (legend) from the strain B31 *(Borrelia burgdorferi sensu stricto).*
Fig. 23 is a bar graph showing the reactivity (as measured by ELISA) of sera from mice immunized with the indicated *Borrelia* protein (OspA or OspC) or recombinant chimeric protein (OspC2-OspA)(X-axis) against the indicated OspA or OspC antigens (legend) from the strain B31 *(Borrelia burgdorferi sensu stricto).* For the ELISA results to the B31 OspA antigen, a purified fragment of B31 OspA (amino acids 18-139) was added in excess to the sera so that the detected immune response was specific for the C-terminal region of OspA.
Fig. 24 is a bar graph showing the reactivity of sera from mice immunized with the indicated *Borrelia* chimeric protein (lipOspA/Bo, lipOspAB/P or OspC-OspAB/P)(X-axis) against the indicated OspA antigens (legend) from strains B31 *(Borrelia burgdorferi sensu stricto),* K48 *(Borrelia garinii)* and Pgau *(Borrelia afzelii*)*.*
Fig. 25 is a bar graph showing the reactivity of sera from mice immunized with the indicated *Borrelia* chimeric protein (lipOspAP/Bo, lipOspAB/P or OspC-OspAB/P)(X-axis) against the indicated OspA (legend) from strains B31 *(Borrelia burgdorferi sensu stricto),* K48 *(Borrelia garinii)* and Pgau *(Borrelia afzelii).* In all cases, a purified fragment of B31 OspA (amino acids 18-139) was added in excess to the sera so that the detected immune response is specific for the C-terminal region of OspA.
Fig. 26 is a bar graph showing the reactivity of sera from mice immunized with the indicated *Borrelia* chimeric protein (OspCB31-OspAB31, OspC2-OspAB31 or lip OspC-B31)(X-axis) against the indicated OspC antigen (legend) from the strain B31 *(Borrelia burgdorferi sensu stricto).*
Fig. 27 is a bar graph showing the reactivity of sera from mice immunized with the indicated *Borrelia* chimeric protein (OspCB31-OspAB31, OspC2-OspAB31 or Lip OspA K/T)(X-axis) against the indicated OspA antigens (legend) from strains B31 (*Borrelia burgdorferi sensu stricto),* K.48 *(Borrelia garinii)* and Pgau *(Borrelia afzelii).*
Fig. 28 is a bar graph showing the reactivity of sera from mice immunized with the indicated *Borrelia* chimeric protein (OspCB31-OspAB/P, OspCB31-OspABPBP or OspCB31-OspAB31)(X-axis) against the indicated OspA antigens (legend) from strains B31 *(Borrelia burgdorferi sensu stricto),* K48 *(Borrelia garinii)* and Pgau *(Borrelia afzelii).*
Fig. 29 is a bar graph showing the reactivity of sera from mice immunized with the indicated *Borrelia* chimeric protein (OspCB31-OspAB/P, OspCB31-OspABPBP or OspCB31-OspAB31)(X-axis) against the indicated OspA (legend) from strains B31 *(Borrelia burgdorferi sensu stricto),* K48 *(Borrelia garinii)* and Pgau *(Borrelia afzelii).* In all cases, a purified fragment of B31 OspA (amino acids 18-139) was added in excess to the sera so that the detected immune response is specific for the C-terminal region of OspA.

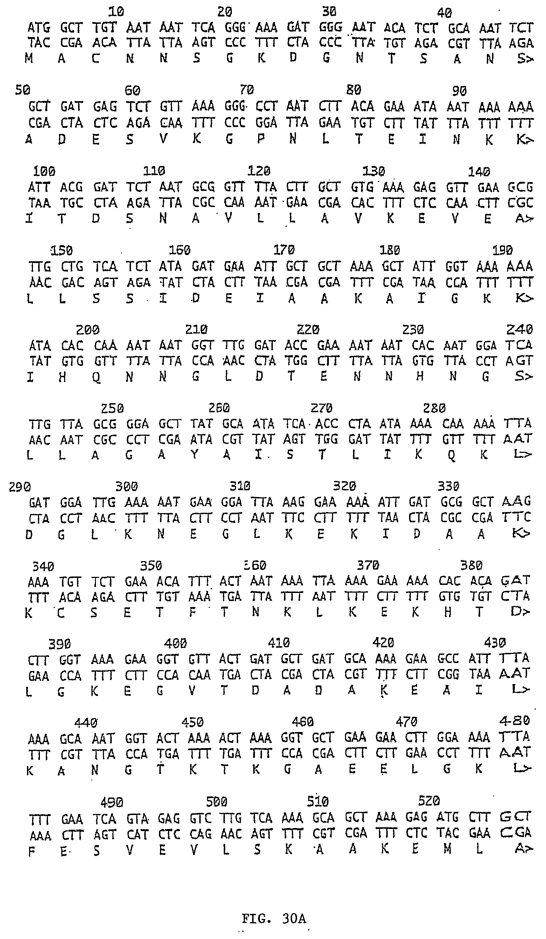

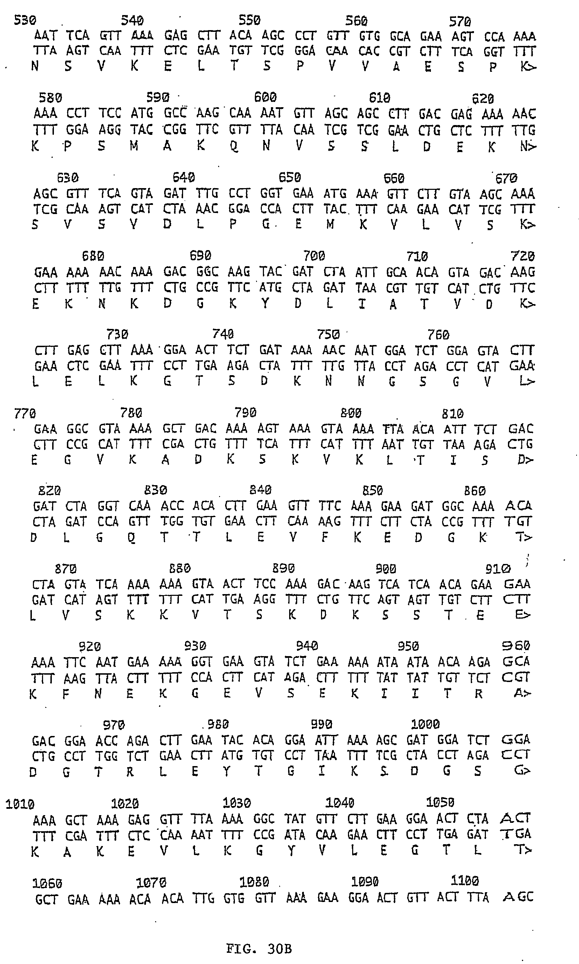

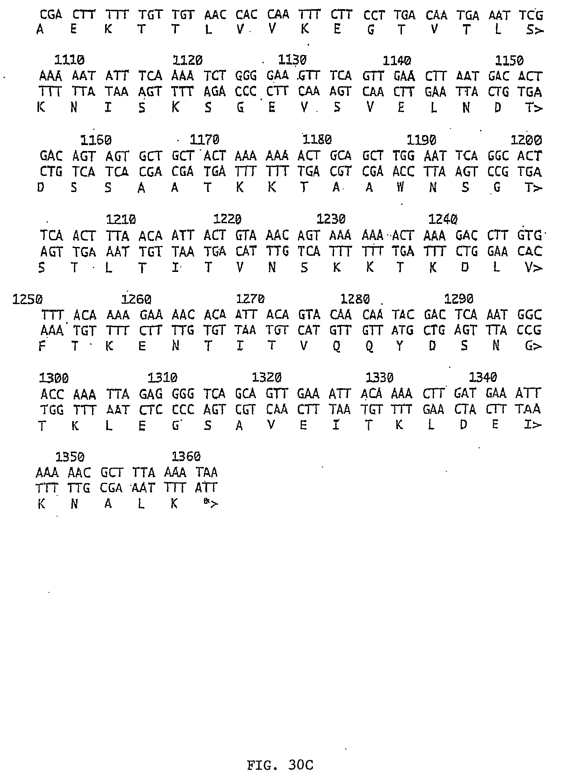

Figs. 30A, 30B and 30C depict the nucleic acid sequence of the OspC-B31 (bp 55-633)/OspA-B31 (bp 52-822) chimer (SEQ ID NO:55) and the encoded chimeric protein sequence (SEQ ID N0:56).

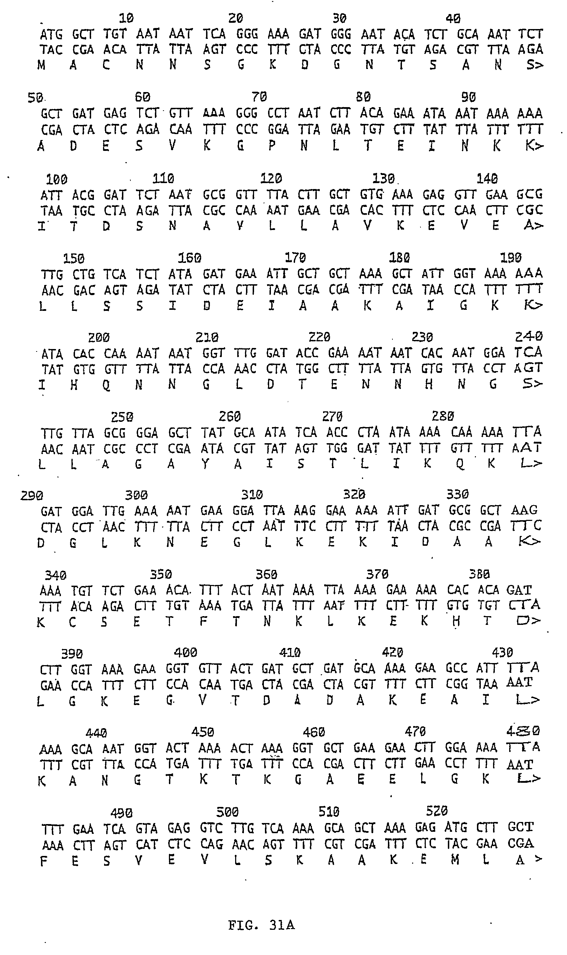

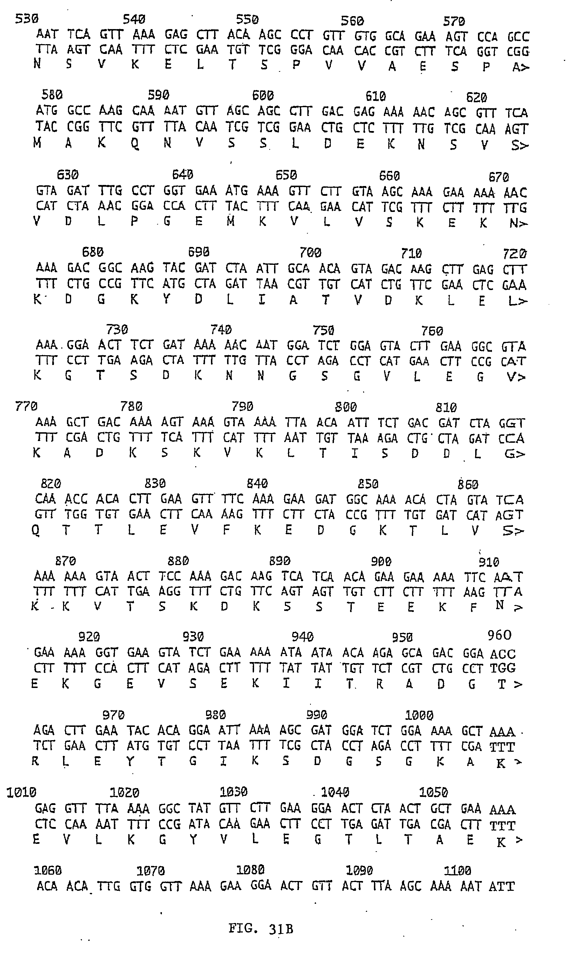

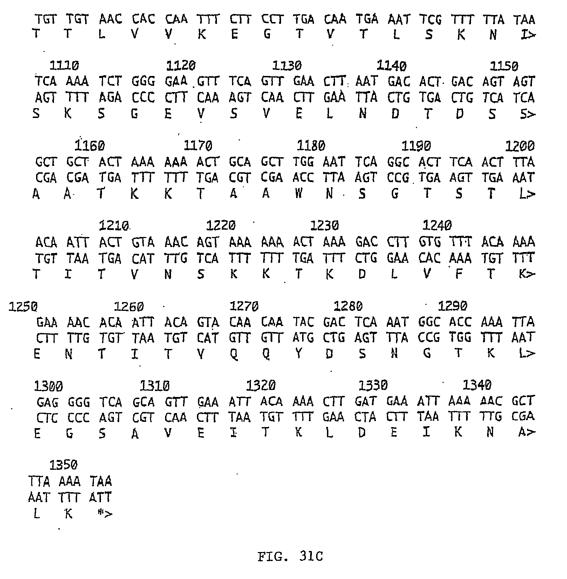

Figs. 31A, 31B and 31C depict the nucleic acid sequence of the OspC-B31 (bp 55-624)/OspA-B31 (bp 52-822) chimer (SEQ ID N0:57) and the encoded chimeric protein sequence (SEQ ID NO:58).

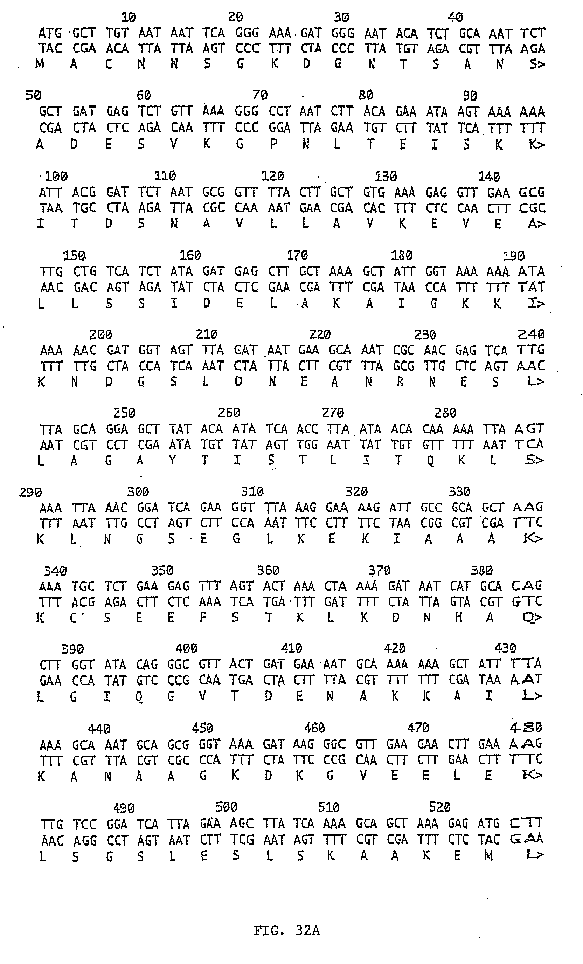

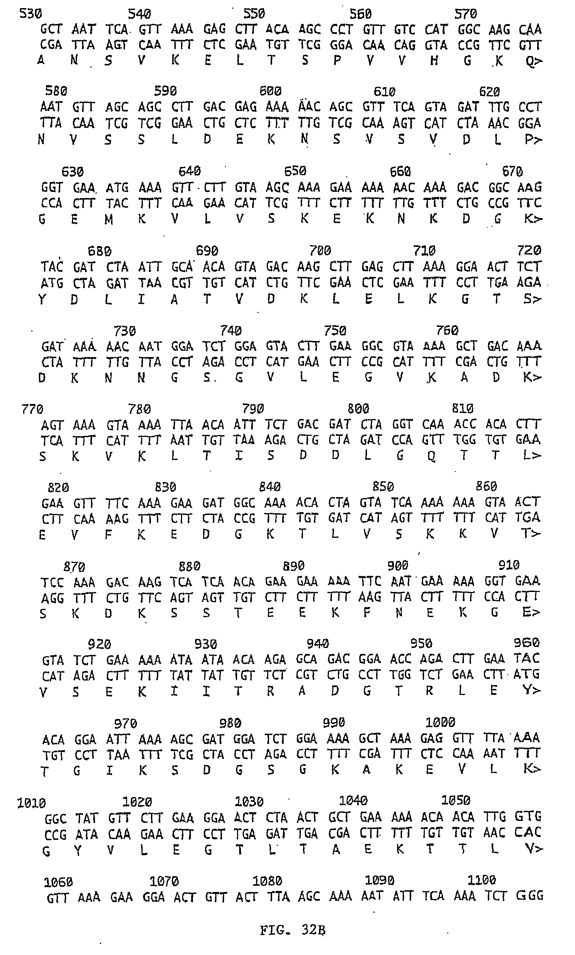

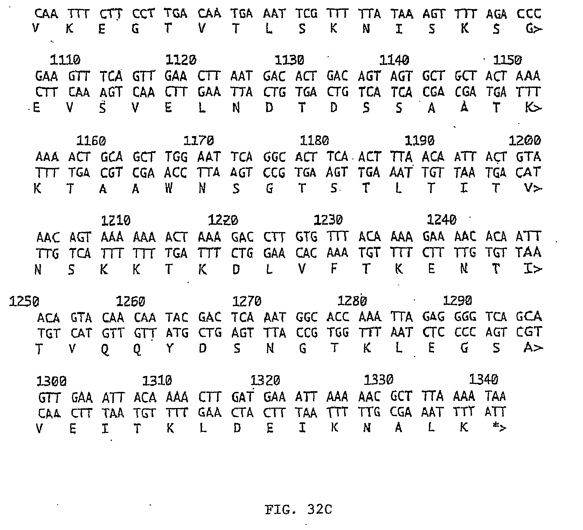

Figs. 32A, 32B and 32C depict the nucleic acid sequence of the OspC-C2 (bp 55-612)/OspA-B31 (bp 52-822) chimer (SEQ ID NO:59) and the encoded chimeric protein sequence (SEQ ID NO:60).

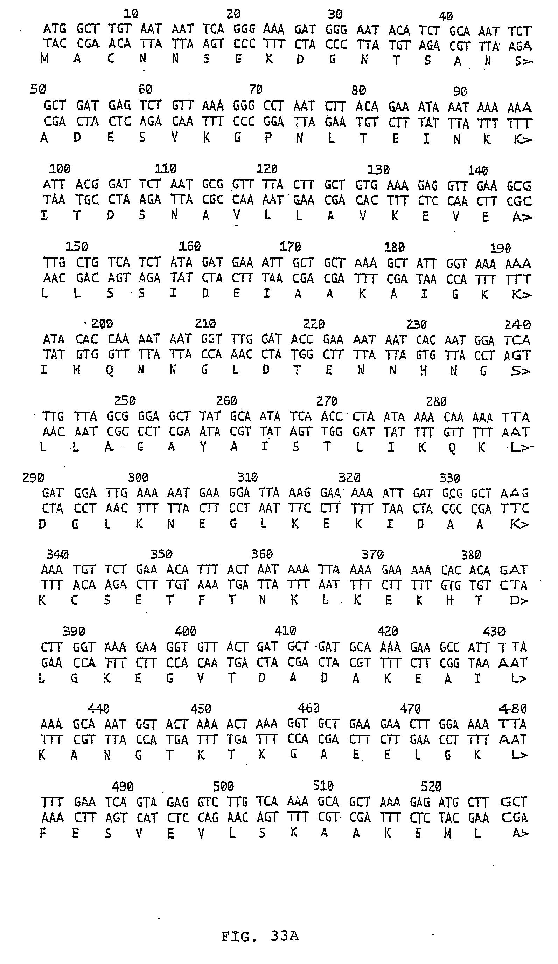

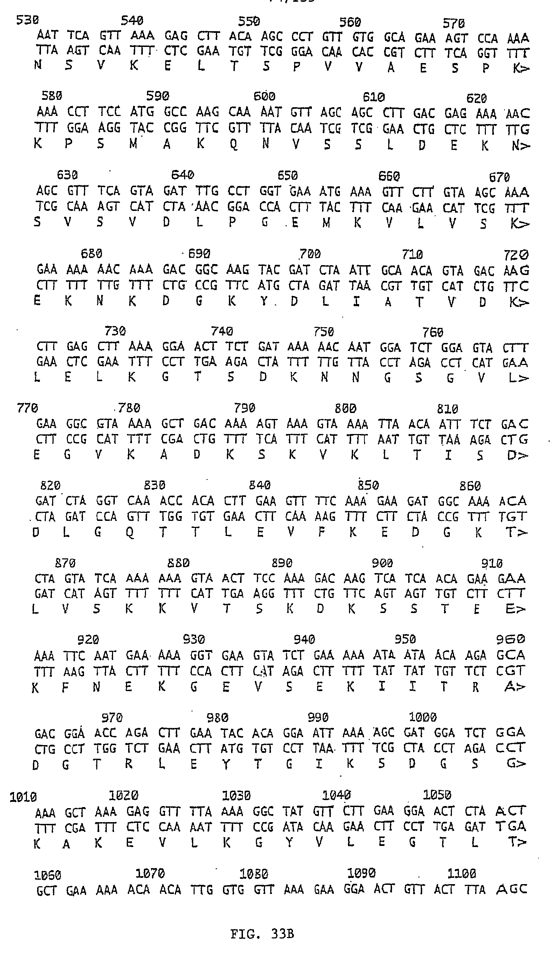

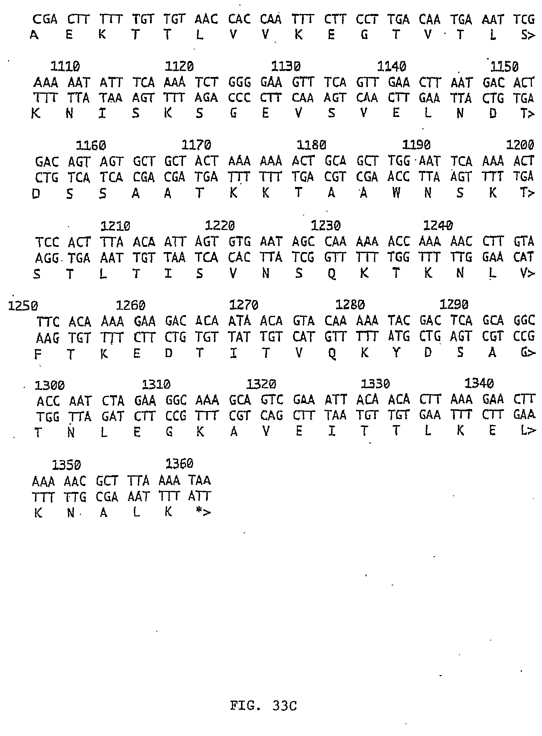

Figs. 33A, 33B, and 33C depict the nucleic acid sequence of the OspC-B31 (bp 55-633)/OspA-B31 (bp 52-651)/OspA-K48 (bp 652-820) chimer (SEQ ID NO:61) and the encoded chimeric protein sequence (SEQ ID NO:62).

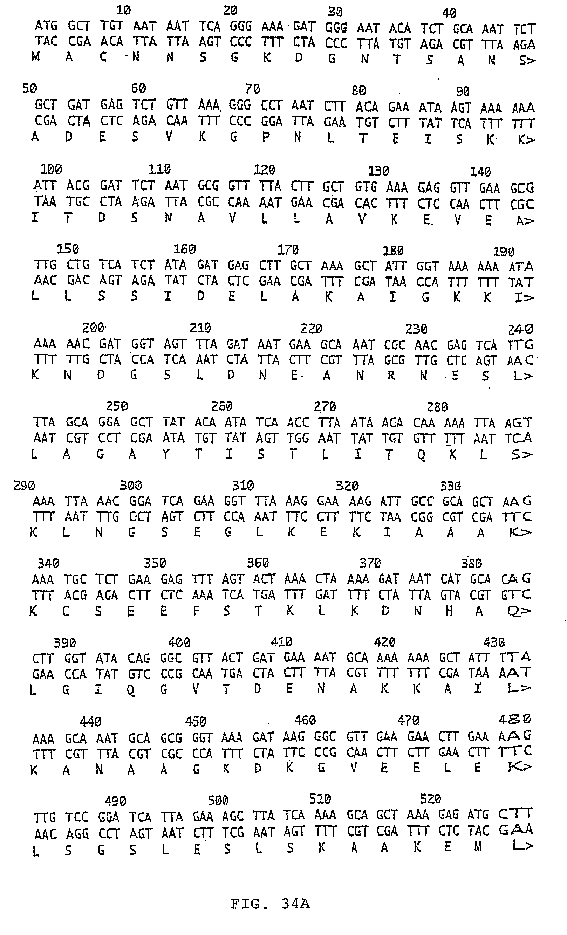

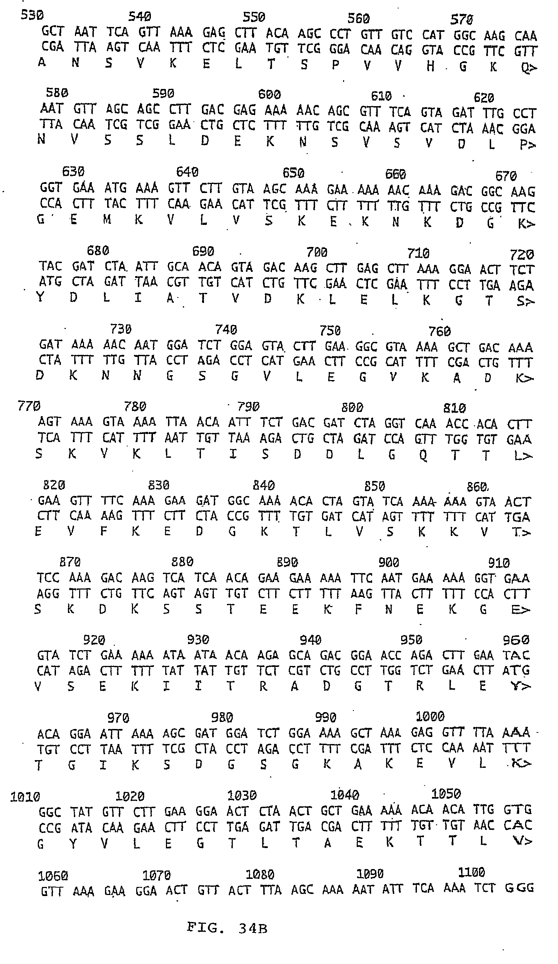

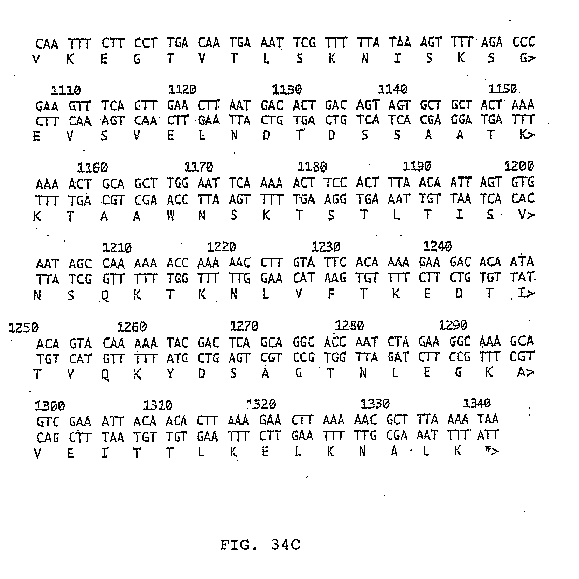

Figs. 34A, 34B and 34C depict the nucleic acid sequence of the OspC-C2 (bp 55-612)/OspA-B31 (bp 52-651)/OspA-K48 (bp 652-820) chimer (SEQ ID NO:63) and the encoded chimeric protein sequence (SEQ ID NO:64).

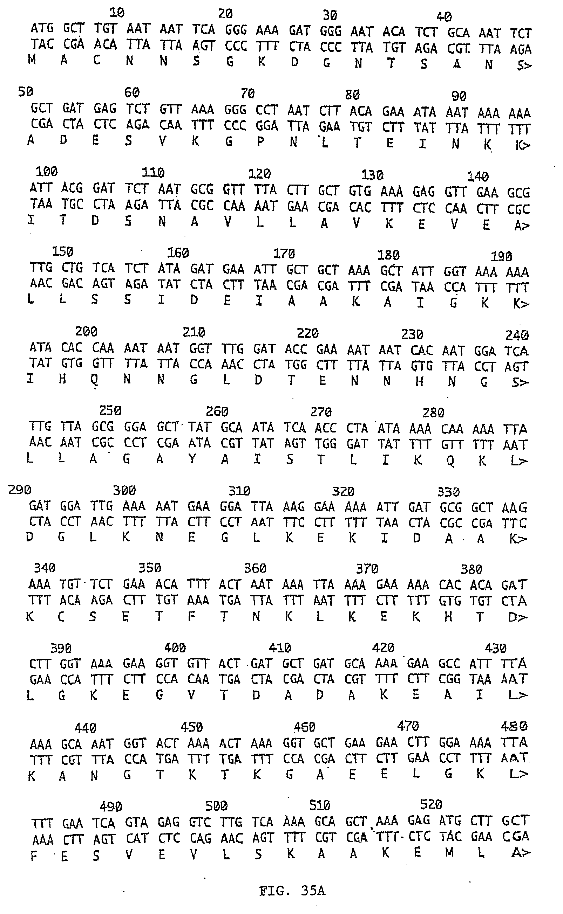

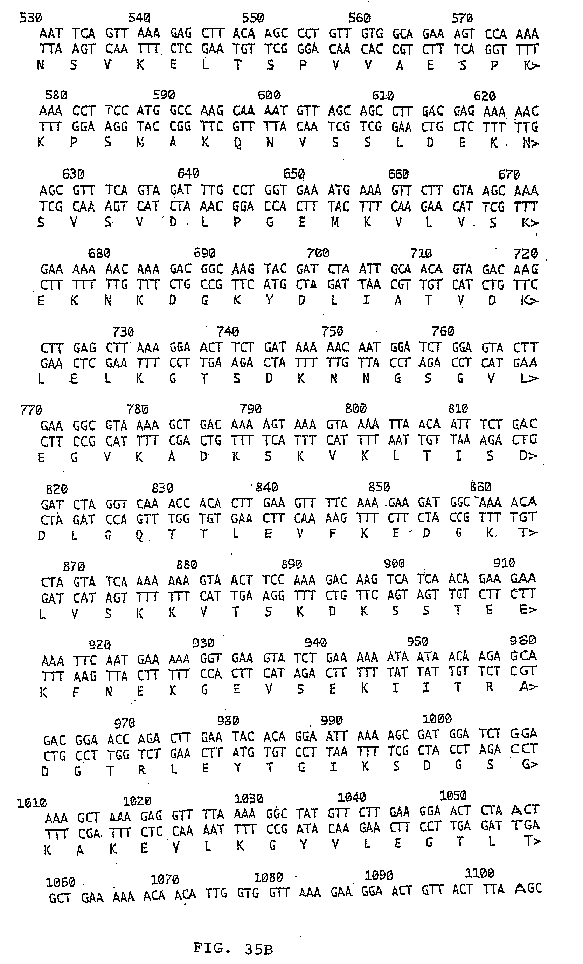

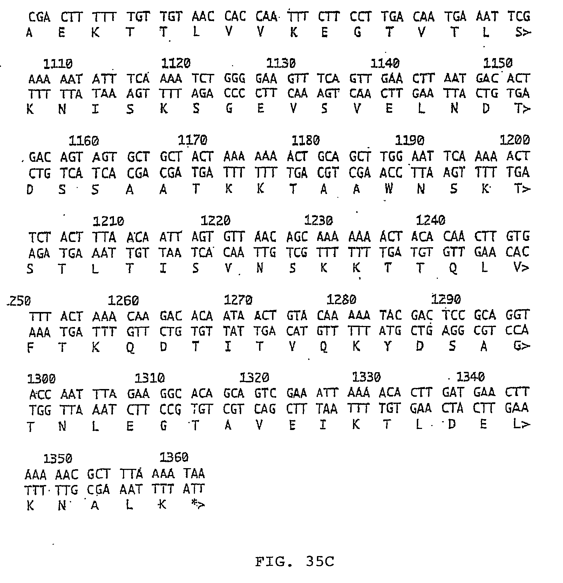

Figs. 35A, 35B and 35C depict the nucleic acid sequence of the OspC-B31 (bp 55-633)/OspA-B31 (bp 52-651)/OspA-Pko (bp 652-820) chimer (SEQ ID NO:65) and the encoded chimeric protein sequence (SEQ ID NO:66).

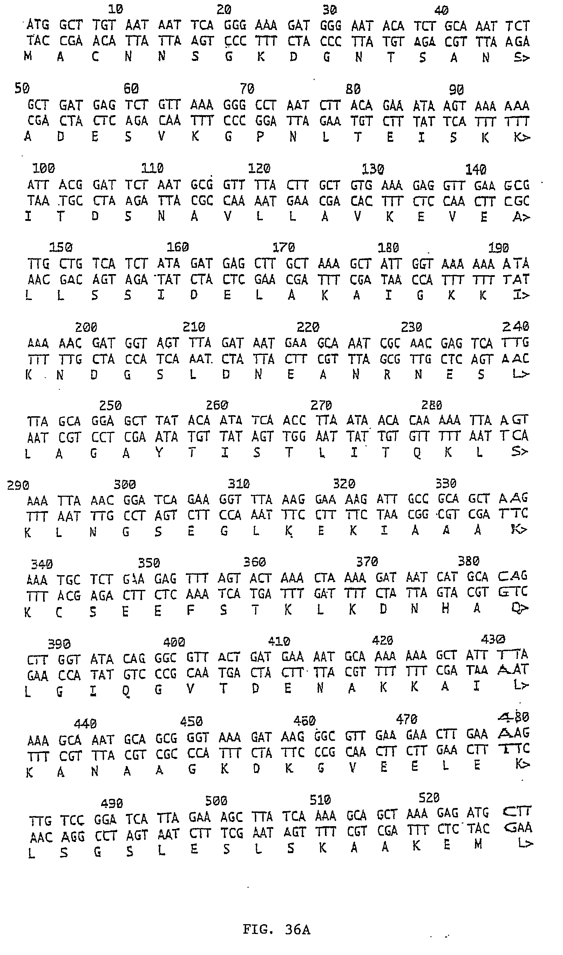

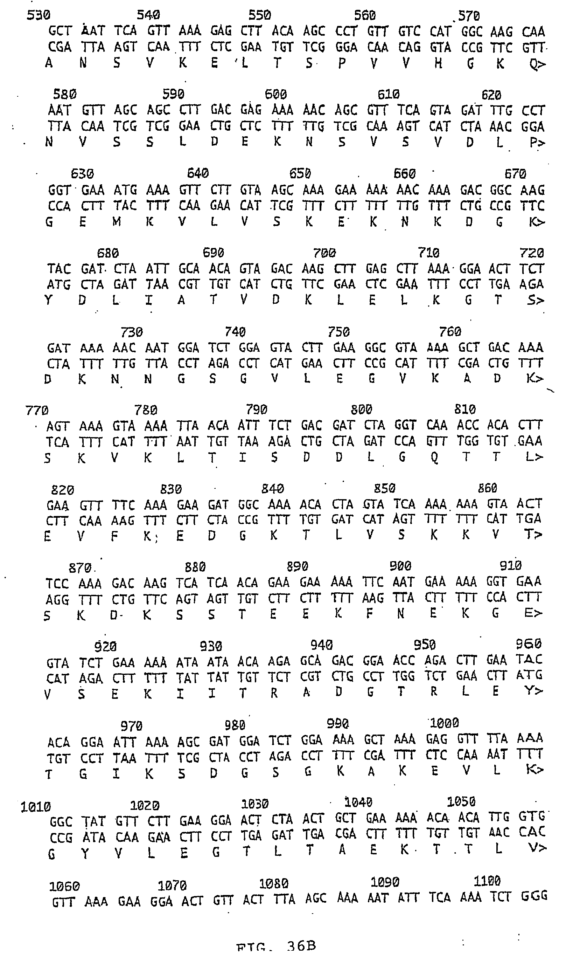

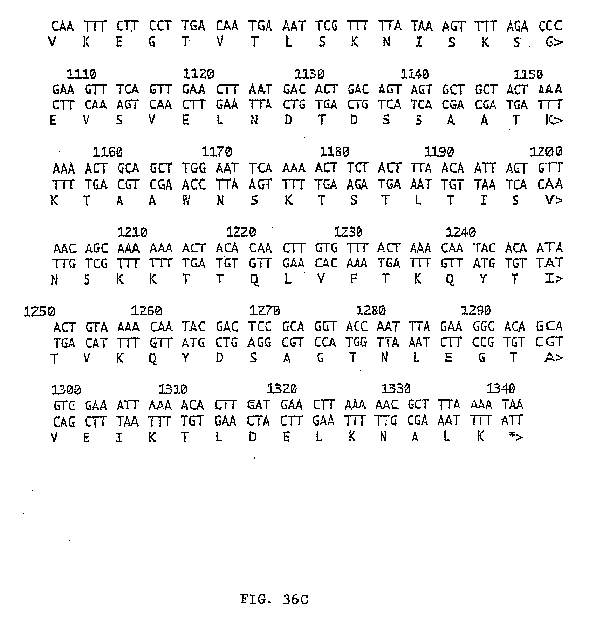

Figs. 36A, 36B and 36C depict the nucleic acid sequence of the OspC-C2 (bp 55-612)/OspA-B31 (bp 52-651)/OspA-Pko (bp 652-820) chimer (SEQ ID NO:67) and the encoded chimeric protein sequence (SEQ ID NO:68).

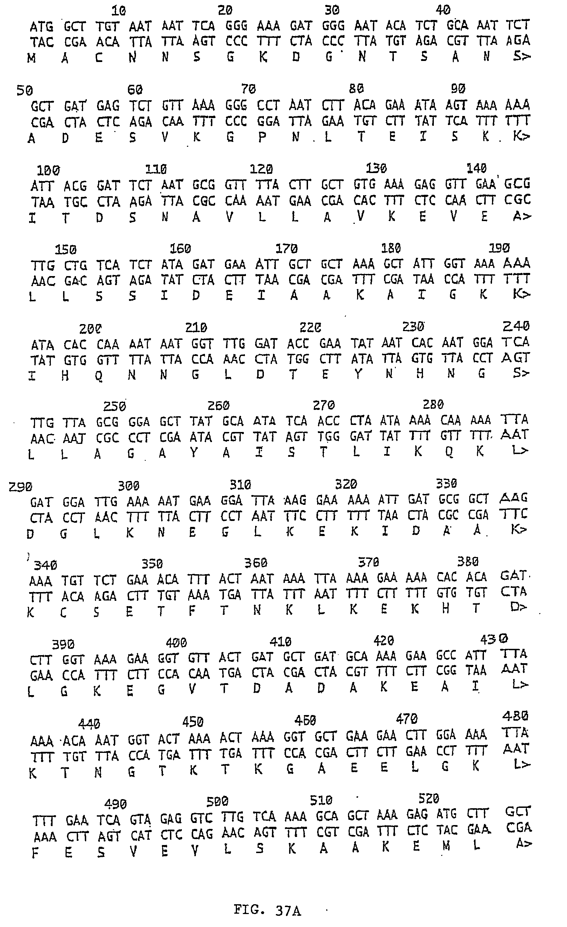

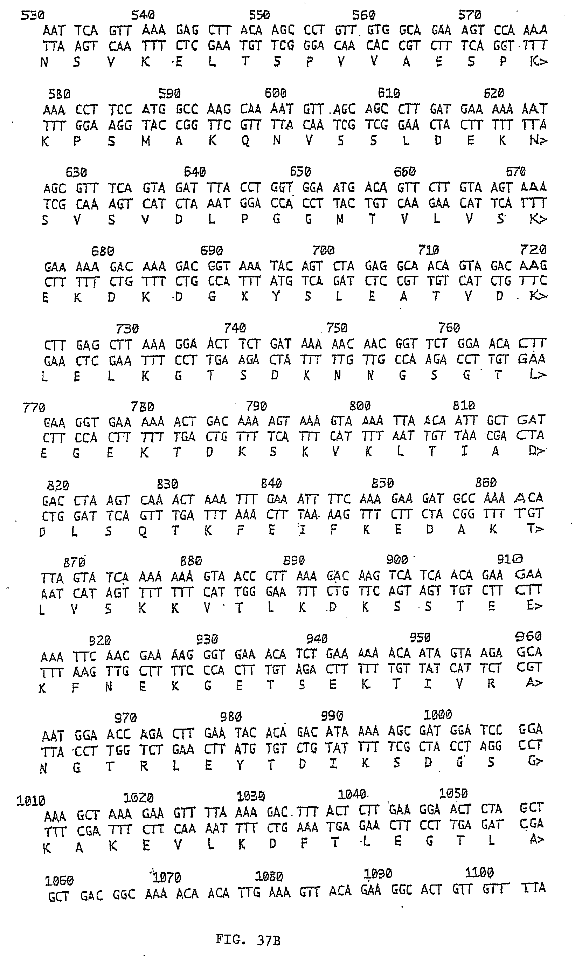

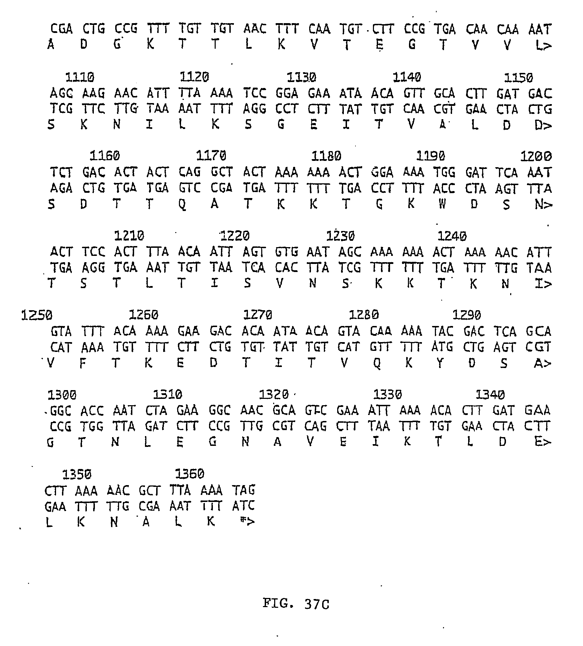

Figs. 37A, 37B and 37C depict the nucleic acid sequence of the OspC-B31 (bp 55-633)/OspA-K48 (bp 52-654)/OspA-Tro (bp 655-819) chimer (SEQ ID NO:69) and the encoded chimeric protein sequence (SEQ ID NO:70).

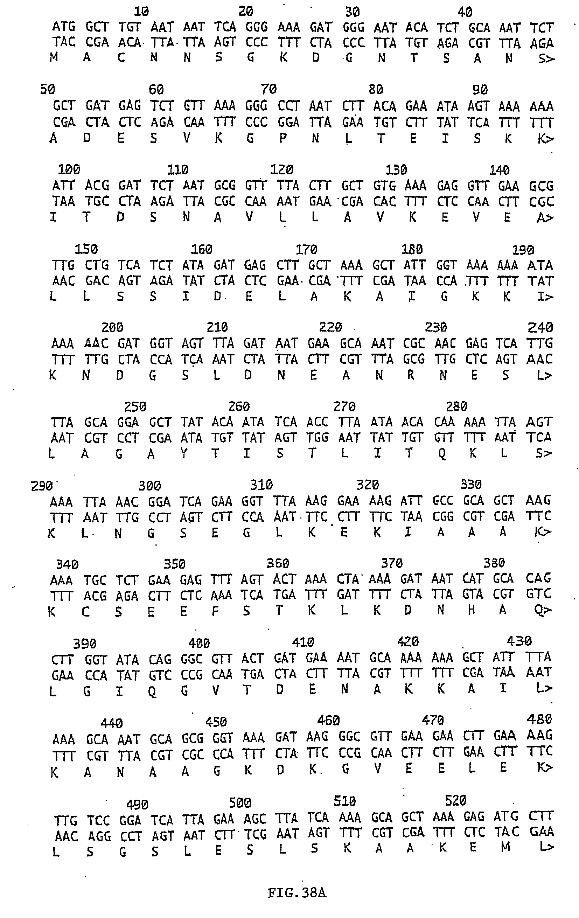

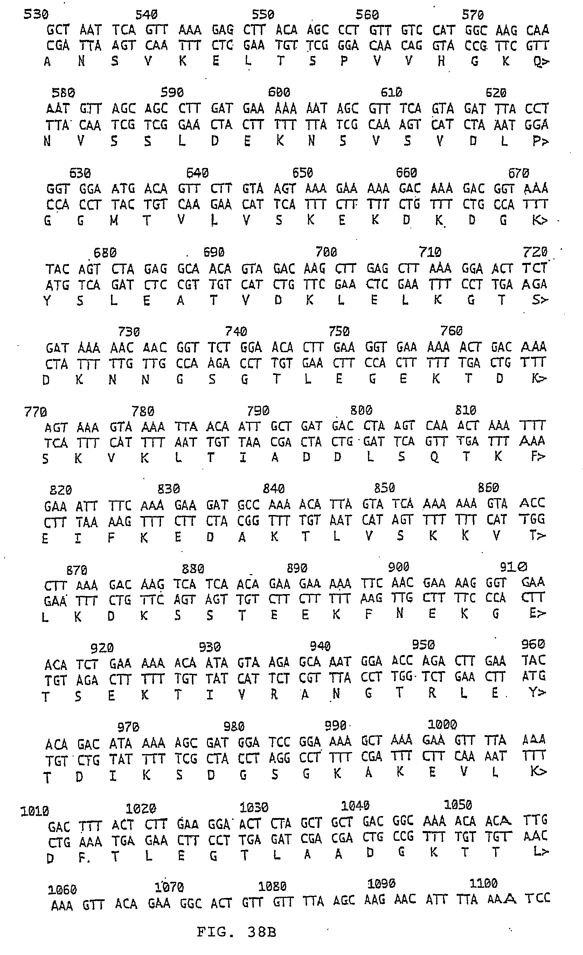

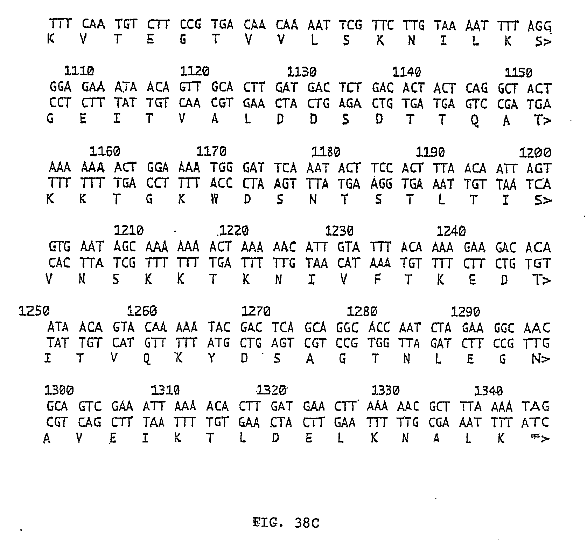

Figs. 38A, 38B and 38C depict the nucleic acid sequence of the OspC-C2 (bp 55-612)/OspA-K48 (bp 52-654)/OspA-Tro (bp 655-819) chimer (SEQ ID NO:71) and the encoded chimeric protein sequence (SEQ ID NO:72).

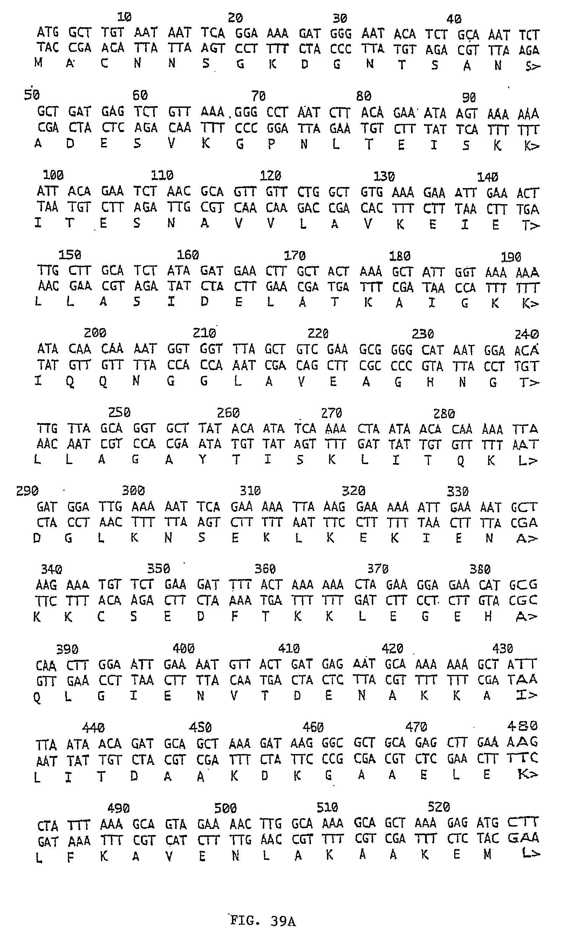

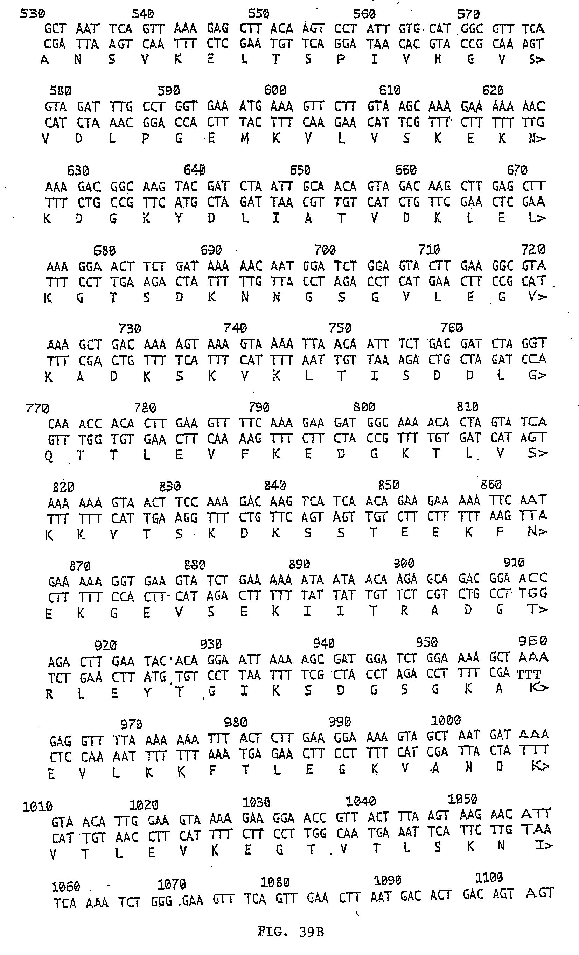

Figs. 39A, 39B, 39C depict the nucleic acid sequence of the OspC-C12 (bp 55-612)/OspA-B31 (bp 88-450)/OspA-Pko (bp 451-537)/OspA-B31 (bp 538-822) chimer (SEQ ID NO:73) and the encoded chimeric protein sequence (SEQ ID NO:74).

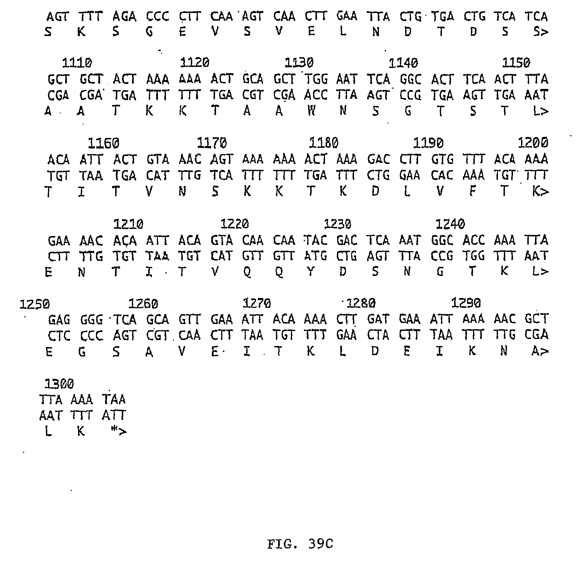

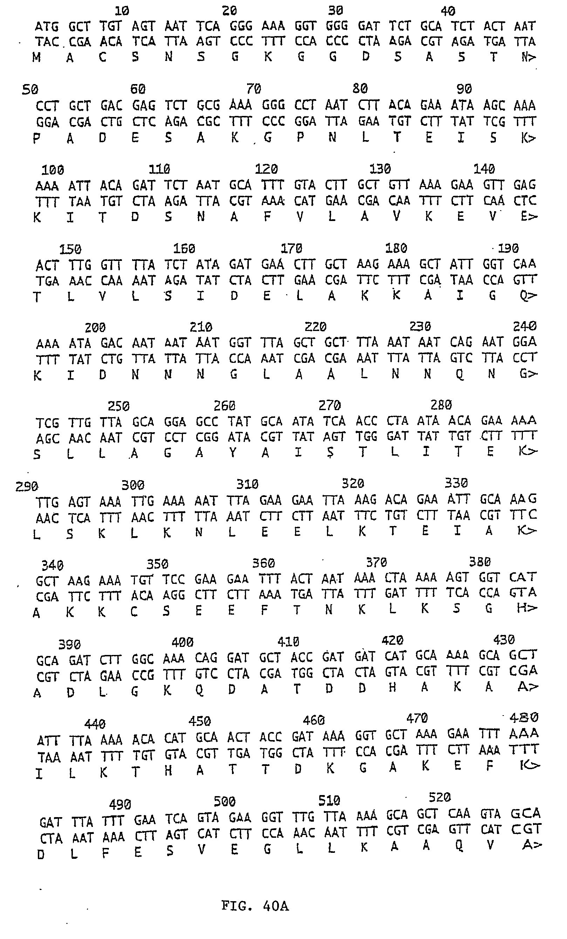

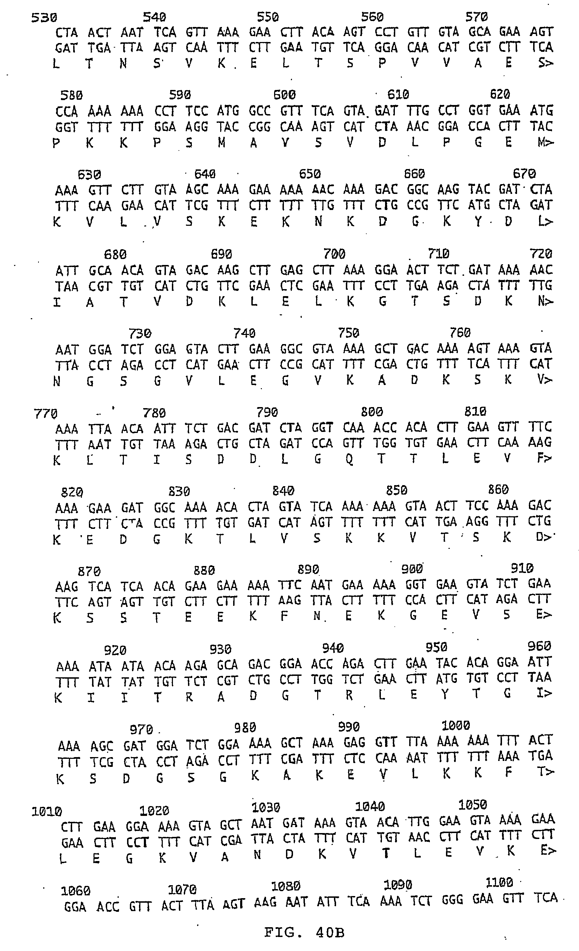

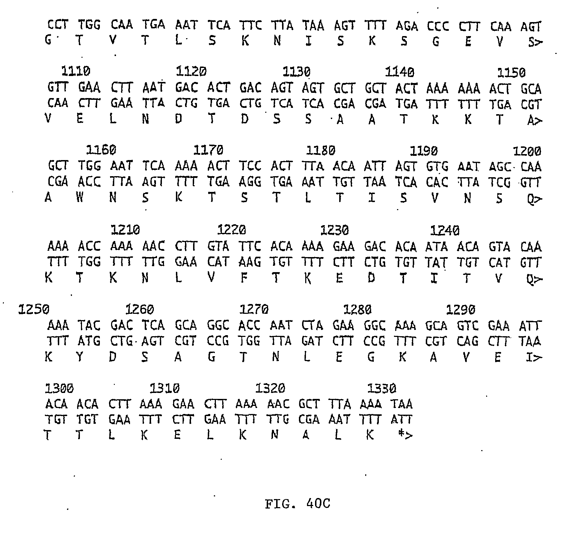

Figs. 40A, 40B and 40C depict the nucleic acid sequence of the OspC-Pko (bp 55-639)/OspA-B31 (bp 88-450)/OspA-Pko (bp 451-537)/OspA-B31 (bp 538-651)/OspA-K48 (bp 652-825) chimer (SEQ ID NO:76) and the encoded chimeric protein sequence (SEQ ID NO:76).

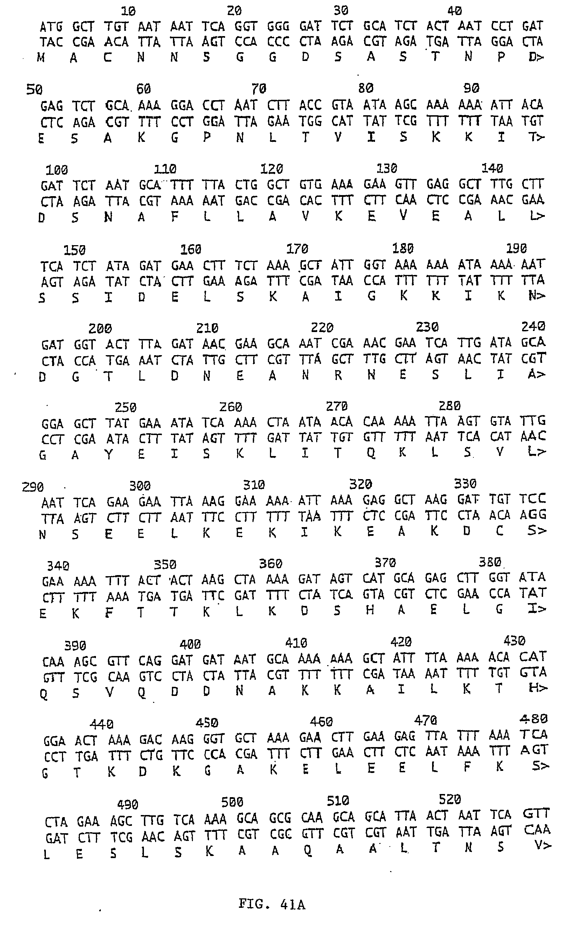

Figs. 41A, 41B and 41C depict the nucleic acid sequence of the OspC-Tro (bp 55-624)/OspA-B31 (bp 88-450)/OspA-Pko (bp 451-537)/OspA-B31 (bp 538-651)/OspA-Pko (bp 652-822) chimer (SEQ ID NO:77) and the encoded chimeric protein sequence (SEQ ID NO:78).

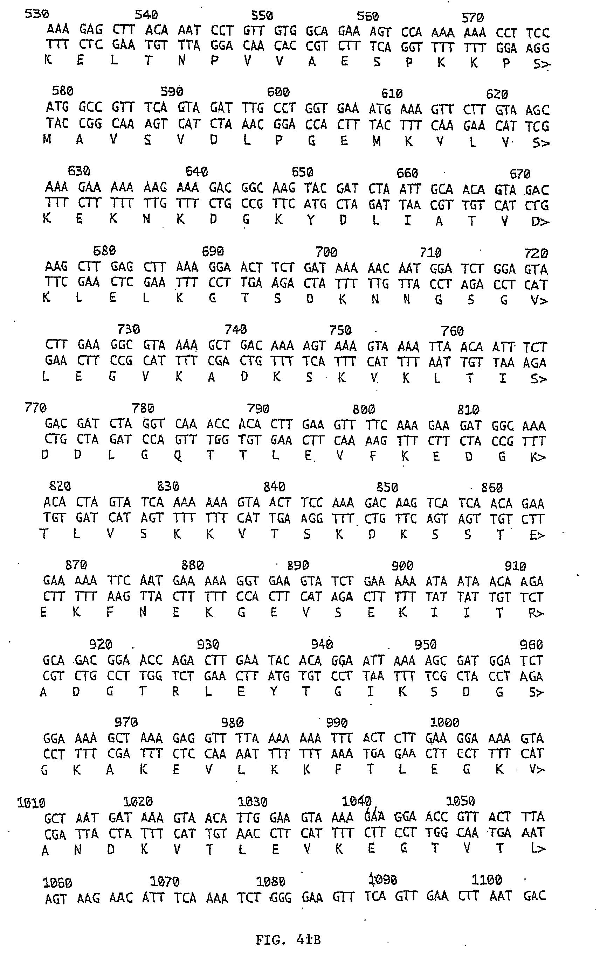

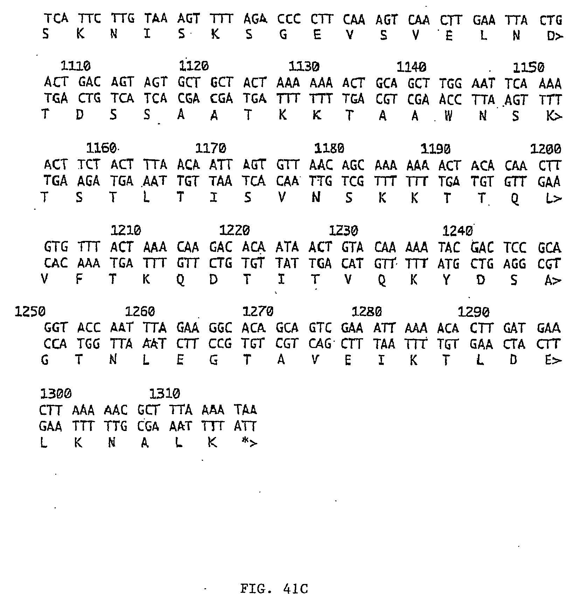

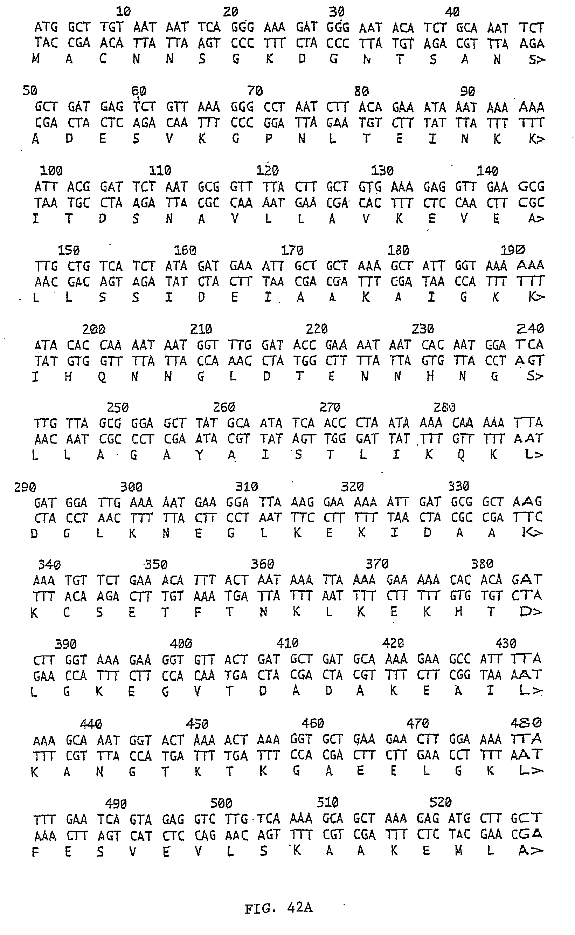

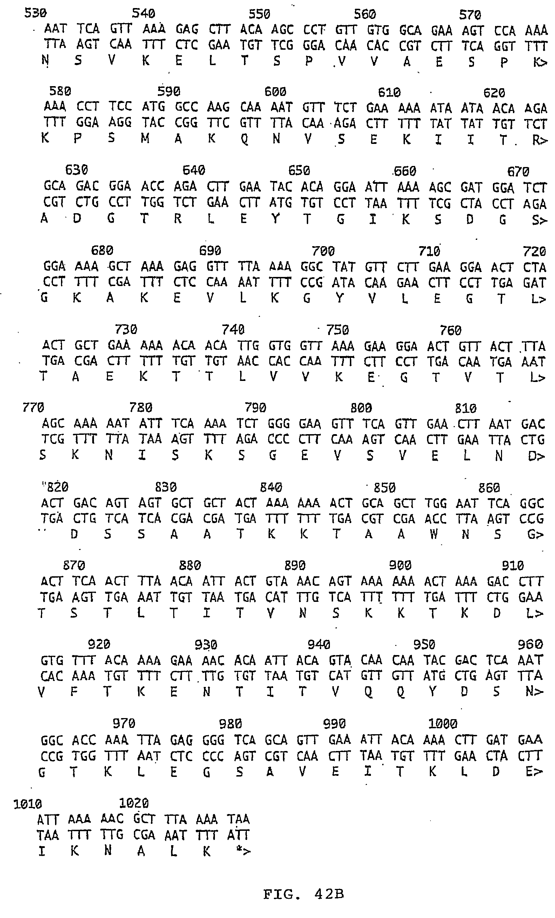

Figs. 42A and 42B depict the nucleic acid sequence of the OspC-B31 (bp 55-633)/OspA-B31 (bp 394-820) chimer (SEQ ID NO:79) and the encoded chimeric protein sequence (SEQ ID NO:80).

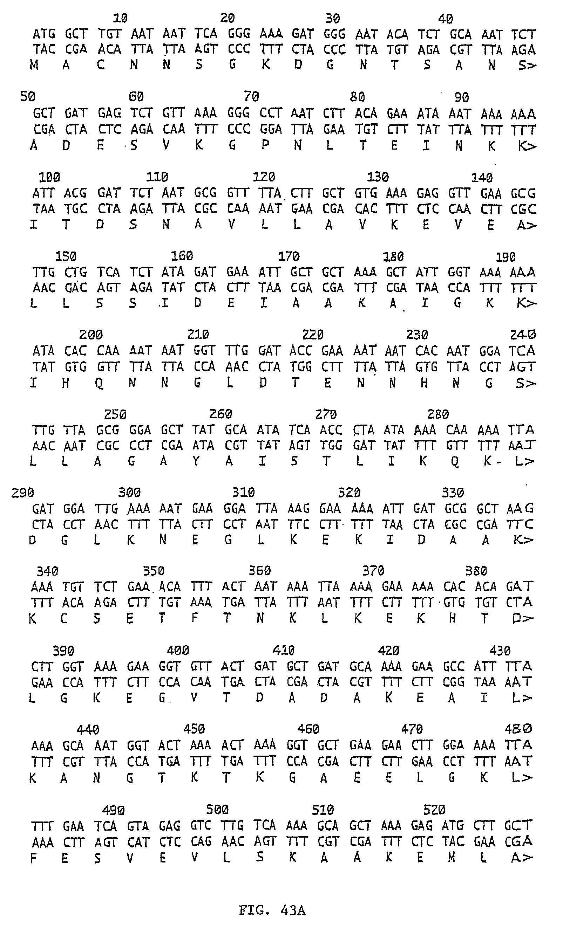

Figs. 43A and 43B depict the nucleic acid sequence of the OspC-B31 (bp 55-631)/OspA-B31 (bp 394-651)/OspA-K48 (bp 652-820) chimer (SEQ ID NO:81) and the encoded chimeric protein sequence (SEQ ID NO:82).

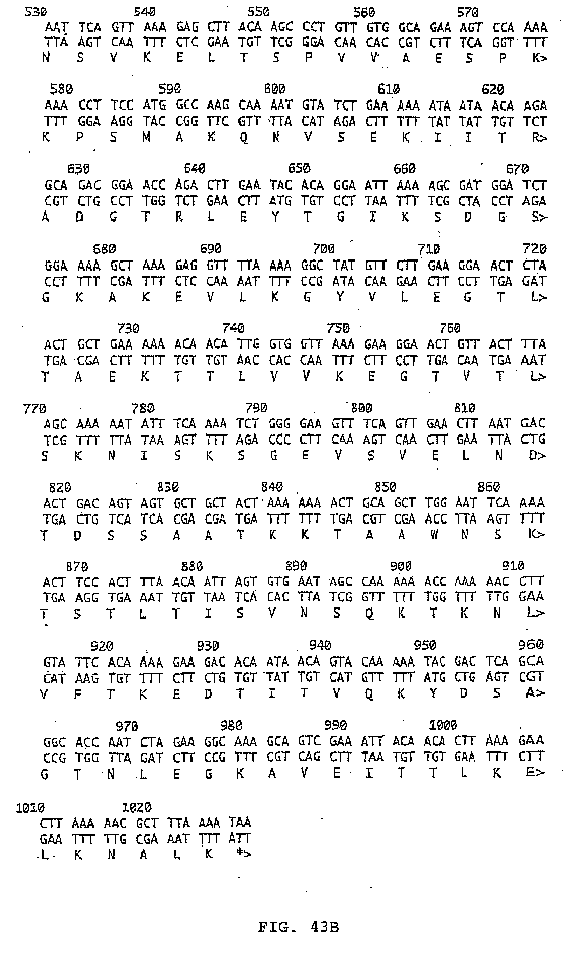

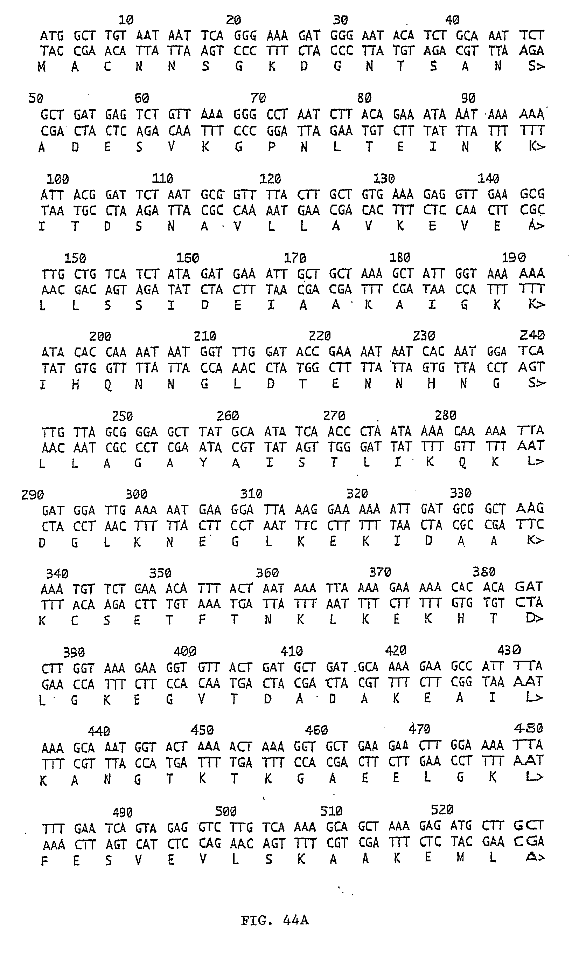

Figs. 44A and 44B depict the nucleic acid sequence of the OspC-B31 (bp 55-633)/OspA-B31 (bp 394-651)/OspA-Pko (bp 652-820) chimer (SEQ ID NO:83) and the encoded chimeric protein sequence (SEQ ID NO: 84).

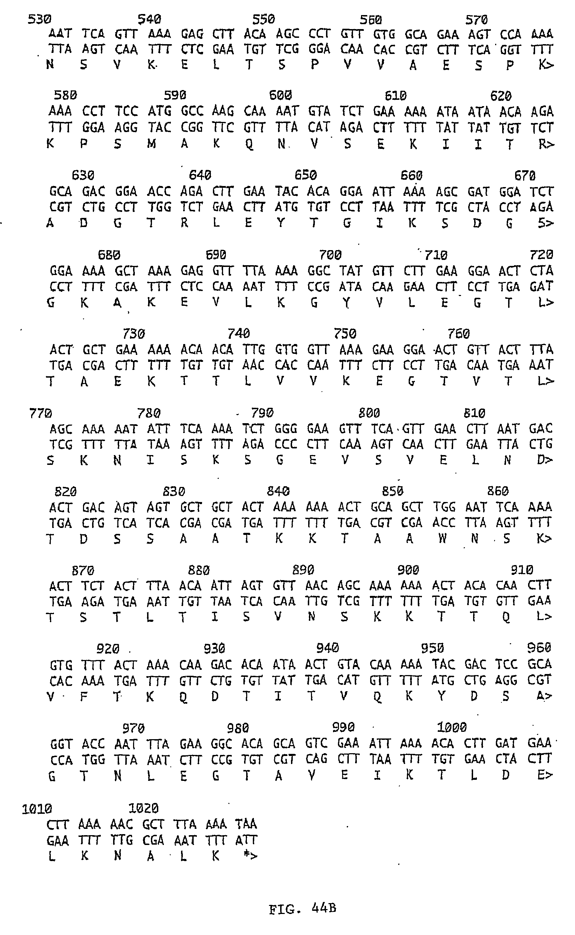

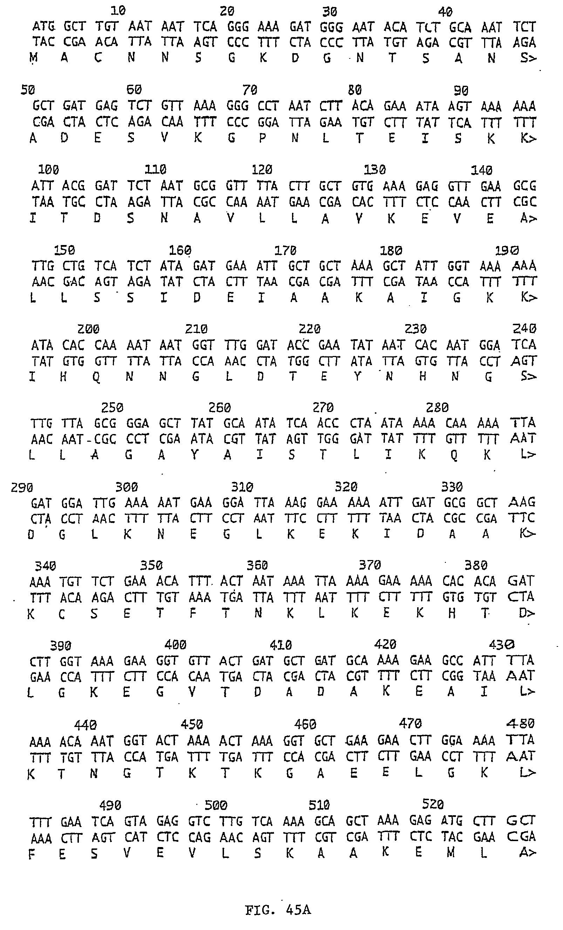

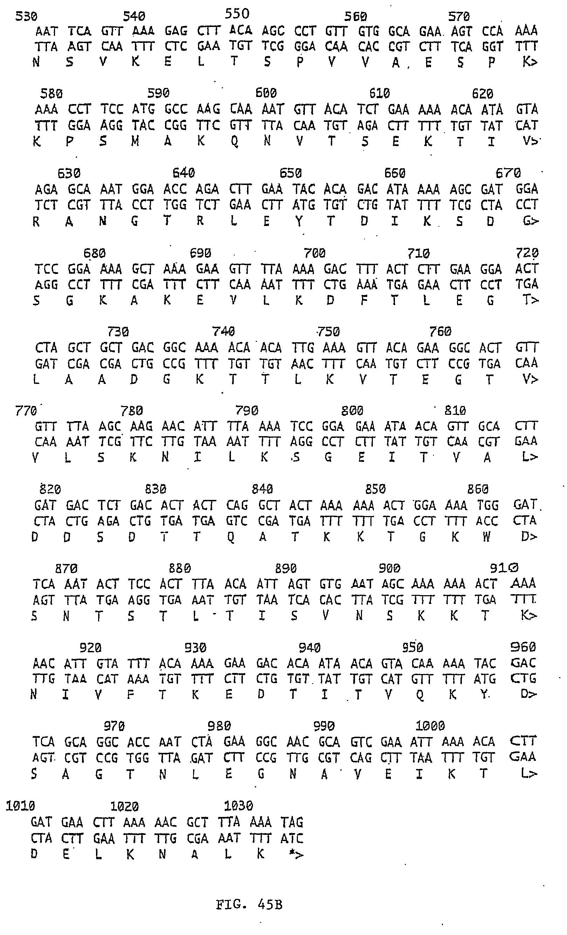

Figs. 45A and 45B depict the nucleic acid sequence of the OspC-B31 (bp 55-633)/OspA-K48 (bp 394-654)/OspA-Tro (bp 655-819) chimer (SEQ ID NO:85) and the encoded chimeric protein sequence (SEQ ID NO:86).

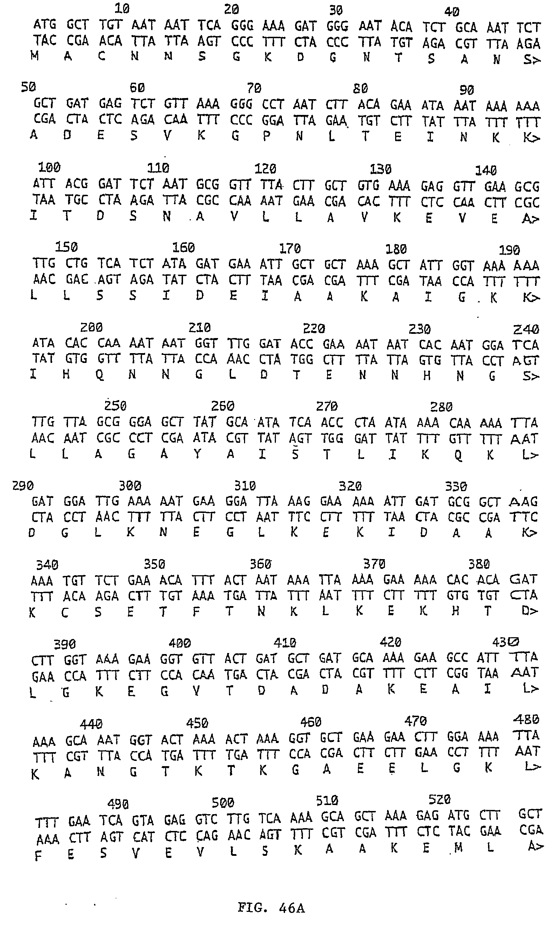

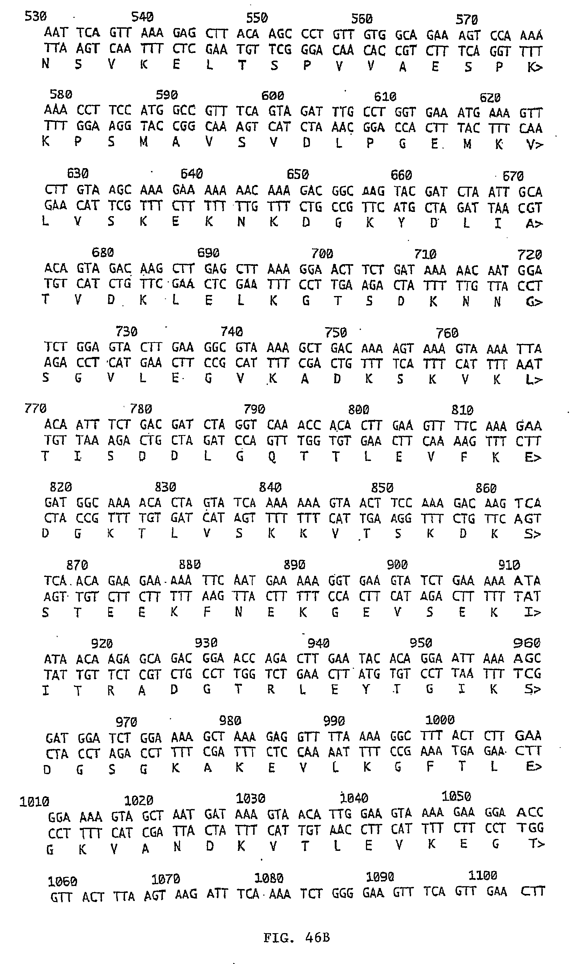

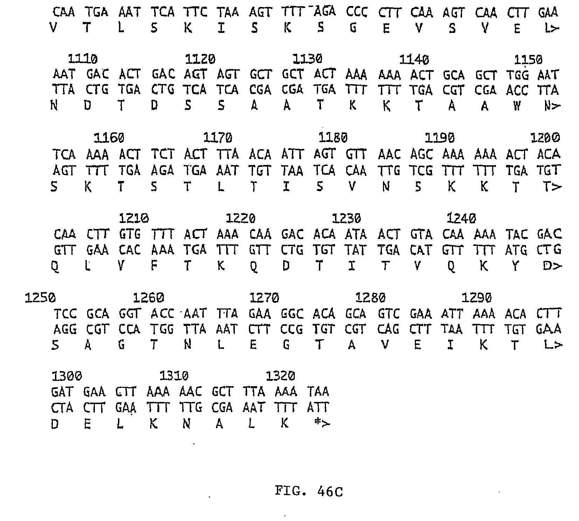

Figs. 46A, 46B and 46C depict the nucleic acid sequence of the OspC-B31 (bp 55-633)/OspA-B31 (bp 88-450)/OspA-Pko (bp 451-537)/OspA-B31 (bp 541-651)/OspA-Pko (bp 652-822) chimer (SEQ ID NO:87) and the encoded chimeric protein sequence (SEQ ID NO:88).

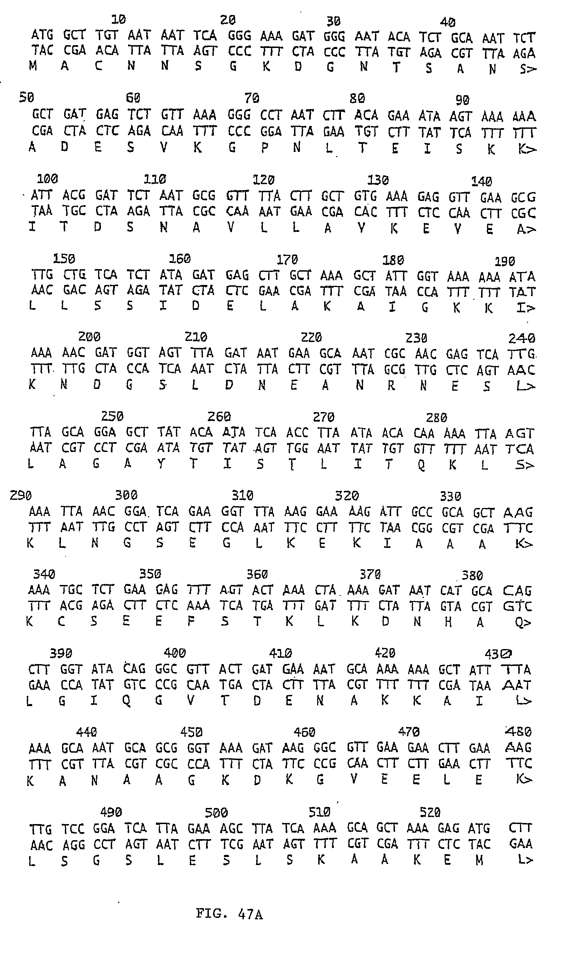

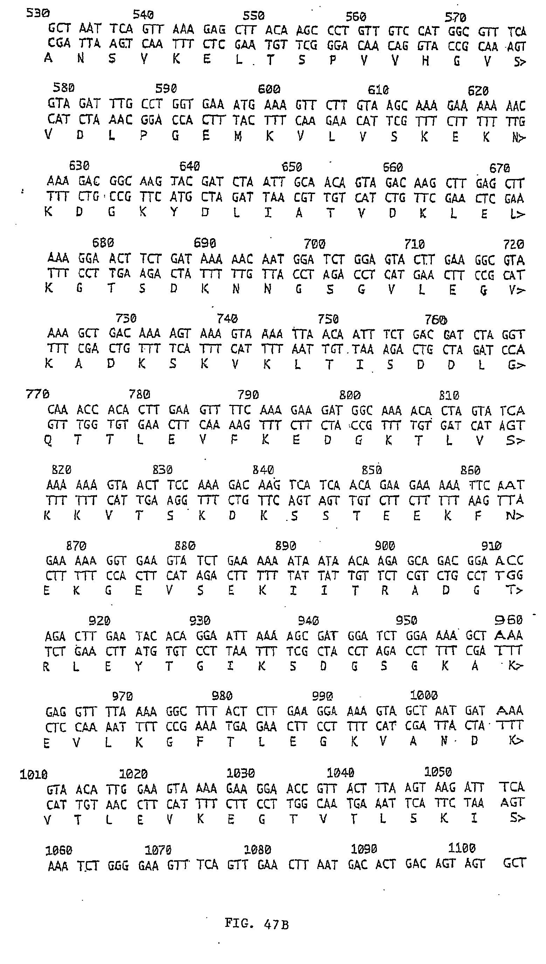

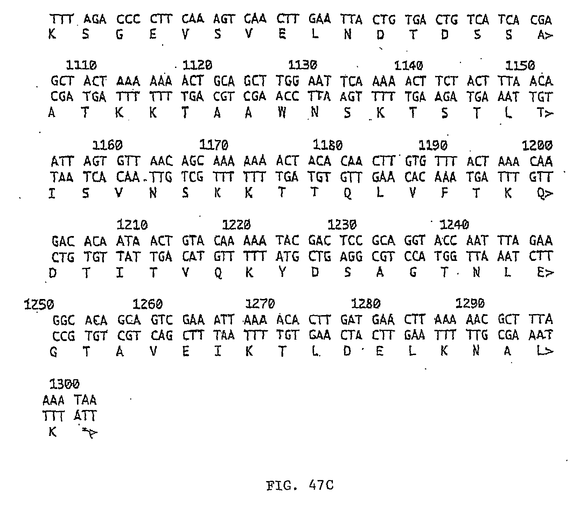

Figs. 47A, 47B, and 47C depict the nucleic acid sequence of the OspC-C2 (bp 55-612)/OspA-B31 (bp 88-450)/OspA-Pko (bp 451-537)/OspA-B31 (bp 541-651)/OspA-Pko (bp 652-822) chimer (SEQ ID NO:89) and the encoded chimeric protein sequence (SEQ ID NO:90).

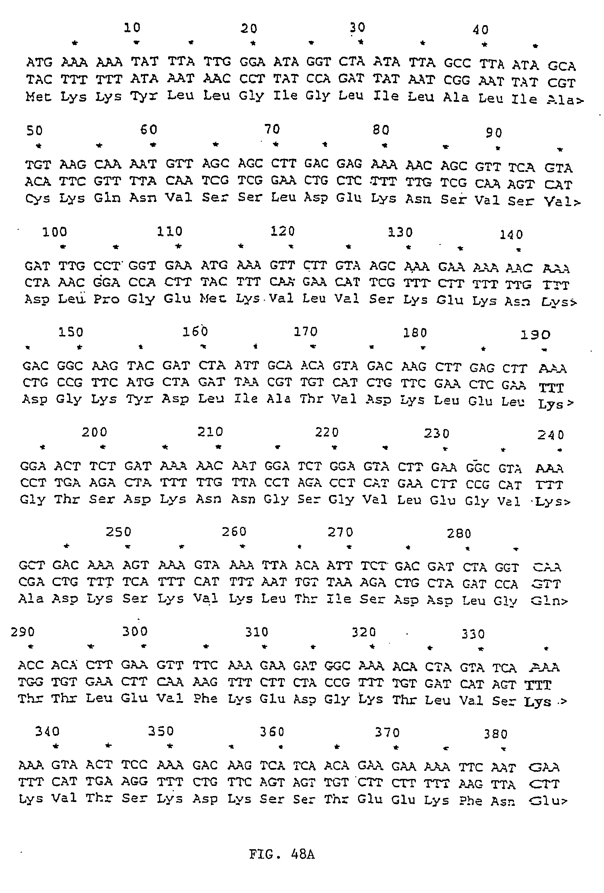

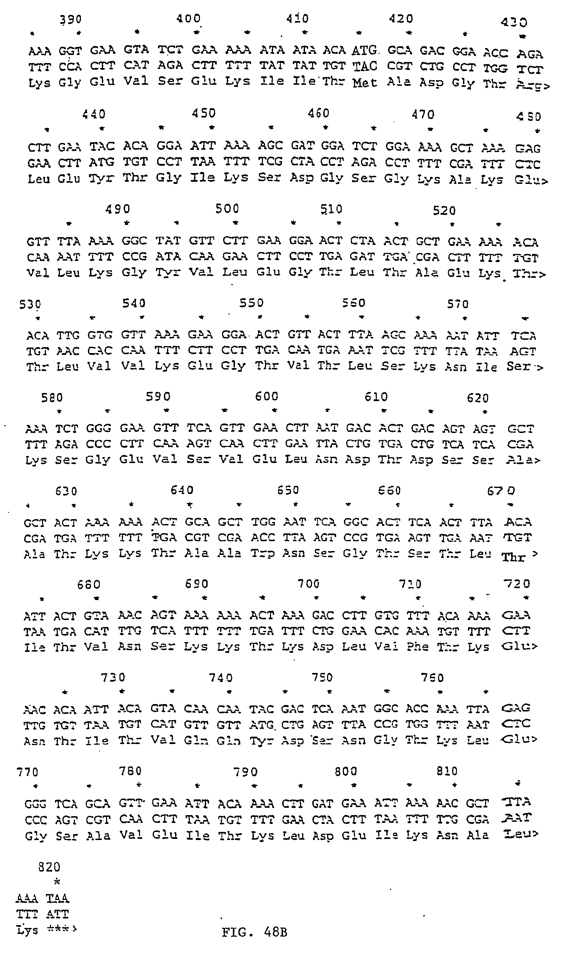

Figs. 48A and 48B depict the nucleic acid and encoded protein sequence of an R139M altered OspA (SEQ ID NO:95 and 96).

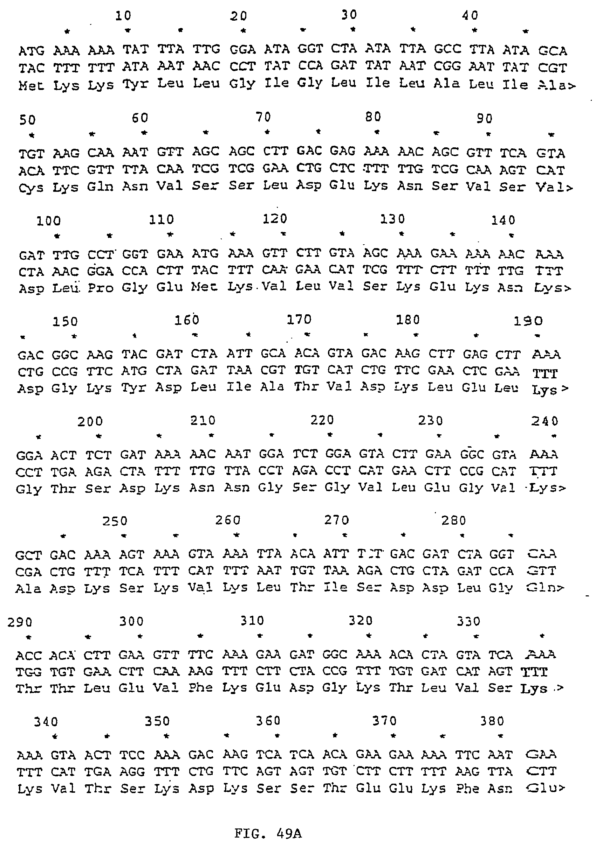

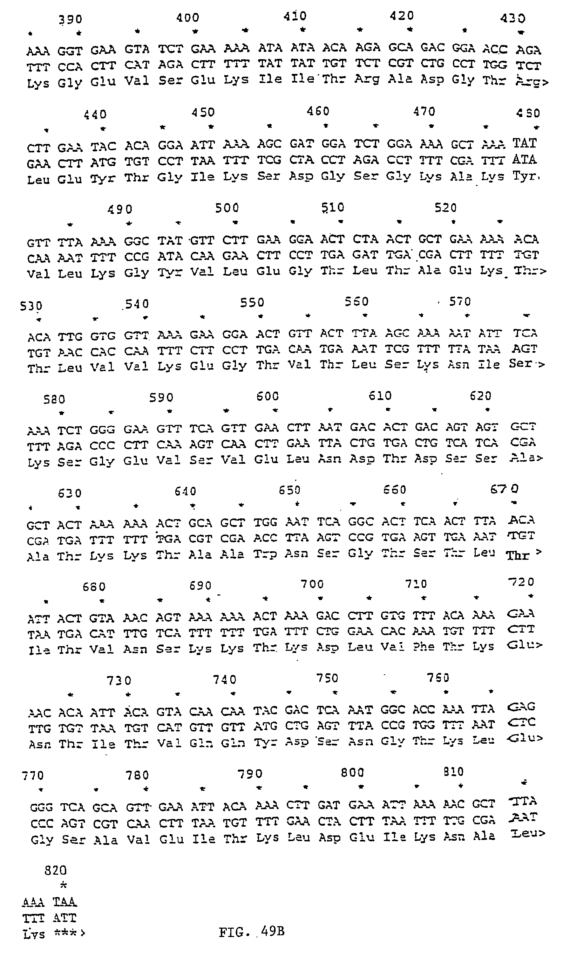

Figs. 49A and 49B depict the nucleic acid and encoded protein sequence of an E160Y altered OspA (SEQ ID NO:97 and 98).

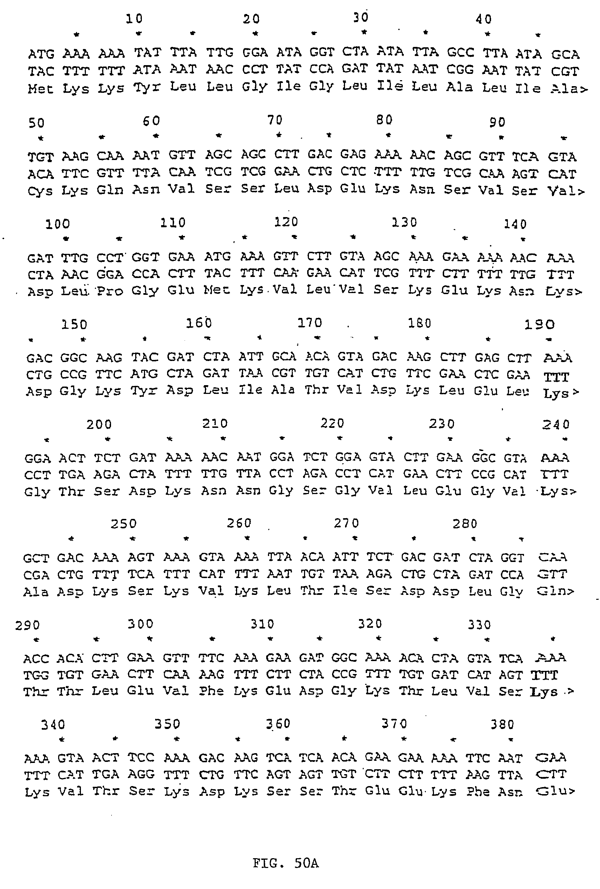

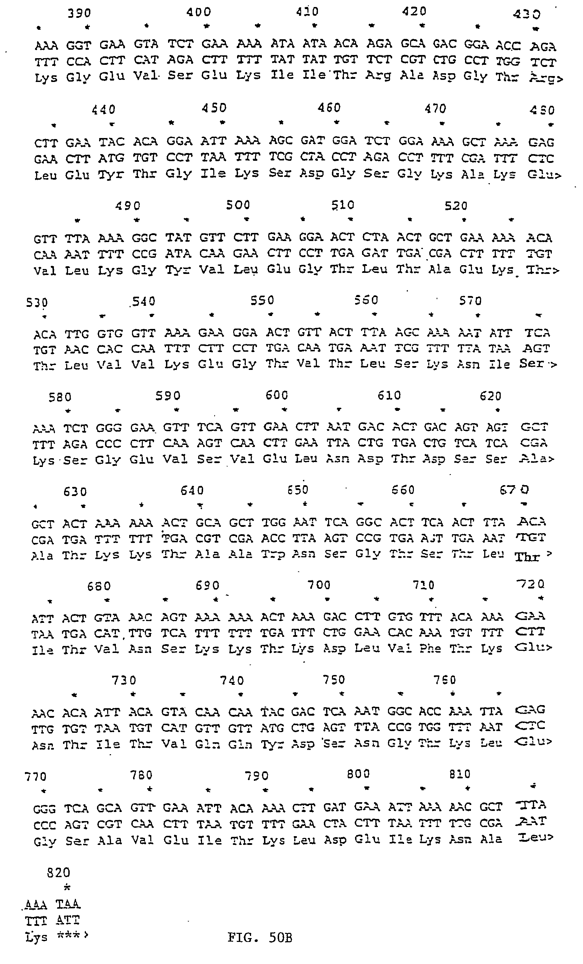

Figs. 50A and 50B depict the nucleic acid and encoded protein sequence of an R139M, E160Y altered OspA (SEQ ID NO:99 and 100).

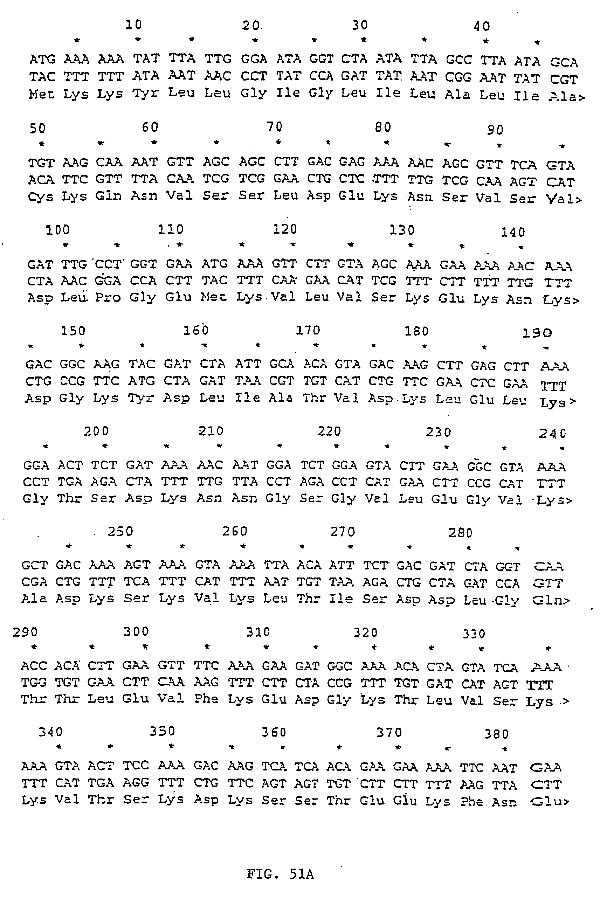

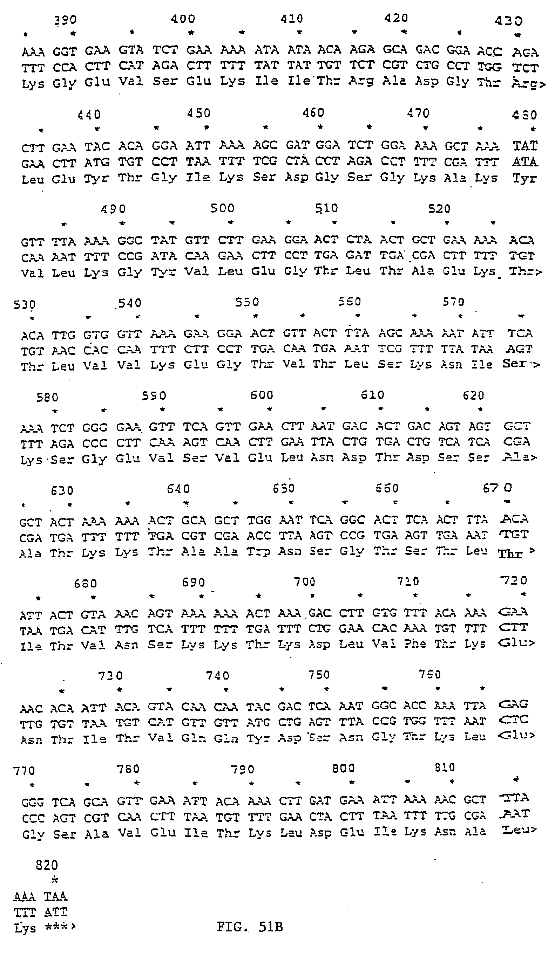

Figs. 51A and 51B depict the nucleic acid and encoded protein sequence of an E160Y, K189F altered OspA (SEQ ID NO:101 and 102).

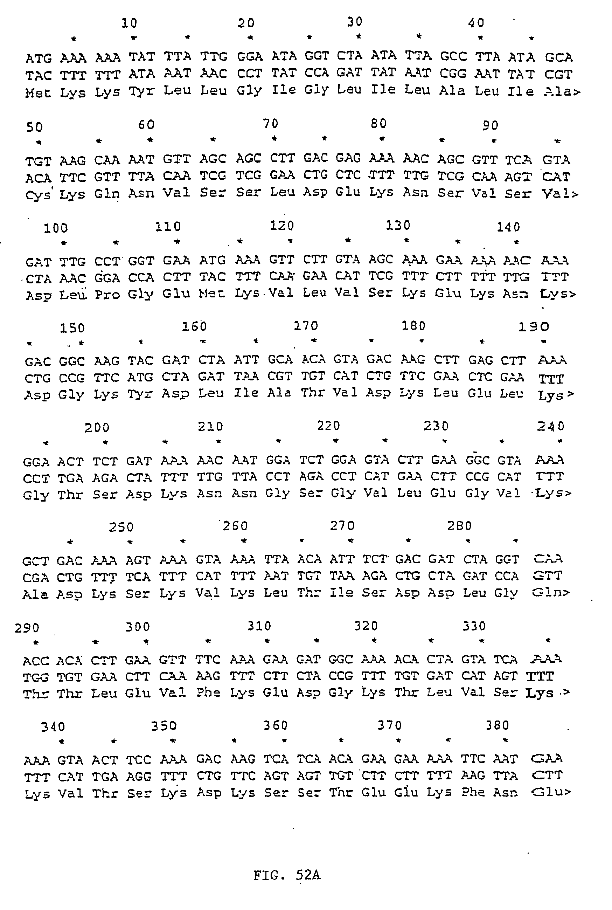

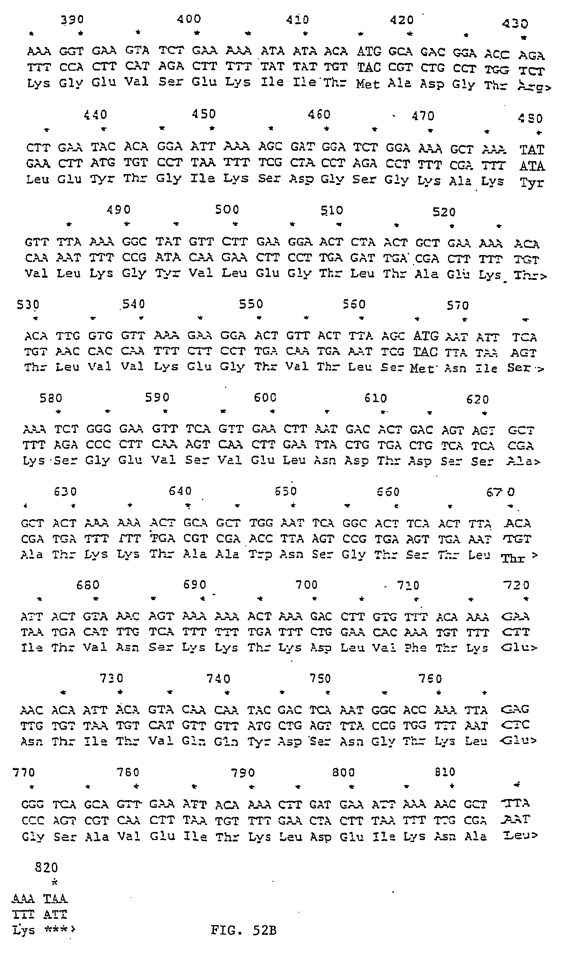

Figs. 52A and 52B depict the nucleic acid and encoded protein sequence of an R139M, E160Y, K189F altered OspA (SEQ ID NO:103 and 104).

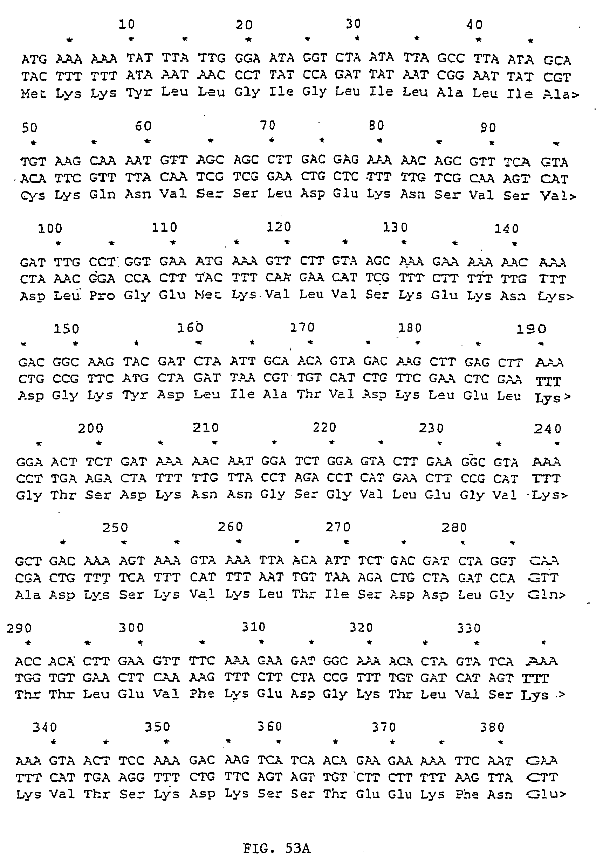

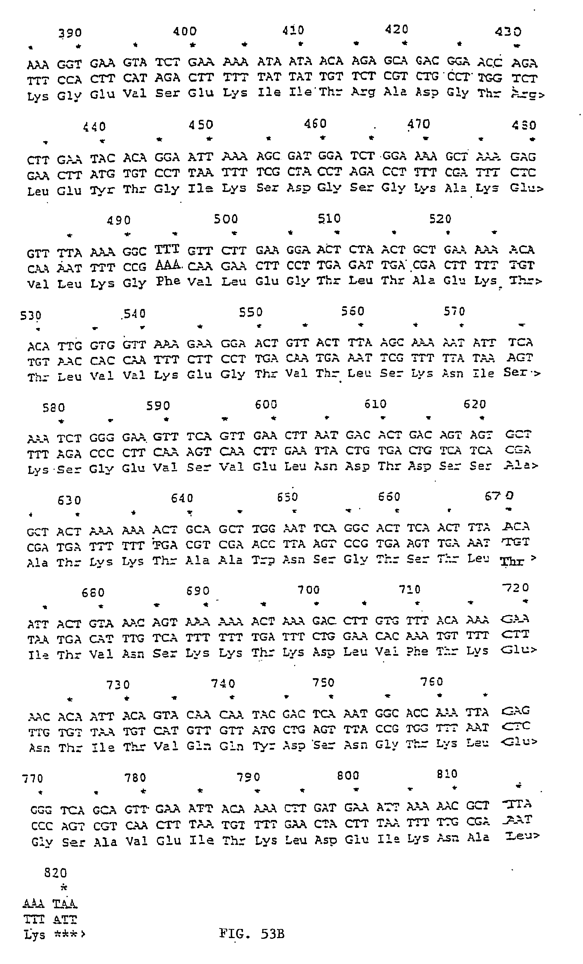

Figs. 53A and 53B depict the nucleic acid and encoded protein sequence of an Y165F altered OspA (SEQ ID NO:105 and 106).

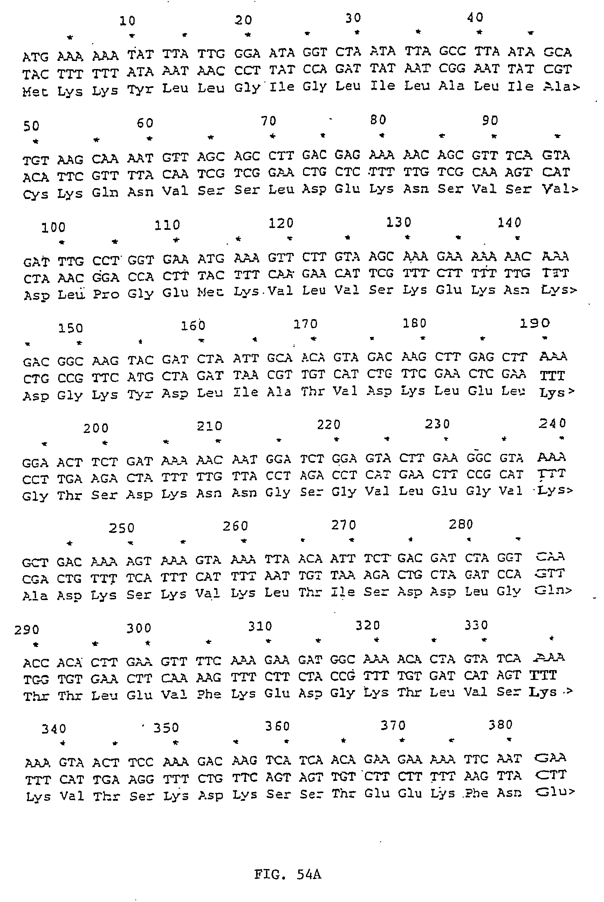

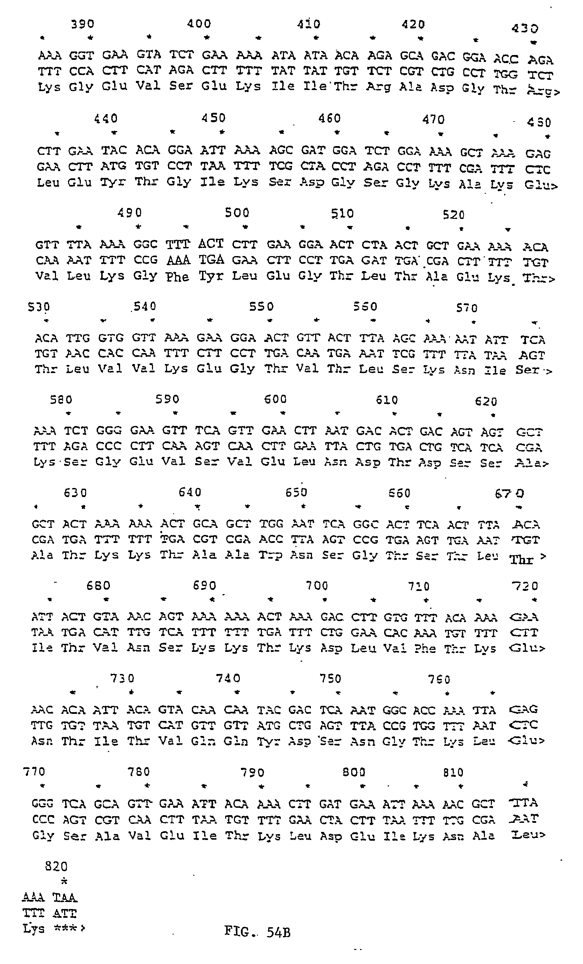

Figs. 54A and 54B depict the nucleic acid and encoded protein sequence of an Y165F, V166Y altered OspA (SEQ ID NO: 107 and 108).

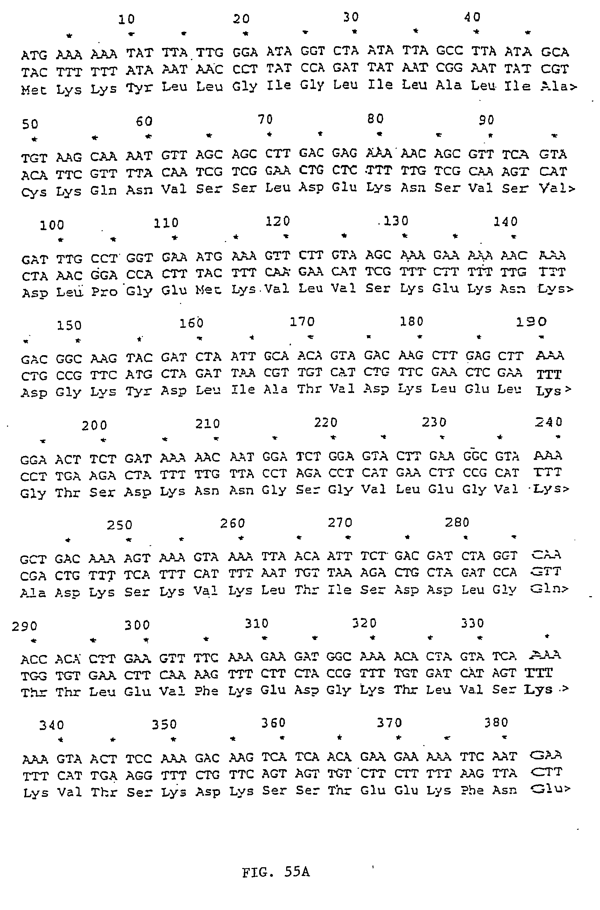

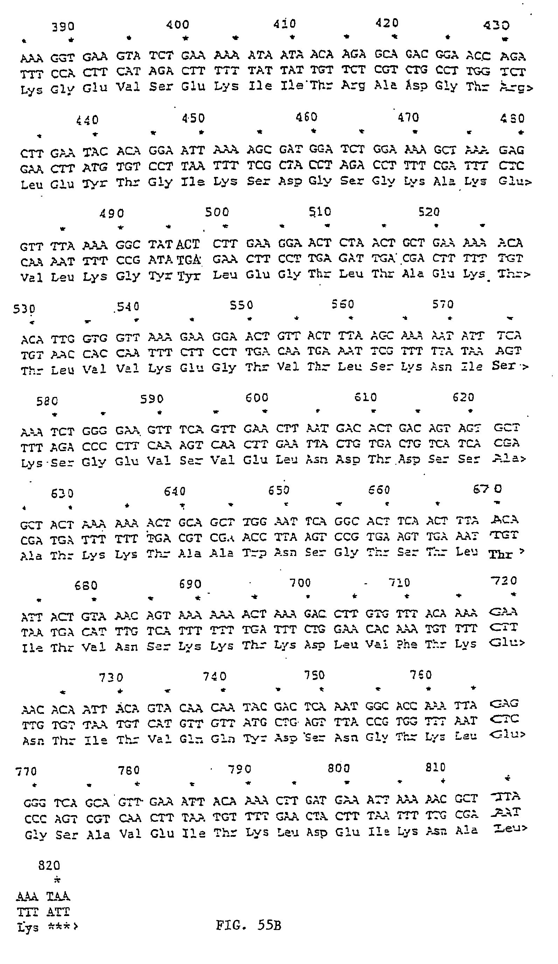

Figs. 55A and 55B depict the nucleic acid and encoded protein sequence of a V166Y altered OspA (SEQ ID NO:109 and 110).

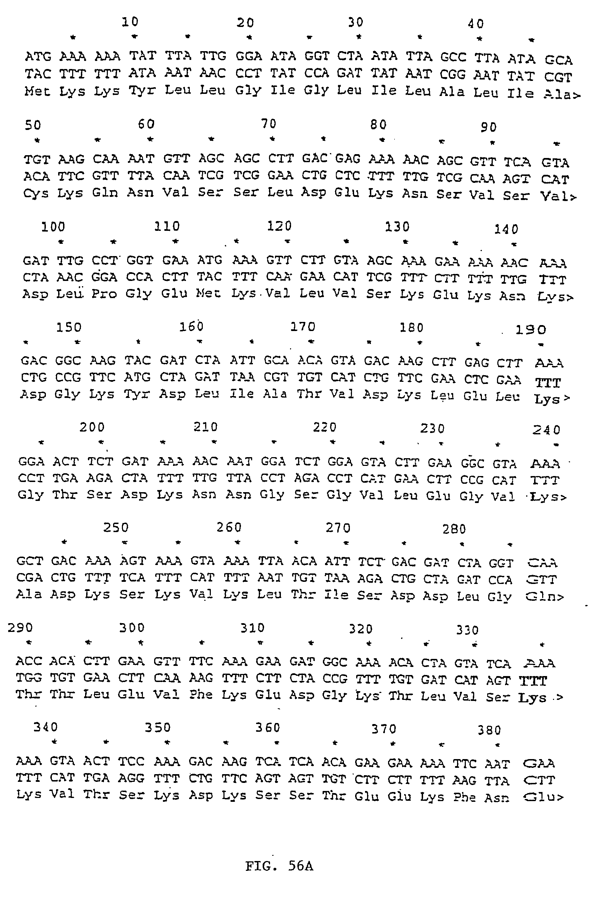

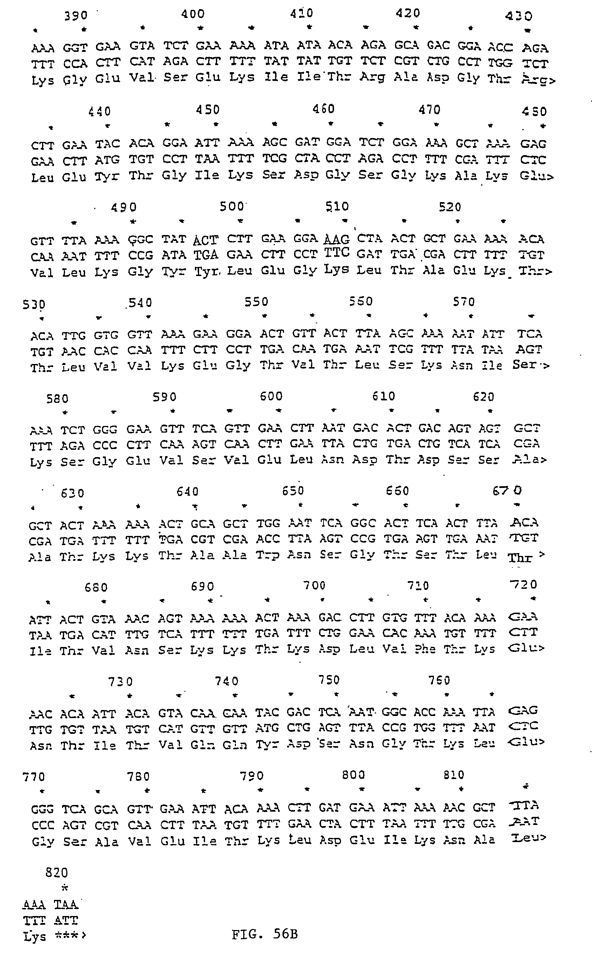

Figs. 56A and 56B depict the nucleic acid and encoded protein sequence of a V166Y, T170K altered OspA (SEQ ID NO: 111 and 112).

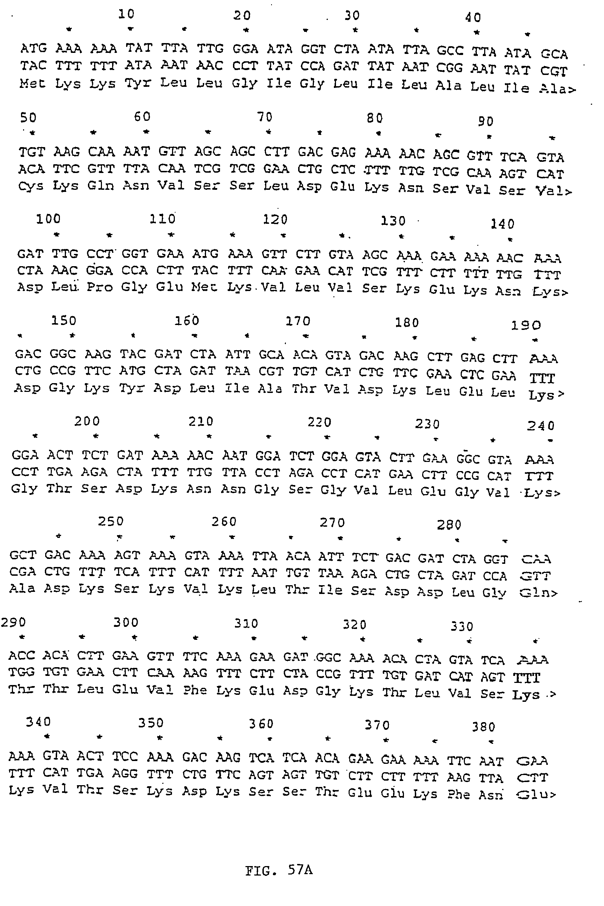

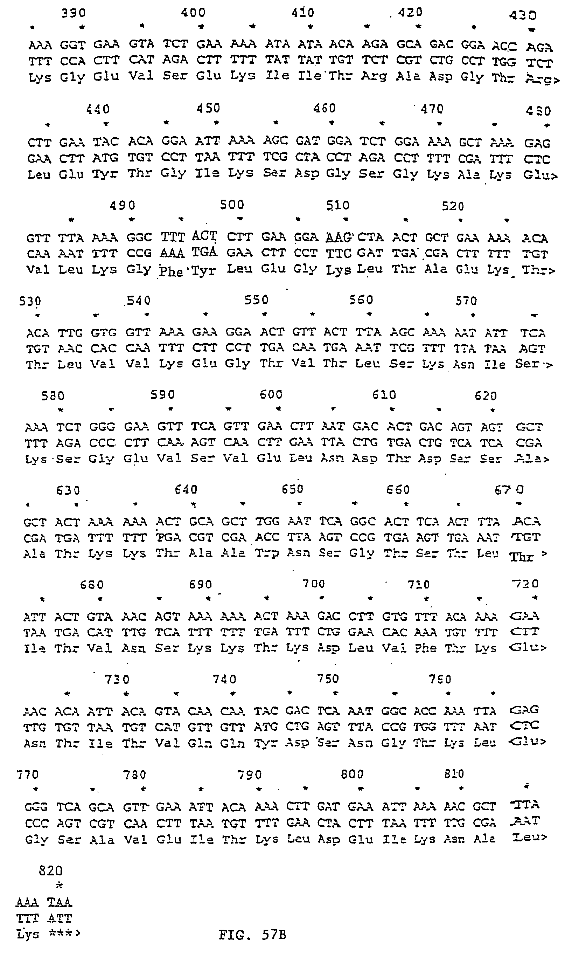

Figs. 57A and 57B depict the nucleic acid and encoded protein sequence of an Y165F, V166Y, T170K altered OspA (SEQ ID NO:113 and 114).

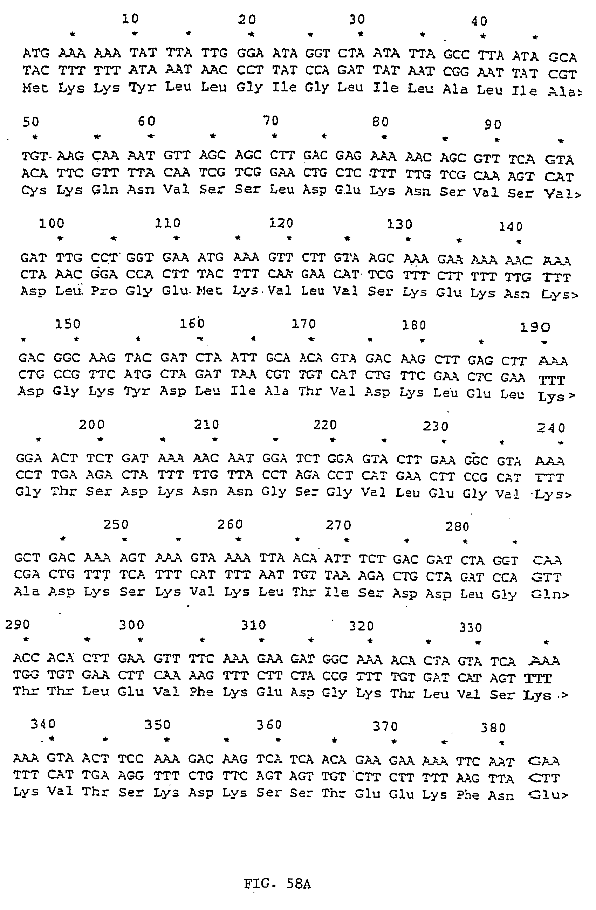

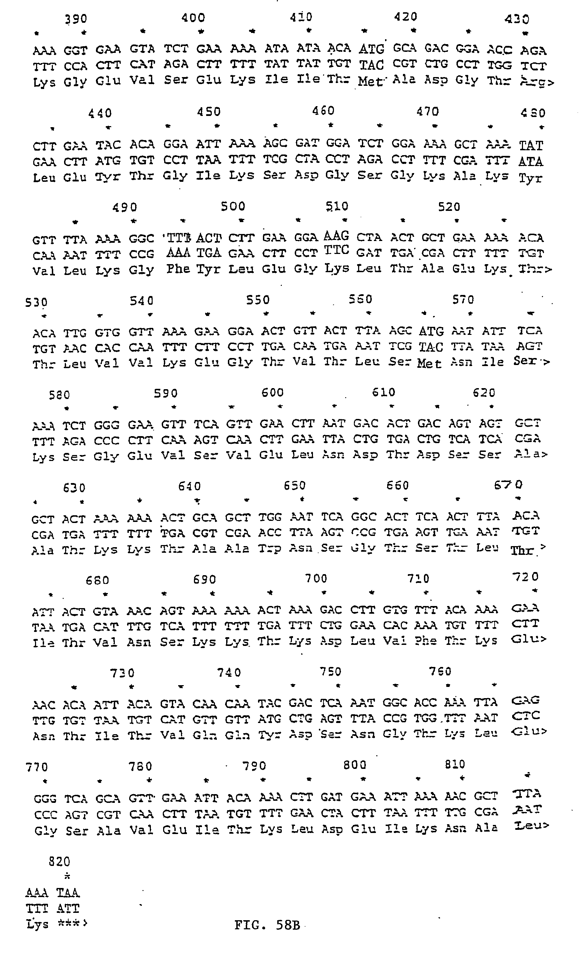

Figs. 58A and 58B depict the nucleic acid and encoded protein sequence of an R139M, E160Y, K189F, Y165F, V166Y, T170K altered OspA (SEQ ID NO:115 and 116).

### DETAILED DESCRIPTION OF THE INVENTION

The present invention is drawn to altered forms of OspA from *Borrelia burgdorferi* that have increased conformational stability while maintaining antigenicity, as indicated, for example, by the ability to be bound by the LA-2 monoclonal antibody. In some embodiments, the altered OspA polypeptides also have decreased cross-reactivity with hLFA-1. The altered OspA polypeptides can comprise all (with the exception of the alterations described herein) or a portion, such as the C-terminal portion, of a wild type OspA polypeptide. Applicants have found that some forms of the OspA protein, such as truncated versions of OspA, do not elicit a strong immunoprotective response when administered to an animal even when the OspA polypeptide has the immunoprotective LA-2 epitope sequence.

The structure for recombinant OspA has been determined to 1.95 Å resolution in a binary complex with the Fab fragment of the nonprotective mouse mAb184.1, which is reactive with the OspA-terminus (Li et al., Proc. Natl. Acad. Sci. U.S.A. 94:3584-3589 (1997)). The OspA polypeptide folds into 21 consecutive antiparallel β- strands followed by a C-terminal α-helix. The structure is conveniently described as two discrete folded domains, an N-terminal sandwich domain and a C-terminal barrel domain, connected by a long central β-sheet. One set of altered polypeptides described herein is designed to remove buried charges and/or salt bridges in the OspA C-terminal portion and replace them with residues that promote hydrophobic interactions.

Accordingly, in one embodiment, the altered OspA polypeptides of the present invention comprise altered OspA protein or polypeptides from *Borrelia burgdorferi* from about residue 139 to about residue 273, wherein the sequence includes at least one alteration selected from the group consisting of: residue 139 changed to methionine, residue 160 changed to tyrosine, residue 189 changed to methionine and combinations thereof. The numbering of the residues corresponds to the numbering of SEQ ID NO:7. In one embodiment, the altered OspA polypeptide has a tyrosine at residue position 160 and a methionine at residue position 189. These alterations have been found to stabilize the conformation of the immunoprotective LA-2 epitope in LA-2-containing OspA polypeptides. In another embodiment, the altered OspA polypeptide has a methionine at residue position 139, a tyrosine at residue position 160 and a methionine at residue position 189. In other embodiments, the altered OspA polypeptides have both increased conformational stability and reduced cross-reactivity to the hLFA-1 protein.

For the alterations at positions 139, 160 and 189, the altered OspA sequence can be from any Lyme borreliosis strain of *Borrelia burgdorferi,* such as strains from *Borrelia burgdorferi sensu stricto, Borrelia afzelii* and *Borrelia garinii.* Strains of *Borrelia burgdorferi* are well known to those of skill in the art. For example, strains of *Borrelia burgdorferi sensu stricto* include B31, strains of *Borrelia afzelii* include Pgau and Pko and strains of *Borrelia garinii* include K48.

In one embodiment, the OspA polypeptides of the present invention comprise an amino acid sequence of OspA protein from *Borrelia burgdorferi* from about residue 160 to about residue 170, wherein the sequence includes at least two alterations selected from the group consisting of: residue 165 changed to phenylalanine, residue 166 changed to threonine and residue 170 changed to lysine, wherein the numbering of the residues corresponds to the numbering of SEQ ID NO:7. In another embodiment, the OspA polypeptides of the present invention comprise an amino acid sequence of OspA protein from a *sensu stricto* strain of *Borrelia burgdorferi* from about residue 160 to about residue 170, wherein the sequence includes at least one alteration selected from the group consisting of: residue 165 changed to phenylalanine, residue 166 changed to threonine, residue 170 changed to lysine and combinations thereof. The numbering of the residues corresponds to the numbering of SEQ ID NO:7. In another embodiment, the altered OspA polypeptide has a phenylalanine at residue position 165 and a threonine at residue position 166. In still another embodiment, the altered OspA polypeptide has a phenylalanine at residue position 165, a threonine at residue position 166 and a lysine at residue position 170. In yet another embodiment, the altered OspA polypeptide includes all of the alterations described herein. In this embodiment, the altered OspA polypeptide has a methionine at residue position 139, a tyrosine at residue position 160, a methionine at residue position 189, a phenylalanine at residue position 165, a threonine at residue position 166 and a lysine at residue position 170.

This invention also pertains to polypeptides comprising SEQ ID NO:96, 98, 100, 102, 104, 106, 108, 110, 112, 114, or 116. The altered OspA polypeptides of the invention can be partially or substantially purified (e.g., purified to homogeneity), and/or substantially free of other proteins.

The present invention is also drawn to polynucleotides encoding the amino acid sequences described herein. As defined herein, the term "polynucleotide" refers to a nucleotide multimer or oligomer which is composed of deoxyribonucleotides or ribonucleotides, or a combination thereof, having from a few, e.g., 2-20, to many, e.g., 20 to several thousand or more, nucleotides. As such, polynucleotides include nucleic acids of any length and further encompass both naturally-occurring and synthetic oligonucleotides and polynucleotides.

The polynucleotides of the present invention include polynucleotides encoding OspA polypeptides from *Borrelia burgdorferi* from about residue 139 to about residue 189, wherein the sequence encodes at least one alteration selected from the group consisting of: codon 139 encoding methionine, codon 160 encoding tyrosine, codon 189 encoding methionine and combinations thereof. The numbering of the residues corresponds to the numbering of SEQ ID NO:7. As described above for the polypeptides, in the case of alterations at positions 139, 160 and 189, the polynucleotide encoding the altered OspA sequence can be from any Lyme borreliosis strain of *Borrelia burgdorferi.*

In another embodiment, the polynucleotide encodes an amino acid sequence of an OspA polypeptide from *Borrelia burgdorferi* from about residue 160 to about residue 170, wherein the sequence encodes at least one alteration selected from the group consisting of: codon 165 encoding phenylalanine, codon 166 encoding threonine, codon 170 encoding lysine and combinations thereof. The numbering of the residues corresponds to the numbering of SEQ ID NO:7.

The polynucleotides of the present invention include polynucleotides selected from the group consisting of: SEQ ID NO:95, 97, 99, 101, 103, 105, 107, 109,111,113and115.

The altered OspA polypeptides of the present invention can be derived from OspA molecules comprising fragments, derivatives, analogs, variants and mutants of the OspA protein (modified OspA) or can be fragmented, derivatized, or otherwise altered after having the alterations described herein inserted. These modified OspA molecules possess OspA antigenic activity.

The present invention is also drawn to a method of generating an altered *Borrelia burgdorferi* OspA polypeptide with increased conformational stability, as compared to the corresponding unaltered *Borrelia burgdorferi* OspA polypeptide. The method comprises selecting a polynucleotide encoding a *Borrelia burgdorferi* OspA polypeptide that includes residues 139, 160 and 189, wherein the numbering corresponds to the numbering of SEQ ID NO:7. The polynucleotide is altered such that residue 139 is methionine, residue 160 is tyrosine or residue 189 is methionine or combination thereof. The altered polynucleotide is expressed, thereby generating an altered *Borrelia burgdorferi* OspA polypeptide with increased conformational stability compared to the corresponding unaltered *Borrelia burgdorferi* OspA polypeptide. Methods of altering a polynucleotide are described below and in the Exemplification and are well known to those of skill in the art. Methods of expressing the altered polypeptides of the invention are also described below and in the Exemplification and are well known to those of skill in the art.

Residues 165-173 on β-strand 13 of OspA have been implicated in induction of Lyme-related arthritis (Gross D.M. et al., Science 281:703-706 (1998)). This region has homology to residues 332-340 of hLFA-1, suggesting that this protein has a cross-reactive T cell epitope (YVLEGTLTA-B31 and YVIEGTSKQ-hLFA-1, respectively). Although *B. burgdorferi sensu stricto* is generally believed to be more arthritogenic that other *Borrelia* strains, a recent study of *ospA* alleles in synovial fluid of patients with Lyme arthritis indicates that *B. garinii* and *B. afzelii* may also cause arthritis (Eiffert, L.F. et al., Scand. J. Infect. Dis. 30:265-268 (1998)).

One way to eliminate the cross-reactive sequence is to replace the β-13 region of OspA-B31 (YVLEGTLTA) with an analogous region from a strain that does not possess the same sequence, such as that from a *B. Afzelii* strain, e.g. Pgau or Pko (U.S. Patent Application entitled "Recombinant Constructs of *Borrelia burgdorferi"* by Luft *et al.,* filed on August 7, 2001, the entire teachings of which are incorporated herein by reference).

Accordingly, in another embodiment, the present invention is drawn to a method of generating an altered *Borrelia burgdorferi* OspA polypeptide with reduced cross-reactivity with the hLFA-1 molecule, as compared to the corresponding unaltered *Borrelia burgdorferi* OspA polypeptide. The method comprises selecting a polynucleotide encoding an OspA polypeptide from *Borrelia burgdorferi* that includes residues 165, 166 and 170, wherein the numbering corresponds to the numbering of SEQ ID NO:7. The polynucleotide is altered such that residue 165 is changed to phenylalanine, residue 166 is changed to tyrosine or residue 170 is changed to lysine or combination thereof. The altered polynucleotide is expressed, thereby generating an altered *Borrelia burgdorferi* OspA polypeptide with reduced cross-reactivity with the hLFA-1 molecule compared to the corresponding unaltered *Borrelia burgdorferi* OspA polypeptide. In another embodiment, an altered OspA polypeptide having reduced cross-reactivity to hLFA-1 while retaining the ability to be bound by LA-2 is generated. In that embodiment, for example, residue 130 is methionine, residue 160 is tyrosine, residue 165 is phenylalanine, residue 166 is tyrosine, residue 170 is lysine and residue 189 is methionine. Polynucleotides encoding *Borrelia burgdorferi* OspA polypeptides can be selected as described herein.

In one embodiment, the altered OspA polypeptide includes the minimal sequence that includes the positions of the alterations. For example, the altered polypeptide can comprise OspA from about residue 139 to about residue 189, wherein the numbering corresponds to SEQ ID NO:7. In another embodiment, the altered polypeptide can comprise OspA from about residue 165 to about residue 170, wherein the numbering corresponds to SEQ ID NO:7. The altered OspA polypeptides of the present invention also include larger fragments of OspA. For example, the altered OspA polypeptides include, but are not limited to, altered OspA polypeptides which comprise OspA from about residue 160 to about residue 170, OspA from about residue 150 to 180, OspA from about residue 131 to 273 or OspA from about residue 17 to 273. Methods of generating and expressing varying-sized fragments of OspA which incorporate one or more of the alterations described herein are described below and are well known to those of skill in the art.

As described herein, the OspA sequence used to generate the altered OspA polypeptide can itself be a chimeric OspA.polypeptide, having two or more segments derived from OspA proteins from different genospecies or strains of *Borrelia.* The size of the altered OspA polypeptide can vary depending on the method used to generate the altered polypeptide and/or the purpose for which it is generated, and such altered OspA chimeric polypeptides can include fragments of OspA. The altered polypeptide can be part of a larger polypeptide, including additional OspA sequences on the N-terminus, the C-terminus or both termini. A fragment of OspA protein can encompass polypeptides that are only a part of the full-length OspA protein. Such OspA fragments typically include at least one of the altered residues described herein and possess at least some of the antigenicity of wild type OspA. OspA fragments can be produced by amino and/or carboxyl terminal deletions, as well as internal deletions. Fragments can also be produced by enzymatic digestion. Such modified OspA molecules can be tested for antigenic activity as described herein or using methods known in the art.

In some embodiments, the altered OspA polypeptide can be part of a cocktail with one or more other proteins, such as other *Borrelia burgdorferi* polypeptides, including but not limited to, OspA, OspB, OspC, OspD, p93 and p41. In other embodiments, the altered OspA polypeptide can be part of a larger molecule, such as a chimeric polypeptide, for example, as described in U.S. Patent No. 6,248,562 and U.S. Patent Application entitled "Recombinant Constructs of *Borrelia burgdorferi",* by Luft *et al.,* filed on August 7, 2001. Such larger polypeptides can include amino acid sequences from other proteins including but not limited to, other *Borrelia burgdorferi* proteins and/or other proteins useful in generating fusion proteins for vaccine and/or immunodiagnostic methods. Additional components, for example, labels (a radioisotope, an epitope label (tag)(e.g., a hemagglutinin (HA) epitope, a hexahistidine tag), an affinity label (e.g., biotin, avidin), a spin label, an enzyme label, a fluorescent group, a chemiluminescent group) can be incorporated into the altered OspA polypeptides of the invention to assist in the isolation and/or purification of the polypeptide. For example, a hexahistidine tag would permit ready purification by nickel chromatography. These and other components can also be incorporated in the altered OspA polypeptides of the invention in order to extend the half life of the polypeptides. Methods of incorporating such components into the polypeptides of the invention are well known to those of skill in the art.

In one embodiment, the altered OspA polypeptide of the invention is a chimeric polypeptide. In a particular embodiment, the altered OspA polypeptide comprises the following: a) an amino acid sequence of a first OspA polypeptide from about residue 1 to about residue 164 from a first strain of *Borrelia burgdorferi;* b) an amino acid sequence of a second OspA polypeptide from about residue 165 to about residue 179 from a second strain of *Borrelia burgdorferi,* wherein said second strain is a different strain from said first strain; c) an amino acid sequence of a third OspA polypeptide from about residue 180 to about residue 216, from a third strain of *Borrelia burgdorferi,* wherein said third strain is a different strain from said second strain; d) an amino acid sequence of a fourth OspA polypeptide from about residue 217 to about residue 273 from a fourth strain *of Borrelia burgdorferi,* wherein said fourth strain is a different strain from said third strain; wherein the sequence includes at least one alteration selected from the group consisting of: residue 139 being methionine, residue 160 being tyrosine, residue 189 being methionine and combinations thereof, wherein the numbering corresponds to the numbering of SEQ ID NO:7.

Polypeptides described herein can be isolated from naturally-occurring sources, chemically synthesized or recombinantly produced. The altered OspA polypeptides of the present invention can be derived from naturally-occurring OspA molecules or from nucleic acids which encode such molecules. The OspA polypeptides of the present invention can comprise fragments, derivatives, analogs, variants and mutants of an OspA protein (modified OspA) and/or can be fragmented, derivatized or otherwise altered after having the alterations described herein inserted (also referred to as modified OspA). Such modified OspA molecules possess at least some OspA antigenic activity. According to the invention, the amino acid sequence of the altered OspA polypeptides of the invention can be that of a naturally-occurring protein or can comprise additional modifications. Such additional modifications include conservative and/or non-conservative amino acid substitutions, additions of one or more amino acids, and/or deletions of one or more amino acids. Such additional modifications should also preserve at least some activity of the encoded protein or polypeptide. For example, the further modified polypeptide or protein should have similar or improved conformational stability, similar or improved immunoprotective activity or reduced cross-reactivity to hLFA-1, as compared to the corresponding altered OspA polypeptide (i.e., the OspA polypeptide comprising one or more of the alterations described herein but not comprising the further modification(s)).

For example, the further modification(s) preferably preserve the three-dimensional configuration of an antibody binding site of the native protein such as the LA-2 binding site. The presence or absence of biological activity or activities can be determined by various functional assays as described herein or using methods that are known in the art, e.g., recognition using an ELISA assay, elicitation of an immune response (e.g., an immunoprotective response) in an animal. Appropriate amino acid alterations that fall within the scope of the invention can be made on the basis of several criteria, including hydrophobicity, basic or acidic character, charge, polarity, size, the presence or absence of a functional group (e.g., -SH or a glycosylation site), and aromatic character, provided that the resulting molecule has at least one of the alterations described herein and maintains increased conformational stability and/or reduced cross-reactivity to hLFA-1. Assignment of various amino acids to similar groups based on the properties above will be readily apparent to the skilled artisan; further appropriate amino acid changes can also be found in Bowie (Science, 247:1306-1310(1990)).

"Variants" and "mutants" of OspA can be produced using *in vitro* and/or in *vivo* techniques well-known to those of skill in the art, for example, site-specific mutagenesis, and oligonucleotide mutagenesis. Manipulations of the OspA polypeptide sequence can be made at the protein level as well. Chemical modifications can be carried out using known techniques including but not limited to, specific chemical cleavage using cyanogen bromide, trypsin and/or papain. OspA can also be structurally modified and/or denatured, for example, using heat. In general, mutations can be conservative or non-conservative amino acid substitutions, amino acid insertions or amino acid deletions.

For example, a nucleic acid (e.g., DNA) encoding a modified OspA polypeptide can be prepared by site-directed mutagenesis of the nucleic acid (e.g., DNA) that encodes a wild type OspA. Site-directed (site-specific) mutagenesis allows the production of OspA variants through the use of specific oligonucleotide sequences that encode the DNA sequence of the desired mutation (e.g., alteration, deletion, insertion), as well as a sufficient number of adjacent nucleotides, to provide a primer sequence of sufficient size and sequence complexity to form a stable duplex on both sides of the desired mutation. Typically, a primer of about 20 to 25 nucleotides in length is preferred, with about 5 to 10 complementary residues on both sides of the mutation of the sequence being altered. In general, the techniques of site-specific mutagenesis are well known in the art, as exemplified by publications such as Edelman et al., DNA, 2:183, 1983. For example, a site-specific mutagenesis technique can employ a phage vector that exists in both a single-stranded and double-stranded form. Typical vectors useful in site-directed mutagenesis include vectors such as the M13 phage, for example, as disclosed by Messing et al., Third Cleveland Symposium on Macromolecules and Recombinant DNA, A. Walton, ed., Elsevier, Amsterdam, 1981. This and other phage vectors are commercially available and their use is well known to those skilled in the art. A versatile and efficient procedure for the construction of oligonucleotide directed site-specific mutations in DNA fragments using M13-derived vectors was published by Zoller, M.J. and Smith, M., Nucleic Acids Res., 10:6487-6500, 1982. In addition, plasmid vectors that contain a single-stranded phage origin of replication can be employed to obtain single-stranded DNA (see for example, Veira et al., Meth Enzymol., 153:3, (1987)).

Alternatively, nucleotide substitutions can be introduced by synthesizing the appropriate DNA fragment *in vitro,* and amplifying it using PCR procedures known in the art.

In general, site-specific mutagenesis can be performed by first obtaining a single-stranded vector that includes within its sequence a DNA sequence that encodes the relevant protein. An oligonucleotide primer bearing the desired mutated sequence is prepared, generally synthetically, for example, by the method of Crea et al., Proc Natl Acad Sci USA., 75:5765, 1978. This primer can then be annealed with the single-stranded protein sequence-containing vector, and subjected to DNA polymerizing enzymes, e.g., *E. coli* polymerase I Klenow fragment, to complete the synthesis of the mutation-bearing strand. Thus, a heteroduplex is formed wherein one strand encodes the original non-mutated sequence and the second strand bears the desired mutation. This heteroduplex vector can then be used to transform appropriate host cells such as JM 101 cells, and clones can be selected that include recombinant vectors bearing the mutated sequence arrangement. Thereafter, the mutated region can be removed and placed in an appropriate expression vector for protein production.

The PCR technique can be used in creating amino acid sequence variants of OspA. When small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, one of the primers can be designed to overlap the position of the mutation and to contain the mutation; the sequence of the other primer is preferably identical to a stretch of sequence of the opposite strand of the plasmid, but this sequence can be located anywhere along the plasmid DNA. It is preferred, however, that the sequence of the second primer be located within 500 nucleotides from that of the first, such that in the end the entire amplified region of DNA bounded by the primers can be easily sequenced. PCR amplification using a primer pair like the one just described results in a population of DNA fragments that differ at the end position of the mutation specified by the primer.

The DNA fragments produced bearing the desired mutation can be used to replace the corresponding region in the plasmid that served as PCR template using standard DNA technology. Mutations at separate positions can be introduced simultaneously by either using a mutant second primer or performing a second PCR with different mutant primers and ligating the two resulting PCR fragments simultaneously to the vector fragment in a three (or more) part ligation.

An additional method for preparing variants, cassette mutagenesis, is based on the technique described by Wells et al., Gene, 34:315, 1985. The starting material can be the plasmid (or vector) comprising the OspA DNA to be mutated. The codon(s) within the OspA to be mutated are identified. There must be unique restriction endonuclease sites on each side of the identified mutation site(s). If such restriction sites do not exist, they can be generated using the above-described oligonucleotide-mediated mutagenesis method to introduce them at appropriate locations in the OspA DNA or they can be generated using PCR and the desired primers as described in the Exemplification. After the restriction sites have been introduced into the plasmid, the plasmid is cut at these sites to linearize it. A double stranded oligonucleotide encoding the sequence of the DNA between the restriction sites but containing the desired mutation(s) is synthesized using standard procedures. The two strands are synthesized separately and then hybridized together using standard techniques. This double-stranded oligonucleotide is referred to as the cassette. This cassette is designed to have 3' and 5' ends that are compatible with the ends of the linearized plasmid, such that it can be directly ligated to the plasmid. The plasmid now contains the mutated OspA DNA sequence, and can be subcloned and/or expressed to produce the modified OspA polypeptide or protein.

The OspA encoding nucleic acid molecules (e.g., polynucleotides) of the present invention have at least one of the alterations described herein and generally hybridize under high stringency hybridization conditions to a polynucleotide-encoding OspA nucleic acid or fragment thereof from a *sensu stricto* strain of *Borrelia burgdorferi,* e.g., SEQ ID NO:7. In one embodiment, the OspA-encoding nucleic acid molecules (e.g., polynucleotides) of the present invention hybridize under high stringency hybridization conditions to a polynucleotide encoding OspA or fragment thereof from *Borrelia afzelii,* e.g., SEQ ID NO:10. In another embodiment, the OspA-encoding nucleic acid molecules (e.g., polynucleotides) of the present invention hybridize under high stringency hybridization conditions to a polynucleotide encoding OspA or fragment thereof from *Borrelia garinii,* e.g., SEQ ID NO:8. Thus, the polynucleotides and polypeptides of the present invention include modified versions of OspA as described herein.

Appropriate selective stringency conditions are known to those skilled in the art or can be found in standard texts such as Current Protocols in Molecular Biology, John Wiley & Sons, N.Y. (1989), 6.3.1-6.3.6. For example, stringent hybridization conditions include a sodium ion concentration of no more than 1 M and a temperature of at least 25°C. In one embodiment, conditions of 5X SSPE (750 mM NaCl, 50 mM NaPhosphate, 5 mM EDTA, pH 7.4) and a temperature of 25-30°C, or equivalent conditions, are suitable for specific hybridization. Equivalent conditions can be determined by varying one or more of the parameters, as is known in the art, while maintaining a similar degree of identity or similarity between the target nucleic acid molecule and the primer or probe used. Hybridizable nucleic acid molecules are useful as probes and primers for diagnostic applications.

Accordingly, the invention pertains to nucleic acid molecules which have a substantial identity with the nucleic acid molecules encoding the altered OspA polypeptides described herein wherein the nucleic acid encodes for one or more of the alterations described herein; particularly preferred are nucleic acid molecules which have at least about 90%, more preferably at least about 95% and most preferably at least about 98% identity with nucleic acid molecules described herein, wherein the nucleic acid encodes for at least one of the alterations described herein. Sequence identity can be determined using publically or commercially available sequence alignment algorithms, using, for example, default parameters.

Thus, DNA molecules which comprise a sequence which is different from the naturally-occurring nucleic acid molecule but which, due to the degeneracy of the genetic code, encode the same protein or polypeptide are encompassed by the present invention. The invention also encompasses variations of the nucleic acid molecules of the invention, such as those encoding portions, analogues or derivatives of the encoded protein or polypeptide. Such variations can be naturally-occurring, such as in the case of allelic variation, or non-naturally-occurring, such as those induced by various mutagens and mutagenic processes, so long as the nucleic acid molecule encodes at least one of the alterations described herein and the encoded protein has either increased conformational stability and/or decreased cross-reactivity to hLFA-1 (as compared to the corresponding unaltered protein) that is conferred by the alterations described herein. Intended variations include, but are not limited to, addition, deletion and/or substitution of one or more nucleotides which can result in conservative or non-conservative amino acid changes, including additions and deletions. Preferably, such nucleotide or amino acid variations are silent; that is, they do not alter one or more characteristics or activity of the encoded altered OspA protein or polypeptide. As used herein, activities of the encoded protein or polypeptide include, but are not limited to, binding function, antigenic function and conformational stability.

The invention also provides expression vectors containing a nucleic acid sequence described herein, operably linked to at least one regulatory sequence. Many such vectors are commercially available, and other suitable vectors can be readily prepared by the skilled artisan. "Operably linked" is intended to mean that the nucleic acid molecule is linked to a regulatory sequence in a manner which allows expression of the nucleic acid sequence. Regulatory sequences are art-recognized and are selected to produce the encoded polypeptide or protein. Accordingly, the term "regulatory sequence" includes promoters, enhancers, and other expression control elements which are described in Goeddel, Gene Expression Technology: Methods in Enzymology 185, Academic Press, San Diego, CA (1990). For example, the native regulatory sequences or regulatory sequences native to the transformed host cell can be employed. It should be understood that the design of the expression vector may depend on such factors as the choice of the host cell to be transformed and/or the type of protein desired to be expressed. For instance, the polypeptides of the present invention can be produced by ligating the cloned gene, or a portion thereof, into a vector suitable for expression in either prokaryotic cells, eukaryotic cells or both (see, for example, Broach, et al., Experimental Manipulation of Gene Expression, ed. M. Inouye (Academic Press, 1983) p. 83; Molecular Cloning: A Laboratory Manual, 2nd Ed., ed. Sambrook et al., (Cold Spring Harbor Laboratory Press, 1989) Chapters 16 and 17). Typically, expression constructs will contain one or more selectable markers, including but not limited to, the gene that encodes dihydrofolate reductase and the genes that confer resistance to neomycin, tetracycline, ampicillin, chloramphenicol, kanamycin or streptomycin.

Prokaryotic and eukaryotic host cells transfected by the described vectors are also provided by this invention. For instance, cells which can be transfected with the vectors of the present invention include, but are not limited to, bacterial cells such as *E. coli* (e.g., *E. coli* K12, BL21, DH5α strains), *Streptomyces, Pseudomonas, Serratia marcescens* and *Salmonella typhimurium,* insect cells (baculovirus) including *Drosophila,* fungal cells, such as yeast cells, plant cells and mammalian cells, such as thymocytes, Chinese hamster ovary cells (CHO) and COS cells.

Thus, a nucleic acid molecule comprising, for example SEQ ID NO:6 with at least one of the specific alterations described herein, or a nucleic acid molecule which encodes, for example SEQ ID NO:7 with at least one of the specific alterations described herein, can be used to produce a recombinant form of the protein via microbial or eukaryotic cellular processes. Ligating the nucleic acid molecule (e.g., polynucleotide) into a gene construct, such as an expression vector, and transforming or transfecting into hosts, either eukaryotic (yeast, avian, insect, plant or mammalian) or prokaryotic (bacterial cells), are standard procedures used in producing other well known proteins. Similar procedures, or modifications thereof, can be employed to prepare recombinant proteins according to the present invention by microbial means or tissue-culture technology. Accordingly, the invention pertains to the production of encoded proteins or polypeptides by recombinant technology.

The proteins and polypeptides of the present invention can be isolated or purified (e.g., to homogeneity) from recombinant cell culture by a variety of processes. These include, but are not limited to, anion or cation exchange chromatography, ethanol precipitation, affinity chromatography and high performance liquid chromatography (HPLC). The particular method used will depend upon the properties of the polypeptide and the selection of the host cell; appropriate methods will be readily apparent to those skilled in the art.

The present invention also pertains to pharmaceutical compositions comprising polypeptides and other compounds described herein. For instance, a polypeptide or protein of the present invention can be formulated with a physiologically acceptable medium to prepare a pharmaceutical composition. The particular physiological medium may include, but is not limited to, water, buffered saline, polyols (e.g., glycerol, propylene glycol, liquid polyethylene glycol) and dextrose solutions. The optimum concentration of the active ingredient(s) in the chosen medium can be determined empirically, according to well known procedures, and will depend on the ultimate pharmaceutical formulation desired. Methods of introduction of exogenous polypeptides at the site of treatment include, but are not limited to, intradermal, intramuscular, intraperitoneal, intravenous, subcutaneous, oral and intranasal. Other suitable methods of introduction can also include gene therapy, rechargeable or biodegradable devices and slow release polymeric devices. The pharmaceutical compositions of this invention can also be administered as part of a combinatorial therapy with other agents.

The altered OspA proteins described herein can be produced so that they are highly soluble, hyper-produced in *E*. *coli,* and non-lipidated. In addition, the altered OspA proteins can be designed to begin or end in a suitable affinity tag (e.g., a His-tag) to facilitate purification. The recombinant proteins described herein have been constructed to maintain high levels of antigenicity and improved conformational stability.

The altered OspA proteins of the current invention are advantageous in that they retain at least some specific reactivity to monoclonal and/or polyclonal antibodies against wild-type *Borrelia* proteins, are immunogenic, and inhibit the growth or induce lysis of *Borrelia in vitro.* The proteins are particularly useful in immunodiagnostic assays. For example, proteins of the present invention can be used as reagents in assays to detect the presence of antibodies to native *Borrelia* in potentially infected individuals. These proteins can also be used as immunodiagnostic reagents, such as in dot blots, Western blots, enzyme-linked immunosorbent assays, or agglutination assays. The altered OspA proteins of the present invention can be produced by known techniques, such as by recombinant methodology, polymerase chain reaction, or mutagenesis.

Furthermore, the proteins of the current invention are useful as vaccine immunogens against *Borrelia* infection. One or more of the altered proteins can be combined with a physiologically acceptable carrier and administered to a vertebrate animal through standard methods (e.g., intravenously or intramuscularly, for example).

The altered forms of the OspA proteins described herein were bioengineered such that at least one immunoprotective domain of the protein was maintained. As described herein, antigenic refers to the ability of a compound to bind products of an immune response, such as antibodies, T-cell receptors or both. Such responses can be measured using standard antibody detection assays, such as ELISA or standard T-cell activation assays. In a preferred embodiment, the altered forms of OspA described herein elicit an immunoprotective response, for example, by eliciting antibodies that recognize the LA-2 epitope.

It is understood that the nucleic acids that encode the polypeptides that comprise the altered OspA protein can include extra nucleotides or fewer nucleotides in order to simplify the construction of the gene encoding the chimeric polypeptide, e.g., to allow for the use of convenient restriction endonuclease sites, or to allow the ligation of the gene fragments such that a contiguous coding region is created. Based on the guidance provided herein, one of ordinary skill in the art would readily be able to add or remove nucleotides from the termini of the gene fragments encoding the polypeptides of the OspA protein in order to generate the altered OspA proteins of the present invention with no experimentation or using only routine experimentation. The altered OspA polypeptides of the present invention can be lipidated or non-lipidated.

To test the antigenicity of the altered OspA polypeptides, mice can be immunized with OspA polypeptides or proteins containing the polypeptide sequences in aluminum hydroxide. Mice are then bled and tested for antibody responses against OspA derived from various strains of *Borrelia.* In additional experiments, these immunized mice can be challenged with ticks infected with *Borrelia burgdorferi* and transmission of infection can be assessed as described in the Exemplification which use OspA, OspC and OspC/OspA chimeric molecules. The results of such a tick challenge reveals whether the animal has developed a protective immune response. For example, an immunized animal that does not seroconvert in response to subsequent tick challenge has likely generated an immunoprotective response to the immunization.

The immunogenic compositions of the present invention can be used to immunize animals including humans. Immunization is understood to elicit specific immunogenic responses, preferably immunoprotective responses, as described above. As described herein, an immunogenic response includes responses that result in at least some level of immune response in the treated animal, where the animal was previously treated with a composition comprising at least one altered OspA polypeptide of the present invention.

Immunity, as described herein, is understood to mean the ability of the treated animal to resist infection, to resist systemic infection, or to overcome infection such as systemic infection more easily or more quickly when compared to non-immunized or non-treated individuals. Immunity can also include an improved ability of the treated individual to sustain an infection with reduced or no clinical symptoms of systemic infection. The individual may be treated with the altered OspA proteins of the present invention either proactively, e.g., once a year or, alternatively, after sustaining a tick bite.

In one embodiment, the altered OspA protein of the present invention, together with suitable excipients and/or adjuvants, is administered to an animal such that the animal develops an immune response to the OspA polypeptide of the composition. The pharmaceutical composition can also be administered with other components suitable for *in vitro* and/or *in vivo* use. These additional components include buffers, carrier proteins, adjuvants, preservatives and combinations thereof. In a preferred embodiment, the individual generates an immunoprotective response, for example, by generating antibodies that recognize the LA-2 epitope.

The present invention is also drawn to a physiological composition comprising an altered OspA protein. The composition is useful to administer to an animal in order to generate an immune response or in the diagnostic methods described herein.

For use as a vaccine, the composition of the present invention can include suitable adjuvants, well known in the art, to enhance immunogenicity, potency or half-life of the chimeric proteins in the treated animal. Adjuvants and their use are well known in the art (see for example PCT Publication WO 96/40290, the entire teachings of which are incorporated herein by reference). The composition can be prepared by known methods of preparing vaccines. For example, the altered OspA polypeptides described herein can be isolated and/or purified using known techniques, such as by size exclusion chromatography, ion exchange chromatography, affinity chromatography, preparative electrophoresis, selective precipitation or combinations thereof. The prepared proteins can be mixed with suitable other reagents as described above, wherein the protein is at a suitable concentration. The dosage of the protein will vary and depends upon the age, weight and/or physical condition of the animal to be treated. The optimal dosage can be determined by routine optimization techniques, using suitable animal models.

The composition to be used as a vaccine can be administered by any suitable technique. In one embodiment, administration is by injection, e.g., subcutaneous, intramuscular, intravenous, or intra peritoneal injection. In another embodiment, the composition is administered to mucosa, e.g., by exposing nasal mucosa to nose drops containing the proteins of chimeric proteins of the present invention. In another embodiment, the immunogenic composition is administered by oral administration. In another embodiment of the present invention the chimeric proteins are administered by DNA immunization using nucleic acids encoding an altered OspA polypeptide.

The present invention is also drawn to a diagnostic kit comprising the altered OspA polypeptides described herein. The kit also includes reagents for detecting antibody-antigen complexes that are formed between the OspA protein and antibodies that are present in an sample, e.g., a user-supplied host sample.

The present invention is also drawn to methods of detecting an immune response to Lyme Disease-causing *Borrelia* in a host sample. The method comprises contacting a host sample with an OspA altered protein, such that anti-OspA antibodies, if present in said sample, bind to said OspA protein. The quantity of antibodies that have bound said OspA protein are measured, thereby detecting an immune response to Lyme disease-causing *Borrelia.*

### EXEMPLIFICATION

### Example 1 Purification of Borrelia burgdorferi Outer Surface Protein A and Analysis of Antibody Binding Domains

This example details a method for the purification of large amounts of native outer surface protein A (OspA) to homogeneity, and describes mapping of the antigenic specificities of several anti-OspA MAbs. OspA was purified to homogeneity by exploiting its resistance to trypsin digestion. Intrinsic labeling with ¹⁴C-palmitic acid confirmed that OspA was lipidated, and partial digestion established lipidation at the amino-terminal cysteine of the molecule.

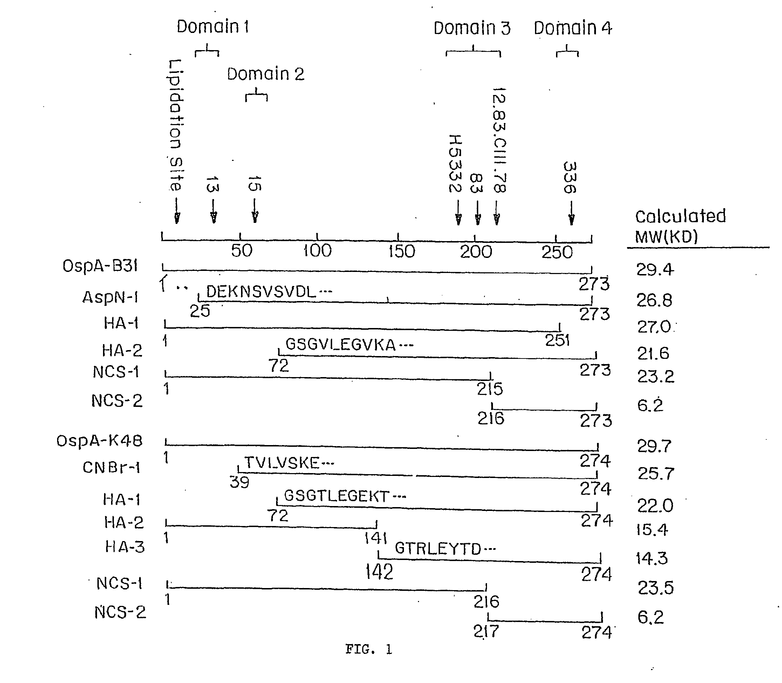

The reactivity of seven anti-OspA murine monoclonal antibodies to nine different *Borrelia* isolates was ascertained by Western blot analysis. The reactivity of the altered OspA polypeptides described herein 15 tested using similar methods. Intact, lipidated or non-lipidated OspA and altered OspA can also be tested using similar methods. Purified OspA was fragmented by enzymatic or chemical cleavage, and the monoclonal antibodies were able to define four distinct immunogenic domains (see Fig. 1). Domain 3, which included residues 190-220 of OspA, was reactive with protective antibodies known to agglutinate the organism *in vitro,* and included distinct specificities, some of which were not restricted to a genotype of *B. burgdorferi.*

### A. Purification of Native OspA

Detergent solubilization of *B. burgdorferi* strips the outer surface proteins and yields partially-purified preparations containing both OspA and outer surface protein B (OspB) (Barbour, A.G. et al., Infect. Immun., 52(5):549-554 (1986); Coleman, J.L. et al., J Infect. Dis., 155 (4):756-765 (1987); Cunningham, T.M. et al., Ann. NY Acad. Sci., 539:376-378 (1988); Brandt, M.E. et al., Infect. Immun., 58: 983-991 (1990); Sambri, V. and R. Cevenini, Microbiol., 14:307-314 (1991)). Although both OspA and OspB are sensitive to proteinase K digestion, in contrast to OspB, OspA is resistant to cleavage by trypsin (Dunn, J. et al., Prot. Exp. Purif., 1:159-168 (1990); Barbour, A.G. et al., Infect. Immun., 45:94-100 (1984)). The relative insensitivity to trypsin is surprising in view of the fact that OspA has a high (16% for B31) lysine content, and may relate to the relative configuration of OspA and B in the outer membrane.

### Intrinsic Radiolabeling of Borrelia

Labeling for lipoproteins was performed as described by Brandt *et al.* (Brandt et al., Infect. Immun., 58:983-991 (1990)). ¹⁴C-palmitic acid (ICN, Irvine, California) was added to the BSK II media to a final concentration of 0.5 µCi per milliliter (ml). Organisms were cultured at 34°C in this medium until a density of 10⁸ cells per ml was achieved.

### Purification of OspA Protein from Borrelia Strain B31

*Borrelia burgdorferi,* either¹⁴C-palmitic acid-labeled or unlabeled, were harvested and washed as described (Brandt, M.E. et al., Infect. Immun., 58:983-991 (1990)). Whole organisms were trypsinized according to the protocol of Barbour et al., (Infect. Immun., 45:94-100 (1984)) with some modifications. The pellet was suspended in phosphate buffered saline (PBS, 10mM, pH 7.2), containing 0.8% tosyl-L-phenylalanine chloromethyl ketone (TPCK)-treated trypsin (Sigma, St. Louis, Missouri), the latter at a ratio of 1 µg per 10⁸ cells. Reaction was carried out at 25°C for 1 hour, following which the cells were centrifuged. The pellet was washed in PBS with 100 µg/ml phenylmethylsulfonyl fluoride (PMSF). Triton X-114 partitioning of the pellet was carried out as described by Brandt *et al.,* (Brandt et al., Infect. Immun., 58:983-991 (1990)). Following trypsin treatment, cells were resuspended in ice-cold 2% (v/v) Triton X-114 in PBS at 10⁹ cells per ml. The suspension was rotated overnight at 4°C, and the insoluble fraction removed as a pellet after centrifugation at 10,000 X g for 15 minutes at 4°C. The supernatant (soluble fraction) was incubated at 37°C for 15 minutes and centrifuged at room temperature at 1000 X g for 15 minutes to separate the aqueous and detergent phases. The aqueous phase was decanted, and ice cold PBS added to the lower Triton phase, mixed, warmed to 37°C, and again centrifuged at 1000 X g for 15 minutes. Washing was repeated twice more. Finally, detergent was removed from the preparation using a spin column of Bio-beads SM2 (BioRad, Melville, New York) as described (Holloway, P.W., Anal. Biochem., 53:304-308 (1973)).

Ion exchange chromatography was carried out as described by Dunn *et al.,* (Dunn et al., Prot. Exp. Purif., 1:159-168 (1990)) with minor modifications. Crude OspA was dissolved in buffer A (1% Triton X-100, 10mM phosphate buffer (pH 5.0)) and loaded onto a SP Sepharose resin (Pharmacia, Piscataway, New Jersey), pre-equilibrated with buffer A at 25°C. After washing the column with 10 bed-volumes of buffer A, the bound OspA was eluted with buffer B (1% Triton X-100, 10mM phosphate buffer (pH 8.0)). OspA fractions were detected by protein assay using the BCA method (Pierce, Rockford, Illinois), or as radioactivity when intrinsically labeled material was fractionated. Triton X-100 was removed using a spin column of Bio-beads SM2.

This method purifies OspA from an outer surface membrane preparation. In the absence of trypsin-treatment, OspA and B were the major components of the soluble fraction obtained after Triton partitioning of strain B31. In contrast, when Triton extraction was carried out after trypsin-treatment, the OspB band is not seen. Further purification of OspA-B31 on a SP Sepharose column resulted in a single band by SDS-PAGE. The yield following removal of detergent was approximately 2 mg per liter of culture. This method of purification of OspA, as described herein for strain B31, can be used for other isolates of *Borrelia* as well. For strains such as strain K48, which lack OspB, trypsin treatment can be omitted.

### Lipidation site of OspA-B31

¹⁴C-palmitic acid labeled OspA from strain B31 was purified as described above and partially digested with endoproteinase Asp-N. Following digestion, a new band of lower molecular weight was apparent by SDS-PAGE, found by direct amino-terminal sequencing to begin at Asp₂₅. This band had no trace of radioactivity by autoradiography. OspA and B contain a signal sequence (L-X-Y-C) similar to the consensus described for lipoproteins of *E. coli,* and it has been predicted that the lipidation site of OspA and B should be the amino-terminal cysteine (Brandt, M.E. et al., Infect. Immun, 58:983-991 (1990)). The results presented herein support this prediction.

### B. Comparison of OspA Antibody Binding Regions in Nine Strains of Borrelia burgdorferi

The availability of the amino acid sequenced for OspA from a number of different isolates, combined with peptide mapping and Western blot analysis, permitted the identification of the antigenic domains recognized by monoclonal antibodies (MAbs) and allowed inference of the key amino acid residues responsible for specific antibody reactivity.

### Strains of Borrelia burgdorferi

Nine strains of *Borrelia,* including seven European strains and two North American strains, were used in this study of antibody binding domains of several proteins. Information concerning the strains is summarized in Table I, below.

**Table I. Representative Borrelia Strains**

| Strain | Location and Source | Reference for Strain |
|---|---|---|
| K48 | Czechoslovakia, *Ixodes ricinus* | none |
| PGau | Germany, human ACA | Wilske, B. et al., J. Clin. Microbiol. 32: 340-350 (1993) |
| DK29 | Denmark, human EM | Wilske, B. *et al.* |
| Pko | Germany, human EM | Wilske, B. *et al.* |
| PTrob | Germany, human skin | Wilske, B. *et al.* |
| Ip3 | Khabarovsk, Russia, *I*. *persulcatus* | Asbrink, E. et al., Acta Derm. Venereol., 64:506-512 (1984) |
| Ip90 | Khabarovsk, Russia, *I. persulcatus* | Asbrink, E. *et al.* |
| 25015 5 *persulcatus* | Millbrook, NY, *I.* | Barbour, A.G. et al., Curr. Microbiol., 8:123-126 (1983) |
| B31 | Shelter Island, NY, *I. scapularis* | Luft, B.J. et al., Infect. Immun, 60: 4309-4321 (1992); ATCC 35210 |
| PKal | Germany, human CSF | Wilske, B. *et al.* |
| ZS7 | Freiburg, Germany, *I. ricinus* | Wallich, R. et al., Nucl. Acids Res, 17: 8864 (1989) |
| N40 | Westchester Co., NY | Fikrig, E. et al., Science, 250:553-556 (1990) |
| PHei | Germany, human CSF | Wilske, B. *et al.* |
| ACAI | Sweden, human ACA | Luft, B. J. et al., FEMS Microbiol. Lett. 93:73-68 (1992) |
| PBo | Germany, human CSF | Wilske, B. *et al.* |

| | | |
|---|---|---|
| ACA = patient with acrodermatitis chronica atrophicans; EM = patient with erythema migrans; CSF = cerebrospinal fluid of patient with Lyme disease | | |

Strains K48, PGau and DK29 were supplied by R. Johnson, University of Minnesota; Pko and pTrob were provided by B. Wilske and V. Preac-Mursic of the Pettenkhofer Institute, Munich, Germany; and Ip3 and Ip90 were supplied by L. Mayer of the Center for Disease Control, Atlanta, Georgia. The North American strains included strain 25015, provided by J. Anderson of the Connecticut Department of Agriculture; and strain B31 (ATCC 35210).

### Monoclonal Antibodies

Seven monoclonal antibodies (MAbs) were utilized in this study. Five of the MAbs (12, 13, 15, 83 and 336) were produced from hybridomas cloned and subcloned as previously described (Schubach, W.H., et al., Infect. Immun., 59(6):1911-1915 (1991)). MAb H5332 (Barbour, A.G. et al., Infect. Immun., 41: 795-804 (1983)) was a gift from Drs. Alan Barbour, University of Texas, and MAb CIII.78 (Sears, J.E. et al., J. Immunol., 147(6):1995-2000 (1991)) was a gift from Richard A. Flavell, Yale University. MAbs 12 and 15 were raised against whole sonicated B3; MAb 336 was produced against whole PGau; and MAbs 13 and 83 were raised to a truncated form of OspA cloned from the K48 strain and expressed in *E. coli* using the T7 RNA polymerase system (McGrath, B.C. et al., Vaccines, Cold Spring Harbor Laboratory Press, Plainview, New York, pp. 365-370 (1993)). All MAbs were typed as being Immunoglobulin G (IgG).

### Methods of Protein Cleavage, Western Blotting and Amino-Terminal Sequencing

Prediction of the various cleavage sites was achieved by knowledge of the primary amino acid sequence derived from the full nucleotide sequences of OspA, many of which are currently available (see Table II, below). Cleavage sites can also be predicted based on the peptide sequence of OspA, which can be determined by standard techniques after isolation and purification of OspA by the method described above. Cleavage of several OspA isolates was conducted to determine the localization of monoclonal antibody binding of the proteins.

Hydroxylamine-HCl (HA), N-chlorosuccinimide (NCS), and cyanogen bromide cleavage of OspA followed the methods described by Bornstein (Biochem. 9 (12):2408-2421 (1970)), Shechter et al., (Biochem., 15 (23):5071-5075 (1976)), and Gross (in Hirs, C.H.W. (ed): Methods in Enzymology, (N.Y. Acad. Press), 11:238-255 (1967)) respectively. Protease cleavage by endoproteinase, Asp-N (Boehringer Mannheim, Indianapolis, Indiana), was performed as described by Cleveland D.W. et al., (J. Biol. Chem., 252:1102-1106 (1977)). Ten micrograms of OspA were used for each reaction. The ratio of enzyme to OspA was approximately 1 to 10 (w/w).

Proteins and peptides generated by cleavage were separated by SDS-polyacrylamide gel electrophoresis (SDS-PAGE) (Laemmli, U.K., Nature (London) 227:680-685 (1970)), and electroblotted onto immobilon Polyvinylidine Difluoride (PVDF) membranes (Ploskal, M.G. et al., Biotechniques, 4:272-283 (1986)). They were detected by amido black staining or by immunostaining with murine MAbs, followed by alkaline phosphatase-conjugated goat anti-mouse IgG. Specific binding was detected using a 5-bromo-4-chloro-3-indolylphosphate (BCIP)/nitroblue tetrazolium (NBT) developer system (KPL Inc., Gathersburg, Maryland).

In addition, amino-terminal amino acid sequence analysis was carried out on several cleavage products, as described by Luft et al., (Infect. Immun., 57:3637-3645 (1989)). Amido black stained bands were excised from PVDF blots and sequenced by Edman degradation using a Biosystems model 475A sequenator with model 120A PTH analyzer and model 900A control/data analyzer.

### Cleavage Products of Outer Surface Protein A Isolates

Purified OspA-B31, labeled with ¹⁴C-palmitic acid, was fragmented with hydroxylamine-HCl (HA) into two peptides, designated HA1 and HA2 (data not shown). The HA1 band migrated at 27 kd and retained its radioactivity, indicating that the peptide included the lipidation site at the N-terminus of the molecule (data not shown). From the predicted cleavage point, HA1 should correspond to residues 1 to 251 of OspA-B31. HA2 had a MW of 21.6 kd by SDS-PAGE, with amino-terminal sequence analysis showing it to begin at Gly72, i.e. residues 72 to 273 of OspA-B31. By contrast, HA cleaved OspA-K48 into three peptides, designated HA1, HA2, and HA3 with apparent MWs of 22 kd, 16 kd and 12 kd, respectively. Amino-terminal sequencing showed HA1 to start at Gly72, and HA3 at Gly142. HA2 was found to have a blocked amino-terminus, as was observed for the full-length OspA protein. HA1, 2 and 3 of OspA-K48 were predicted to be residues 72-274, 1 to 141 and 142 to 274, respectively.

N-Chlorosuccinimide (NCS) cleaves tryptophan (W), which is at residue 216 of OspA-B31 or residue 217 of OspA-K48 (data not shown). NCS cleaved OspA-B31 into 2 fragments, NCS1, with MW of 23 kd, residues 1-216 of the protein, and NCS2 with a MW of 6.2 kd, residues 217 to 273 (data not shown). Similarly, K48 OspA was divided into 2 pieces, NCS1 residues 1-217, and NCS2 residues 218 to 274 (data not shown).

Cleavage of OspA by cyanogen bromide (CNBr) occurs at the carboxy side of methionine, residue 39. The major fragment, CNBr1, has a MW of 25.7 kd, residues 39-274 by amino-terminal amino acid sequence analysis (data not shown). CNBr2 (about 4 kd) could not be visualized by amido black staining; instead, lightly stained bands of about 20 kd MW were seen. These bands reacted with anti-OspA MAbs, and most likely were degradation products due to cleavage by formic acid.

### Determination of Antibody Binding Domains for Anti-OspA Monoclonal Antibodies

The cleavage products of OspA-B31 and OspA-K48 were analyzed by Western blot to assess their ability to bind to the six different MAbs. Preliminary Western blot analysis of the cleavage products demonstrated that strains K48 and DK29 have similar patterns of reactivity, as do IP3, PGau and Pko. The OspA of strain PTrob was immunologically distinct from the others, being recognized only by MAb 336. MAb 12 recognized only the two North American strains, B31 and 25015. When the isolates were separated into genogroups, it was remarkable that all the MAbs, except MAb 12, crossed over to react with multiple genogroups.

MAb12, specific for OspA-B31, bound to both HA1 and HA2 ofOspA-B31. However, cleavage of OspA-B31 by NCS at residue Trp216 created fragments which did not react with MAb12, suggesting that the relevant domain is near or is structurally dependent upon the integrity of this residue (data not shown). MAb 13 bound only to OspA-K48, and to peptides containing the amino-terminus of that molecule (e.g. HA2; NCS1). It did not bind to CNBr1 residues 39 to 274. Thus the domain recognized by MAb13 is in the amino-terminal end of OspA-K48, near Met38.

MAb15 reacts with the OspA of both the B31 and K48 strains, and to peptides containing the N-terminus of OspA, such as HA1 of OspA-B31 and NCS1, but not to peptides HA2 of OspA-B31 and HA1 of OspA-K48 (data not shown). Both peptides include residue 72 to the C-terminus of the molecules. MAb15 bound to CNBr1 of OspA-K48, indicating the domain for this antibody to be residues 39 to 72, specifically near Gly72 (data not shown).

MAb83 binds to OspA-K48, and to peptides containing the C-terminal portion of the molecule, such as HA1. They do not bind to HA2 of OspA-K48, most likely because the C-terminus of HA2 of OspA-K48 ends at 141. Similar to MAb 12 and OspA-B31, binding of MAbs 83 and CIII.78 is eliminated by cleavage of OspA at the tryptophan residue. Thus binding of MAbs 12, 83 and CIII.78 to OspA depends on the structural integrity of the Trp₂₁₆ residue, which appears to be critical for antigenicity. Also apparent is that, although these MAbs bind to a common antigenic domain, the precise epitopes which they recognize are distinct from one another given the varying degrees of cross-reactivity to these MAbs among strains.

Although there is similar loss of binding activity of MAb336 with cleavage at Trp₂₁₆, this MAb does not bind to HA1 of OspA-B31, suggesting the domain for this antibody includes the carboxy-terminal end of the molecule, inclusive of residues 251 to 273. Low MW peptides, such as HA3 (10 kd) and NCS2 (6 kd), of OspA-K48 do not bind this MAb on Western blots. In order to confirm this observation, we tested binding of the 6 MAbs with a recombinant fusion construct p3AJEC that contains a trpE leader protein fused with residues 217 to 273 of OspA-B31 (Schubach, W.H. et al., Infect. Immun., 59(6):1911-1915 (1991)). Only MAb336 reacted with this construct (data not shown). Peptides and antigenic domains localized by fragmentation of OspA are summarized in Fig. 1.

### Example 2 Site-Directed Mutagenesis within Hypervariable Domains A., (residues 120 -140), B., (residues 150-180) and C., (residues 200-216 or 217).

Site-directed mutagenesis was performed to convert residues within the 204-219 domain of the recombinant B31 OspA to the analogous residues of a European OspA variant, K48. In the region of OspA between residues 204 and 219, there are seven amino acid differences between OspA-B31 and OspA-K48. Three oligonucleotides were generated, each containing nucleotide changes which would incorporate K48 amino acids at their analogous positions in the B31 OspA protein. The oligonucleotides used to create the site-directed mutants were:
5'-CTTAATGACTCTGACACTAGTGC-3' (#613, which converts threonine at position 204 to serine, and serine at 206 to threonine (Thr204-Ser, Thr206-Ser)) (SEQ ID NO:1);
5'-GCTACTAAAAAAACCGGGAAATGGAATTCA-3' (#625, which converts alanine at 214 to glycine, and alanine at 215 to lysine (Ala214-Gly, Ala215-Lys)) (SEQ ID NO:2); and 5'-GCAGCTTGGGATTCAAAAACATCCACTTTAACA-3' (#640, which converts asparagine at 217 to aspartate, and glycine at 219 to lysine (Asn217-Asp, Gly219-Lys)) (SEQ ID NO:3).

Site-directed mutagenesis was carried out by performing mutagenesis with pairs of the above oligonucleotides. Three site-directed mutants were created, each with two changes: OspA 613 (Thr204-Ser, Thr206-Ser), OspA 625 (Ala214-Gly, Ala215-Lys), and 640 (Asn217-Asp, Gly219-Lys). There were also two proteins with four changes: OspA 613/625 (Thr204-Ser, Thr206-Ser, Ala214-Gly, Ala215-Lys) and OspA 613/640 (Thr204-Ser, Thr206-Ser, Asn217-Asp, Gly219-Lys).

### Specificity of Antibody Binding to Epitopes of the Non-mutated Hypervariable Region

Monoclonal antibodies that agglutinate spirochetes, including several which are neutralizing *in vitro,* recognize epitopes that map to the hypervariable region around Trp216 (Barbour, A.G. et al., Infect. and Immun., 41:759 (1983); Schubach, W.H. et al., Infect. and Immun., 59:1911 (1991)). Western Blot analysis demonstrated that chemical cleavage of OspA from the B31 strain at Trp 216 abolishes reactivity of the protein with the agglutinating MAB 105, a monoclonal raised against B31 spirochetes. The reagent, n-chlorosuccinimide (NCS), cleaves OspA at the Trp 216, forming a 23.2 kd fragment and a 6.2 kd peptide which is not retained on the Imobilon-P membrane after transfer. The uncleaved material binds MAb 105; however, the 23.2 kd fragment is unreactive. Similar Western blots with a TrpE-OspA fusion protein containing the carboxy-terminal portion of the OspA protein demonstrated that the small 6.2 kd piece also fails to bind MAb 105 (Schubach, W.H. et al., Infect. and Immun., 59:1911 (1991)).

Monoclonal antibodies H5332 and H3TS (Barbour, A.G. et al., Infect. and Immun., 41:759 (1983)) have been shown by immunofluorescence to decorate the surface of fixed spirochetes (Wilske, B. et al., World J. Microbiol., 7:130 (1991)). These monoclonal antibodies also inhibit the growth of the organism in culture. Epitope mapping with fusion proteins has confirmed that the epitopes which bind these MAbs are conformationally determined and reside in the carboxy half of the protein. MAb H5332 is cross-reactive among all of the known phylogenetic groups, whereas MAb H3TS and MAb 105 seem to be specific to the B31 strain to which they were raised. Like MAb 105, the reactivities of H5332 and H3TS to OspA are abrogated by fragmentation of the protein at Trp216. MAb 336 was raised to whole spirochetes of the strain PGau. It cross-reacts to OspA from group 1 (the group to which B31 belongs) but not to group 2 (of which K48 is a member). Previous studies using fusion proteins and chemical cleavage have indicated that this antibody recognizes a domain of OspA in the region between residues 217 and 273. All of these MAbs agglutinate the B31 spirochete.

### Western Blot Analysis of Antibody Binding to Mutated Hypervariable Regions

MAbs were used for Western Blot analysis of the site-directed OspA mutants induced in *E. coli* using the T7 expression system (Dunn, J.J. et al., Protein Expression and Purification, 1:159 (1990)). *E. coli* cells carrying pET9c plasmids having a site-directed OspA mutant insert were induced at mid-log phase growth with IPTG for four hours at 37°C. Cell lysates were made by boiling an aliquot of the induced cultures in SDS gel loading dye, and this material was then loaded onto a 12% SDS gel (BioRad mini-Protean II), and subjected to electrophoresis. The proteins were then transferred to Imobilon-P membranes (Millipore) 70V, 2 hour at 4°C using the BioRad mini transfer system. Western analysis was carried out as described by Schubach et al., (Infect. Immun., 59:1911 (1991)).

Western Blot analysis indicated that only the 625 mutant (A1a214-Gly and A1a215-Lys) retained binding to the agglutinating monoclonal H3TS. However, the 613/625 mutant which has additional alterations to the amino terminus of Trp216 (Ser204-Thr and Thr206-Ser) did not bind this monoclonal. Both 640 and 613/640 OspAs which have the Asn217-Asp and Gly219-Lys changes on the carboxy-terminal side of Trp216 also failed to bind MAb H3TS. This indicated that the epitope of the B31 OspA which binds H3TS is comprised of amino acid side-chains on both sides of Trp216.

The 613/625 mutant failed to bind MAbs 105 and H5332, while the other mutants retained their ability to bind these MAbs. This is important in light of the data using fusion proteins that indicate that MAb 105 behaves more like MAb H3TS in terms of its serotype specificity and binding to OspA (Wilske, B. et al., Med. Microbiol. Immunol., 181:191 (1992)). The 613/625 protein has, in addition to the differences at residues Thr204 and Ser206, changes immediately amino-terminal to Trp216 (Ala214-Gly and Ala215-Lys). The abrogation of reactivity of MAbs 105 and H5332 to this protein indicated that the epitopes of OspA which bind these monoclonal antibodies are comprised of residues on the amino-terminal side of Trp216.

The two proteins carrying the Asn217-Asp and Gly219-Lys replacements on the carboxy-terminal side of Trp216 (OspAs 640 and 613/640) retained binding to MAbs 105 and H5332; however, they failed to react with MAb 336, a monoclonal which has been mapped with TrpE-OspA fusion proteins and by chemical cleavage to a more carboxy-terminal domain. This result may explain why MAb 336 failed to recognize the K48-type of OspA (Group 2).

It is clear that amino acids Ser204 and Thr206 play an important part in the agglutinating epitopes in the region of the B31 OspA flanking Trp216. Replacement of these two residues altered the epitopes of OspA that bind MAbs 105, H3TS and H5332. The ability of the 640 changes alone to abolish reactivity of MAb 336 indicated that Thr204 and Ser206 are not involved in direct interaction with MAb 336.

The results indicated that the epitopes of OspA which are available to MAbs that agglutinate spirochetes are comprised at least in part by amino acids in the immediate vicinity of Trp216. Since circular dichroism analysis indicated that the structures of B31 and K48 OspA differ very little within this domain, it is unlikely that the changes made by mutation have radically altered the overall structure of the OspA protein (France, L.L. et al., Biochem. Biophys. Acta, 1120:59 (1992); and France et al., Biochem. Biophys Acta, submitted (1993)). This hypothesis is supported by the finding that the recombinant, mutant OspAs exhibit the same high solubility and purification properties as the parent B31 protein (data not shown).

In summary, amino acid side-chains at Ser204 and Thr206 are important for many of the agglutinating epitopes. However, a limited set of conservative changes at these sites were not sufficient to abolish binding of all of the agglutinating MAbs. These results suggested that the agglutinating epitopes of OspA are distinct, yet may have some overlap. The results also supported the hypothesis that the surface-exposed epitope around Trp216 which is thought to be important for immune recognition and neutralization is a conformationally-determined and complex domain of OspA.

### Example 3 Borrelia Strains and Proteins

### A. Genes Encoding Borrelia Proteins

The altered OspA polypeptides of the current invention can be part of a cocktail with other proteins or can be joined to other proteins to form a chimeric protein. The other polypeptides of the cocktail or chimeric can be derived from any *Borrelia.* Representative proteins include OspA, OspB, OspC, OspD, p12, p39, p41 (fla), p66, and p93. Nucleic acid sequences encoding several *Borrelia* proteins are available (see Table II for examples); alternatively, nucleic acid sequences encoding *Borrelia* proteins can be isolated and characterized using methods such as those described below.

**Table II. References for Nucleic Acid Sequences for Several Proteins of Various Borrelia Strains**

| Strain | OspA |
|---|---|
| K48 | X62624 (SID 8) |
| PGau | X62387 (SID 10) |
| DK29 | X63412 (SID 21) |
| Pko | X65599 (SID 25) |
| PTrob | X65598 (SID 45) |
| Ip3 | X70365 (SID 24) |
| Ip90 | Kryuchechnikov, V.N. et al., J.Microbiol. Epid. Immunobiol. 12: 41-44 (1988)(SID 22) |
| 25015 | Fikrig, E.S. et al., J. Immunol. 7:2256-2260 (1992)(SID 12) |
| B31 | Bergstrom, S. et al., Mol. Microbiol. 3: 479-486 (1989) (SID 6) |
| PKa1 | X69606 (SID 42) |
| ZS7 | Jonsson, M. et al., Infect. Immun. 60:1845-1853 (1992)(SID 44) |
| N40 | Kryuchechnikov, V.N. *et al.* (SID 43) |
| PHei | X65600 (SID 46) |
| ACAI | Kryuchechnikov, V.N. *et al.* (SID 26) |
| PBo | X65605 (SID 23) |

| | |
|---|---|
| Numbers with an "X" prefix are GenBank data base accession numbers. SID = SEQ ID NO. | |

### B. Isolation of Borrelia Genes

Nucleic acid sequences encoding full length, lipidated proteins from known *Borrelia* strains were isolated using the polymerase chain reaction (PCR) as described below. In addition, nucleic acid sequences were generated which encoded truncated proteins (proteins in which the lipidation signal has been removed, such as by eliminating the nucleic acid sequence encoding the first 18 amino acids, resulting in non-lipidated proteins). Other proteins were generated which encoded polypeptides of a particular gene (i.e., encoding a segment of the protein which has a different number of amino acids than the protein does in nature). Using similar methods as those described below, primers can be generated from known nucleic acid sequences encoding *Borrelia* proteins and used to isolate other genes encoding *Borrelia* proteins. Primers can be designed to amplify all of a gene, as well as to amplify a nucleic acid sequence encoding truncated protein sequences, such as described below for OspC, or nucleic acid sequences encoding a polypeptide derived from a *Borrelia* protein. Primers can also be designed to incorporate unique restriction enzyme cleavage sites into the amplified nucleic acid sequences. Sequence analysis of the amplified nucleic acid sequences can then be performed using standard techniques.

### Cloning and Sequencing of OspA Genes and Relevant Nucleic Acid Sequences

*Borrelia* OspA sequences were isolated in the following manner: 100 µl reaction mixtures containing 50 mM KCl, 10 mM TRIS-HCl (pH 8,3), 1.5 mM MgCl₂, 200 µM each NTP, 2.5 units of TaqI DNA polymerase (Amplitaq, Perkin-Elmer/Cetus) and 100 pmol each of the 5' and 3' primers (described below) were used. Amplification was performed in a Perkin-Elmer/Cetus thermal cycler as described (Schubach, W.H. et al., Infect. Immun., 59:1811-1915 (1991)). The amplicon was visualized on an agarose gel by ethidium bromide staining. Twenty nanograms of the chloroform-extracted PCR product were cloned directly into the PC-TA vector (Invitrogen) by following the manufacturer's instructions. Recombinant colonies containing the amplified fragment were selected, the plasmids were prepared, and the nucleic acid sequence of each OspA was determined by the dideoxy chain-termination technique using the Sequenase kit (United States Biochemical). Directed sequencing was performed with M13 primers followed by OspA-specific primers derived from sequences, previously obtained with M13 primers.

Because the 5' and 3' ends of the OspA gene are highly conserved (Fikrig, E.S. et al., J. Immunol., 7:2256-2260 (1992); Bergstrom, S. et al., Mol. Microbiol. 3:479-486 (1989); Zumstein, G. et al., Med. Microbiol. Immunol., 181:57-70 (1992)), the 5' and 3' primers for cloning can be based upon any known OspA sequences. For example, the following primers based upon the OspA nucleic acid sequence from strain B31 were used:
5'-GGAGAATATATTATGAAA-3' (-12 to +6) (SEQ ID NO:4); and
5'-CTCCTTATTTTAAAGCG-3' (+826 to +809) (SEQ ID NO:5). (Schubach, W.H. et al., Infect. Immun, 59:1811-1915 (1991)).

OspA genes isolated in this manner include those for strains B31, K48, PGau, and 25015; the nucleic acid sequences are depicted in the sequence listing as SEQ ID NO:6 (OspA-B31), SEQ ID NO:8 (OspA-K48), SEQ ID NO:10 (OspA-PGau), and SEQ ID NO:12 (OspA-25015). An alignment of these and other OspA nucleic acid sequences is shown in Fig. 17. The amino acid sequences of the proteins encoded by these nucleic acid sequences are represented as SEQ ID NO:7 (OspA-B31), SEQ ID NO:9 (OspA-K48), SEQ ID NO:11 (OspA-PGau), and SEQ ID NO:13 (OspA-25015).

The following primers were used to generate specific nucleic acid sequences of the OspA gene:
5'-GTCTGCAAAAACCATGACAAG-3' (plus strand primer #369) (SEQ ID NO:14);
5'-GTCATCAACAGAAGAAAAATTC-3' (plus strand primer #357) (SEQ ID NO:15);
5'-CCGGATCCATATGAAAAAATATTTATTGGG-3' (plus strand primer #607) (SEQ ID NO:16);
5'-CCGGGATCCATATGGCTAAGCAAAATGTTAGC-3' (plus strand primer #584) (SEQ ID NO:17);
5'-GCGTTCAAGTACTCCAGA-3' (minus strand primer #200) (SEQ ID NO:18);
5'-GATATCTAGATCTTATTTTAAAGCGTT-3'(minus strand primer #586) (SEQ ID NO:19); and
5'-GGATCCGGTGACCTTTTAAAGCGTTTTTAAT-3' (minus strand primer #1169) (SEQ ID NO:20).

### C. Expression of Proteins from Borrelia Genes

The nucleic acid sequences described above can be incorporated into expression plasmids, using standard techniques, and transfected into compatible host cells in order to express the proteins encoded by the nucleic acid sequences. As an example, the expression of the p12 gene and the isolation of p12 protein is set forth.

Amplification of the p12 nucleic acid sequence was conducted with primers that included a NdeI restriction site into the nucleic acid sequence. The PCR product was extracted with phenol/chloroform and precipitated with ethanol. The precipitated product was digested and ligated into an expression plasmid as follows: 15 µl (approximately 1 µg) of PCR DNA was combined with 2 µl 10X restriction buffer for NdeI (Gibco/BRL), 1 µl NdeI (Gibco/BRL), and 2 µl distilled water, and incubated overnight at 37°C. This mixture was subsequently combined with 3 µl 10X buffer (buffer 3, New England BioLabs), 1 µl BamHI (NEB), and 6 µl distilled water, and incubated at 37° for two hours. The resultant material was purified by preparative gel electrophoresis using low melting point agarose, and the band was visualized under long wave ultraviolet light and excised from the gel. The gel slice was treated with Gelase using conditions recommended by the manufacturer (Epicentre Technologies). The resulting DNA pellet was resuspended in 25-50 µl of 10 mM TRIS-CL (pH 8.0) and 1 mM EDTA (TE). An aliquot of this material was ligated into the pET9c expression vector (Dunn, J. J. et al., Protein Expression and Purification, 1:159 (1990)).

To ligate the material into the pET9c expression vector, 20-50 ng of p12 nucleic acid sequences cut and purified as described above was combined with 5 µl 10 One-Phor-All (OPA) buffer (Pharmacia), 30-60 ng pET9c cut with NdeI and BamHI, 2.5 µl 20 mM ATP, 2 µl T4 DNA ligase (Pharmacia) diluted 1:5 in 1X OPA buffer, and sufficient distilled water to bring the final volume to 50 µl. The mixture was incubated at 12°C overnight.

The resultant ligations were transformed into competent DH5-alpha cells and plated on nutrient agar plates containing 50 µg/ml kanamycin and incubated overnight at 37 °C. DH5-alpha is used as a "storage strain" for T7 expression clones, because it is RecA deficient, so that recombination and concatenation are not problematic, and because it lacks the T7 RNA polymerase gene necessary to express the cloned gene. The use of this strain allows for cloning of potentially toxic gene products while minimizing the chance of deletion and/or rearrangement of the desired genes. Other cell lines having similar properties may also be used.

Kanamycin resistant colonies were single-colony purified on nutrient agar plates supplemented with kanamycin at 50 µg/ml. A colony from each isolate was inoculated into 3-5 ml of liquid medium containing 50 µg/ml kanamycin, and incubated at 37°C without agitation. Plasmid DNA was obtained from 1 ml of each isolate using a hot alkaline lysis procedure (Maniatis, T. et al., Molecular Cloning: A Laboratory Manual, Cold Spring Harbor Laboratory, Cold Spring Harbor, NY (1982)).

Plasmid DNA was digested with EcoRI and Bg1II in the following manner: 15 µl plasmid DNA was combined with 2 µl 10X buffer 3 (NEB), 1 µl EcoRI (NEB), 1 µl BglII (NEB) and 1 µl distilled water, and incubated for two hours at 37°C. The entire reaction mixture was subjected to electrophoresis on an analytical agarose gel. Plasmids carrying the p12 insert were identified by the presence of a band corresponding to 925 base-pairs (full length p12) or 875 base-pairs (nonlipidated p12). One or two plasmid DNAs from the full length and nonlipidated p12 clones in pET9c were used to transform BL21 DE3 pLysS to kanamycin resistance as described by Studier et al., (Methods in Enzymology, Goeddel, D. (Ed.), Academic Press, 185: 60-89 (1990)). One or two transformants of the full length and nonlipidated clones were single-colony purified on nutrient plates containing 25 µg/ml chloramphenicol (to maintain pLysS) and 50 µg/ml kanamycin at 37 °C. One colony of each isolate was inoculated into liquid medium supplemented with chloramphenicol and kanamycin and incubated overnight at 37°C. The overnight culture was subcultured the following morning into 500 ml of liquid broth with chloramphenicol (25 µg/ml) and kanamycin (50 µg/ml) and grown with aeration at 37°C in an orbital air-shaker until the absorbance at 600 nm reached 0.4-0.7. Isopropyl-thio-galactoside (IPTG) was added to a final concentration of 0.5 mM, for induction, and the culture was incubated for 3-4 hours at 37° as before. The induced cells were pelleted by centrifugation and resuspended in 25 ml of 20 mM NaPO₄ (pH 7.7). A small aliquot was removed for analysis by gel electrophoresis. Expressing clones produced proteins which migrated at the 12 kd position.

A crude cell lysate was prepared from the culture as described for recombinant OspA by Dunn, J.J. et al., (Protein Expression and Purification, 1:159 (1990)). The crude lysate was first passed over a Q-sepharose column (Pharmacia) which had been pre-equilibrated in Buffer A: 10 mM NaPO₄ (pH 7.7), 10 mM NaCl, 0.5 mM PMSF. The column was washed with 10 mM NaPO₄, 50 mM NaCl and 0.5 mM PMSF and then p12 was eluted in 10 mM NaPO₄, 0.5 mM PMSF with a NaCl gradient from 50-400 mM. p12 eluted approximately halfway through the gradient between 100 and 200 mM NaCl. The peak fractions were pooled and dialyzed against 10 mM NaPo4 (pH 7.7), 10 mM NaCl, 0.5 mM PMSF. The protein was then concentrated and applied to a Sephadex G50 gel filtration column of approximately 50 ml bed volume (Pharmacia), in 10 mM NaPO₄, 200 mM NaCl, 0.5 mM PMSF. p12 would typically elute shortly after the excluded volume marker. Peak fractions were determined by running small aliquots of all fractions on a gel. The p12 peak was pooled and stored in small aliquots at -20°C.

### Example 4 Generation of Chimeric Nucleic Acid Sequences and Chimeric Proteins

### A. General Protocol for Creation of Chimeric Nucleic Acid Sequences

The megaprimer method of site directed mutagenesis and a modification were used to generate chimeric nucleic acid sequences (Sarkar and Sommer, Biotechniques, 8(4): 404-407 (1990); Aiyar, A. and J. Leis, Biotechniques, 14(3): 366-369 (1993)). A 5' primer for the first genomic template and a 3' fusion oligo are used to amplify the desired region. The fusion primer consists of a 3' end of the first template (DNA that encodes the amino-proximal polypeptide of the fusion protein), coupled to a 5' end of the second template (DNA that encodes the carboxy-proximal polypeptide of the fusion protein).

The PCR amplifications are performed using Taq DNA polymerase, 10X PCR buffer, and MgCl₂ (Promega Corp., Madison, WI), and Ultrapure dNTPs (Pharmacia, Piscataway, NJ). One µg of genomic template 1, 5 µl of 10 µM 5' oligo and 5 µl of 10 µM fusion oligo are combined with the following reagents at indicated final concentrations: 10X Buffer-Mg FREE (1X), MgCl₂ (2 mM), dNTP mix (200 µM each dNTP), Taq DNA polymerase (2.5 units), water to bring final volume to 100 µl. A Thermal Cycler (Perkin Elmer Cetus, Norwalk, CT) is used to amplify under the following conditions: 35 cycles at 95°C for one minute, 55°C for two minutes, and 72° for three minutes. This procedure results in a "megaprimer".

The resulting megaprimer is run on a 1X TAE, 4% low-melt agarose gel. The megaprimer band is cut from the gel and purified using the Promega Magic PCR Preps DNA purification system. Purified megaprimer is then used in a second PCR step. One µg of genomic template 2, approximately 0.5 µg of the megaprimer, and 5 µl of 10 µM 3' oligo are added to a cocktail of 10X buffer, MgCl₂, dNTPs and Taq at the same final concentrations as noted above, and brought to 100 µl with water. PCR conditions are the same as above. The fusion product resulting from this amplification is also purified using the Promega Magic PCR Preps DNA purification system.

The fusion product is then ligated into TA vector and transformed into *E*. *coli* using the Invitrogen (San Diego, CA) TA Cloning Kit. Approximately 50 ng of PCR fusion product is ligated to 50 ng of pCRII vector with 1X Ligation Buffer, 4 units of T4 ligase, and brought to 10 µl with water. This ligated product mixture is incubated at 12°C overnight (approximately 14 hours). Two µl of the ligation product mixture is added to 50 µl competent INC F' cells and 2 µl beta mercaptoethanol. The cells are then incubated for 30 minutes, followed by heat shock treatment at 42°C for 60 seconds, and an ice quenching for two minutes. 450 µl of warmed SOC media is then added to the cells, resulting in a transformed cell culture which is incubated at 37°C for one hour with slight shaking. 50 µl of the transformed cell culture is plated on LB + 50 µg/µl ampicillin plates and incubated overnight at 37°C. Single white colonies are picked and added to individual overnight cultures containing 3 ml LB with ampicillin (50 µg/µl).

The individual overnight cultures are prepared using Promega's Magic Miniprep DNA purification system. A small amount of the resulting DNA is cut using a restriction digest as a check. DNA sequencing is then performed to check the sequence of the fusion nucleic acid sequence, using the United States Biochemical (Cleveland, OH) Sequenase Version 2.0 DNA sequencing kit. Three to five µg of plasmid DNA is used per reaction. 2 µl 2M NaOH/2mM EDTA are added to the DNA, and the volume is brought to 20 µl with water. The mixture is then incubated at room temperature for five minutes. 7 µl water, 3 µl 3M NaAc, 75 µl ethanol are added. The resultant mixture is mixed by vortex and incubated for ten minutes at -70°C, and then subjected to microcentrifugation. After microcentrifugating for ten minutes, the supernatant is aspirated off, and the pellet is dried in the speed vac for 30 second. 6 µl water, 2 µl annealing buffer, and 2 µl of 10 µM of the appropriate oligo is then added. This mixture is incubated for 10 minutes at 37°C and then allowed to stand at room temperature for 10 minutes. Subsequently, 5.5 µl of label cocktail (described above) is added to each sample of the mixture, which are incubated at room temperature for an additional five minutes. 3.5 µl labeled DNA is then added to each sample which is then incubated for five minutes at 37°C. 4 µl stop solution is added to each well. The DNA is denatured at 95° for two minutes, and then placed on ice.

Clones with the desired fusion nucleic acid sequences are then recloned in frame in the pET expression system in the lipidated (full length) and non-lipidated (truncated, i.e., without first 17 amino acids) forms. The product is amplified using restriction sites contained in the PCR primers. The vector and product are cut with the same enzymes and ligated together with T4 ligase. The resultant plasmid is transformed into competent *E. coli* using standard transformation techniques. Colonies are screened as described earlier and positive clones are transformed into expression cells, such as *E*. *coli* BL21, for protein expression with IPTG for induction. The expressed protein in its bacterial culture lysate form and/or purified form is then injected in mice for antibody production. The mice are bled, and the sera collected for agglutination, *in vitro* growth inhibition, and complement-dependent and -independent lysis tests.

A specific example of chimeric OspA is as follows. Other OspA chimeras can be made using the same method with suitable primers.

### OspA-K48/OspA-PGau

A chimer of OspA from strain K48 (OspA-K48) and OspA from strain PGau (OspA-PGau) was generated using the method described above. This chimeric nucleic acid sequence included bp 1-654 from OspA-K48, followed by bp 655-820 from OspA-PGau. Primers used included: the amino-terminal sequence of OspA primer #607 (SEQ ID NO:16); the fusion primer, 5'-AAAGTAGAAGTTTTTGAATCCCATTTTCCAGTTTTTTT-3' (minus strand primer #668-654) (SEQ ID NO:27); the carboxy-terminal sequence of OspA primer #586 (SEQ ID NO:19); and the sequence primers #369 (SEQ ID NO:14) and #357 (SEQ ID NO:15). The chimeric nucleic acid sequence is presented as SEQ ID NO:28; the chimeric protein encoded by this chimeric nucleic acid sequence is presented as SEQ ID NO:29.

### C. Purification of Proteins Generated by Chimeric Nucleic Acid Sequences

The chimeric nucleic acid sequences described above, as well as chimeric nucleic acid sequences produced by the methods described above, are used to produce chimeric proteins encoded by the nucleic acid sequences. Standard methods, such as those described above in Example 3, concerning the expression of proteins from *Borrelia* genes, can be used to express the proteins in a compatible host organism. The chimeric proteins can then be isolated and purified using standard techniques.

Nucleic acid encoding altered versions of OspA can be used to generate OspA chimeras. In addition, nucleic acid encoding OspA chimeras can be used to generate the altered OspA polypeptides of the present invention.

If the chimeric protein is soluble, it can be purified on a Sepharose column. Insoluble proteins can be solubilized in guanidine and purified on a Ni²⁺ column; alternatively, they can be solubilized in 10 mM NaPO₄ with 0.1-1% TRIXON X 114, and subsequently purified over an S column (Pharmacia). Lipidated proteins were generally purified by the latter method. Solubility was determined by separating both soluble and insoluble fractions of cell lysate on a 12% PAGE gel, and checking for the localization of the protein by Coomassie staining, or by Western blotting with monoclonal antibodies directed to an antigenic polypeptide of the chimeric protein.

### Example 5 Generation of OspC/OspA Chimeric Nucleic Acids and Chimeric Proteins

### A. General Protocol for Creation of Chimeric Nucleic Acid Sequences

A large number of chimeric nucleic acid sequences encoding proteins comprising at least a first and a second polypeptide from *Borrelia burgdorferi* were generated. These chimeric nucleic acid sequences were produced such that the encoded chimeric protein comprised a *Borrelia burgdorferi* OspC polypeptide upstream of (or N-terminal to) a *Borrelia burgdorferi* OspA polypeptide. The chimeric nucleic acid sequences were also produced such that the nucleic acid encoding one polypeptide was in the same reading frame as the nucleic acid sequence encoding the next polypeptide in the chimeric protein.

The general cloning strategy used to construct the chimeric nucleic acid sequences was as follows. The desired fragment of OspC was amplified using a 5' primer containing a restriction site suitable for cloning the resultant product into a vector of interest and a 3' primer containing a restriction site suitable for ligating the OspC fragment to the OspA fragment. The OspC product was cloned into a suitable vector. For the OspA portion of the chimeric nucleic acid, the desired OspA fragment was amplified using a 5' primer containing a restriction site for ligating the resultant OspA fragment to the OspC fragment and a 3' primer containing a restriction site suitable for cloning the resultant OspA product into the vector with the OspC product. The use of a restriction site to allow ligation of the OspC and OspA fragments results in the insertion of 0 to about 3 amino acids between the OspC and OspA fragments.

A specific example of such a construction follows. It is understood that other suitable restriction sites could be used with no or only routine experimentation. The resultant OspC/OspA Chimer could have, therefore, the addition of 0 to about 3 amino acids or more between the OspC and OspA fragments, depending on the restriction site used.

For the OspC portions of the chimeric nucleic acids, desired fragments of OspC genes from various strains or genospecies were PCR amplified using a 5' primer containing an NdeI site and a 3' primer containing a NcoI site and a BamHI site. The amplified OspC product was then cloned into the NdeI and BamHI sites of the T7 promoter driven expression vector, pET9c. For the OspA portion of the chimeric nucleic acid, desired fragments of OspA genes from a strain of interest or genospecies of interest were PCR amplified using a 5' primer containing an NcoI site and a 3' primer containing a BamHI site. This OspA portion could then be directly cloned into the NcoI and BamHI sites of the pET9c vector containing the desired OspC sequence, thereby producing the desired OspC-OspA construct. By including the sequence for the NcoI restriction site in the primers, a nine nucleotide linker sequence encoding the amino acids Ser-Met-Ala was produced at the junction between the N-terminal OspC sequence and the C-terminal OspA sequence. The use of the NcoI restriction enzyme (CCATGG) in this cloning strategy was a suitable choice as *Borrelia* is an AT-rich organism which possesses only a few NcoI sites in its genome. One of ordinary skill in the art would know that different restriction sites wold be utilized and would know how to generate the chimeric OspC/A constructs for use in subsequent alterations described herein with no or only routine experimentation.

As an example, OspC-OspA chimeric nucleic acids which contain nonlipidated OspC B31 were generated using the following primers:
(5'OspC-NdeI): 5'-GT CAT ATG GCT TGT AAT AAT TCA GGG AAA GA-3' (SEQ ID NO:91); and
(3'OspC-NcoI): 5'-T TTC CAT GGA AGG TTT TTT TGG ACT TTC TG-3'(SEQ ID NO:92).

For OspC-OspA chimeric nucleic acids which contain nonlipidated OspA B31, the following primers were used:
(5'OspA-NcoI:) 5'-TT TCC ATG GCC AAG CAA AAT GTT AGC AGC C-3' (SEQ ID NO:93); and
(3'OspA-BamHI): 5'-TAA GGA TCC TTA TTT TAA AGC GTT TTT-3' (SEQ ID NO:94).

Lipidated versions of the OspC/OspA chimeras can be constructed by designing primers to amplify the a portion of the template that includes the lipidation signal sequence or by generating a nucleic acid construct with a suitable lipidation signal sequence. The leader sequence comprising a lipidation signal can be, for example, from a gene encoding the OspA, OspB or OspC polypeptides.

### B. Protein Expression

As described in the previous examples, it is possible to express and purify *Borrelia* proteins or polypeptides, for example, OspA polypeptides, OspC polypeptides, chimeric OspC/OspA polypeptides and polypeptides comprising the OspA alterations described herein. This is accomplished by incorporating the desired nucleic acid sequence, which encodes the protein of choice, into an expression plasmid, for example, using standard techniques. This expression plasmid can then be transfected into a compatible host cell in order to express the desired protein.

For example, the purified OspA, OspC or OspC/OspA chimeric proteins that were used to immunize mice and in the ELISA tests described below, were generated and purified by cloning either OspA, OspC or OspC/OspA chimeric nucleic acid sequences, in frame, into the pET expression plasmid. The expression plasmid was then transfected into the compatible expression cell line *Escherichia coli* strain BL21 (pLysS) or strain B834 (DE3). The BL21 or B834 cells were grown in 10 mL LB media (5 g/L NaCl, 10 g/L tryptone, 5 g/L yeast extract, 25 mg/L chloramphenicol and 50 mg/L ampicillin) at 37°C, with shaking. When the optical density at 600λ reached 0.3-0.4 units, recombinant protein expression was induced by adding IPTG (isopropyl B-D-thiogalactopyranoside) to a final concentration of 0.5 mM and the cells were grown for an additional three hours. The cultures were harvested by centrifugation at 3800xg for five minutes. The cells were resuspended in 20 mM NaPO₄, pH 7.7, and stored at -20°C overnight. Once thawed, the crude extracts were incubated with DNase (2 µg/mL) in the presence of 2.5 mM MgCl₂ at room temperature for thirty minutes and then spun at 14,000 rpm (Eppendorf 5417C) for five minutes.

To purify the OspC proteins described below, the crude extracts from the OspC-expressing cells were loaded onto an anion exchange column (Q Sepharose Fast Flow, 2.2x10cm, Pharmacia) which had been pre-equilibrated with 20 mM Tris-Cl, pH 9.3. The column was washed in the same buffer (20 mM Tris-Cl, pH 9.3) which eluted the OspC protein. The wash fractions that contained OspC were concentrated using Amicon 10K and then were dialyzed with a solution containing 20 mM NaPO₄, pH 8.0, and 250 mM NaCl. The partially purified OspC was then passed over a Ni²⁺ metal affinity column (Chelating Sepharose Fast Flow 2.2x10 cm, Pharmacia) equilibrated with 20 mM NaPO₄, pH 8.0, and 250 mM NaCl. The column was washed using a decreasing pH gradient of 20 mM sodium acetic acid and 250 mM NaCl and the bound OspC eluted around pH 5.7. The OspC fractions were then concentrated by ultrafiltration and stored at -70°C.

For purification of OspA proteins, the same procedure was followed, except that the dialysis step, after the Amicon 10K cutoff, was done in 20 mM NaPO₄, pH 6.0. The partially purified OspA was then applied to a cation exchange column (S Sepharose Fast Flow 2.2x10 cm, Pharmacia) equilibrated with 20 nM NaPO₄, pH 6.0. The column was washed using an increasing NaCl gradient from 0 to 100 mM. The OspA-containing fractions were concentrated by ultrafiltration and stored at -70°C.

As previously indicated, both lipidated and non-lipidated (e.g., truncated, lacking the first 17 amino acids) forms of OspC, OspA and OspC/OspA chimeric proteins were generated.

The above-described techniques were also used to express proteins comprising the altered OspA polypeptides of the present invention.

### C. Immunization of Mice and Serologic Characterization Using ELISA (Enzyme-Linked Immunosorbent Assay) Immunization of Mice

Mice, either C3H-J or ICR, were immunized with 3 µg of lipidated OspC/OspA chimeric protein or 6 µg of non-lipidated OspC/OspA chimer in 100 mL of aluminum hydroxide adjuvant (concentration of 1.8 mg/mL) by (SC) subcutaneous injection. As a negative control, mice were immunized with 100 mL of aluminum hydroxide adjuvant only. All mice received a total of three injections which were given at two week intervals. One week after the final immunization, blood was drawn from each mouse (including negative control mice) and the serum was tested for IgG reactivity using the ELISA method described below, for the presence of anti-OspA antibodies to three different purified OspA proteins (*Borrelia burgdorferi sensu stricto* (B31), *Borrelia garinii* (K48) and *Borrelia afzelii* (PGau). The sera was tested at a dilution of 1:1000.

Mice were immunized with the chimeric proteins described in Table III.

**Table III. Chimeric Proteins Used to Immunize Mice**

| Name | Description (amino acid) | SEQ ID NO: (nucleic acid) | SEQ ID NO: (polypeptide) | Fig. No: |
|---|---|---|---|---|
| OspA | OspA-B31(18-273) | 6 | 7 | 22,23 |
| OspC | OspC-B31(19-211) | * | * | 22,23 |
| OspC2-OspA | OspC-C2(19-204)/ OspA-B31(18-273) | 59 | 60 | 22,23 |
| ¹lipOspAP/Bo | OspA-PGau(1-217)/ OspA-Bo(218-273) | 49 | 50 | 24,25 |
| ¹lipOspAB/P | OspA-B31(1-216)/ OspA-Pko(217-273) | * | * | 24,25 |
| OspC-OspAB/P | OspC-B31(19-211)/ OspA-B31 (18-216)/ OspA-Pko(217-273) | 65 | 66 | 24,25, 27, 28, 29 |
| OspCB31-OspAB31 | OspC-B31(19-211)/ OspA-B31(18-273) | 55 | 56 | 26,27, 28,29 |
| OspC2-OspAB31 | OspC-C2(19-204)/ OspA-B31(18-273) | 59 | 60 | 26,27 |
| ¹lip OspA K/T | OspA-K48(1-217)/ OspA-Tro(218-273) | * | * | 27 |
| ¹lip OspC-B31 | OspC-B31(1-211) | * | * | 26 |
| OspCB31-OspABPBP | OspC-B31(19-211)/ OspA-B31(30-150)/ OspA-Pko(151-179)/ OspA-B31 (180-216) (190 N deletion)/ OspA-Pko(217-273) B31/B31/Pko | 87 | 88 | 28,29 |

| | | | | |
|---|---|---|---|---|
| ¹"lip" means the chimeric protein contains its native N-terminal lipidation signal Serologic Characterization Using ELISA (Enzyme-Linked Immunosorbent Assay) | | | | |

### Immobilization of antigen onto ELISA Plates

A solution of purified recombinant OspC or OspA protein from each of the *Borrelia burgdorferi* strains B31 *(Borrelia burgdorferi sensu stricto),* K48 *(Borrelia garinii)* and PGau *(Borrelia afzelii)* was added to sodium phosphate buffer, pH 9.0, and was used to coat a commercial microwell plate (MaxiSorp^{®}, Nunc). The coating procedure was as follows: 100 µl of a solution containing the appropriate OspA or OspC protein (made up at a concentration of 250 ng/ml in the following coating buffer: 100 mM Bis-Tris propane, pH 9.7) was added to each well of a microtiter plate which was incubated for one hour at 37°C. The antigen solution was removed from the wells, the plate was washed three times with phosphate buffered saline (PBS) pH 9.0, and 300 µl of blocking buffer solution was added (3% dry milk, 0.1% polyoxyethylenesorbitan (referred to herein as Tween 20^{™}), 0.02% NaN₃ in 100 nM Bis-Tris propane, pH 9.7). Following a one hour incubation at 37°C, the plates were washed four times with TBS-Tween 20^{™} wash buffer (20 mM Tris-Cl, pH 7.5, 136 mM NaCl, 0.1% Tween 20^{™} and 0.02% NaN₃) and then were allowed to dry. The plates were then wrapped in plastic and stored at 4°C until they were used.

### ELISA (Enzyme-Linked Immunosorbent Assay) Tests

The standard procedure for the ELISA tests was as follows: mouse serum was diluted 1:1000 in sample dilution buffer (1% dry milk, 136 mM NaCl, 0.1% Tween 20^{™}, 0.02% NaN₃ in 20 mM Tris-Cl, pH7.5) and 100 µl of the diluted serum was added to the ELISA microtiter plate wells that had been coated with antigen as described above. Following incubation for 1 hour at 37°C, the samples were removed and the plates were washed four times in TBS-Tween^{™} (20 mM Tris-Cl, pH 7.5; 136 mM NaCl; 0.1% Tween 20^{™} and 0.02% NaN₃). For the secondary antibody, goat anti-mouse antisera conjugated to alkaline phosphatase-specific for either IgM (Fc) or IgG (Fab), (Jackson Immuno Research Laboratories) was diluted 1:750 in sample dilution buffer (1% dry milk, 136 mM NaCl, 0.1% Tween 20^{™}, 0.02% NaN₃ in 20 mM Tris-Cl, pH7.5) and 100 µl of the diluted secondary antibody was added to each well. Following incubation for thirty minutes at 37°C, the plates were washed three times with TBS-Tween^{™} and 100 µl of Phosphatase Substrate solution (5 mg of p-nitrophenylphosphate tablets dissolved in 1X diethanolamine substrate buffer to yield a 2 mg/ml solution - Kirkegaard Perry Laboratory) was added to each well. The plates were incubated for thirty minutes at 37°C and 100 µl of stop solution (5 % EDTA) was added to each well. The absorbance at 405 nm was read on a microplate reader (Dynatech). A sample was considered positive if it produced an average absorbance greater than the mean of the negative controls plus three standard deviations.

Previous work has demonstrated that it is the carboxy-terminal region of OspA that contains the antigenic sites that provide the immunoprotective response. Thus, in addition to the ELISA test described above, a modified ELISA was performed (herein referred to as the Protective ELISA Test), wherein the purified N-terminal region of B31 OspA (amino acids 18-139) was used in excess to block any antibodies present in the mouse serum that had specificity to this N-terminal OspA region. These protective ELISA tests were performed as above, except that 80 µg/ml of a purified B31 OspA fragment (amino acids 18-139) was added to the diluted mouse serum prior to adding the sera to the antigen-coated ELISA microtiter plate wells.

### Results of ELISA Tests

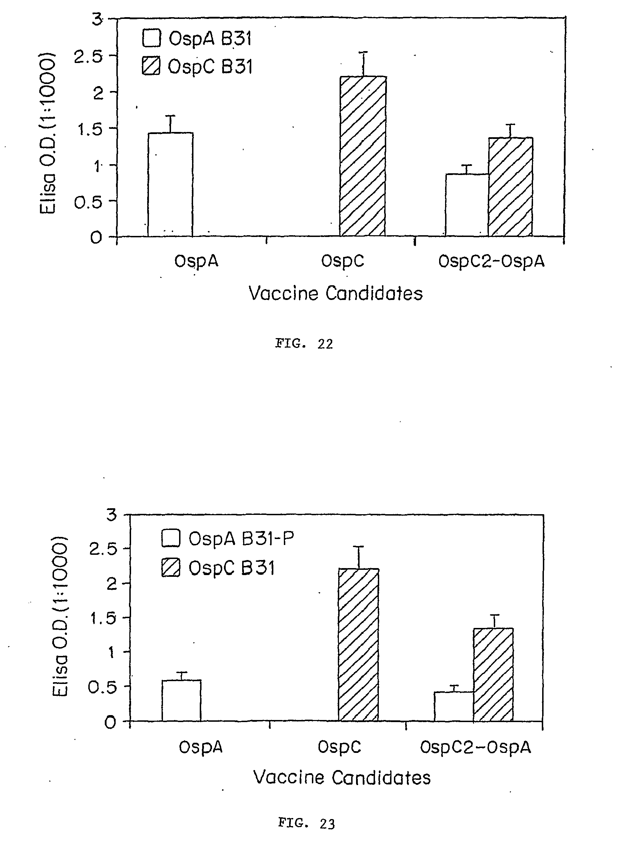

Using the above-described ELISA tests, it was demonstrated that mice immunized with a non-lipidated OspC/OspA chimeric protein (OspC2-OspA - composed of OspC (a.a.19-204 from strain C2)/OspA (a.a. 18-273 from strain B31) (SEQ ID NO:60) produced an immune response both to OspA and OspC that was comparable to the immune response generated to non-lipidated OspA (OspA - a.a. 18-273 from strain B31) and non-lipidated OspC (OspC - a.a. 19-211 from strain B31) control proteins (Fig. 22). As indicated in Fig. 22 and described above, mice were immunized with OspA, OspC or OspC2-OspA proteins and immune responses of the sera were measured against B31 OspA antigen (stippled bars) and B31 OspC antigen (solid bars).

Using the above-described Protective ELISA Test, it was also shown that mice immunized with the same non-lipidated OspC/OspA chimeric protein (OspC2-OspA - composed of OspC (a.a.19-204 from strain C2)/OspA (a.a. 18-273 from strain B31) (SEQ ID NO: 60) produced an immune response to the C-terminal portion of OspA that was comparable to the immune response generated to the C-terminal portion of a non-lipidated OspA (OspA - a.a. 18-273 from strain B31) control protein (Fig. 23). As indicated in Fig. 23, mice were immunized with OspA, OspC or OspC2-OspA proteins and immune responses of the sera were measured against B31 OspA antigen. The protective antibody response to B31 OspA antigen is indicated in the stippled bars.

Thus, these results clearly demonstrate that non-lipidated chimeric OspC/OspA proteins are able to induce immune responses in mice that are comparable to the immune response generated against non-lipidated OspC and OspA control proteins.

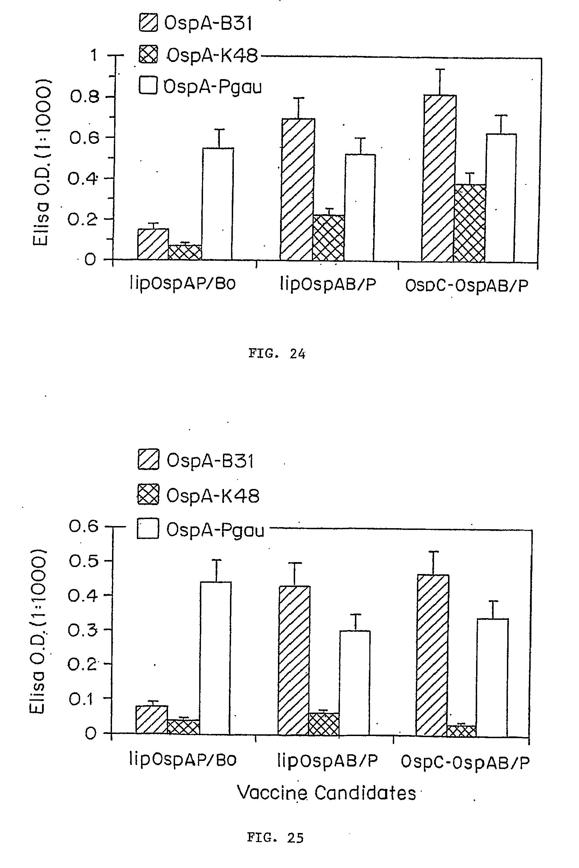

It had been previously thought that the lipidation signals that are present on *Borrelia burgdorferi* outer surface proteins were required for immunogenicity and that OspC and OspA proteins that lacked this lipidation signal would be less or non-immunogenic. To test this idea, mice were immunized with a non-lipidated OspC/OspA chimeric protein (OspC-OspAB/P - composed of OspC (a.a.19-211 from strain B31)/OspA (a.a. 18-216 from strain B31)/OspA (a.a. 217-273 from strain Pko)(SEQ ID NO:66) as well as two lipidated OspA proteins, lipOspAP/Bo (composed of OspA (a.a. 1-217 from strain PGau)/OspA (a.a. 218-273 from strain Bo)) and lipOspAB/P (composed of OspA (a.a. 1-216 from strain B31)/OspA (a.a. 217-273 from strain Pko)) and were subjected ELISA tests. Mice immunized with the non-lipidated OspC/OspA chimeric protein (OspC-OspAB/P) produced an immune response to OspA from each of the *Borrelia burgdorferi* strains B31 *(Borrelia burgdorferi sensu stricto),* K48 *(Borrelia garinii)* and PGau *(Borrelia afzelii),* that was equivalent or greater than the immune response generated to the two lipidated OspA control proteins (lipOspAP/Bo and lipOspAb/P) (Fig. 24).

Similar results to these were obtained using the Protective ELISA Test described above. Mice immunized with the non-lipidated OspC/OspA chimeric protein (OspC-OspAB/P) produced an immune response to the C-terminal region of OspA from each of the *Borrelia burgdorferi* strains B31 *(Borrelia burgdorferi sensu stricto),* K48 *(Borrelia garinii)* and PGau *(Borrelia afzelii),* that was equivalent or greater than the immune response generated to the C-terminal region of OspA from the two lipidated OspA control proteins (lipOspAP/Bo and lipOspAb/P) (Fig. 25).

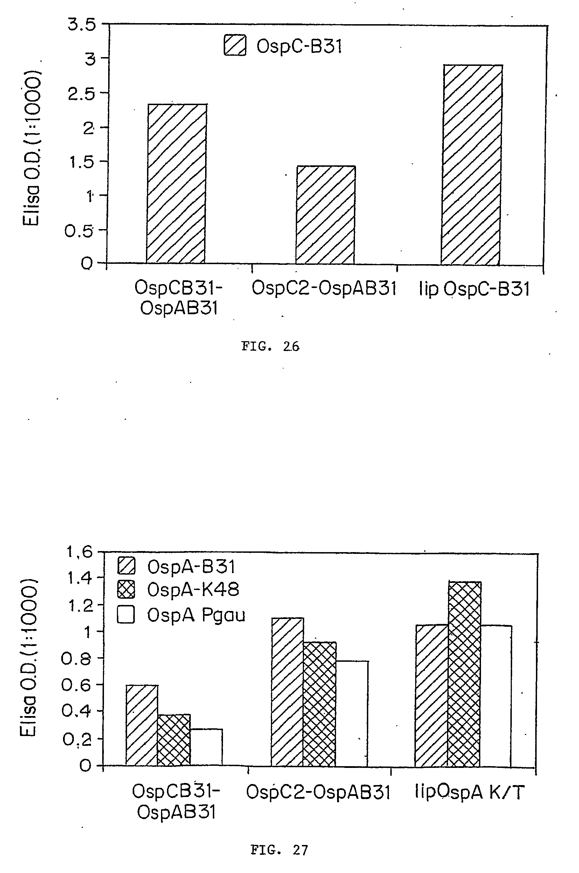

In addition to the comparisons between non-lipidated OspC/OspA chimeric proteins and lipidated OspA control proteins, experiments were also performed to compare non-lipidated OspC/OspA chimeric proteins with a lipidated OspC control protein (Fig. 26). Mice that were immunized with either the non-lipidated OspC/OspA chimeric protein OspCB31-OspAB31 (composed of OspC (a.a. 19-211 from strain B31)/OspA (a.a. 18-273 from strain B31) (SEQ ID NO:56) or the non-lipidated OspC/OspA chimeric protein OspC2-OspAB31 (composed of OspC (a.a. 19-204 from strain C2)/OspA (a.a. 18-273 from strain B31) (SEQ ID NO:60) produced an immune response to OspC derived from the *Borrelia burgdorferi* strain B31 that was comparable to the immune response produced by a lipidated OspC control protein (lip OspC-B31 - composed of OspC (a.a. 1-211 from strain B31)) (Fig. 26).

Thus, these results clearly demonstrate that non-lipidated chimeric OspC/OspA proteins are able to induce immune responses against OspA and OspC that are comparable to the immune response generated against OspA and OspC using lipidated OspA or OspC control proteins. The use ofunlipidated forms of these proteins as vaccine immunogens or diagnostic antigens is highly desirable because the product yield is much greater and the proteins are much easier to purify. For these reasons, the production of these proteins is less expensive.

The OspC/OspA chimeric proteins of the present invention are also able to generate immune responses against OspA proteins that are derived from strains that are not represented in the chimeric protein. Mice immunized with the OspC/OspA chimeric proteins, OspCB31-OspAB31 (SEQ ID NO:56) and OspC2-OspAB31 (SEQ ID NO:60), are not only able to generate immune responses that recognize OspA derived from strain B31 *(Borrelia burgdorferi sensu stricto),* but also recognize OspA derived from strain K48 *(Borrelia garinii)* and strain PGau *(Borrelia afzelii)* (Fig. 27). For comparison, mice were also immunized with the lipidated OspA chimeric protein, Lip OspA K/T (composed of OspA (a.a. 1-217 from strain K48)/OspA (a.a. 218-273 from strain Tro)) (Fig. 27).

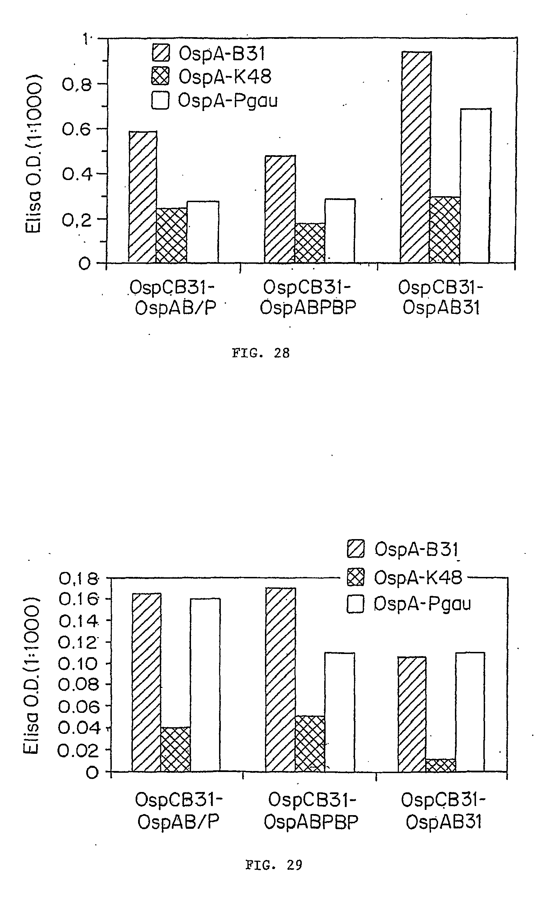

Additional antibody responses to OspA derived from strain B31 (*Borrelia burgdorferi sensu stricto),* strain K48 *(Borrelia garinii)* and strain PGau *(Borrelia afzelii)* are also presented for sera from mice immunized with other OspC/OspA chimeric proteins. Thus, Fig. 28 presents the ELISA results from mice immunized with either OspCB31-OspAB/P (SEQ ID NO:66), OspCB31-OspABPBP (SEQ ID NO:88) or OspCB31-OspAB31 (SEQ ID NO:56). In each case, sera from the immunized mice was tested against OspA derived from each of strain B31 *(Borrelia burgdorferi sensu stricto),* K48 *(Borrelia garinii)* and PGau *(Borrelia afzelii).* In all cases, a strong immune response was generated (Fig. 28). As with the previously described OspC/OspA chimeric proteins, the three OspC/OspA chimeric proteins used to immunize the mice in Fig. 27 also elicited a strong immune response to the C-terminal region of OspA when examined using the Protective ELISA Test described above (Fig. 29).

The above-described techniques are also used to immunize mice and to serologically characterize the immune response against the proteins comprising the altered OspA polypeptides of the present invention.

### Tick Challenge of Immunized Mice

Mice, either C3H-J or JCR, that had been immunized as described above, were also challenged with either laboratory-infected nympha or field nympha. The immunized mice were placed in isolation cages and each mouse received 5-10 nymphs. All of the nymphs were collected at counted after 6 days. Four week after challenge, the mice were bled and sera was tested using commercially-available Western blot strips to *Borrelia burgdorferi sensu stricto* strain B31(MarDx strips) and/or *Borrelia garinii* (MRL strips). Eight weeks after challenge, the mice were bled, sera was tested again by Western blot and ear punch and bladder samples were cultured. As a positive control, mice which had been immunized with only aluminum hydroxide adjuvant, as described above, were subjected to the same challenge.

The results of the tick challenge studies (Table IV) demonstrate that while immunization with lipidated OspC protein was unable to protect the mice, as evidenced by a positive Western blot signal (in 4 out of 5 mice), immunization with two different OspC/OspA chimeric proteins (SEQ ID NO:56 and SEQ ID NO:62) did provide protection, as indicated by the absence of Western blot signal (in 0 out of 8 mice and 0 out of 3 mice) (Table IV). The sham positive control showed that the challenge by the ticks was successful in all cases, as evidenced by 100% positive signal in Western blots (Table IV). Results from the tick challenge experiments are shown in Table IV.

**Table IV. Effect of Vaccination on Transmission of Borrelia from Ticks**

| Vaccine Candidate | Mouse | Tick-nymph | Seroconversion (Western Blots) Vaccinated | Seroconversion (Western Blots) Sham |
|---|---|---|---|---|
| OspC1-OspAB31 | C3H-J | Long Island | 0+/8 | 8+/8 |
| OspC2-OspAB31 | C3H-J | Long Island | 0+/3 | 4+/4 |
| Lip OspC12 | ICR | Long Island | 4+/5 | 5+/5 |

The above-described techniques are also used to measure the ability of mice immunized with proteins comprising the altered OspA polypeptides of the present invention to resist or respond to transmission *of Borrelia* from ticks.

### Example 6 Generation of OspA M1, M2, M3, J1, J2 and J3 constructs using site directed mutagenesis and PCR

All constructs were made using Stratagene's QuikChange site-directed mutagenesis kit. Site-directed mutagenesis was performed using Pfu Turbo DNA polymerase II and a thermal temperature cycler. Pfu Turbo DNA polymerase replicates both plasmid strands with high fidelity and without displacing the mutant oligonucleotide primers. The basic procedure used a supercoiled double-stranded DNA vector (pET 9c for all constructs) with an insert of interest and two synthetic oligonucleotide primers containing the desired mutation(s). The oligonucleotide primers, each complementary to opposite strands of the vector, were extended during the temperature cycling by the Pfu Turbo DNA polymerase. Incorporation of the oligonucleotide primers created a mutated plasmid which contained staggered nicks. Following temperature cycling, the linear product was treated with the restriction enzyme Dpn I, which is specific for methylated DNA. DNA isolated from most *E. coli* strains is dam methylated and therefore is susceptible to Dpn I digestion. Digestion with Dpn I therefore destroyed the original template DNA leaving only the nicked plasmid DNA (which was not methylated) containing the desired mutation(s). This nicked vector DNA (containing the desired mutation(s)) was then transformed into competent *E. coli* which were plated on antibiotic-containing plates. Colonies containing the plasmid (which encodes for antibiotic resistance in addition to the altered OspA polypeptide) were grown and plasmids were purified and sequenced to confirm that they possessed the desired mutation(s).

### 1) M1, M2 and M3 mutants

The mutations described herein are made using nucleic acids (e.g., polynucleotides) encoding OspA polypeptides or fragments from any strain of Lyme Disease-causing *Borrelia,* such as *Borrelia burgdorferi sensu stricto, Borrelia afzelii* or *Borrelia garinii.* As an example, the generation of the M3 mutations in an OspA chimera, called "BPBP", is described below. The creation of the construct OspA BPBP M3 was as follows. BPBP is a chimeric OspA polypeptide, wherein residues 1-164 are from OspA of B31, residues 165-179 are from OspA ofPko or PGau, residues 180-216 are from OspA of B31 and residues 217-273 are from OspA of Pko (wherein the numbering is as shown in SEQ ID NO:7).

The first step in creating this construct was to make the construct BPBP M1. The M1 mutation consists of a mutation of codon 139 (aga) of OspA from the amino acid arginine to the amino acid methionine (codon atg). A PCR reaction was set up as described above containing a polynucleotide encoding BPBP as the template DNA and the following oligonucleotide primers:
a) 5' R139M:
   5' gaa aaa ata ata aca atg gca gac gga acc 3' (SEQ ID NO:117).
b) 3' R139M:
   5' ggt tcc gtc tgc cat tgt tat tat ttt ttc 3' (SEQ ID NO:118)
The PCR reaction also contained reaction buffer, 10ng of the BPBP DNA template, 125 ng of each oligonucleotide primer, and the dNTP mix. The parameters for the PCR were as outlined in Table V.

**Table V. Parameters for PCR**

| Segment | cycles | temp | time |
|---|---|---|---|
| 1 | 1 | 95°C | 30 sec. |
| 2 | 18 | 95°C | 30 sec. |
| 3 | | 50°C | 1 min. |
| 4 | | 68°C | 12 min |

Following PCR, the product was transformed into competent *E*. *coli* XLl-Blue cells. Colonies formed on the selective agar plates (containing kanamycin) were grown and plasmids were purified and sequenced to confirm that they possessed the desired mutation. The construct BPBP M1 was then used as template to make the plasmid BPBP M2, which in addition to the R139M mutation, also contains a mutation that changes the glutamic acid codon (gag) at position 160 to the codon (tat) which encodes for tyrosine. The oligonucleotide primers used to generate this plasmid were:
a) 5' E160Y
   5' gga aaa gct aaa tat gtt tta aaa ggc 3' (SEQ ID NO:119)
b) 3' E160Y
   5' gcc ttt taa aac ata ttt agc ttt tcc 3' (SEQ ID NO:120)
The parameters for the PCR were as outlined in Table VII. The final mutation, M3, which alters a lysine residue at position 189 to a methionine residue was performed using BPBP M2 as a DNA template. The oligonucleotide primers used to generate this plasmid were:
a) 5' K189M
   5' gtt act tta agc atg aat att tca aaa tc 3' (SEQ ID NO:121)
b) 3' K189M
   5' ga ttt tga aat att cat gct taa agt aac 3' (SEQ ID NO:122)
This final mutation yielded the desired construct, BPBP M3.

### 2) J1, J2 and J3 mutants

J1, J2 and J3 mutants were made using the same protocol described for the generation of the M1, M2 and M3 mutants. Such mutants can be made using nucleic acids (e.g., polynucleotides) encoding OspA polypeptides fragments from any strain of Lyme Disease-causing *Borrelia,* such as *Borrelia burgdorferi sensu stricto, Borrelia afzelii* or *Borrelia garinii.*

The oligonucleotide primers which were used to generate the J1 mutation (which contains a Y165F mutation (codon tat to ttt) and a V166Y mutation (codon gtt to act)) were as follows:
a) 5' B31 YV-FY
   5' gag gtt tta aaa ggc ttt act ctt gaa gga act c 3' (SEQ ID NO:123)
b) 3' B31 YV-FY
   5' gag ttc ctt caa gag taa agc ctt tta aaa cct g 3' (SEQ ID NO:124)

The oligonucleotide primers which were used to generate the J2 mutation (which contains a T170K mutation (codon act to aag)) were as follows:
a) 5' B31 T-K
   5' tct tga agg aaa gct aac tgc tg 3' (SEQ ID NO:125)
b) 3' B31 T-K
   5' cag cag tta gct ttc ctt caa ga 3'(SEQ ID NO:126)

To generate the J3 mutant (which contains a Y165F mutation (codon tat to ttt), a V166Y mutation (codon gtt to act) and a T170K mutation (codon act to aag)), the template containing the J1 mutations was used with the oligonucleotide primers for generating the J2 mutation (5' B31 T-K (SEQ ID NO:125) and 3' B31 T-K (SEQ ID NO:126)).

The altered OspA polypeptides described herein are expressed, used to immunize mice and characterized using ELISA as described in the Experiments above.

While this invention has been particularly shown and described with references to preferred embodiments thereof, it will be understood by those skilled in the art that various changes in form and details may be made therein without departing from the scope of the invention encompassed by the appended claims.

## Claims

1. A polypeptide, comprising an amino acid sequence of OspA protein from *Borrelia burgdorferi* from about residue 139 to about residue 273 of *Borrelia burgdorferi* OspA protein, wherein the sequence includes at least one alteration selected from the group consisting of: residue 139 being methionine, residue 160 being tyrosine, residue 189 being methionine and combinations thereof, wherein the numbering corresponds to the numbering of SEQ ID NO:7.

2. The polypeptide of Claim 1, wherein the polypeptide has increased conformational stability compared to the corresponding unaltered OspA polypeptide.

3. The polypeptide of Claim 1, wherein residue 160 is tyrosine and residue 189 is methionine.

4. The polypeptide of Claim 1, wherein residue 139 is methionine, residue 160 is tyrosine and residue 189 is methionine.

5. The polypeptide of Claim 1, wherein the polypeptide comprises residues 131 through 273 of the *Borrelia burgdorferi* OspA protein.

6. The polypeptide of Claim 1, wherein the polypeptide comprises residues 17 through 273 of the *Borrelia burgdorferi* OspA protein.

7. The polypeptide of Claim 1, wherein the polypeptide is derived from an OspA protein of a *sensu stricto* strain of *Borrelia burgdorferi.*

8. A polypeptide, comprising an amino acid sequence of OspA protein from a *sensu stricto* strain of *Borrelia burgdorferi* from about residue 160 to about residue 170, wherein the sequence includes at least one alteration selected from the group consisting of: residue 165 being phenylalanine, residue 166 being threonine, residue 170 being lysine and combinations thereof, wherein the numbering corresponds to the numbering of SEQ ID NO:7.

9. The polypeptide of Claim 8, wherein cross-reactivity to an hLFA-1 molecule is reduced compared to the corresponding unaltered OspA polypeptide.

10. The polypeptide of Claim 8, wherein residue 165 is phenylalanine and residue 166 is threonine.

11. The polypeptide of Claim 8, wherein residue 165 is phenylalanine, residue 166 is threonine and residue 170 is lysine.

12. The polypeptide of Claim 8, wherein residue 139 is methionine, residue 160 is tyrosine and residue 189 is methionine.

13. The polypeptide of Claim 8, wherein the polypeptide comprises residues 150 through 180 of the *Borrelia burgdorferi* OspA protein.

14. The polypeptide of Claim 8, wherein the polypeptide comprises residues 17 through 273 of the *Borrelia burgdorferi* OspA protein.

15. A polypeptide selected from the group consisting of: SEQ ID NO:96,98, 100, 102, 104, 106, 108, 110, 112, 114 or 116.

16. A polynucleotide encoding an amino acid sequence of an OspA protein from *Borrelia burgdorferi* from about residue 139 to about residue 273, wherein the sequence encodes at least one alteration selected from the group consisting of: codon 139 encoding methionine, codon 160 encoding tyrosine, codon 189 encoding methionine and combinations thereof, wherein the numbering corresponds to the numbering of SEQ ID NO:7.

17. The polynucleotide of Claim 16, wherein codon 160 encodes tyrosine and codon 189 encodes methionine.

18. The polynucleotide of Claim 16, wherein codon 139 encodes methionine, codon 160 encodes tyrosine and codon 189 encodes methionine.

19. The polynucleotide of Claim 16, wherein the encoded polypeptide comprises residues 131 through 273 of the *Borrelia burgdorferi* OspA protein.

20. The polynucleotide of Claim 16, wherein the encoded polypeptide comprises residues 17 through 273 of the *Borrelia burgdorferi* OspA protein.

21. The polynucleotide of Claim 16, wherein the encoded polypeptide corresponds to OspA protein of a *sensu stricto* strain *of Borrelia burgdorferi.*

22. A polynucleotide encoding an amino acid sequence of an OspA protein from *Borrelia burgdorferi* from about residue 160 to about residue 170, wherein the sequence encodes at least one alteration selected from the group consisting of: codon 165 encoding phenylalanine, codon 166 encoding threonine, codon 170 encoding lysine and combinations thereof, wherein the numbering corresponds to the numbering of SEQ ID NO:7.

23. The polynucleotide of Claim 22, wherein codon 165 encodes phenylalanine and codon 166 encodes threonine.

24. The polynucleotide of Claim 22, wherein codon 165 encodes phenylalanine, codon 166 encodes threonine and codon 170 encodes lysine.

25. A polynucleotide selected from the group consisting of: SEQ ID NO:95, 97, 99, 101, 103, 105, 107, 109, 111, 113 and 115.

26. A method of generating an altered *Borrelia burgdorferi* OspA polypeptide with increased conformational stability compared to the corresponding unaltered *Borrelia burgdorferi* OspA polypeptide comprising;
a) selecting a polynucleotide encoding a *Borrelia burgdorferi* OspA polypeptide that includes residues 139, 160 and 189, wherein the numbering corresponds to the numbering of SEQ m NO:7;
b) altering the polynucleotide such that residue 139 is methionine, residue 160 is tyrosine and residue 189 is methionine; and
c) expressing said altered polynucleotide;
thereby generating an altered *Borrelia burgdorferi* OspA polypeptide with increased conformational stability compared to the corresponding unaltered *Borrelia burgdorferi* OspA polypeptide.

27. A method of generating an altered *Borrelia burgdorferi* OspA polypeptide with reduced cross-reactivity with an hLFA-1 molecule, compared to the corresponding unaltered *Borrelia burgdorferi* OspA polypeptide, comprising;
a) selecting a polynucleotide encoding an OspA polypeptide from *Borrelia burgdorferi* that includes residues 165, 166 and 170, wherein the numbering corresponds to the numbering of SEQ ID NO:7;
b) altering the polynucleotide such that residue 165 is phenylalanine, residue 166 is tyrosine and residue 170 is lysine ; and
c) expressing said altered polynucleotide;
thereby generating an altered *Borrelia burgdorferi* OspA polypeptide with reduced cross-reactivity with the hLFA-1 molecule compared to the corresponding unaltered *Borrelia burgdorferi* OspA polypeptide.

28. Use of an altered OspA polypeptide for the manufacture of a medicament for immunizing a mammal, *e.g.* a human against Lyme disease, wherein the altered OspA polypeptide comprises either
a) an amino acid sequence of OspA protein from *Borrelia burgdorferi* from about residue 139 to about residue 273 *of Borrelia burgdorferi* OspA protein, wherein the sequence includes at least one alteration selected from the group consisting of: residue 139 being methionine, residue 160 being tyrosine, residue 189 being methionine and combinations thereof, wherein the polypeptide has increased conformational stability compared to a corresponding wild type OspA polypeptide, or
b) an amino acid sequence of OspA protein from *Borrelia bufgdorferi* from about residue 160 to about residue 170, wherein the sequence includes at least one alteration selected from the group consisting of: residue 165 being phenylalanine, residue 166 being threonine, residue 170 being lysine and combinations thereof, wherein the polypeptide has decreased cross-reactivity to hLFA-1 compared to a corresponding wild type OspA polypeptide;
wherein the numbering corresponds to the numbering of SEQ ID NO:7.

29. A polypeptide, comprising an amino acid sequence of OspA protein from *Borrelia burgdorferi* from about residue 160 to about residue 170, wherein the sequence includes at least two alterations selected from the group consisting of: residue 165 being phenylalanine, residue 166 being threonine and residue 170 being lysine, wherein the numbering corresponds to the numbering of SEQ ID NO:7.

30. A chimeric polypeptide, comprising:
a) an amino acid sequence of a first OspA polypeptide from about residue 1 to about residue 164 from a first strain of *Borrelia burgdorferi;*
b) an amino acid sequence of a second OspA polypeptide from about residue 165 to about residue 179 from a second strain of *Borrelia burgdorferi,* wherein said second strain is a different strain from said first strain;
c) an amino acid sequence of a third OspA polypeptide from about residue 180 to about residue 216 from a third strain of *Borrelia burgdorferi,* wherein said third strain is a different strain from said second strain;
d) an amino acid sequence of a fourth OspA polypeptide from about residue 217 to about residue 273 from a fourth strain *of Borrelia burgdorferi,* wherein said fourth strain is a different strain from said third strain;
wherein the sequence includes at least one alteration selected from the group consisting of: residue 139 being methionine, residue 160 being tyrosine, residue 189 being methionine and combinations thereof, wherein the numbering corresponds to the numbering of SEQ ID NO:7.


### Annex to the application documents - subsequently filed sequences listing

SEQUENCE LISTING

<110> Research Foundation of the State University of New York
      Brookhaven Sciences Associates, LLC
      University of Rochester

<120> Altered OspA of Borrelia burgdorferi

<130> 15939EP div1

<140> 07011369.1
<141> 2001-08-17

<150> US 60/226,484
<151> 2000-08-18

<160> 130

<170> FastSEQ for Windows Version 4.0

<210> 1
<211> 23
<212> DNA
<213> Artificial Sequence

<220>
<223> Oligonucleotide Primer

<400> 1
cttaatgact ctgacactag tgc                                              23

<210> 2
<211> 30
<212> DNA
<213> Artificial Sequence

<220>
<223> Oligonucleotide Primer

<400> 2
gctactaaaa aaaccgggaa atggaattca                                       30

<210> 3
<211> 33
<212> DNA
<213> Artificial Sequence

<220>
<223> Oligonucleotide Primer

<400> 3
gcagcttggg attcaaaaac atccacttta aca                                   33

<210> 4
<211> 18
<212> DNA
<213> Artificial Sequence

<220>
<223> Oligonucleotide Primer

<400> 4
ggagaatata ttatgaaa                                                    18

<210> 5
<211> 17
<212> DNA

<213> Artificial Sequence

<220>
<223> Oligonucleotide Primer

<400> 5
ctccttattt taaagcg                                                    17

<210> 6
<211> 822
<212> DNA
<213> Borrelia burgdorferi

<220>
<221> CDS
<222> (1)...(822)

<400> 6
atg aaa aaa tat tta ttg gga ata ggt cta ata tta gcc tta ata gca      48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta      96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala

195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 7
<211> 273
<212> PRT
<213> Borrelia burgdorferi

<400> 7
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 8

<211> 825
<212> DNA
<213> Borrelia burgdorferi

<220>
<221> CDS
<222> (1)...(825)

<400> 8
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gat gaa aaa aat agc gtt tca gta     96
Cys Lys Gln Asn Val Ser Ser Leu Asp Glu Lys Asn Ser Val Ser Val
            20                  25                  30

gat tta cct ggt gga atg aca gtt ctt gta agt aaa gaa aaa gac aaa     144
Asp Leu Pro Gly Gly Met Thr Val Leu Val Ser Lys Glu Lys Asp Lys
        35                  40                  45

gac ggt aaa tac agt cta gag gca aca gta gac aag ctt gag ctt aaa     192
Asp Gly Lys Tyr Ser Leu Glu Ala Thr Val Asp Lys Leu Glu Leu Lys
    50                  55                  60

gga act tct gat aaa aac aac ggt tct gga aca ctt gaa ggt gaa aaa     240
Gly Thr Ser Asp Lys Asn Asn Gly Ser Gly Thr Leu Glu Gly Glu Lys
 65                  70                  75                  80

act gac aaa agt aaa gta aaa tta aca att gct gat gac cta agt caa     288
Thr Asp Lys Ser Lys Val Lys Leu Thr Ile Ala Asp Asp Leu Ser Gln
                 85                  90                  95

act aaa ttt gaa att ttc aaa gaa gat gcc aaa aca tta gta tca aaa     336
Thr Lys Phe Glu Ile Phe Lys Glu Asp Ala Lys Thr Leu Val Ser Lys
            100                 105                 110

aaa gta acc ctt aaa gac aag tca tca aca gaa gaa aaa ttc aac gaa     384
Lys Val Thr Leu Lys Asp Lys Ser Ser Thr Glu Glu Lys Phe Asn Glu
        115                 120                 125

aag ggt gaa aca tct gaa aaa aca ata gta aga gca aat gga acc aga     432
Lys Gly Glu Thr Ser Glu Lys Thr Ile Val Arg Ala Asn Gly Thr Arg
    130                 135                 140

ctt gaa tac aca gac ata aaa agc gat gga tcc gga aaa gct aaa gaa     480
Leu Glu Tyr Thr Asp Ile Lys Ser Asp Gly Ser Gly Lys Ala Lys Glu
145                 150                 155                 160

gtt tta aaa gac ttt act ctt gaa gga act cta gct gct gac ggc aaa     528
Val Leu Lys Asp Phe Thr Leu Glu Gly Thr Leu Ala Ala Asp Gly Lys
                165                 170                 175

aca aca ttg aaa gtt aca gaa ggc act gtt gtt tta agc aag aac att     576
Thr Thr Leu Lys Val Thr Glu Gly Thr Val Val Leu Ser Lys Asn Ile
            180                 185                 190

tta aaa tcc gga gaa ata aca gtt gca ctt gat gac tct gac act act     624
Leu Lys Ser Gly Glu Ile Thr Val Ala Leu Asp Asp Ser Asp Thr Thr
        195                 200                 205

cag gct act aaa aaa act gga aaa tgg gat tca aaa act tcc act tta     672
Gln Ala Thr Lys Lys Thr Gly Lys Trp Asp Ser Lys Thr Ser Thr Leu
    210                 215                 220

aca att agt gtg aat agc caa aaa acc aaa aac ctt gta ttc aca aaa     720
Thr Ile Ser Val Asn Ser Gln Lys Thr Lys Asn Leu Val Phe Thr Lys
225                 230                 235                 240

gaa gac aca ata aca gta caa aaa tac gac tca gca ggc acc aat cta     768
Glu Asp Thr Ile Thr Val Gln Lys Tyr Asp Ser Ala Gly Thr Asn Leu
                245                 250                 255

gaa ggc aaa gca gtc gaa att aca aca ctt aaa gaa ctt aaa aac gct     816
Glu Gly Lys Ala Val Glu Ile Thr Thr Leu Lys Glu Leu Lys Asn Ala
            260                 265                 270

tta aaa taa                                                         825
Leu Lys  *

<210> 9
<211> 274
<212> PRT
<213> Borrelia burgdorferi

<400> 9
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Gly Met Thr Val Leu Val Ser Lys Glu Lys Asp Lys
        35                  40                  45
Asp Gly Lys Tyr Ser Leu Glu Ala Thr Val Asp Lys Leu Glu Leu Lys
    50                  55                  60
Gly Thr Ser Asp Lys Asn Asn Gly Ser Gly Thr Leu Glu Gly Glu Lys
65                  70                  75                  80
Thr Asp Lys Ser Lys Val Lys Leu Thr Ile Ala Asp Asp Leu Ser Gln
                85                  90                  95
Thr Lys Phe Glu Ile Phe Lys Glu Asp Ala Lys Thr Leu Val Ser Lys
            100                 105                 110
Lys Val Thr Leu Lys Asp Lys Ser Ser Thr Glu Glu Lys Phe Asn Glu
        115                 120                 125
Lys Gly Glu Thr Ser Glu Lys Thr Ile Val Arg Ala Asn Gly Thr Arg
    130                 135                 140
Leu Glu Tyr Thr Asp Ile Lys Ser Asp Gly Ser Gly Lys Ala Lys Glu
145                 150                 155                 160
Val Leu Lys Asp Phe Thr Leu Glu Gly Thr Leu Ala Ala Asp Gly Lys
                165                 170                 175
Thr Thr Leu Lys Val Thr Glu Gly Thr Val Val Leu Ser Lys Asn Ile
            180                 185                 190
Leu Lys Ser Gly Glu Ile Thr Val Ala Leu Asp Asp Ser Asp Thr Thr
        195                 200                 205
Gln Ala Thr Lys Lys Thr Gly Lys Trp Asp Ser Lys Thr Ser Thr Leu
    210                 215                 220
Thr Ile Ser Val Asn Ser Gln Lys Thr Lys Asn Leu Val Phe Thr Lys
225                 230                 235                 240
Glu Asp Thr Ile Thr Val Gln Lys Tyr Asp Ser Ala Gly Thr Asn Leu
                245                 250                 255
Glu Gly Lys Ala Val Glu Ile Thr Thr Leu Lys Glu Leu Lys Asn Ala
            260                 265                 270
Leu Lys

<210> 10
<211> 822
<212> DNA
<213> Borrelia burgdorferi

<220>
<221> CDS
<222> (1)...(822)

<400> 10

atg aaa aaa tat tta ttg gga ata ggt cta ata tta gcc tta ata gca     48
Met Lys Lys Tyr Leu Leu Gly Ile Gly Leu Ile Leu Ala Leu Ile Ala
1               5                   10                  15

tgc aag caa aat gtt agc agc ctt gat gaa aaa aac agc gct tca gta     96
Cys Lys Gln Asn Val Ser Ser Leu Asp Glu Lys Asn Ser Ala Ser Val
            20                  25                  30

gat ttg cct ggt gag atg aaa gtt ctt gta agt aaa gaa aaa gac aaa     144
Asp Leu Pro Gly Glu Met Lys Val Leu Val Ser Lys Glu Lys Asp Lys
        35                  40                  45

gac ggt aag tac agt cta aag gca aca gta gac aag att gag cta aaa     192
Asp Gly Lys Tyr Ser Leu Lys Ala Thr Val Asp Lys Ile Glu Leu Lys
    50                  55                  60

gga act tct gat aaa gac aat ggt tct gga gtg ctt gaa ggt aca aaa     240
Gly Thr Ser Asp Lys Asp Asn Gly Ser Gly Val Leu Glu Gly Thr Lys
65                  70                  75                  80

gat gac aaa agt aaa gca aaa tta aca att gct gac gat cta agt aaa     288
Asp Asp Lys Ser Lys Ala Lys Leu Thr Ile Ala Asp Asp Leu Ser Lys
                85                  90                  95

acc aca ttc gaa ctt tta aaa gaa gat ggc aaa aca tta gtg tca aga     336
Thr Thr Phe Glu Leu Leu Lys Glu Asp Gly Lys Thr Leu Val Ser Arg
            100                 105                 110

aaa gta agt tct aga gac aaa aca tca aca gat gaa atg ttc aat gaa     384
Lys Val Ser Ser Arg Asp Lys Thr Ser Thr Asp Glu Met Phe Asn Glu
        115                 120                 125

aaa ggt gaa ttg tct gca aaa acc atg aca aga gaa aat gga acc aaa     432
Lys Gly Glu Leu Ser Ala Lys Thr Met Thr Arg Glu Asn Gly Thr Lys
    130                 135                 140

ctt gaa tat aca gaa atg aaa agc gat gga acc gga aaa gct aaa gaa     480
Leu Glu Tyr Thr Glu Met Lys Ser Asp Gly Thr Gly Lys Ala Lys Glu
145                 150                 155                 160

gtt tta aaa aag ttt act ctt gaa gga aaa gta gct aat gat aaa gta     528
Val Leu Lys Lys Phe Thr Leu Glu Gly Lys Val Ala Asn Asp Lys Val
                165                 170                 175

aca ttg gaa gta aaa gaa gga acc gtt act tta agt aag gaa att gca     576
Thr Leu Glu Val Lys Glu Gly Thr Val Thr Leu Ser Lys Glu Ile Ala
            180                 185                 190

aaa tct gga gaa gta aca gtt gct ctt aat gac act aac act act cag     624
Lys Ser Gly Glu Val Thr Val Ala Leu Asn Asp Thr Asn Thr Thr Gln
        195                 200                 205

gct act aaa aaa act ggc gca tgg gat tca aaa act tct act tta aca     672
Ala Thr Lys Lys Thr Gly Ala Trp Asp Ser Lys Thr Ser Thr Leu Thr
    210                 215                 220

att agt gtt aac agc aaa aaa act aca caa ctt gtg ttt act aaa caa     720
Ile Ser Val Asn Ser Lys Lys Thr Thr Gln Leu Val Phe Thr Lys Gln
225                 230                 235                 240

tac aca ata act gta aaa caa tac gac tcc gca ggt acc aat tta gaa     768
Tyr Thr Ile Thr Val Lys Gln Tyr Asp Ser Ala Gly Thr Asn Leu Glu
                245                 250                 255

ggc aca gca gtc gaa att aaa aca ctt gat gaa ctt aaa aac gct tta     816
Gly Thr Ala Val Glu Ile Lys Thr Leu Asp Glu Leu Lys Asn Ala Leu
            260                 265                 270

aaa taa                                                          822
Lys  *

<210> 11
<211> 273
<212> PRT
<213> Borrelia burgdorferi

<400> 11
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Ala Ser Val
            20                  25                  30
Asp Leu Pro Gly Glu Met Lys Val Leu Val Ser Lys Glu Lys Asp Lys
        35                  40                  45
Asp Gly Lys Tyr Ser Leu Lys Ala Thr Val Asp Lys Ile Glu Leu Lys
    50                  55                  60
Gly Thr Ser Asp Lys Asp Asn Gly Ser Gly Val Leu Glu Gly Thr Lys
65                  70                  75                  80
Asp Asp Lys Ser Lys Ala Lys Leu Thr Ile Ala Asp Asp Leu Ser Lys
                85                  90                  95
Thr Thr Phe Glu Leu Leu Lys Glu Asp Gly Lys Thr Leu Val Ser Arg
            100                 105                 110
Lys Val Ser Ser Arg Asp Lys Thr Ser Thr Asp Glu Met Phe Asn Glu
        115                 120                 125
Lys Gly Glu Leu Ser Ala Lys Thr Met Thr Arg Glu Asn Gly Thr Lys
    130                 135                 140
Leu Glu Tyr Thr Glu Met Lys Ser Asp Gly Thr Gly Lys Ala Lys Glu
145                 150                 155                 160
Val Leu Lys Lys Phe Thr Leu Glu Gly Lys Val Ala Asn Asp Lys Val
                165                 170                 175
Thr Leu Glu Val Lys Glu Gly Thr Val Thr Leu Ser Lys Glu Ile Ala
            180                 185                 190
Lys Ser Gly Glu Val Thr Val Ala Leu Asn Asp Thr Asn Thr Thr Gln
        195                 200                 205
Ala Thr Lys Lys Thr Gly Ala Trp Asp Ser Lys Thr Ser Thr Leu Thr
    210                 215                 220
Ile Ser Val Asn Ser Lys Lys Thr Thr Gln Leu Val Phe Thr Lys Gln
225                 230                 235                 240
Tyr Thr Ile Thr Val Lys Gln Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Lys

<210> 12
<211> 819
<212> DNA
<213> Borrelia burgdorferi

<220>
<221> CDS
<222> (1)...(819)

<400> 12
atg aaa aaa tat tta ttg gga ata ggt cta ata tta gct tta ata gca      48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta      96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa gac aaa     144

Asp Leu Pro Gly Glu Met Lys Val Leu Val Ser Lys Glu Lys Asp Lys
        35                  40                  45

gac ggc aag tac agt cta atg gca aca gta gac aag ctt gag ctt aaa     192
Asp Gly Lys Tyr Ser Leu Met Ala Thr Val Asp Lys Leu Glu Leu Lys
    50                  55                  60

gga aca tct gat aaa aac aat gga tct ggg gtg ctt gaa ggc gta aaa     240
Gly Thr Ser Asp Lys Asn Asn Gly Ser Gly Val Leu Glu Gly Val Lys
65                  70                  75                  80

gct gac aaa agc aaa gta aaa tta aca gtt tct gac gat cta agc aca     288
Ala Asp Lys Ser Lys Val Lys Leu Thr Val Ser Asp Asp Leu Ser Thr
                85                  90                  95

acc aca ctt gaa gtt tta aaa gaa gat ggc aaa aca tta gtg tca aaa     336
Thr Thr Leu Glu Val Leu Lys Glu Asp Gly Lys Thr Leu Val Ser Lys
            100                 105                 110

aaa aga act tct aaa gat aag tca tca aca gaa gaa aag ttc aat gaa     384
Lys Arg Thr Ser Lys Asp Lys Ser Ser Thr Glu Glu Lys Phe Asn Glu
        115                 120                 125

aaa ggc gaa tta gtt gaa aaa ata atg gca aga gca aac gga acc ata     432
Lys Gly Glu Leu Val Glu Lys Ile Met Ala Arg Ala Asn Gly Thr Ile
    130                 135                 140

ctt gaa tac aca gga att aaa agc gat gga tcc gga aaa gct aaa gaa     480
Leu Glu Tyr Thr Gly Ile Lys Ser Asp Gly Ser Gly Lys Ala Lys Glu
145                 150                 155                 160

act tta aaa gaa tat gtt ctt gaa gga act cta act gct gaa aaa gca     528
Thr Leu Lys Glu Tyr Val Leu Glu Gly Thr Leu Thr Ala Glu Lys Ala
                165                 170                 175

aca ttg gtg gtt aaa gaa gga act gtt act tta agt aag cac att tca     576
Thr Leu Val Val Lys Glu Gly Thr Val Thr Leu Ser Lys His Ile Ser
            180                 185                 190

aaa tct gga gaa gta aca gct gaa ctt aat gac act gac agt act caa     624
Lys Ser Gly Glu Val Thr Ala Glu Leu Asn Asp Thr Asp Ser Thr Gln
        195                 200                 205

gct act aaa aaa act ggg aaa tgg gat gca ggc act tca act tta aca     672
Ala Thr Lys Lys Thr Gly Lys Trp Asp Ala Gly Thr Ser Thr Leu Thr
    210                 215                 220

att act gta aac aac aaa aaa act aaa gcc ctt gta ttt aca aaa caa     720
Ile Thr Val Asn Asn Lys Lys Thr Lys Ala Leu Val Phe Thr Lys Gln
225                 230                 235                 240

gac aca att aca tca caa aaa tac gac tca gca gga acc aac ttg gaa     768
Asp Thr Ile Thr Ser Gln Lys Tyr Asp Ser Ala Gly Thr Asn Leu Glu
                245                 250                 255

ggc aca gca gtc gaa att aaa aca ctt gat gaa ctt aaa aac gct tta     816
Gly Thr Ala Val Glu Ile Lys Thr Leu Asp Glu Leu Lys Asn Ala Leu
            260                 265                 270

aga                                                                 819
Arg

<210> 13
<211> 273
<212> PRT
<213> Borrelia burgdorferi

<400> 13
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asp Lys
        35                  40                  45
Asp Gly Lys Tyr Ser Leu Met Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Val Ser Asp Asp Leu Ser Thr
                85                  90                  95
Thr Thr Leu Glu Val Leu Lys Glu Asp Gly Lys Thr Leu Val Ser Lys
            100                 105                 110
Lys Arg Thr Ser Lys Asp Lys Ser Ser Thr Glu Glu Lys Phe Asn Glu
        115                 120                 125
Lys Gly Glu Leu Val Glu Lys Ile Met Ala Arg Ala Asn Gly Thr Ile
    130                 135                 140
Leu Glu Tyr Thr Gly Ile Lys Ser Asp Gly Ser Gly Lys Ala Lys Glu
145                 150                 155                 160
Thr Leu Lys Glu Tyr Val Leu Glu Gly Thr Leu Thr Ala Glu Lys Ala
                165                 170                 175
Thr Leu Val Val Lys Glu Gly Thr Val Thr Leu Ser Lys His Ile Ser
            180                 185                 190
Lys Ser Gly Glu Val Thr Ala Glu Leu Asn Asp Thr Asp Ser Thr Gln
        195                 200                 205
Ala Thr Lys Lys Thr Gly Lys Trp Asp Ala Gly Thr Ser Thr Leu Thr
    210                 215                 220
Ile Thr Val Asn Asn Lys Lys Thr Lys Ala Leu Val Phe Thr Lys Gln
225                 230                 235                 240
Asp Thr Ile Thr Ser Gln Lys Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Arg

<210> 14
<211> 21
<212> DNA
<213> Artificial Sequence

<220>
<223> Oligonucleotide Primer

<400> 14
gtctgcaaaa accatgacaa g                                                21

<210> 15
<211> 22
<212> DNA
<213> Artificial Sequence

<220>
<223> Oligonucleotide Primer

<400> 15
gtcatcaaca gaagaaaaat tc                                               22

<210> 16
<211> 30
<212> DNA
<213> Artificial Sequence

<220>

<223> Oligonucleotide Primer

<400> 16
ccggatccat atgaaaaaat atttattggg                                        30

<210> 17
<211> 32
<212> DNA
<213> Artificial Sequence

<220>
<223> Oligonucleotide Primer

<400> 17
ccgggatcca tatggctaag caaaatgtta gc                                     32

<210> 18
<211> 18
<212> DNA
<213> Artificial Sequence

<220>
<223> Oligonucleotide Primer

<400> 18
gcgttcaagt actccaga                                                     18

<210> 19
<211> 27
<212> DNA
<213> Artificial Sequence

<220>
<223> Oligonucleotide Primer

<400> 19
gatatctaga tcttatttta aagcgtt                                           27

<210> 20
<211> 31
<212> DNA
<213> Artificial Sequence

<220>
<223> Oligonucleotide Primer

<400> 20
ggatccggtg accttttaaa gcgtttttaa t                                      31

<210> 21
<211> 825
<212> DNA
<213> Borrelia burgdorferi

<400> 21
atgaaaaaat atttattggg aataggtcta atattagcct taatagcatg taagcaaaat 60
gttagcagcc ttgatgaaaa aaatagcgtt tcagtagatt tacctggtgg aatgacagtt 120
cttgtaagta aagaaaaaga caaagacggt aaatacagtc tagaggcaac agtagacaag 180
cttgagctta aaggaacttc tgataaaaac aacggttctg gaacacttga aggtgaaaaa 240
actgacaaaa gtaaagtaaa atcaacaatt gctgatgacc taagtcaaac taaatttgaa 300
attttcaaag aagatggcaa aacattagta tcaaaaaaag taacccttaa agacaagtca 360
tcaacagaag aaaaattcaa cggaaagggt gaaacatctg aaaaaacaat agtaagagca 420
aatggaacca gacttgaata cacagacata aaaagcgatg gatccggaaa agctaaagaa 480
gttttaaaag actttactct tgaaggaact ctagctgctg acggcaaaac aacattgaaa 540
gttacagaag gcactgttgt tttaagcaag aacattttaa aatccggaga aataacagct 600
gcacttgatg actctgacac tactcgggct actaaaaaaa ctggaaaatg ggattcaaag 660
acttccactt taacaattag tgtgaatagc caaaaaacca aaaaccttgt attcacaaaa 720
gaagacacaa taacagtaca aagatacgac tcagcaggca ccaatctaga aggcaaagca 780

gtcgaaatta caacacttaa agaacttaaa aacgctttaa aataa 825

<210> 22
<211> 824
<212> DNA
<213> Borrelia burgdorferi

<400> 22
atgaaaaaat atttattggg aataggtcta atattagcat taatagcatg taagcaaaat 60
gttagcagcc ttgatgaaaa aaatagcgtt tcagtagatt tacctggtgg aatgcaagtt 120
cttgtaagta aagaaaaaga caaagatggt aaatacagtc taatggcaac agtagacaag 180
cttgagctta aaggaacttc tgataaaaac aacggttctg gaacacttga aggtgaaaaa 240
actgacaaaa gtaaagcaaa attaacaatt gctgaggatc taagtaaaac cacatttgaa 300
atcttcaaag aagatggcaa aacattagta tcaaaaaaag taacccttaa agacaagtca 360
tcaacagaag aaaaattcaa cgcaaagggt gaagcatctg aaaaaacaat agtaagagca 420
aatggaacca gacttgaata cacagacata aaaagcgata aaaccggaaa agctaaagaa 480
gttttaaaag actttgctct tgaaggaact ctagctgctg acggcaaaac aacattaaaa 540
gttacagaag gcactgttgt tttaagcaaa cacatttcaa actctggaga aataacagtt 600
gagcttaatg actctgacac tactcaggct actaaaaaaa ctggaacatg ggattcaaag 660
acttccactt taacaattag tgtgaatagc cgaaaaacca aaaaccttgt attcacaaaa 720
gaagacacaa taacagtaca aaaatacgac tcagcaggca ccaatctaga aggcaaagca 780
gtcgaaatta caacgcttaa agaacttaaa gatgctttaa aata 824

<210> 23
<211> 821
<212> DNA
<213> Borrelia burgdorferi

<400> 23
atgaaaaaat atttattggg aataggtcta atattagcct taatagcatg caagcaaaat 60
gttagcagcc ttgatgaaaa aaacagcgct tcagtagatt tgcctggtga gatgaaagtt 120
cttgtaagta aagaaaaaga caaagacggt aagtacagtc taaaggcaac agtagacaag 180
attgagctaa aaggaacttc tgataaagac aatggttctg ggtgcttgga aggtacaaaa 240
gatgacaaaa gtaaagcaaa attaacaatt gctgacgatc taggtaaaac cacattcgaa 300
cttttcaaag aagatggcaa aacattagtg tcaagaaaag taagttctaa agacaaaaca 360
tcaacagatg aaatgttcaa tgaaaaaggt gaattgtctg caaaaaccat gacaagagaa 420
aatggaacca aacttgaata tacagaaatg aaaagcgatg gaaccggaaa agctaaagaa 480
gttttaaaaa actttactct tgaaggaaaa gtagctaatg ataaagtaac attggaagta 540
aaagaaggaa ccgttacttt aagtaaggaa attgcaaaat ctggagaagt aacagttgct 600
cttaatgaca ctaacactac tcaggctact aaaaaaactg gcgcatggga ttcaaaaact 660
tctactttaa caattagtgt taacagcaaa aaaactacac aacttgtgtt tactaaacaa 720
gacacaataa ctgtacaaaa atacgactcc gcaggtacca atttagaagg cacagcagtc 780
gaaattaaaa cacttgatga acttaaaaac gctttaaaat a 821

<210> 24
<211> 821
<212> DNA
<213> Borrelia burgdorferi

<400> 24
atgaaaaaat atttattggg aataggtcta atattagcct taatagcatg caagcaaaat 60
gttagcagcc ttgatgaaaa aaacagcgct tcagtagatt tgcctggtga gattaaagtt 120
cttgtaagta aagaaaaaga caaagacggt aagtacagtc taaaggcaac agtagacaag 180
attgagctaa aaggaacttc tgataaagac aatggttctg gagtgcttga aggtacaaaa 240
gatgacaaaa gtaaagcaaa attaacaatt gctgacgatc taagtaaaac cacattcgaa 300
cttttcaaag aagatggcaa aacattagtg tcaagaaaag taagttctaa agacaaaaca 360
tcaacagatg aaatgttcaa tgaaaaaggt gaattgtctg caaaaaccat gacaagagaa 420
aatggaacca aacttgaata tacagaaatg aaaagcgatg gaaccggaaa agctaaagaa 480
gttttaaaaa actttactct tgaaggaaaa gtagctaatg ataaagtaac attggaagta 540
aaagaaggaa ccgttacttt aagtaaggaa attgcaaaat ctggagaagt aacagttgct 600
cttaatgaca ctaacactac tcaggctact aaaaaaactg gcgcatggga ttcaaaaact 660
tctactttaa caattagtgt taacagtaaa aaaactacac aacttgtgtt tactaaacaa 720
gacacaataa ctgtacaaaa atacgactcc gcaggtacca atttagaagg cacagcagtc 780
gaaattaaaa cacttgatga acttaaaaac gctttaaaat a 821

<210> 25
<211> 821
<212> DNA

<213> Borrelia burgdorferi

<400> 25
atgaaaaaat atttattggg aataggtcta atattagcct taatagcatg caagcaaaat 60
gttagcagcc ttgatgaaaa aaacagcgct tcagtagatt tgcctggtga gatgaaagtt 120
cttgtaagta aagaaaaaga caaagacggt aagtacagtc taaaggcaac agtagacaag 180
attgagctaa aaggaacttc tgataaagac aatggttctg gagtgcttga aggtacaaaa 240
gatgacaaaa gtaaagcaaa attaacaatt gctgacgatc taggtaaaac cacattcgaa 300
cttttcaaag aagatggcaa aacattagtg tcaagaaaag taagttctaa agacaaaaca 360
tcaacagatg aaatgttcaa tgaaaaaggt gaattgtctg caaaaaccat gacaagagaa 420
aatggaacca aacttgaata tacagaaatg aaaagcgatg gaaccggaaa agctaaagaa 480
gttttaaaaa actttactct tgaaggaaaa gtagctaatg ataaagtaac attggaagta 540
aaagaaggaa ccgttacttt aagtaaggaa attgcaaaat ctggagaagt aacagttgct 600
cttaatgaca ctaacactac tcaggctact aaaaaaactg gcgcatggga ttcaaaaact 660
tctactttaa caattagtgt taacagcaaa aaaactacac aacttgtgtt tactaaacaa 720
gacacaataa ctgtacaaaa atacgactcc gcaggtacca atttagaagg cacagcagtc 780
gaaattaaaa cacttgatga acttaaaaac gctttaaaat a 821

<210> 26
<211> 822
<212> DNA
<213> Borrelia burgdorferi

<400> 26
atgaaaaaat atttattggg aataggtcta atattagcct taatagcatg caagcaaaat 60
gttagcagcc ttgatgaaaa aaacagcgct tcagtagatt tgcctggtga gatgaaagtt 120
cttgtaagta aagaaaaaga caaagacggt aagtacagtc taaaggcaac agtagacaag 180
attgagctaa aaggaacttc tgataaagac aatggttctg gagtgcttga aggtacaaaa 240
gatgacaaaa gtaaagcaaa attaacaatt gctgacgatc taagtaaaac cacattcgaa 300
cttttcaaag aagatggcaa aacattagtg tcaagaaaag taagttctaa agacaaaaca 360
tcaacagatg aaatgttcaa tgaaaaaggt gaattgtctg caaaaaccat gacaagagaa 420
aatggaacca aacttgaata tacagaaatg aaaagcgatg gaaccggaaa agctaaagaa 480
gttttaaaaa actttactct tgaaggaaaa gtagctaatg ataaagtaac attggaagta 540
aaagaaggaa ccgttacttt aagtaaggaa attgcaaaat ctggagaagt aacagttgct 600
cttaatgaca ctaacactac tcaggctact aaaaaaactg gcgcatggga ttcaaaaact 660
tctactttaa caattagtgt taacagcaaa aaaactacac aacttgtgtt tactaaacaa 720
gacacaataa ctgtacaaaa atacgactcc gcaggtacca atttagaagg cacagcagtc 780
gaaattaaaa cacttgatga acttaaaaac gctttgaaat aa 822

<210> 27
<211> 38
<212> DNA
<213> Artificial Sequence

<220>
<223> Oligonucleotide Primer

<400> 27
aaagtagaag tttttgaatc ccattttcca gttttttt 38

<210> 28
<211> 825
<212> DNA
<213> Artificial Sequence

<220>
<223> Chimeric nucleic acid

<221> CDS
<222> (1)...(825)

<400> 28
atg aaa aaa tat tta ttg gga ata ggt cta ata tta gcc tta ata gca 48
Met Lys Lys Tyr Leu Leu Gly Ile Gly Leu Ile Leu Ala Leu Ile Ala
1               5                   10                  15

tgt aag caa aat gtt agc agc ctt gat gaa aaa aat agc gtt tca gta 96
Cys Lys Gln Asn Val Ser Ser Leu Asp Glu Lys Asn Ser Val Ser Val

20                      25                      30

gat tta cct ggt gga atg aca gtt ctt gta agt aaa gaa aaa gac aaa      144
Asp Leu Pro Gly Gly Met Thr Val Leu Val Ser Lys Glu Lys Asp Lys
            35                  40                  45

gac ggt aaa tac agt cta gag gca aca gta gac aag ctt gag ctt aaa      192
Asp Gly Lys Tyr Ser Leu Glu Ala Thr Val Asp Lys Leu Glu Leu Lys
    50                  55                  60

gga act tct gat aaa aac aac ggt tct gga aca ctt gaa ggt gaa aaa      240
Gly Thr Ser Asp Lys Asn Asn Gly Ser Gly Thr Leu Glu Gly Glu Lys
65                  70                  75                  80

act gac aaa agt aaa gta aaa tta aca att gct gat gac cta agt caa      288
Thr Asp Lys Ser Lys Val Lys Leu Thr Ile Ala Asp Asp Leu Ser Gln
                85                  90                  95

act aaa ttt gaa att ttc aaa gaa gat gcc aaa aca tta gta tca aaa      336
Thr Lys Phe Glu Ile Phe Lys Glu Asp Ala Lys Thr Leu Val Ser Lys
            100                 105                 110

aaa gta acc ctt aaa gac aag tca tca aca gaa gaa aaa ttc aac gaa      384
Lys Val Thr Leu Lys Asp Lys Ser Ser Thr Glu Glu Lys Phe Asn Glu
        115                 120                 125

aag ggt gaa aca tct gaa aaa aca ata gta aga gca aat gga acc aga      432
Lys Gly Glu Thr Ser Glu Lys Thr Ile Val Arg Ala Asn Gly Thr Arg
    130                 135                 140

ctt gaa tac aca gac ata aaa agc gat gga tcc gga aaa gct aaa gaa      480
Leu Glu Tyr Thr Asp Ile Lys Ser Asp Gly Ser Gly Lys Ala Lys Glu
145                 150                 155                 160

gtt tta aaa gac ttt act ctt gaa gga act cta gct gct gac ggc aaa      528
Val Leu Lys Asp Phe Thr Leu Glu Gly Thr Leu Ala Ala Asp Gly Lys
                165                 170                 175

aca aca ttg aaa gtt aca gaa ggc act gtt gtt tta agc aag aac att      576
Thr Thr Leu Lys Val Thr Glu Gly Thr Val Val Leu Ser Lys Asn Ile
            180                 185                 190

tta aaa tcc gga gaa ata aca gtt gca ctt gat gac tct gac act act      624
Leu Lys Ser Gly Glu Ile Thr Val Ala Leu Asp Asp Ser Asp Thr Thr
        195                 200                 205

cag gct act aaa aaa act gga aaa tgg gat tca aaa act tct act tta      672
Gln Ala Thr Lys Lys Thr Gly Lys Trp Asp Ser Lys Thr Ser Thr Leu
    210                 215                 220

aca att agt gtt aac agc aaa aaa act aca caa ctt gtg ttt act aaa      720
Thr Ile Ser Val Asn Ser Lys Lys Thr Thr Gln Leu Val Phe Thr Lys
225                 230                 235                 240

caa tac aca ata act gta aaa caa tac gac tcc gca ggt acc aat tta      768
Gln Tyr Thr Ile Thr Val Lys Gln Tyr Asp Ser Ala Gly Thr Asn Leu
                245                 250                 255

gaa ggc aca gca gtc gaa att aaa aca ctt gat gaa ctt aaa aac gct      816
Glu Gly Thr Ala Val Glu Ile Lys Thr Leu Asp Glu Leu Lys Asn Ala
            260                 265                 270

tta aaa taa                                                          825
Leu Lys  *

<210> 29

<211> 274
<212> PRT
<213> Artificial Sequence

<220>
<223> Chimeric protein

<400> 29
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Gly Met Thr Val Leu Val Ser Lys Glu Lys Asp Lys
        35                  40                  45
Asp Gly Lys Tyr Ser Leu Glu Ala Thr Val Asp Lys Leu Glu Leu Lys
    50                  55                  60
Gly Thr Ser Asp Lys Asn Asn Gly Ser Gly Thr Leu Glu Gly Glu Lys
65                  70                  75                  80
Thr Asp Lys Ser Lys Val Lys Leu Thr Ile Ala Asp Asp Leu Ser Gln
                85                  90                  95
Thr Lys Phe Glu Ile Phe Lys Glu Asp Ala Lys Thr Leu Val Ser Lys
            100                 105                 110
Lys Val Thr Leu Lys Asp Lys Ser Ser Thr Glu Glu Lys Phe Asn Glu
        115                 120                 125
Lys Gly Glu Thr Ser Glu Lys Thr Ile Val Arg Ala Asn Gly Thr Arg
    130                 135                 140
Leu Glu Tyr Thr Asp Ile Lys Ser Asp Gly Ser Gly Lys Ala Lys Glu
145                 150                 155                 160
Val Leu Lys Asp Phe Thr Leu Glu Gly Thr Leu Ala Ala Asp Gly Lys
                165                 170                 175
Thr Thr Leu Lys Val Thr Glu Gly Thr Val Val Leu Ser Lys Asn Ile
            180                 185                 190
Leu Lys Ser Gly Glu Ile Thr Val Ala Leu Asp Asp Ser Asp Thr Thr
        195                 200                 205
Gln Ala Thr Lys Lys Thr Gly Lys Trp Asp Ser Lys Thr Ser Thr Leu
    210                 215                 220
Thr Ile Ser Val Asn Ser Lys Lys Thr Thr Gln Leu Val Phe Thr Lys
225                 230                 235                 240
Gln Tyr Thr Ile Thr Val Lys Gln Tyr Asp Ser Ala Gly Thr Asn Leu
                245                 250                 255
Glu Gly Thr Ala Val Glu Ile Lys Thr Leu Asp Glu Leu Lys Asn Ala
            260                 265                 270
Leu Lys



<210> 30
<211> 822
<212> DNA
<213> Artificial Sequence

<220>
<221> CDS
<222> (1)...(822)

<223> Chimeric nucleic acid

<400> 30
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
1               5                   10                  15

tgc aag caa aat gtt agc agc ctt gat gaa aaa aac agc gct tca gta     96
Cys Lys Gln Asn Val Ser Ser Leu Asp Glu Lys Asn Ser Ala Ser Val
            20                  25                  30

gat ttg cct ggt gag atg aaa gtt ctt gta agt aaa gaa aaa gac aaa     144
Asp Leu Pro Gly Glu Met Lys Val Leu Val Ser Lys Glu Lys Asp Lys

        35                  40                  45

gac ggt aag tac agt cta aag gca aca gta gac aag att gag cta aaa 192
Asp Gly Lys Tyr Ser Leu Lys Ala Thr Val Asp Lys Ile Glu Leu Lys
    50                  55                  60

gga act tct gat aaa gac aat ggt tct gga gtg ctt gaa ggt aca aaa 240
Gly Thr Ser Asp Lys Asp Asn Gly Ser Gly Val Leu Glu Gly Thr Lys
65                  70                  75                  80

gat gac aaa agt aaa gca aaa tta aca att gct gac gat cta agt aaa 288
Asp Asp Lys Ser Lys Ala Lys Leu Thr Ile Ala Asp Asp Leu Ser Lys
                85                  90                  95

acc aca ttc gaa ctt tta aaa gaa gat ggc aaa aca tta gtg tca aga 336
Thr Thr Phe Glu Leu Leu Lys Glu Asp Gly Lys Thr Leu Val Ser Arg
            100                 105                 110

aaa gta agt tct aga gac aaa aca tca aca gat gaa atg ttc aat gaa 384
Lys Val Ser Ser Arg Asp Lys Thr Ser Thr Asp Glu Met Phe Asn Glu
        115                 120                 125

aaa ggt gaa ttg tct gca aaa acc atg aca aga gaa aat gga acc aaa 432
Lys Gly Glu Leu Ser Ala Lys Thr Met Thr Arg Glu Asn Gly Thr Lys
    130                 135                 140

ctt gaa tat aca gaa atg aaa agc gat gga acc gga aaa gct aaa gaa 480
Leu Glu Tyr Thr Glu Met Lys Ser Asp Gly Thr Gly Lys Ala Lys Glu
145                 150                 155                 160

gtt tta aaa aag ttt act ctt gaa gga aaa gta gct aat gat aaa gta 528
Val Leu Lys Lys Phe Thr Leu Glu Gly Lys Val Ala Asn Asp Lys Val
                165                 170                 175

aca ttg gaa gta aaa gaa gga acc gtt act tta agt aag gaa att gca 576
Thr Leu Glu Val Lys Glu Gly Thr Val Thr Leu Ser Lys Glu Ile Ala
            180                 185                 190

aaa tct gga gaa gta aca gtt gct ctt aat gac act aac act act cag 624
Lys Ser Gly Glu Val Thr Val Ala Leu Asn Asp Thr Asn Thr Thr Gln
        195                 200                 205

gct act aaa aaa act ggc gca tgg gat tca aaa act tct act tta aca 672
Ala Thr Lys Lys Thr Gly Ala Trp Asp Ser Lys Thr Ser Thr Leu Thr
    210                 215                 220

att agt gtt aac agc aaa aaa act aca caa ctt gtg ttt act aaa caa 720
Ile Ser Val Asn Ser Lys Lys Thr Thr Gln Leu Val Phe Thr Lys Gln
225                 230                 235                 240

tac aca ata act gta aaa caa tac gac tcc gca ggt acc aat tta gaa 768
Tyr Thr Ile Thr Val Lys Gln Tyr Asp Ser Ala Gly Thr Asn Leu Glu
                245                 250                 255

ggc aca gca gtc gaa att aaa aca ctt gat gaa ctt aaa aac gct tta 816
Gly Thr Ala Val Glu Ile Lys Thr Leu Asp Glu Leu Lys Asn Ala Leu
            260                 265                 270

aaa taa 822
Lys *

<210> 31
<211> 273
<212> PRT
<213> Artificial Sequence

<220>
<223> Chimeric protein

<400> 31
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Ala Ser Val
            20                  25                  30
Asp Leu Pro Gly Glu Met Lys Val Leu Val Ser Lys Glu Lys Asp Lys
        35                  40                  45
Asp Gly Lys Tyr Ser Leu Lys Ala Thr Val Asp Lys Ile Glu Leu Lys
    50                  55                  60
Gly Thr Ser Asp Lys Asp Asn Gly Ser Gly Val Leu Glu Gly Thr Lys
65                  70                  75                  80
Asp Asp Lys Ser Lys Ala Lys Leu Thr Ile Ala Asp Asp Leu Ser Lys
                85                  90                  95
Thr Thr Phe Glu Leu Leu Lys Glu Asp Gly Lys Thr Leu Val Ser Arg
            100                 105                 110
Lys Val Ser Ser Arg Asp Lys Thr Ser Thr Asp Glu Met Phe Asn Glu
        115                 120                 125
Lys Gly Glu Leu Ser Ala Lys Thr Met Thr Arg Glu Asn Gly Thr Lys
    130                 135                 140
Leu Glu Tyr Thr Glu Met Lys Ser Asp Gly Thr Gly Lys Ala Lys Glu
145                 150                 155                 160
Val Leu Lys Lys Phe Thr Leu Glu Gly Lys Val Ala Asn Asp Lys Val
                165                 170                 175
Thr Leu Glu Val Lys Glu Gly Thr Val Thr Leu Ser Lys Glu Ile Ala
            180                 185                 190
Lys Ser Gly Glu Val Thr Val Ala Leu Asn Asp Thr Asn Thr Thr Gln
        195                 200                 205
Ala Thr Lys Lys Thr Gly Ala Trp Asp Ser Lys Thr Ser Thr Leu Thr
    210                 215                 220
Ile Ser Val Asn Ser Lys Lys Thr Thr Gln Leu Val Phe Thr Lys Gln
225                 230                 235                 240
Tyr Thr Ile Thr Val Lys Gln Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Lys

<210> 32
<211> 822
<212> DNA
<213> Artificial Sequence

<220>
<223> Chimeric nucleic acid

<221> CDS
<222> (1)...(822)

<400> 32
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
1               5                   10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys

50                  55                  60

gga act tct gat aaa aac aat gga tct gga gta ctt gaa ggc gta aaa      240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa      288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa      336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa      384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga      432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag      480
Leu Glu Tyr Thr Gly Ile Lys Ser Asp Gly Ser Gly Lys Ala Lys Glu
145                 150                 155                 160

gtt tta aaa ggc tat gtt ctt gaa gga act cta act gct gaa aaa aca      528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca      576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct      624
Lys Ser Gly Glu Val Ser Val Glu Leu Asn Asp Thr Asp Ser Ser Ala
        195                 200                 205

gct act aaa aaa act gca gct tgg aat tca aaa act tcc act tta aca      672
Ala Thr Lys Lys Thr Ala Ala Trp Asn Ser Lys Thr Ser Thr Leu Thr
    210                 215                 220

att agt gtg aat agc caa aaa acc aaa aac ctt gta ttc aca aaa gaa      720
Ile Ser Val Asn Ser Gln Lys Thr Lys Asn Leu Val Phe Thr Lys Glu
225                 230                 235                 240

gac aca ata aca gta caa aaa tac gac tca gca ggc acc aat cta gaa      768
Asp Thr Ile Thr Val Gln Lys Tyr Asp Ser Ala Gly Thr Asn Leu Glu
                245                 250                 255

ggc aaa gca gtc gaa att aca aca ctt aaa gaa ctt aaa aac gct tta      816
Gly Lys Ala Val Glu Ile Thr Thr Leu Lys Glu Leu Lys Asn Ala Leu
            260                 265                 270

aaa taa                                                              822
Lys  *

<210> 33
<211> 273
<212> PRT
<213> Artificial Sequence

<220>
<223> Chimeric protein

<400> 33

Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Lys Thr Ser Thr Leu Thr
    210                 215                 220
Ile Ser Val Asn Ser Gln Lys Thr Lys Asn Leu Val Phe Thr Lys Glu
225                 230                 235                 240
Asp Thr Ile Thr Val Gln Lys Tyr Asp Ser Ala Gly Thr Asn Leu Glu
                245                 250                 255
Gly Lys Ala Val Glu Ile Thr Thr Leu Lys Glu Leu Lys Asn Ala Leu
            260                 265                 270
Lys

<210> 34
<211> 819
<212> DNA
<213> Artificial Sequence

<220>
<223> Chimeric nucleic acid

<221> CDS
<222> (1)...(819)

<400> 34
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
1               5                   10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys

65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat gca ggc act tca act tta aca     672
Ala Thr Lys Lys Thr Ala Ala Trp Asn Ala Gly Thr Ser Thr Leu Thr
    210                 215                 220

att act gta aac aac aaa aaa act aaa gcc ctt gta ttt aca aaa caa     720
Ile Thr Val Asn Asn Lys Lys Thr Lys Ala Leu Val Phe Thr Lys Gln
225                 230                 235                 240

gac aca att aca tca caa aaa tac gac tca gca gga acc aac ttg gaa     768
Asp Thr Ile Thr Ser Gln Lys Tyr Asp Ser Ala Gly Thr Asn Leu Glu
                245                 250                 255

ggc aca gca gtc gaa att aaa aca ctt gat gaa ctt aaa aac gct tta     816
Gly Thr Ala Val Glu Ile Lys Thr Leu Asp Glu Leu Lys Asn Ala Leu
            260                 265                 270

aga                                                                 819
Arg

<210> 35
<211> 273
<212> PRT
<213> Artificial Sequence

<220>
<223> Chimeric protein

<400> 35
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30

Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ala Gly Thr Ser Thr Leu Thr
    210                 215                 220
Ile Thr Val Asn Asn Lys Lys Thr Lys Ala Leu Val Phe Thr Lys Gln
225                 230                 235                 240
Asp Thr Ile Thr Ser Gln Lys Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Arg

<210> 36
<211> 822
<212> DNA
<213> Artificial Sequence

<220>
<223> Chimeric nucleic acid

<221> CDS
<222> (1)...(822)

<400> 36
atg aaa aaa tat tta ttg gga ata ggt cta ata tta gcc tta ata gca      48
Met Lys Lys Tyr Leu Leu Gly Ile Gly Leu Ile Leu Ala Leu Ile Ala
 1               5                  10                  15

tgt aag caa aat gtt agc agc ctt gat gaa aaa aat agc gtt tca gta      96
Cys Lys Gln Asn Val Ser Ser Leu Asp Glu Lys Asn Ser Val Ser Val
            20                  25                  30

gat tta cct ggt gga atg aca gtt ctt gta agt aaa gaa aaa gac aaa     144
Asp Leu Pro Gly Gly Met Thr Val Leu Val Ser Lys Glu Lys Asp Lys
        35                  40                  45

gac ggt aaa tac agt cta gag gca aca gta gac aag ctt gag ctt aaa     192
Asp Gly Lys Tyr Ser Leu Glu Ala Thr Val Asp Lys Leu Glu Leu Lys
    50                  55                  60

gga act tct gat aaa aac aac ggt tct gga aca ctt gaa ggt gaa aaa     240
Gly Thr Ser Asp Lys Asn Asn Gly Ser Gly Thr Leu Glu Gly Glu Lys
65                  70                  75                  80

act gac aaa agt aaa gta aaa tta aca att gct gat gac cta agt caa     288
Thr Asp Lys Ser Lys Val Lys Leu Thr Ile Ala Asp Asp Leu Ser Gln

                85                  90                  95

act aaa ttt gaa att ttc aaa gaa gat gcc aaa aca tta gta tca aaa 336
Thr Lys Phe Glu Ile Phe Lys Glu Asp Ala Lys Thr Leu Val Ser Lys
            100                 105                 110

aaa gta acc ctt aaa gac aag tca tca aca gaa gaa aaa ttc aac gaa 384
Lys Val Thr Leu Lys Asp Lys Ser Ser Thr Glu Glu Lys Phe Asn Glu
        115                 120                 125

aag ggt gaa aca tct gaa aaa aca ata gta aga gca aat gga acc aga 432
Lys Gly Glu Thr Ser Glu Lys Thr Ile Val Arg Ala Asn Gly Thr Arg
    130                 135                 140

ctt gaa tac aca gac ata aaa agc gat gga tcc gga aaa gct aaa gaa 480
Leu Glu Tyr Thr Asp Ile Lys Ser Asp Gly Ser Gly Lys Ala Lys Glu
145                 150                 155                 160

gtt tta aaa gac ttt act ctt gaa gga act cta gct gct gac ggc aaa 528
Val Leu Lys Asp Phe Thr Leu Glu Gly Thr Leu Ala Ala Asp Gly Lys
                165                 170                 175

aca aca ttg aaa gtt aca gaa ggc act gtt gtt tta agc aag att tca 576
Thr Thr Leu Lys Val Thr Glu Gly Thr Val Val Leu Ser Lys Ile Ser
            180                 185                 190

aaa tct ggg gaa gtt tca gtt gaa ctt aat gac act gac agt agt gct 624
Lys Ser Gly Glu Val Ser Val Glu Leu Asn Asp Thr Asp Ser Ser Ala
        195                 200                 205

gct act aaa aaa act gca gct tgg aat tca aaa act tcc act tta aca 672
Ala Thr Lys Lys Thr Ala Ala Trp Asn Ser Lys Thr Ser Thr Leu Thr
    210                 215                 220

att agt gtg aat agc caa aaa acc aaa aac ctt gta ttc aca aaa gaa 720
Ile Ser Val Asn Ser Gln Lys Thr Lys Asn Leu Val Phe Thr Lys Glu
225                 230                 235                 240

gac aca ata aca gta caa aaa tac gac tca gca ggc acc aat cta gaa 768
Asp Thr Ile Thr Val Gln Lys Tyr Asp Ser Ala Gly Thr Asn Leu Glu
                245                 250                 255

ggc aaa gca gtc gaa att aca aca ctt aaa gaa ctt aaa aac gct tta 816
Gly Lys Ala Val Glu Ile Thr Thr Leu Lys Glu Leu Lys Asn Ala Leu
            260                 265                 270

aaa taa 822
Lys *

<210> 37
<211> 273
<212> PRT
<213> Artificial Sequence

<220>
<223> Chimeric protein

<400> 37
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Gly Met Thr Val Leu Val Ser Lys Glu Lys Asp Lys
        35                  40                  45
Asp Gly Lys Tyr Ser Leu Glu Ala Thr Val Asp Lys Leu Glu Leu Lys
    50                  55                  60

Gly Thr Ser Asp Lys Asn Asn Gly Ser Gly Thr Leu Glu Gly Glu Lys
65                  70                  75                  80
Thr Asp Lys Ser Lys Val Lys Leu Thr Ile Ala Asp Asp Leu Ser Gln
                85                  90                  95
Thr Lys Phe Glu Ile Phe Lys Glu Asp Ala Lys Thr Leu Val Ser Lys
            100                 105                 110
Lys Val Thr Leu Lys Asp Lys Ser Ser Thr Glu Glu Lys Phe Asn Glu
        115                 120                 125
Lys Gly Glu Thr Ser Glu Lys Thr Ile Val Arg Ala Asn Gly Thr Arg
    130                 135                 140
Leu Glu Tyr Thr Asp Ile Lys Ser Asp Gly Ser Gly Lys Ala Lys Glu
145                 150                 155                 160
Val Leu Lys Asp Phe Thr Leu Glu Gly Thr Leu Ala Ala Asp Gly Lys
                165                 170                 175
Thr Thr Leu Lys Val Thr Glu Gly Thr Val Val Leu Ser Lys Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Lys Thr Ser Thr Leu Thr
    210                 215                 220
Ile Ser Val Asn Ser Gln Lys Thr Lys Asn Leu Val Phe Thr Lys Glu
225                 230                 235                 240
Asp Thr Ile Thr Val Gln Lys Tyr Asp Ser Ala Gly Thr Asn Leu Glu
                245                 250                 255
Gly Lys Ala Val Glu Ile Thr Thr Leu Lys Glu Leu Lys Asn Ala Leu
            260                 265                 270
Lys

<210> 38
<211> 822
<212> DNA
<213> Artificial Sequence

<220>
<223> Chimeric nucleic acid

<221> CDS
<222> (1)...(822)

<400> 38
atg aaa aaa tat tta ttg gga ata ggt cta ata tta gcc tta ata gca      48
Met Lys Lys Tyr Leu Leu Gly Ile Gly Leu Ile Leu Ala Leu Ile Ala
 1               5                  10                  15

tgt aag caa aat gtt agc agc ctt gat gaa aaa aat agc gtt tca gta      96
Cys Lys Gln Asn Val Ser Ser Leu Asp Glu Lys Asn Ser Val Ser Val
            20                  25                  30

gat tta cct ggt gga atg aca gtt ctt gta agt aaa gaa aaa gac aaa     144
Asp Leu Pro Gly Gly Met Thr Val Leu Val Ser Lys Glu Lys Asp Lys
        35                  40                  45

gac ggt aaa tac agt cta gag gca aca gta gac aag ctt gag ctt aaa     192
Asp Gly Lys Tyr Ser Leu Glu Ala Thr Val Asp Lys Leu Glu Leu Lys
    50                  55                  60

gga act tct gat aaa aac aac ggt tct gga aca ctt gaa ggt gaa aaa     240
Gly Thr Ser Asp Lys Asn Asn Gly Ser Gly Thr Leu Glu Gly Glu Lys
 65                  70                  75                  80

act gac aaa agt aaa gta aaa tta aca att gct gat gac cta agt caa     288
Thr Asp Lys Ser Lys Val Lys Leu Thr Ile Ala Asp Asp Leu Ser Gln
                85                  90                  95

act aaa ttt gaa att ttc aaa gaa gat gcc aaa aca tta gta tca aaa     336
Thr Lys Phe Glu Ile Phe Lys Glu Asp Ala Lys Thr Leu Val Ser Lys

100                 105                 110

aaa gta acc ctt aaa gac aag tca tca aca gaa gaa aaa ttc aac gaa     384
Lys Val Thr Leu Lys Asp Lys Ser Ser Thr Glu Glu Lys Phe Asn Glu
        115                 120                 125

aag ggt gaa aca tct gaa aaa aca ata gta aga gca aat gga acc aga     432
Lys Gly Glu Thr Ser Glu Lys Thr Ile Val Arg Ala Asn Gly Thr Arg
    130                 135                 140

ctt gaa tac aca gac ata aaa agc gat gga tcc gga aaa gct aaa gaa     480
Leu Glu Tyr Thr Asp Ile Lys Ser Asp Gly Ser Gly Lys Ala Lys Glu
145                 150                 155                 160

gtt tta aaa gac ttt act ctt gaa gga act cta gct gct gac ggc aaa     528
Val Leu Lys Asp Phe Thr Leu Glu Gly Thr Leu Ala Ala Asp Gly Lys
                165                 170                 175

aca aca ttg aaa gtt aca gaa ggc act gtt gtt tta agc aag att tca     576
Thr Thr Leu Lys Val Thr Glu Gly Thr Val Val Leu Ser Lys Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca aaa act tcc act tta aca     672
Ala Thr Lys Lys Thr Ala Ala Trp Asn Ser Lys Thr Ser Thr Leu Thr
    210                 215                 220

att agt gtg aat agc caa aaa acc aaa aac ctt gta ttc aca aaa gaa     720
Ile Ser Val Asn Ser Gln Lys Thr Lys Asn Leu Val Phe Thr Lys Glu
225                 230                 235                 240

gac aca ata aca gta caa aaa tac gac tca gca ggc acc aat cta gaa     768
Asp Thr Ile Thr Val Gln Lys Tyr Asp Ser Ala Gly Thr Asn Leu Glu
                245                 250                 255

ggc aaa gca gtc gaa att aca aca ctt aaa gaa ctt aaa aac gct tta     816
Gly Lys Ala Val Glu Ile Thr Thr Leu Lys Glu Leu Lys Asn Ala Leu
            260                 265                 270

aaa taa                                                             822
Lys  *

<210> 39
<211> 273
<212> PRT
<213> Artificial Sequence

<220>
<223> Chimeric protein

<400> 39
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Gly Met Thr Val Leu Val Ser Lys Glu Lys Asp Lys
        35                  40                  45
Asp Gly Lys Tyr Ser Leu Glu Ala Thr Val Asp Lys Leu Glu Leu Lys
    50                  55                  60
Gly Thr Ser Asp Lys Asn Asn Gly Ser Gly Thr Leu Glu Gly Glu Lys
65                  70                  75                  80
Thr Asp Lys Ser Lys Val Lys Leu Thr Ile Ala Asp Asp Leu Ser Gln
                85                  90                  95

Thr Lys Phe Glu Ile Phe Lys Glu Asp Ala Lys Thr Leu Val Ser Lys
            100                 105                 110
Lys Val Thr Leu Lys Asp Lys Ser Ser Thr Glu Glu Lys Phe Asn Glu
        115                 120                 125
Lys Gly Glu Thr Ser Glu Lys Thr Ile Val Arg Ala Asn Gly Thr Arg
    130                 135                 140
Leu Glu Tyr Thr Asp Ile Lys Ser Asp Gly Ser Gly Lys Ala Lys Glu
145                 150                 155                 160
Val Leu Lys Asp Phe Thr Leu Glu Gly Thr Leu Ala Ala Asp Gly Lys
                165                 170                 175
Thr Thr Leu Lys Val Thr Glu Gly Thr Val Val Leu Ser Lys Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Lys Thr Ser Thr Leu Thr
    210                 215                 220
Ile Ser Val Asn Ser Gln Lys Thr Lys Asn Leu Val Phe Thr Lys Glu
225                 230                 235                 240
Asp Thr Ile Thr Val Gln Lys Tyr Asp Ser Ala Gly Thr Asn Leu Glu
                245                 250                 255
Gly Lys Ala Val Glu Ile Thr Thr Leu Lys Glu Leu Lys Asn Ala Leu
            260                 265                 270
Lys

<210> 40
<211> 822
<212> DNA
<213> Artificial Sequence

<220>
<223> Chimeric nucleic acid

<221> CDS
<222> (1)...(822)

<400> 40
atg aaa aaa tat tta ttg gga ata ggt cta ata tta gcc tta ata gca      48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta      96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu

115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat gac agt act agc act tta aca     672
Ala Thr Lys Lys Thr Ala Ala Trp Asn Asp Ser Thr Ser Thr Leu Thr
    210                 215                 220

att agt gct gac agc aaa aaa act aaa gat ttg gtg ttc tta aca gat     720
Ile Ser Ala Asp Ser Lys Lys Thr Lys Asp Leu Val Phe Leu Thr Asp
225                 230                 235                 240

ggt aca att aca gta caa caa tac aac aca gct gga acc agc cta gaa     768
Gly Thr Ile Thr Val Gln Gln Tyr Asn Thr Ala Gly Thr Ser Leu Glu
                245                 250                 255

gga tca gca agt gaa att aaa aat ctt tca gag ctt aaa aac gct tta     816
Gly Ser Ala Ser Glu Ile Lys Asn Leu Ser Glu Leu Lys Asn Ala Leu
            260                 265                 270

aaa taa                                                             822
Lys  *

<210> 41
<211> 273
<212> PRT
<213> Artificial Sequence

<220>
<223> Chimeric protein

<400> 41
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125

Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Asp Ser Thr Ser Thr Leu Thr
    210                 215                 220
Ile Ser Ala Asp Ser Lys Lys Thr Lys Asp Leu Val Phe Leu Thr Asp
225                 230                 235                 240
Gly Thr Ile Thr Val Gln Gln Tyr Asn Thr Ala Gly Thr Ser Leu Glu
                245                 250                 255
Gly Ser Ala Ser Glu Ile Lys Asn Leu Ser Glu Leu Lys Asn Ala Leu
            260                 265                 270
Lys

<210> 42
<211> 822
<212> DNA
<213> Borrelia burgdorferi

<400> 42
atgaaaaaat atttattggg aataggtcta atattagcct taatagcatg taagcaaaat 60
gttagcagcc ttgacgagaa aaacagcgtt tcagtagatt tgcctggtga aatgaaagtt 120
cttgtaagca aagaaaaaaa caaagacggc aagtacgatc taattgcaac agtagacaag 180
cttgagctta aaggaacttc tgataaaaac aatggatctg gagtacttga aggcgtaaaa 240
gctgacaaaa gtaaagtaaa attaacaatt tctgacgatc taggtcaaac cacacttgaa 300
gttttcaaag aagatggcaa aacactagta tcaaaaaaag taacttccaa agacaagtca 360
tcaacagaag aaaaattcaa tgaaaaaggt gaagtatctg aaaaaataat aacaagagca 420
gacggaacca gacttgaata cacaggaatt aaaagcgatg gatctggaaa agctaaagag 480
gttttaaaag gctatgttct tgaaggaact ctaactgctg aaaaaacaac attggtggtt 540
aaagaaggaa ctgttacttt aagcaaaaat atttcaaaat ctggggaagt ttcagttgaa 600
cttaatgaca ctgacagtag tgctgctact aaaaaaactg cagcttggaa ttcaggcact 660
tcaactttaa caattactgt aaacagtaaa aaaactaaag accttgtgtt tacaaaagaa 720
aacacaatta cagtacaaca atacgactca aatggcacca aattagaggg gtcagcagtt 780
gaaattacaa aacttgatga aattaaaaac gctttaaaat aa                    822

<210> 43
<211> 822
<212> DNA
<213> Borrelia burgdorferi

<400> 43
atgaaaaaat atttattggg aataggtcta atattagcct taatagcatg taagcaaaat 60
gttagcagcc ttgacgagaa aaacagcgtt tcagtagatt tgcctggtga aatgaacgtt 120
cttgtaagca aagaaaaaaa caaagacggc aagtacgatc taattgcaac agtagacaag 180
cttgagctta aaggaacttc tgataaaaac aatggatctg gagtacttga aggcgtaaaa 240
gctgacaaaa gtaaagtaaa attaacaatt tctgacgatc taggtcaaac cacacttgaa 300
gttttcaaag aagatggcaa aacactagta tcaaaaaaag taacttccaa agacaagtca 360
tcaacagaag aaaaattcaa tgaaaaaggt gaagtatctg aaaaaataat aacaagagca 420
gacggaacca gacttgaata cacagaaatt aaaagcgatg gatctggaaa agctaaagag 480
gttttaaaag gctatgttct tgaaggaact ctaactgctg aaaaaacaac attggtggtt 540
aaagaaggaa ctgttacttt aagcaaaaat atttcaaaat ctggggaagt ttcagttgaa 600
cttaatgaca ctgacagtag tgctgctact aaaaaaactg cagcttggaa ttcaggcact 660
tcaactttaa caattactgt aaacagtaaa aaaactaaag accttgtgtt tacaaaagaa 720
aacacaatta cagtacaaca atacgactca aatggcacca aattagaggg gtcagcagtt 780
gaaattacaa aacttgatga aattaaaaac gctttaaaat aa                    822

<210> 44
<211> 822
<212> DNA
<213> Borrelia burgdorferi

<400> 44
atgaaaaaat atttattggg aataggtcta atattagcct taatagcatg taagcaaaat 60
gttagcagcc ttgacgagaa aaacagcgtt tcagtagatt tgcctggtga aatgaacgtt 120
cttgtaagca aagaaaaaaa caaagacggc aagtacgatc taattgcaac agtagacaag 180
cttgagctta aaggaacttc tgataaaaac aatggatctg gagtacttga aggcgtaaaa 240
gctgacaaaa gtaaagtaaa attaacaatt tctgacgatc taggtcaaac cacacttgaa 300
gttttcaaag aagatggcaa aacactagta tcaaaaaaag taacttccaa agacaagtca 360
tcaacagaag aaaaattcaa tgaaaaaggt gaagtatctg aaaaaataat aacaagagca 420
gacggaacca gacttgaata cacagaaatt aaaagcgatg gatctggaaa agctaaagag 480
gttttaaaaa gctatgttct tgaaggaact ttaactgctg aaaaaacaac attggtggtt 540
aaagaaggaa ctgttacttt aagcaaaaat atttcaaaat ctggggaagt ttcagttgaa 600
cttaatgaca ctgacagtag tgctgctact aaaaaaactg cagcttggaa ttcaggcact 660
tcaactttaa caattactgt aaacagtaaa aaaactaaag accttgtgtt tacaaaagaa 720
aacacaatta cagtacaaca atacgactca aatggcacca aattagaggg gtcagcagtt 780
gaaattacaa aacttgatga aattaaaaac gctttaaaat aa 822

<210> 45
<211> 821
<212> DNA
<213> Borrelia burgdorferi

<400> 45
atgaaaaaat atttattggg aataggtcta atattagcct taatagcatg taagcaaaat 60
gttagcagcc ttgatgagaa aaacagcgtt tcagtagatt tacctggtga aatgaaagtt 120
cttgtaagca aagaaaaaga caaagatggt aaatacagtc taatggcaac agtagacaag 180
ctagagctta aaggaacttc tgataaaagc aacggttctg gaacacttga aggtgaaaaa 240
tctgacaaaa gtaaagcaaa attaacaatt tctgaagatc taagtaaaac cacatttgaa 300
attttcaaag aagatggcaa aacattagta tcaaaaaaag taaattctaa agataagtca 360
tcaatagaag aaaaattcaa cgcaaaaggt gaattatctg aaaaaacaat actaagagca 420
aacggaacca ggcttgaata cacagaaata aaaagcgatg gaaccggaaa agctaaagaa 480
gctttaaaag actttgctct tgaaggaact ctagctgccg acaaaacaac attgaaagtt 540
acagaaggca ctgttgtttt aagcaaacac attccaaact ctggagaaat aacagttgag 600
cttaatgact ctaactctac tcaggctact aaaaaaactg gaaaatggga ttcaaatact 660
tccactttaa caattagtgt gaatagcaaa aaaactaaaa acattgtatt tacaaaagaa 720
gacacaataa cagtacaaaa atacgactca gcaggcacca atctagaagg caacgcagtc 780
gaaattaaaa cacttgatga acttaaaaac gctttaaaat a 821

<210> 46
<211> 821
<212> DNA
<213> Borrelia burgdorferi

<400> 46
atgaaaaaat atttattggg aataggtcta atattagcct taatagcatg taagcaaaat 60
gttagcagcc ttgatgaaaa aaatagcgtt tcagtagatt tacctggtgg aatgaaagtt 120
cttgtaagta aagaaaaaga caaagatggt aaatacagtc taatggcaac agtagaaaag 180
cttgagctta aaggaacttc tgataaaaac aacggttctg gaacacttga aggtgaaaaa 240
actgacaaaa gtaaagtaaa attaacaatt gctgaggatc taagtaaaac cacatttgaa 300
atcttcaaag aagatggcaa aacattagta tcgaaaaaag taacccttaa agacaagtca 360
tcaacagaag aaaaattcaa cgaaaagggt gaaatatctg aaaaaacaat agtaagagca 420
aatggaacca gacttgaata cacagacata aaaagcgata aaaccggaaa agctaaagaa 480
gttttaaaag actttactct tgaaggaact ctagctgctg acggcaaaac aacattgaaa 540
gttacagagg gcactgttac tttaagcaag aacatttcaa aatccggaga aataacagtt 600
gcacttgatg acactgactc tagcggcaat aaaaaatccg gaacatggga ttcaggtact 660
tctactttaa caattagtaa aaacagacaa aaaactaaac aacttgtatt cacaaaagaa 720
gacacaataa cagtacaaaa ctacgactca gcaggcacca atctagaagg caaagcagtc 780
gaaattacaa cacttaaaga acttaaaaac gctttaaaat a 821

<210> 47
<211> 825
<212> DNA
<213> Artificial Sequence

<220>
<223> Chimeric nucleic acid

<221> CDS

<222> (1)...(825)

<400> 47
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gat gaa aaa aat agc gtt tca gta     96
Cys Lys Gln Asn Val Ser Ser Leu Asp Glu Lys Asn Ser Val Ser Val
            20                  25                  30

gat tta cct ggt gga atg aca gtt ctt gta agt aaa gaa aaa gac aaa    144
Asp Leu Pro Gly Gly Met Thr Val Leu Val Ser Lys Glu Lys Asp Lys
        35                  40                  45

gac ggt aaa tac agt cta gag gca aca gta gac aag ctt gag ctt aaa    192
Asp Gly Lys Tyr Ser Leu Glu Ala Thr Val Asp Lys Leu Glu Leu Lys
    50                  55                  60

gga act tct gat aaa aac aac ggt tct gga aca ctt gaa ggt gaa aaa    240
Gly Thr Ser Asp Lys Asn Asn Gly Ser Gly Thr Leu Glu Gly Glu Lys
65                  70                  75                  80

act gac aaa agt aaa gta aaa tta aca att gct gat gac cta agt caa    288
Thr Asp Lys Ser Lys Val Lys Leu Thr Ile Ala Asp Asp Leu Ser Gln
                85                  90                  95

act aaa ttt gaa att ttc aaa gaa gat gcc aaa aca tta gta tca aaa    336
Thr Lys Phe Glu Ile Phe Lys Glu Asp Ala Lys Thr Leu Val Ser Lys
            100                 105                 110

aaa gta acc ctt aaa gac aag tca tca aca gaa gaa aaa ttc aac gaa    384
Lys Val Thr Leu Lys Asp Lys Ser Ser Thr Glu Glu Lys Phe Asn Glu
        115                 120                 125

aag ggt gaa aca tct gaa aaa aca ata gta aga gca aat gga acc aga    432
Lys Gly Glu Thr Ser Glu Lys Thr Ile Val Arg Ala Asn Gly Thr Arg
    130                 135                 140

ctt gaa tac aca gac ata aaa agc gat gga tcc gga aaa gct aaa gaa    480
Leu Glu Tyr Thr Asp Ile Lys Ser Asp Gly Ser Gly Lys Ala Lys Glu
145                 150                 155                 160

gtt tta aaa gac ttt act ctt gaa gga act cta gct gct gac ggc aaa    528
Val Leu Lys Asp Phe Thr Leu Glu Gly Thr Leu Ala Ala Asp Gly Lys
                165                 170                 175

aca aca ttg aaa gtt aca gaa ggc act gtt gtt tta agc aag aac att    576
Thr Thr Leu Lys Val Thr Glu Gly Thr Val Val Leu Ser Lys Asn Ile
            180                 185                 190

tta aaa tcc gga gaa ata aca gtt gca ctt gat gac tct gac act act    624
Leu Lys Ser Gly Glu Ile Thr Val Ala Leu Asp Asp Ser Asp Thr Thr
        195                 200                 205

cag gct act aaa aaa act gga aaa tgg gat tca aat act tcc act tta    672
Gln Ala Thr Lys Lys Thr Gly Lys Trp Asp Ser Asn Thr Ser Thr Leu
    210                 215                 220

aca att agt gtg aat agc aaa aaa act aaa aac att gta ttt aca aaa    720
Thr Ile Ser Val Asn Ser Lys Lys Thr Lys Asn Ile Val Phe Thr Lys
225                 230                 235                 240

gaa gac aca ata aca gta caa aaa tac gac tca gca ggc acc aat cta    768
Glu Asp Thr Ile Thr Val Gln Lys Tyr Asp Ser Ala Gly Thr Asn Leu
                245                 250                 255

gaa ggc aac gca gtc gaa att aaa aca ctt gat gaa ctt aaa aac gct    816

Glu Gly Asn Ala Val Glu Ile Lys Thr Leu Asp Glu Leu Lys Asn Ala
            260                 265                 270

tta aaa tag                                                       825
Leu Lys  *

<210> 48
<211> 274
<212> PRT
<213> Artificial Sequence

<220>
<223> Chimeric protein

<400> 48
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Gly Met Thr Val Leu Val Ser Lys Glu Lys Asp Lys
        35                  40                  45
Asp Gly Lys Tyr Ser Leu Glu Ala Thr Val Asp Lys Leu Glu Leu Lys
    50                  55                  60
Gly Thr Ser Asp Lys Asn Asn Gly Ser Gly Thr Leu Glu Gly Glu Lys
65                  70                  75                  80
Thr Asp Lys Ser Lys Val Lys Leu Thr Ile Ala Asp Asp Leu Ser Gln
                85                  90                  95
Thr Lys Phe Glu Ile Phe Lys Glu Asp Ala Lys Thr Leu Val Ser Lys
            100                 105                 110
Lys Val Thr Leu Lys Asp Lys Ser Ser Thr Glu Glu Lys Phe Asn Glu
        115                 120                 125
Lys Gly Glu Thr Ser Glu Lys Thr Ile Val Arg Ala Asn Gly Thr Arg
    130                 135                 140
Leu Glu Tyr Thr Asp Ile Lys Ser Asp Gly Ser Gly Lys Ala Lys Glu
145                 150                 155                 160
Val Leu Lys Asp Phe Thr Leu Glu Gly Thr Leu Ala Ala Asp Gly Lys
                165                 170                 175
Thr Thr Leu Lys Val Thr Glu Gly Thr Val Val Leu Ser Lys Asn Ile
            180                 185                 190
Leu Lys Ser Gly Glu Ile Thr Val Ala Leu Asp Asp Ser Asp Thr Thr
        195                 200                 205
Gln Ala Thr Lys Lys Thr Gly Lys Trp Asp Ser Asn Thr Ser Thr Leu
    210                 215                 220
Thr Ile Ser Val Asn Ser Lys Lys Thr Lys Asn Ile Val Phe Thr Lys
225                 230                 235                 240
Glu Asp Thr Ile Thr Val Gln Lys Tyr Asp Ser Ala Gly Thr Asn Leu
                245                 250                 255
Glu Gly Asn Ala Val Glu Ile Lys Thr Leu Asp Glu Leu Lys Asn Ala
            260                 265                 270
Leu Lys

<210> 49
<211> 822
<212> DNA
<213> Artificial Sequence

<220>
<223> Chimeric nucleic acid

<221> CDS
<222> (1)...(822)

<400> 49
atg aaa aaa tat tta ttg gga ata ggt cta ata tta gcc tta ata gca    48

Met Lys Lys Tyr Leu Leu Gly Ile Gly Leu Ile Leu Ala Leu Ile Ala
1               5                   10                  15

tgc aag caa aat gtt agc agc ctt gat gaa aaa aac agc gct tca gta     96
Cys Lys Gln Asn Val Ser Ser Leu Asp Glu Lys Asn Ser Ala Ser Val
            20                  25                  30

gat ttg cct ggt gag atg aaa gtt ctt gta agt aaa gaa aaa gac aaa     144
Asp Leu Pro Gly Glu Met Lys Val Leu Val Ser Lys Glu Lys Asp Lys
        35                  40                  45

gac ggt aag tac agt cta aag gca aca gta gac aag att gag cta aaa     192
Asp Gly Lys Tyr Ser Leu Lys Ala Thr Val Asp Lys Ile Glu Leu Lys
    50                  55                  60

gga act tct gat aaa gac aat ggt tct gga gtg ctt gaa ggt aca aaa     240
Gly Thr Ser Asp Lys Asp Asn Gly Ser Gly Val Leu Glu Gly Thr Lys
65                  70                  75                  80

gat gac aaa agt aaa gca aaa tta aca att gct gac gat cta agt aaa     288
Asp Asp Lys Ser Lys Ala Lys Leu Thr Ile Ala Asp Asp Leu Ser Lys
                85                  90                  95

acc aca ttc gaa ctt tta aaa gaa gat ggc aaa aca tta gtg tca aga     336
Thr Thr Phe Glu Leu Leu Lys Glu Asp Gly Lys Thr Leu Val Ser Arg
            100                 105                 110

aaa gta agt tct aga gac aaa aca tca aca gat gaa atg ttc aat gaa     384
Lys Val Ser Ser Arg Asp Lys Thr Ser Thr Asp Glu Met Phe Asn Glu
        115                 120                 125

aaa ggt gaa ttg tct gca aaa acc atg aca aga gaa aat gga acc aaa     432
Lys Gly Glu Leu Ser Ala Lys Thr Met Thr Arg Glu Asn Gly Thr Lys
    130                 135                 140

ctt gaa tat aca gaa atg aaa agc gat gga acc gga aaa gct aaa gaa     480
Leu Glu Tyr Thr Glu Met Lys Ser Asp Gly Thr Gly Lys Ala Lys Glu
145                 150                 155                 160

gtt tta aaa aag ttt act ctt gaa gga aaa gta gct aat gat aaa gta     528
Val Leu Lys Lys Phe Thr Leu Glu Gly Lys Val Ala Asn Asp Lys Val
                165                 170                 175

aca ttg gaa gta aaa gaa gga acc gtt act tta agt aag gaa att gca     576
Thr Leu Glu Val Lys Glu Gly Thr Val Thr Leu Ser Lys Glu Ile Ala
            180                 185                 190

aaa tct gga gaa gta aca gtt gct ctt aat gac act aac act act cag     624
Lys Ser Gly Glu Val Thr Val Ala Leu Asn Asp Thr Asn Thr Thr Gln
        195                 200                 205

gct act aaa aaa act ggc gca tgg gat tca aaa act tct act tta aca     672
Ala Thr Lys Lys Thr Gly Ala Trp Asp Ser Lys Thr Ser Thr Leu Thr
    210                 215                 220

att agt gtt aac agc aaa aaa act aca caa ctt gtg ttt act aaa caa     720
Ile Ser Val Asn Ser Lys Lys Thr Thr Gln Leu Val Phe Thr Lys Gln
225                 230                 235                 240

gac aca ata act gta caa aaa tac gac tcc gca ggt acc aat tta gaa     768
Asp Thr Ile Thr Val Gln Lys Tyr Asp Ser Ala Gly Thr Asn Leu Glu
                245                 250                 255

ggt aca gca gtc gaa att aaa aca ctt gat gaa ctt aaa aac gct tta     816
Gly Thr Ala Val Glu Ile Lys Thr Leu Asp Glu Leu Lys Asn Ala Leu
            260                 265                 270

aaa tag                                                             822

Lys *

<210> 50
<211> 273
<212> PRT
<213> Artificial Sequence

<220>
<223> Chimeric protein

<400> 50
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Ala Ser Val
            20                  25                  30
Asp Leu Pro Gly Glu Met Lys Val Leu Val Ser Lys Glu Lys Asp Lys
        35                  40                  45
Asp Gly Lys Tyr Ser Leu Lys Ala Thr Val Asp Lys Ile Glu Leu Lys
    50                  55                  60
Gly Thr Ser Asp Lys Asp Asn Gly Ser Gly Val Leu Glu Gly Thr Lys
65                  70                  75                  80
Asp Asp Lys Ser Lys Ala Lys Leu Thr Ile Ala Asp Asp Leu Ser Lys
                85                  90                  95
Thr Thr Phe Glu Leu Leu Lys Glu Asp Gly Lys Thr Leu Val Ser Arg
            100                 105                 110
Lys Val Ser Ser Arg Asp Lys Thr Ser Thr Asp Glu Met Phe Asn Glu
        115                 120                 125
Lys Gly Glu Leu Ser Ala Lys Thr Met Thr Arg Glu Asn Gly Thr Lys
    130                 135                 140
Leu Glu Tyr Thr Glu Met Lys Ser Asp Gly Thr Gly Lys Ala Lys Glu
145                 150                 155                 160
Val Leu Lys Lys Phe Thr Leu Glu Gly Lys Val Ala Asn Asp Lys Val
                165                 170                 175
Thr Leu Glu Val Lys Glu Gly Thr Val Thr Leu Ser Lys Glu Ile Ala
            180                 185                 190
Lys Ser Gly Glu Val Thr Val Ala Leu Asn Asp Thr Asn Thr Thr Gln
        195                 200                 205
Ala Thr Lys Lys Thr Gly Ala Trp Asp Ser Lys Thr Ser Thr Leu Thr
    210                 215                 220
Ile Ser Val Asn Ser Lys Lys Thr Thr Gln Leu Val Phe Thr Lys Gln
225                 230                 235                 240
Asp Thr Ile Thr Val Gln Lys Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Lys

<210> 51
<211> 822
<212> DNA
<213> Artificial Sequence

<220>
<223> Chimeric nucleic acid

<221> CDS
<222> (1)...(822)

<400> 51
atg aaa aaa tat tta ttg gga ata ggt cta ata tta gcc tta ata gca 48
Met Lys Lys Tyr Leu Leu Gly Ile Gly Leu Ile Leu Ala Leu Ile Ala
1               5                   10                  15

tgc aag caa aat gtt agc agc ctt gat gaa aaa aac agc gct tca gta 96

Cys Lys Gln Asn Val Ser Ser Leu Asp Glu Lys Asn Ser Ala Ser Val
            20                  25                  30

gat ttg cct ggt gag atg aaa gtt ctt gta agt aaa gaa aaa gac aaa 144
Asp Leu Pro Gly Glu Met Lys Val Leu Val Ser Lys Glu Lys Asp Lys
        35                  40                  45

gac ggt aag tac agt cta aag gca aca gta gac aag att gag cta aaa 192
Asp Gly Lys Tyr Ser Leu Lys Ala Thr Val Asp Lys Ile Glu Leu Lys
    50                  55                  60

gga act tct gat aaa gac aat ggt tct gga gtg ctt gaa ggt aca aaa 240
Gly Thr Ser Asp Lys Asp Asn Gly Ser Gly Val Leu Glu Gly Thr Lys
65                  70                  75                  80

gat gac aaa agt aaa gca aaa tta aca att gct gac gat cta agt aaa 288
Asp Asp Lys Ser Lys Ala Lys Leu Thr Ile Ala Asp Asp Leu Ser Lys
                85                  90                  95

acc aca ttc gaa ctt tta aaa gaa gat ggc aaa aca tta gtg tca aga 336
Thr Thr Phe Glu Leu Leu Lys Glu Asp Gly Lys Thr Leu Val Ser Arg
            100                 105                 110

aaa gta agt tct aga gac aaa aca tca aca gat gaa atg ttc aat gaa 384
Lys Val Ser Ser Arg Asp Lys Thr Ser Thr Asp Glu Met Phe Asn Glu
        115                 120                 125

aaa ggt gaa ttg tct gca aaa acc atg aca aga gaa aat gga acc aaa 432
Lys Gly Glu Leu Ser Ala Lys Thr Met Thr Arg Glu Asn Gly Thr Lys
    130                 135                 140

ctt gaa tat aca gaa atg aaa agc gat gga acc gga aaa gct aaa gaa 480
Leu Glu Tyr Thr Glu Met Lys Ser Asp Gly Thr Gly Lys Ala Lys Glu
145                 150                 155                 160

gtt tta aaa aag ttt act ctt gaa gga aaa gta gct aat gat aaa gta 528
Val Leu Lys Lys Phe Thr Leu Glu Gly Lys Val Ala Asn Asp Lys Val
                165                 170                 175

aca ttg gaa gta aaa gaa gga acc gtt act tta agt aag gaa att tca 576
Thr Leu Glu Val Lys Glu Gly Thr Val Thr Leu Ser Lys Glu Ile Ser
            180                 185                 190

aaa tct ggg gaa gtt tca gtt gaa ctt aat gac act gac agt agt gct 624
Lys Ser Gly Glu Val Ser Val Glu Leu Asn Asp Thr Asp Ser Ser Ala
        195                 200                 205

gct act aaa aaa act gca gct tgg aat tca aaa act tcc act tta aca 672
Ala Thr Lys Lys Thr Ala Ala Trp Asn Ser Lys Thr Ser Thr Leu Thr
    210                 215                 220

att agt gtg aat agc caa aaa acc aaa aac ctt gta ttc aca aaa gaa 720
Ile Ser Val Asn Ser Gln Lys Thr Lys Asn Leu Val Phe Thr Lys Glu
225                 230                 235                 240

gac aca ata aca gta caa aaa tac gac tca gca ggc acc aat cta gaa 768
Asp Thr Ile Thr Val Gln Lys Tyr Asp Ser Ala Gly Thr Asn Leu Glu
                245                 250                 255

ggc aaa gca gtc gaa att aca aca ctt aaa gaa ctt aaa aac gct tta 816
Gly Lys Ala Val Glu Ile Thr Thr Leu Lys Glu Leu Lys Asn Ala Leu
            260                 265                 270

aaa taa 822
Lys *

<210> 52
<211> 273
<212> PRT
<213> Artificial Sequence

<220>
<223> Chimeric protein

<400> 52
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Ala Ser Val
            20                  25                  30
Asp Leu Pro Gly Glu Met Lys Val Leu Val Ser Lys Glu Lys Asp Lys
        35                  40                  45
Asp Gly Lys Tyr Ser Leu Lys Ala Thr Val Asp Lys Ile Glu Leu Lys
    50                  55                  60
Gly Thr Ser Asp Lys Asp Asn Gly Ser Gly Val Leu Glu Gly Thr Lys
65                  70                  75                  80
Asp Asp Lys Ser Lys Ala Lys Leu Thr Ile Ala Asp Asp Leu Ser Lys
                85                  90                  95
Thr Thr Phe Glu Leu Leu Lys Glu Asp Gly Lys Thr Leu Val Ser Arg
            100                 105                 110
Lys Val Ser Ser Arg Asp Lys Thr Ser Thr Asp Glu Met Phe Asn Glu
        115                 120                 125
Lys Gly Glu Leu Ser Ala Lys Thr Met Thr Arg Glu Asn Gly Thr Lys
    130                 135                 140
Leu Glu Tyr Thr Glu Met Lys Ser Asp Gly Thr Gly Lys Ala Lys Glu
145                 150                 155                 160
Val Leu Lys Lys Phe Thr Leu Glu Gly Lys Val Ala Asn Asp Lys Val
                165                 170                 175
Thr Leu Glu Val Lys Glu Gly Thr Val Thr Leu Ser Lys Glu Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Lys Thr Ser Thr Leu Thr
    210                 215                 220
Ile Ser Val Asn Ser Gln Lys Thr Lys Asn Leu Val Phe Thr Lys Glu
225                 230                 235                 240
Asp Thr Ile Thr Val Gln Lys Tyr Asp Ser Ala Gly Thr Asn Leu Glu
                245                 250                 255
Gly Lys Ala Val Glu Ile Thr Thr Leu Lys Glu Leu Lys Asn Ala Leu
            260                 265                 270
Lys

<210> 53
<211> 822
<212> DNA
<213> Artificial Sequence

<220>
<223> Chimeric nucleic acid

<221> CDS
<222> (1)...(822)

<400> 53
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
1               5                   10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144

Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca aat gga acc aaa     432
Lys Gly Glu Val Ser Glu Lys Ile Ile Thr Arg Ala Asn Gly Thr Lys
    130                 135                 140

ctt gaa tat aca gaa atg aaa agc gat gga acc gga aaa gct aaa gaa     480
Leu Glu Tyr Thr Glu Met Lys Ser Asp Gly Thr Gly Lys Ala Lys Glu
145                 150                 155                 160

gtt tta aaa aag ttt act ctt gaa gga aaa gta gct aat gat aaa gta     528
Val Leu Lys Lys Phe Thr Leu Glu Gly Lys Val Ala Asn Asp Lys Val
                165                 170                 175

aca ttg gaa gta aaa gaa gga acc gtt act tta agt aag gaa att tca     576
Thr Leu Glu Val Lys Glu Gly Thr Val Thr Leu Ser Lys Glu Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca aaa act tcc act tta aca     672
Ala Thr Lys Lys Thr Ala Ala Trp Asn Ser Lys Thr Ser Thr Leu Thr
    210                 215                 220

att agt gtg aat agc caa aaa acc aaa aac ctt gta ttc aca aaa gaa     720
Ile Ser Val Asn Ser Gln Lys Thr Lys Asn Leu Val Phe Thr Lys Glu
225                 230                 235                 240

gac aca ata aca gta caa aaa tac gac tca gca ggc acc aat cta gaa     768
Asp Thr Ile Thr Val Gln Lys Tyr Asp Ser Ala Gly Thr Asn Leu Glu
                245                 250                 255

ggc aaa gca gtc gaa att aca aca ctt aaa gaa ctt aaa aac gct tta     816
Gly Lys Ala Val Glu Ile Thr Thr Leu Lys Glu Leu Lys Asn Ala Leu
            260                 265                 270

aaa taa                                                             822
Lys  *

<210> 54
<211> 273
<212> PRT
<213> Artificial Sequence

<220>
<223> Chimeric protein

<400> 54
Met Lys Lys Tyr Leu Leu Gly Ile Gly Leu Ile Leu Ala Leu Ile Ala
 1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asn Gly Thr Lys
    130                 135                 140
Leu Glu Tyr Thr Glu Met Lys Ser Asp Gly Thr Gly Lys Ala Lys Glu
145                 150                 155                 160
Val Leu Lys Lys Phe Thr Leu Glu Gly Lys Val Ala Asn Asp Lys Val
                165                 170                 175
Thr Leu Glu Val Lys Glu Gly Thr Val Thr Leu Ser Lys Glu Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Lys Thr Ser Thr Leu Thr
    210                 215                 220
Ile Ser Val Asn Ser Gln Lys Thr Lys Asn Leu Val Phe Thr Lys Glu
225                 230                 235                 240
Asp Thr Ile Thr Val Gln Lys Tyr Asp Ser Ala Gly Thr Asn Leu Glu
                245                 250                 255
Gly Lys Ala Val Glu Ile Thr Thr Leu Lys Glu Leu Lys Asn Ala Leu
            260                 265                 270
Lys

<210> 55
<211> 1362
<212> DNA
<213> Artificial Sequence

<220>
<223> Chimeric nucleic acid

<221> CDS
<222> (1)...(1362)

<400> 55
atg gct tgt aat aat tca ggg aaa gat ggg aat aca tct gca aat tct    48
Met Ala Cys Asn Asn Ser Gly Lys Asp Gly Asn Thr Ser Ala Asn Ser
 1               5                   10                  15

gct gat gag tct gtt aaa ggg cct aat ctt aca gaa ata aat aaa aaa    96
Ala Asp Glu Ser Val Lys Gly Pro Asn Leu Thr Glu Ile Asn Lys Lys
            20                  25                  30

att acg gat tct aat gcg gtt tta ctt gct gtg aaa gag gtt gaa gcg    144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa    192

Leu Leu Ser Ser Ile Asp Glu Ile Ala Ala Lys Ala Ile Gly Lys Lys
    50                  55                  60

ata cac caa aat aat ggt ttg gat acc gaa aat aat cac aat gga tca 240
Ile His Gln Asn Asn Gly Leu Asp Thr Glu Asn Asn His Asn Gly Ser
65                  70                  75                  80

ttg tta gcg gga gct tat gca ata tca acc cta ata aaa caa aaa tta 288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag 336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat 384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta 432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta 480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct 528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa 576
Asn Ser Val Lys Glu Leu Thr Ser Pro Val Val Ala Glu Ser Pro Lys
            180                 185                 190

aaa cct tcc atg gcc aag caa aat gtt agc agc ctt gac gag aaa aac 624
Lys Pro Ser Met Ala Lys Gln Asn Val Ser Ser Leu Asp Glu Lys Asn
        195                 200                 205

agc gtt tca gta gat ttg cct ggt gaa atg aaa gtt ctt gta agc aaa 672
Ser Val Ser Val Asp Leu Pro Gly Glu Met Lys Val Leu Val Ser Lys
    210                 215                 220

gaa aaa aac aaa gac ggc aag tac gat cta att gca aca gta gac aag 720
Glu Lys Asn Lys Asp Gly Lys Tyr Asp Leu Ile Ala Thr Val Asp Lys
225                 230                 235                 240

ctt gag ctt aaa gga act tct gat aaa aac aat gga tct gga gta ctt 768
Leu Glu Leu Lys Gly Thr Ser Asp Lys Asn Asn Gly Ser Gly Val Leu
                245                 250                 255

gaa ggc gta aaa gct gac aaa agt aaa gta aaa tta aca att tct gac 816
Glu Gly Val Lys Ala Asp Lys Ser Lys Val Lys Leu Thr Ile Ser Asp
            260                 265                 270

gat cta ggt caa acc aca ctt gaa gtt ttc aaa gaa gat ggc aaa aca 864
Asp Leu Gly Gln Thr Thr Leu Glu Val Phe Lys Glu Asp Gly Lys Thr
        275                 280                 285

cta gta tca aaa aaa gta act tcc aaa gac aag tca tca aca gaa gaa 912
Leu Val Ser Lys Lys Val Thr Ser Lys Asp Lys Ser Ser Thr Glu Glu
    290                 295                 300

aaa ttc aat gaa aaa ggt gaa gta tct gaa aaa ata ata aca aga gca 960
Lys Phe Asn Glu Lys Gly Glu Val Ser Glu Lys Ile Ile Thr Arg Ala
305                 310                 315                 320

gac gga acc aga ctt gaa tac aca gga att aaa agc gat gga tct gga 1008

Asp Gly Thr Arg Leu Glu Tyr Thr Gly Ile Lys Ser Asp Gly Ser Gly
                325                 330                 335

aaa gct aaa gag gtt tta aaa ggc tat gtt ctt gaa gga act cta act      1056
Lys Ala Lys Glu Val Leu Lys Gly Tyr Val Leu Glu Gly Thr Leu Thr
            340                 345                 350

gct gaa aaa aca aca ttg gtg gtt aaa gaa gga act gtt act tta agc      1104
Ala Glu Lys Thr Thr Leu Val Val Lys Glu Gly Thr Val Thr Leu Ser
        355                 360                 365

aaa aat att tca aaa tct ggg gaa gtt tca gtt gaa ctt aat gac act      1152
Lys Asn Ile Ser Lys Ser Gly Glu Val Ser Val Glu Leu Asn Asp Thr
    370                 375                 380

gac agt agt gct gct act aaa aaa act gca gct tgg aat tca ggc act      1200
Asp Ser Ser Ala Ala Thr Lys Lys Thr Ala Ala Trp Asn Ser Gly Thr
385                 390                 395                 400

tca act tta aca att act gta aac agt aaa aaa act aaa gac ctt gtg      1248
Ser Thr Leu Thr Ile Thr Val Asn Ser Lys Lys Thr Lys Asp Leu Val
                405                 410                 415

ttt aca aaa gaa aac aca att aca gta caa caa tac gac tca aat ggc      1296
Phe Thr Lys Glu Asn Thr Ile Thr Val Gln Gln Tyr Asp Ser Asn Gly
            420                 425                 430

acc aaa tta gag ggg tca gca gtt gaa att aca aaa ctt gat gaa att      1344
Thr Lys Leu Glu Gly Ser Ala Val Glu Ile Thr Lys Leu Asp Glu Ile
        435                 440                 445

aaa aac gct tta aaa taa                                              1362
Lys Asn Ala Leu Lys  *
    450


<210> 56
<211> 453
<212> PRT
<213> Artificial Sequence

<220>
<223> Chimeric protein

<400> 56
Met Ala Cys Asn Asn Ser Gly Lys Asp Gly Asn Thr Ser Ala Asn Ser
 1               5                  10                  15
Ala Asp Glu Ser Val Lys Gly Pro Asn Leu Thr Glu Ile Asn Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys

180                 185                 190
Lys Pro Ser Met Ala Lys Gln Asn Val Ser Ser Leu Asp Glu Lys Asn
        195                 200                 205
Ser Val Ser Val Asp Leu Pro Gly Glu Met Lys Val Leu Val Ser Lys
    210                 215                 220
Glu Lys Asn Lys Asp Gly Lys Tyr Asp Leu Ile Ala Thr Val Asp Lys
225                 230                 235                 240
Leu Glu Leu Lys Gly Thr Ser Asp Lys Asn Asn Gly Ser Gly Val Leu
                245                 250                 255
Glu Gly Val Lys Ala Asp Lys Ser Lys Val Lys Leu Thr Ile Ser Asp
            260                 265                 270
Asp Leu Gly Gln Thr Thr Leu Glu Val Phe Lys Glu Asp Gly Lys Thr
        275                 280                 285
Leu Val Ser Lys Lys Val Thr Ser Lys Asp Lys Ser Ser Thr Glu Glu
    290                 295                 300
Lys Phe Asn Glu Lys Gly Glu Val Ser Glu Lys Ile Ile Thr Arg Ala
305                 310                 315                 320
Asp Gly Thr Arg Leu Glu Tyr Thr Gly Ile Lys Ser Asp Gly Ser Gly
                325                 330                 335
Lys Ala Lys Glu Val Leu Lys Gly Tyr Val Leu Glu Gly Thr Leu Thr
            340                 345                 350
Ala Glu Lys Thr Thr Leu Val Val Lys Glu Gly Thr Val Thr Leu Ser
        355                 360                 365
Lys Asn Ile Ser Lys Ser Gly Glu Val Ser Val Glu Leu Asn Asp Thr
    370                 375                 380
Asp Ser Ser Ala Ala Thr Lys Lys Thr Ala Ala Trp Asn Ser Gly Thr
385                 390                 395                 400
Ser Thr Leu Thr Ile Thr Val Asn Ser Lys Lys Thr Lys Asp Leu Val
                405                 410                 415
Phe Thr Lys Glu Asn Thr Ile Thr Val Gln Gln Tyr Asp Ser Asn Gly
            420                 425                 430
Thr Lys Leu Glu Gly Ser Ala Val Glu Ile Thr Lys Leu Asp Glu Ile
        435                 440                 445
Lys Asn Ala Leu Lys
    450

<210> 57
<211> 1353
<212> DNA
<213> Artificial Sequence

<220>
<223> Chimeric nucleic acid

<221> CDS
<222> (1)...(1353)

<400> 57
atg gct tgt aat aat tca ggg aaa gat ggg aat aca tct gca aat tct     48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa     96
Ala Asp Glu Ser Val Lys Gly Pro Asn Leu Thr Glu Ile Asn Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa     192
Leu Leu Ser Ser Ile Asp Glu Ile Ala Ala Lys Ala Ile Gly Lys Lys
    50                  55                  60

ata cac caa aat aat ggt ttg gat acc gaa aat aat cac aat gga tca     240
Ile His Gln Asn Asn Gly Leu Asp Thr Glu Asn Asn His Asn Gly Ser
65                  70                  75                  80

ttg tta gcg gga gct tat gca ata tca acc cta ata aaa caa aaa tta 288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag 336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat 384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta 432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta 480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct 528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca gcc 576
Asn Ser Val Lys Glu Leu Thr Ser Pro Val Val Ala Glu Ser Pro Ala
            180                 185                 190

atg gcc aag caa aat gtt agc agc ctt gac gag aaa aac agc gtt tca 624
Met Ala Lys Gln Asn Val Ser Ser Leu Asp Glu Lys Asn Ser Val Ser
        195                 200                 205

gta gat ttg cct ggt gaa atg aaa gtt ctt gta agc aaa gaa aaa aac 672
Val Asp Leu Pro Gly Glu Met Lys Val Leu Val Ser Lys Glu Lys Asn
    210                 215                 220

aaa gac ggc aag tac gat cta att gca aca gta gac aag ctt gag ctt 720
Lys Asp Gly Lys Tyr Asp Leu Ile Ala Thr Val Asp Lys Leu Glu Leu
225                 230                 235                 240

aaa gga act tct gat aaa aac aat gga tct gga gta ctt gaa ggc gta 768
Lys Gly Thr Ser Asp Lys Asn Asn Gly Ser Gly Val Leu Glu Gly Val
                245                 250                 255

aaa gct gac aaa agt aaa gta aaa tta aca att tct gac gat cta ggt 816
Lys Ala Asp Lys Ser Lys Val Lys Leu Thr Ile Ser Asp Asp Leu Gly
            260                 265                 270

caa acc aca ctt gaa gtt ttc aaa gaa gat ggc aaa aca cta gta tca 864
Gln Thr Thr Leu Glu Val Phe Lys Glu Asp Gly Lys Thr Leu Val Ser
        275                 280                 285

aaa aaa gta act tcc aaa gac aag tca tca aca gaa gaa aaa ttc aat 912
Lys Lys Val Thr Ser Lys Asp Lys Ser Ser Thr Glu Glu Lys Phe Asn
    290                 295                 300

gaa aaa ggt gaa gta tct gaa aaa ata ata aca aga gca gac gga acc 960
Glu Lys Gly Glu Val Ser Glu Lys Ile Ile Thr Arg Ala Asp Gly Thr
305                 310                 315                 320

aga ctt gaa tac aca gga att aaa agc gat gga tct gga aaa gct aaa 1008
Arg Leu Glu Tyr Thr Gly Ile Lys Ser Asp Gly Ser Gly Lys Ala Lys
                325                 330                 335

gag gtt tta aaa ggc tat gtt ctt gaa gga act cta act gct gaa aaa 1056
Glu Val Leu Lys Gly Tyr Val Leu Glu Gly Thr Leu Thr Ala Glu Lys
            340                 345                 350

aca aca ttg gtg gtt aaa gaa gga act gtt act tta agc aaa aat att     1104
Thr Thr Leu Val Val Lys Glu Gly Thr Val Thr Leu Ser Lys Asn Ile
        355                 360                 365

tca aaa tct ggg gaa gtt tca gtt gaa ctt aat gac act gac agt agt     1152
Ser Lys Ser Gly Glu Val Ser Val Glu Leu Asn Asp Thr Asp Ser Ser
    370                 375                 380

gct gct act aaa aaa act gca gct tgg aat tca ggc act tca act tta     1200
Ala Ala Thr Lys Lys Thr Ala Ala Trp Asn Ser Gly Thr Ser Thr Leu
385                 390                 395                 400

aca att act gta aac agt aaa aaa act aaa gac ctt gtg ttt aca aaa     1248
Thr Ile Thr Val Asn Ser Lys Lys Thr Lys Asp Leu Val Phe Thr Lys
                405                 410                 415

gaa aac aca att aca gta caa caa tac gac tca aat ggc acc aaa tta     1296
Glu Asn Thr Ile Thr Val Gln Gln Tyr Asp Ser Asn Gly Thr Lys Leu
            420                 425                 430

gag ggg tca gca gtt gaa att aca aaa ctt gat gaa att aaa aac gct     1344
Glu Gly Ser Ala Val Glu Ile Thr Lys Leu Asp Glu Ile Lys Asn Ala
        435                 440                 445

tta aaa taa                                                         1353
Leu Lys  *
    450


<210> 58
<211> 450
<212> PRT
<213> Artificial Sequence

<220>
<223> Chimeric protein

<400> 58
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Asn Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Ala
            180                 185                 190
Met Ala Lys Gln Asn Val Ser Ser Leu Asp Glu Lys Asn Ser Val Ser
        195                 200                 205
Val Asp Leu Pro Gly Glu Met Lys Val Leu Val Ser Lys Glu Lys Asn
    210                 215                 220
Lys Asp Gly Lys Tyr Asp Leu Ile Ala Thr Val Asp Lys Leu Glu Leu

225                 230                 235                 240
Lys Gly Thr Ser Asp Lys Asn Asn Gly Ser Gly Val Leu Glu Gly Val
                245                 250                 255
Lys Ala Asp Lys Ser Lys Val Lys Leu Thr Ile Ser Asp Asp Leu Gly
            260                 265                 270
Gln Thr Thr Leu Glu Val Phe Lys Glu Asp Gly Lys Thr Leu Val Ser
        275                 280                 285
Lys Lys Val Thr Ser Lys Asp Lys Ser Ser Thr Glu Glu Lys Phe Asn
    290                 295                 300
Glu Lys Gly Glu Val Ser Glu Lys Ile Ile Thr Arg Ala Asp Gly Thr
305                 310                 315                 320
Arg Leu Glu Tyr Thr Gly Ile Lys Ser Asp Gly Ser Gly Lys Ala Lys
                325                 330                 335
Glu Val Leu Lys Gly Tyr Val Leu Glu Gly Thr Leu Thr Ala Glu Lys
            340                 345                 350
Thr Thr Leu Val Val Lys Glu Gly Thr Val Thr Leu Ser Lys Asn Ile
        355                 360                 365
Ser Lys Ser Gly Glu Val Ser Val Glu Leu Asn Asp Thr Asp Ser Ser
    370                 375                 380
Ala Ala Thr Lys Lys Thr Ala Ala Trp Asn Ser Gly Thr Ser Thr Leu
385                 390                 395                 400
Thr Ile Thr Val Asn Ser Lys Lys Thr Lys Asp Leu Val Phe Thr Lys
                405                 410                 415
Glu Asn Thr Ile Thr Val Gln Gln Tyr Asp Ser Asn Gly Thr Lys Leu
            420                 425                 430
Glu Gly Ser Ala Val Glu Ile Thr Lys Leu Asp Glu Ile Lys Asn Ala
        435                 440                 445
Leu Lys
    450


<210> 59
<211> 1341
<212> DNA
<213> Artificial Sequence

<220>
<223> Chimeric nucleic acid

<221> CDS
<222> (1)...(1341)

<400> 59
atg gct tgt aat aat tca ggg aaa gat ggg aat aca tct gca aat tct      48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata agt aaa aaa      96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gag ctt gct aaa gct att ggt aaa aaa ata     192
Leu Leu Ser Ser Ile Asp Glu Leu Ala Lys Ala Ile Gly Lys Lys Ile
    50                  55                  60

aaa aac gat ggt agt tta gat aat gaa gca aat cgc aac gag tca ttg     240
Lys Asn Asp Gly Ser Leu Asp Asn Glu Ala Asn Arg Asn Glu Ser Leu
65                  70                  75                  80

tta gca gga gct tat aca ata tca acc tta ata aca caa aaa tta agt     288
Leu Ala Gly Ala Tyr Thr Ile Ser Thr Leu Ile Thr Gln Lys Leu Ser
                85                  90                  95

aaa tta aac gga tca gaa ggt tta aag gaa aag att gcc gca gct aag     336

Lys Leu Asn Gly Ser Glu Gly Leu Lys Glu Lys Ile Ala Ala Ala Lys
            100                 105                 110

aaa tgc tct gaa gag ttt agt act aaa cta aaa gat aat cat gca cag     384
Lys Cys Ser Glu Glu Phe Ser Thr Lys Leu Lys Asp Asn His Ala Gln
        115                 120                 125

ctt ggt ata cag ggc gtt act gat gaa aat gca aaa aaa gct att tta     432
Leu Gly Ile Gln Gly Val Thr Asp Glu Asn Ala Lys Lys Ala Ile Leu
    130                 135                 140

aaa gca aat gca gcg ggt aaa gat aag ggc gtt gaa gaa ctt gaa aag     480
Lys Ala Asn Ala Ala Gly Lys Asp Lys Gly Val Glu Glu Leu Glu Lys
145                 150                 155                 160

ttg tcc gga tca tta gaa agc tta tca aaa gca gct aaa gag atg ctt     528
Leu Ser Gly Ser Leu Glu Ser Leu Ser Lys Ala Ala Lys Glu Met Leu
                165                 170                 175

gct aat tca gtt aaa gag ctt aca agc cct gtt gtc cat ggc aag caa     576
Ala Asn Ser Val Lys Glu Leu Thr Ser Pro Val Val His Gly Lys Gln
            180                 185                 190

aat gtt agc agc ctt gac gag aaa aac agc gtt tca gta gat ttg cct     624
Asn Val Ser Ser Leu Asp Glu Lys Asn Ser Val Ser Val Asp Leu Pro
        195                 200                 205

ggt gaa atg aaa gtt ctt gta agc aaa gaa aaa aac aaa gac ggc aag     672
Gly Glu Met Lys Val Leu Val Ser Lys Glu Lys Asn Lys Asp Gly Lys
    210                 215                 220

tac gat cta att gca aca gta gac aag ctt gag ctt aaa gga act tct     720
Tyr Asp Leu Ile Ala Thr Val Asp Lys Leu Glu Leu Lys Gly Thr Ser
225                 230                 235                 240

gat aaa aac aat gga tct gga gta ctt gaa ggc gta aaa gct gac aaa     768
Asp Lys Asn Asn Gly Ser Gly Val Leu Glu Gly Val Lys Ala Asp Lys
                245                 250                 255

agt aaa gta aaa tta aca att tct gac gat cta ggt caa acc aca ctt     816
Ser Lys Val Lys Leu Thr Ile Ser Asp Asp Leu Gly Gln Thr Thr Leu
            260                 265                 270

gaa gtt ttc aaa gaa gat ggc aaa aca cta gta tca aaa aaa gta act     864
Glu Val Phe Lys Glu Asp Gly Lys Thr Leu Val Ser Lys Lys Val Thr
        275                 280                 285

tcc aaa gac aag tca tca aca gaa gaa aaa ttc aat gaa aaa ggt gaa     912
Ser Lys Asp Lys Ser Ser Thr Glu Glu Lys Phe Asn Glu Lys Gly Glu
    290                 295                 300

gta tct gaa aaa ata ata aca aga gca gac gga acc aga ctt gaa tac     960
Val Ser Glu Lys Ile Ile Thr Arg Ala Asp Gly Thr Arg Leu Glu Tyr
305                 310                 315                 320

aca gga att aaa agc gat gga tct gga aaa gct aaa gag gtt tta aaa     1008
Thr Gly Ile Lys Ser Asp Gly Ser Gly Lys Ala Lys Glu Val Leu Lys
                325                 330                 335

ggc tat gtt ctt gaa gga act cta act gct gaa aaa aca aca ttg gtg     1056
Gly Tyr Val Leu Glu Gly Thr Leu Thr Ala Glu Lys Thr Thr Leu Val
            340                 345                 350

gtt aaa gaa gga act gtt act tta agc aaa aat att tca aaa tct ggg     1104
Val Lys Glu Gly Thr Val Thr Leu Ser Lys Asn Ile Ser Lys Ser Gly
        355                 360                 365

gaa gtt tca gtt gaa ctt aat gac act gac agt agt gct gct act aaa     1152

Glu Val Ser Val Glu Leu Asn Asp Thr Asp Ser Ser Ala Ala Thr Lys
    370                 375                 380

aaa act gca gct tgg aat tca ggc act tca act tta aca att act gta    1200
Lys Thr Ala Ala Trp Asn Ser Gly Thr Ser Thr Leu Thr Ile Thr Val
385                 390                 395                 400

aac agt aaa aaa act aaa gac ctt gtg ttt aca aaa gaa aac aca att    1248
Asn Ser Lys Lys Thr Lys Asp Leu Val Phe Thr Lys Glu Asn Thr Ile
                405                 410                 415

aca gta caa caa tac gac tca aat ggc acc aaa tta gag ggg tca gca    1296
Thr Val Gln Gln Tyr Asp Ser Asn Gly Thr Lys Leu Glu Gly Ser Ala
            420                 425                 430

gtt gaa att aca aaa ctt gat gaa att aaa aac gct tta aaa taa        1341
Val Glu Ile Thr Lys Leu Asp Glu Ile Lys Asn Ala Leu Lys  *
        435                 440                 445

<210> 60
<211> 446
<212> PRT
<213> Artificial Sequence

<220>
<223> Chimeric protein

<400> 60
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Ser Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Leu Ala Lys Ala Ile Gly Lys Lys Ile
    50                  55                  60
Lys Asn Asp Gly Ser Leu Asp Asn Glu Ala Asn Arg Asn Glu Ser Leu
65                  70                  75                  80
Leu Ala Gly Ala Tyr Thr Ile Ser Thr Leu Ile Thr Gln Lys Leu Ser
                85                  90                  95
Lys Leu Asn Gly Ser Glu Gly Leu Lys Glu Lys Ile Ala Ala Ala Lys
            100                 105                 110
Lys Cys Ser Glu Glu Phe Ser Thr Lys Leu Lys Asp Asn His Ala Gln
        115                 120                 125
Leu Gly Ile Gln Gly Val Thr Asp Glu Asn Ala Lys Lys Ala Ile Leu
    130                 135                 140
Lys Ala Asn Ala Ala Gly Lys Asp Lys Gly Val Glu Glu Leu Glu Lys
145                 150                 155                 160
Leu Ser Gly Ser Leu Glu Ser Leu Ser Lys Ala Ala Lys Glu Met Leu
                165                 170                 175
Ala Asn Ser Val Lys Glu Leu Thr Ser Pro Val Val His Gly Lys Gln
            180                 185                 190
Asn Val Ser Ser Leu Asp Glu Lys Asn Ser Val Ser Val Asp Leu Pro
        195                 200                 205
Gly Glu Met Lys Val Leu Val Ser Lys Glu Lys Asn Lys Asp Gly Lys
    210                 215                 220
Tyr Asp Leu Ile Ala Thr Val Asp Lys Leu Glu Leu Lys Gly Thr Ser
225                 230                 235                 240
Asp Lys Asn Asn Gly Ser Gly Val Leu Glu Gly Val Lys Ala Asp Lys
                245                 250                 255
Ser Lys Val Lys Leu Thr Ile Ser Asp Asp Leu Gly Gln Thr Thr Leu
            260                 265                 270
Glu Val Phe Lys Glu Asp Gly Lys Thr Leu Val Ser Lys Lys Val Thr
        275                 280                 285
Ser Lys Asp Lys Ser Ser Thr Glu Glu Lys Phe Asn Glu Lys Gly Glu
    290                 295                 300

Val Ser Glu Lys Ile Ile Thr Arg Ala Asp Gly Thr Arg Leu Glu Tyr
305                 310                 315                 320
Thr Gly Ile Lys Ser Asp Gly Ser Gly Lys Ala Lys Glu Val Leu Lys
                325                 330                 335
Gly Tyr Val Leu Glu Gly Thr Leu Thr Ala Glu Lys Thr Thr Leu Val
            340                 345                 350
Val Lys Glu Gly Thr Val Thr Leu Ser Lys Asn Ile Ser Lys Ser Gly
        355                 360                 365
Glu Val Ser Val Glu Leu Asn Asp Thr Asp Ser Ser Ala Ala Thr Lys
    370                 375                 380
Lys Thr Ala Ala Trp Asn Ser Gly Thr Ser Thr Leu Thr Ile Thr Val
385                 390                 395                 400
Asn Ser Lys Lys Thr Lys Asp Leu Val Phe Thr Lys Glu Asn Thr Ile
                405                 410                 415
Thr Val Gln Gln Tyr Asp Ser Asn Gly Thr Lys Leu Glu Gly Ser Ala
            420                 425                 430
Val Glu Ile Thr Lys Leu Asp Glu Ile Lys Asn Ala Leu Lys
        435                 440                 445

<210> 61
<211> 1362
<212> DNA
<213> Artificial Sequence

<220>
<223> Chimeric nucleic acid

<221> CDS
<222> (1)...(1362)

<400> 61
atg gct tgt aat aat tca ggg aaa gat ggg aat aca tct gca aat tct     48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa     96
Ala Asp Glu Ser Val Lys Gly Pro Asn Leu Thr Glu Ile Asn Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa     192
Leu Leu Ser Ser Ile Asp Glu Ile Ala Ala Lys Ala Ile Gly Lys Lys
    50                  55                  60

ata cac caa aat aat ggt ttg gat acc gaa aat aat cac aat gga tca     240
Ile His Gln Asn Asn Gly Leu Asp Thr Glu Asn Asn His Asn Gly Ser
65                  70                  75                  80

ttg tta gcg gga gct tat gca ata tca acc cta ata aaa caa aaa tta     288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag     336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat     384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta     432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta     480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct     528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa     576
Asn Ser Val Lys Glu Leu Thr Ser Pro Val Val Ala Glu Ser Pro Lys
            180                 185                 190

aaa cct tcc atg gcc aag caa aat gtt agc agc ctt gac gag aaa aac     624
Lys Pro Ser Met Ala Lys Gln Asn Val Ser Ser Leu Asp Glu Lys Asn
        195                 200                 205

agc gtt tca gta gat ttg cct ggt gaa atg aaa gtt ctt gta agc aaa     672
Ser Val Ser Val Asp Leu Pro Gly Glu Met Lys Val Leu Val Ser Lys
    210                 215                 220

gaa aaa aac aaa gac ggc aag tac gat cta att gca aca gta gac aag     720
Glu Lys Asn Lys Asp Gly Lys Tyr Asp Leu Ile Ala Thr Val Asp Lys
225                 230                 235                 240

ctt gag ctt aaa gga act tct gat aaa aac aat gga tct gga gta ctt     768
Leu Glu Leu Lys Gly Thr Ser Asp Lys Asn Asn Gly Ser Gly Val Leu
                245                 250                 255

gaa ggc gta aaa gct gac aaa agt aaa gta aaa tta aca att tct gac     816
Glu Gly Val Lys Ala Asp Lys Ser Lys Val Lys Leu Thr Ile Ser Asp
            260                 265                 270

gat cta ggt caa acc aca ctt gaa gtt ttc aaa gaa gat ggc aaa aca     864
Asp Leu Gly Gln Thr Thr Leu Glu Val Phe Lys Glu Asp Gly Lys Thr
        275                 280                 285

cta gta tca aaa aaa gta act tcc aaa gac aag tca tca aca gaa gaa     912
Leu Val Ser Lys Lys Val Thr Ser Lys Asp Lys Ser Ser Thr Glu Glu
    290                 295                 300

aaa ttc aat gaa aaa ggt gaa gta tct gaa aaa ata ata aca aga gca     960
Lys Phe Asn Glu Lys Gly Glu Val Ser Glu Lys Ile Ile Thr Arg Ala
305                 310                 315                 320

gac gga acc aga ctt gaa tac aca gga att aaa agc gat gga tct gga     1008
Asp Gly Thr Arg Leu Glu Tyr Thr Gly Ile Lys Ser Asp Gly Ser Gly
                325                 330                 335

aaa gct aaa gag gtt tta aaa ggc tat gtt ctt gaa gga act cta act     1056
Lys Ala Lys Glu Val Leu Lys Gly Tyr Val Leu Glu Gly Thr Leu Thr
            340                 345                 350

gct gaa aaa aca aca ttg gtg gtt aaa gaa gga act gtt act tta agc     1104
Ala Glu Lys Thr Thr Leu Val Val Lys Glu Gly Thr Val Thr Leu Ser
        355                 360                 365

aaa aat att tca aaa tct ggg gaa gtt tca gtt gaa ctt aat gac act     1152
Lys Asn Ile Ser Lys Ser Gly Glu Val Ser Val Glu Leu Asn Asp Thr
    370                 375                 380

gac agt agt gct gct act aaa aaa act gca gct tgg aat tca aaa act     1200
Asp Ser Ser Ala Ala Thr Lys Lys Thr Ala Ala Trp Asn Ser Lys Thr
385                 390                 395                 400

tcc act tta aca att agt gtg aat agc caa aaa acc aaa aac ctt gta     1248
Ser Thr Leu Thr Ile Ser Val Asn Ser Gln Lys Thr Lys Asn Leu Val
                405                 410                 415

ttc aca aaa gaa gac aca ata aca gta caa aaa tac gac tca gca ggc     1296
Phe Thr Lys Glu Asp Thr Ile Thr Val Gln Lys Tyr Asp Ser Ala Gly
            420                 425                 430

acc aat cta gaa ggc aaa gca gtc gaa att aca aca ctt aaa gaa ctt     1344
Thr Asn Leu Glu Gly Lys Ala Val Glu Ile Thr Thr Leu Lys Glu Leu
        435                 440                 445

aaa aac gct tta aaa taa                                             1362
Lys Asn Ala Leu Lys  *
    450


<210> 62
<211> 453
<212> PRT
<213> Artificial Sequence

<220>
<223> Chimeric protein

<400> 62
Met Ala Cys Asn Asn Ser Gly Lys Asp Gly Asn Thr Ser Ala Asn Ser
 1               5                  10                  15
Ala Asp Glu Ser Val Lys Gly Pro Asn Leu Thr Glu Ile Asn Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Lys Gln Asn Val Ser Ser Leu Asp Glu Lys Asn
        195                 200                 205
Ser Val Ser Val Asp Leu Pro Gly Glu Met Lys Val Leu Val Ser Lys
    210                 215                 220
Glu Lys Asn Lys Asp Gly Lys Tyr Asp Leu Ile Ala Thr Val Asp Lys
225                 230                 235                 240
Leu Glu Leu Lys Gly Thr Ser Asp Lys Asn Asn Gly Ser Gly Val Leu
                245                 250                 255
Glu Gly Val Lys Ala Asp Lys Ser Lys Val Lys Leu Thr Ile Ser Asp
            260                 265                 270
Asp Leu Gly Gln Thr Thr Leu Glu Val Phe Lys Glu Asp Gly Lys Thr
        275                 280                 285
Leu Val Ser Lys Lys Val Thr Ser Lys Asp Lys Ser Ser Thr Glu Glu
    290                 295                 300
Lys Phe Asn Glu Lys Gly Glu Val Ser Glu Lys Ile Ile Thr Arg Ala
305                 310                 315                 320
Asp Gly Thr Arg Leu Glu Tyr Thr Gly Ile Lys Ser Asp Gly Ser Gly
                325                 330                 335
Lys Ala Lys Glu Val Leu Lys Gly Tyr Val Leu Glu Gly Thr Leu Thr
            340                 345                 350
Ala Glu Lys Thr Thr Leu Val Val Lys Glu Gly Thr Val Thr Leu Ser
        355                 360                 365

Lys Asn Ile Ser Lys Ser Gly Glu Val Ser Val Glu Leu Asn Asp Thr
    370                 375                 380
Asp Ser Ser Ala Ala Thr Lys Lys Thr Ala Ala Trp Asn Ser Lys Thr
385                 390                 395                 400
Ser Thr Leu Thr Ile Ser Val Asn Ser Gln Lys Thr Lys Asn Leu Val
                405                 410                 415
Phe Thr Lys Glu Asp Thr Ile Thr Val Gln Lys Tyr Asp Ser Ala Gly
            420                 425                 430
Thr Asn Leu Glu Gly Lys Ala Val Glu Ile Thr Thr Leu Lys Glu Leu
        435                 440                 445
Lys Asn Ala Leu Lys
    450

<210> 63
<211> 1341
<212> DNA
<213> Artificial Sequence

<220>
<223> Chimeric nucleic acid

<221> CDS
<222> (1)...(1341)

<400> 63
atg gct tgt aat aat tca ggg aaa gat ggg aat aca tct gca aat tct      48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata agt aaa aaa      96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gag ctt gct aaa gct att ggt aaa aaa ata     192
Leu Leu Ser Ser Ile Asp Glu Leu Ala Lys Ala Ile Gly Lys Lys Ile
    50                  55                  60

aaa aac gat ggt agt tta gat aat gaa gca aat cgc aac gag tca ttg     240
Lys Asn Asp Gly Ser Leu Asp Asn Glu Ala Asn Arg Asn Glu Ser Leu
65                  70                  75                  80

tta gca gga gct tat aca ata tca acc tta ata aca caa aaa tta agt     288
Leu Ala Gly Ala Tyr Thr Ile Ser Thr Leu Ile Thr Gln Lys Leu Ser
                85                  90                  95

aaa tta aac gga tca gaa ggt tta aag gaa aag att gcc gca gct aag     336
Lys Leu Asn Gly Ser Glu Gly Leu Lys Glu Lys Ile Ala Ala Ala Lys
            100                 105                 110

aaa tgc tct gaa gag ttt agt act aaa cta aaa gat aat cat gca cag     384
Lys Cys Ser Glu Glu Phe Ser Thr Lys Leu Lys Asp Asn His Ala Gln
        115                 120                 125

ctt ggt ata cag ggc gtt act gat gaa aat gca aaa aaa gct att tta     432
Leu Gly Ile Gln Gly Val Thr Asp Glu Asn Ala Lys Lys Ala Ile Leu
    130                 135                 140

aaa gca aat gca gcg ggt aaa gat aag ggc gtt gaa gaa ctt gaa aag     480
Lys Ala Asn Ala Ala Gly Lys Asp Lys Gly Val Glu Glu Leu Glu Lys
145                 150                 155                 160

ttg tcc gga tca tta gaa agc tta tca aaa gca gct aaa gag atg ctt     528
Leu Ser Gly Ser Leu Glu Ser Leu Ser Lys Ala Ala Lys Glu Met Leu

                165                 170                 175

gct aat tca gtt aaa gag ctt aca agc cct gtt gtc cat ggc aag caa 576
Ala Asn Ser Val Lys Glu Leu Thr Ser Pro Val Val His Gly Lys Gln
            180                 185                 190

aat gtt agc agc ctt gac gag aaa aac agc gtt tca gta gat ttg cct 624
Asn Val Ser Ser Leu Asp Glu Lys Asn Ser Val Ser Val Asp Leu Pro
        195                 200                 205

ggt gaa atg aaa gtt ctt gta agc aaa gaa aaa aac aaa gac ggc aag 672
Gly Glu Met Lys Val Leu Val Ser Lys Glu Lys Asn Lys Asp Gly Lys
    210                 215                 220

tac gat cta att gca aca gta gac aag ctt gag ctt aaa gga act tct 720
Tyr Asp Leu Ile Ala Thr Val Asp Lys Leu Glu Leu Lys Gly Thr Ser
225                 230                 235                 240

gat aaa aac aat gga tct gga gta ctt gaa ggc gta aaa gct gac aaa 768
Asp Lys Asn Asn Gly Ser Gly Val Leu Glu Gly Val Lys Ala Asp Lys
                245                 250                 255

agt aaa gta aaa tta aca att tct gac gat cta ggt caa acc aca ctt 816
Ser Lys Val Lys Leu Thr Ile Ser Asp Asp Leu Gly Gln Thr Thr Leu
            260                 265                 270

gaa gtt ttc aaa gaa gat ggc aaa aca cta gta tca aaa aaa gta act 864
Glu Val Phe Lys Glu Asp Gly Lys Thr Leu Val Ser Lys Lys Val Thr
        275                 280                 285

tcc aaa gac aag tca tca aca gaa gaa aaa ttc aat gaa aaa ggt gaa 912
Ser Lys Asp Lys Ser Ser Thr Glu Glu Lys Phe Asn Glu Lys Gly Glu
    290                 295                 300

gta tct gaa aaa ata ata aca aga gca gac gga acc aga ctt gaa tac 960
Val Ser Glu Lys Ile Ile Thr Arg Ala Asp Gly Thr Arg Leu Glu Tyr
305                 310                 315                 320

aca gga att aaa agc gat gga tct gga aaa gct aaa gag gtt tta aaa 1008
Thr Gly Ile Lys Ser Asp Gly Ser Gly Lys Ala Lys Glu Val Leu Lys
                325                 330                 335

ggc tat gtt ctt gaa gga act cta act gct gaa aaa aca aca ttg gtg 1056
Gly Tyr Val Leu Glu Gly Thr Leu Thr Ala Glu Lys Thr Thr Leu Val
            340                 345                 350

gtt aaa gaa gga act gtt act tta agc aaa aat att tca aaa tct ggg 1104
Val Lys Glu Gly Thr Val Thr Leu Ser Lys Asn Ile Ser Lys Ser Gly
        355                 360                 365

gaa gtt tca gtt gaa ctt aat gac act gac agt agt gct gct act aaa 1152
Glu Val Ser Val Glu Leu Asn Asp Thr Asp Ser Ser Ala Ala Thr Lys
    370                 375                 380

aaa act gca gct tgg aat tca aaa act tcc act tta aca att agt gtg 1200
Lys Thr Ala Ala Trp Asn Ser Lys Thr Ser Thr Leu Thr Ile Ser Val
385                 390                 395                 400

aat agc caa aaa acc aaa aac ctt gta ttc aca aaa gaa gac aca ata 1248
Asn Ser Gln Lys Thr Lys Asn Leu Val Phe Thr Lys Glu Asp Thr Ile
                405                 410                 415

aca gta caa aaa tac gac tca gca ggc acc aat cta gaa ggc aaa gca 1296
Thr Val Gln Lys Tyr Asp Ser Ala Gly Thr Asn Leu Glu Gly Lys Ala
            420                 425                 430

gtc gaa att aca aca ctt aaa gaa ctt aaa aac gct tta aaa taa 1341
Val Glu Ile Thr Thr Leu Lys Glu Leu Lys Asn Ala Leu Lys *

435                 440                 445

<210> 64
<211> 446
<212> PRT
<213> Artificial Sequence

<220>
<223> Chimeric protein

<400> 64
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Ser Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Leu Ala Lys Ala Ile Gly Lys Lys Ile
    50                  55                  60
Lys Asn Asp Gly Ser Leu Asp Asn Glu Ala Asn Arg Asn Glu Ser Leu
65                  70                  75                  80
Leu Ala Gly Ala Tyr Thr Ile Ser Thr Leu Ile Thr Gln Lys Leu Ser
                85                  90                  95
Lys Leu Asn Gly Ser Glu Gly Leu Lys Glu Lys Ile Ala Ala Ala Lys
            100                 105                 110
Lys Cys Ser Glu Glu Phe Ser Thr Lys Leu Lys Asp Asn His Ala Gln
        115                 120                 125
Leu Gly Ile Gln Gly Val Thr Asp Glu Asn Ala Lys Lys Ala Ile Leu
    130                 135                 140
Lys Ala Asn Ala Ala Gly Lys Asp Lys Gly Val Glu Glu Leu Glu Lys
145                 150                 155                 160
Leu Ser Gly Ser Leu Glu Ser Leu Ser Lys Ala Ala Lys Glu Met Leu
                165                 170                 175
Ala Asn Ser Val Lys Glu Leu Thr Ser Pro Val Val His Gly Lys Gln
            180                 185                 190
Asn Val Ser Ser Leu Asp Glu Lys Asn Ser Val Ser Val Asp Leu Pro
        195                 200                 205
Gly Glu Met Lys Val Leu Val Ser Lys Glu Lys Asn Lys Asp Gly Lys
    210                 215                 220
Tyr Asp Leu Ile Ala Thr Val Asp Lys Leu Glu Leu Lys Gly Thr Ser
225                 230                 235                 240
Asp Lys Asn Asn Gly Ser Gly Val Leu Glu Gly Val Lys Ala Asp Lys
                245                 250                 255
Ser Lys Val Lys Leu Thr Ile Ser Asp Asp Leu Gly Gln Thr Thr Leu
            260                 265                 270
Glu Val Phe Lys Glu Asp Gly Lys Thr Leu Val Ser Lys Lys Val Thr
        275                 280                 285
Ser Lys Asp Lys Ser Ser Thr Glu Glu Lys Phe Asn Glu Lys Gly Glu
    290                 295                 300
Val Ser Glu Lys Ile Ile Thr Arg Ala Asp Gly Thr Arg Leu Glu Tyr
305                 310                 315                 320
Thr Gly Ile Lys Ser Asp Gly Ser Gly Lys Ala Lys Glu Val Leu Lys
                325                 330                 335
Gly Tyr Val Leu Glu Gly Thr Leu Thr Ala Glu Lys Thr Thr Leu Val
            340                 345                 350
Val Lys Glu Gly Thr Val Thr Leu Ser Lys Asn Ile Ser Lys Ser Gly
        355                 360                 365
Glu Val Ser Val Glu Leu Asn Asp Thr Asp Ser Ser Ala Ala Thr Lys
    370                 375                 380
Lys Thr Ala Ala Trp Asn Ser Lys Thr Ser Thr Leu Thr Ile Ser Val
385                 390                 395                 400
Asn Ser Gln Lys Thr Lys Asn Leu Val Phe Thr Lys Glu Asp Thr Ile
                405                 410                 415
Thr Val Gln Lys Tyr Asp Ser Ala Gly Thr Asn Leu Glu Gly Lys Ala
            420                 425                 430
Val Glu Ile Thr Thr Leu Lys Glu Leu Lys Asn Ala Leu Lys

435                 440                 445

<210> 65
<211> 1362
<212> DNA
<213> Artificial Sequence

<220>
<223> Chimeric nucleic acid


<221> CDS
<222> (1)...(1362)

<400> 65
atg gct tgt aat aat tca ggg aaa gat ggg aat aca tct gca aat tct      48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa      96
Ala Asp Glu Ser Val Lys Gly Pro Asn Leu Thr Glu Ile Asn Lys Lys
            20                  25                  30

att acg tca tct aat gcg gtt tta ctt gct gtg aaa gag gtt gaa gcg     144
Ile Thr Ser Ser Asn Ala Val Leu Leu Ala Val Lys Glu Val Glu Ala
        35                  40                  45

ttg ctg gat tct ata gat gaa att gct gct aaa gct att ggt aaa aaa     192
Leu Leu Asp Ser Ile Asp Glu Ile Ala Ala Lys Ala Ile Gly Lys Lys
    50                  55                  60

ata cac caa aat aat ggt ttg gat acc gaa aat aat cac aat gga tca     240
Ile His Gln Asn Asn Gly Leu Asp Thr Glu Asn Asn His Asn Gly Ser
65                  70                  75                  80

ttg tta gcg gga gct tat gca ata tca acc cta ata aaa caa aaa tta     288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag     336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat     384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta     432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta     480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct     528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa     576
Asn Ser Val Lys Glu Leu Thr Ser Pro Val Val Ala Glu Ser Pro Lys
            180                 185                 190

aaa cct tcc atg gcc aag caa aat gtt agc agc ctt gac gag aaa aac     624
Lys Pro Ser Met Ala Lys Gln Asn Val Ser Ser Leu Asp Glu Lys Asn
        195                 200                 205

agc gtt tca gta gat ttg cct ggt gaa atg aaa gtt ctt gta agc aaa 672
Ser Val Ser Val Asp Leu Pro Gly Glu Met Lys Val Leu Val Ser Lys
    210                 215                 220

gaa aaa aac aaa gac ggc aag tac gat cta att gca aca gta gac aag 720
Glu Lys Asn Lys Asp Gly Lys Tyr Asp Leu Ile Ala Thr Val Asp Lys
225                 230                 235                 240

ctt gag ctt aaa gga act tct gat aaa aac aat gga tct gga gta ctt 768
Leu Glu Leu Lys Gly Thr Ser Asp Lys Asn Asn Gly Ser Gly Val Leu
                245                 250                 255

gaa ggc gta aaa gct gac aaa agt aaa gta aaa tta aca att tct gac 816
Glu Gly Val Lys Ala Asp Lys Ser Lys Val Lys Leu Thr Ile Ser Asp
            260                 265                 270

gat cta ggt caa acc aca ctt gaa gtt ttc aaa gaa gat ggc aaa aca 864
Asp Leu Gly Gln Thr Thr Leu Glu Val Phe Lys Glu Asp Gly Lys Thr
        275                 280                 285

cta gta tca aaa aaa gta act tcc aaa gac aag tca tca aca gaa gaa 912
Leu Val Ser Lys Lys Val Thr Ser Lys Asp Lys Ser Ser Thr Glu Glu
    290                 295                 300

aaa ttc aat gaa aaa ggt gaa gta tct gaa aaa ata ata aca aga gca 960
Lys Phe Asn Glu Lys Gly Glu Val Ser Glu Lys Ile Ile Thr Arg Ala
305                 310                 315                 320

gac gga acc aga ctt gaa tac aca gga att aaa agc gat gga tct gga 1008
Asp Gly Thr Arg Leu Glu Tyr Thr Gly Ile Lys Ser Asp Gly Ser Gly
                325                 330                 335

aaa gct aaa gag gtt tta aaa ggc tat gtt ctt gaa gga act cta act 1056
Lys Ala Lys Glu Val Leu Lys Gly Tyr Val Leu Glu Gly Thr Leu Thr
            340                 345                 350

gct gaa aaa aca aca ttg gtg gtt aaa gaa gga act gtt act tta agc 1104
Ala Glu Lys Thr Thr Leu Val Val Lys Glu Gly Thr Val Thr Leu Ser
        355                 360                 365

aaa aat att tca aaa tct ggg gaa gtt tca gtt gaa ctt aat gac act 1152
Lys Asn Ile Ser Lys Ser Gly Glu Val Ser Val Glu Leu Asn Asp Thr
    370                 375                 380

gac agt agt gct gct act aaa aaa act gca gct tgg aat tca aaa act 1200
Asp Ser Ser Ala Ala Thr Lys Lys Thr Ala Ala Trp Asn Ser Lys Thr
385                 390                 395                 400

tct act tta aca att agt gtt aac agc aaa aaa act aca caa ctt gtg 1248
Ser Thr Leu Thr Ile Ser Val Asn Ser Lys Lys Thr Thr Gln Leu Val
                405                 410                 415

ttt act aaa caa gac aca ata act gta caa aaa tac gac tcc gca ggt 1296
Phe Thr Lys Gln Asp Thr Ile Thr Val Gln Lys Tyr Asp Ser Ala Gly
            420                 425                 430

acc aat tta gaa ggc aca gca gtc gaa att aaa aca ctt gat gaa ctt 1344
Thr Asn Leu Glu Gly Thr Ala Val Glu Ile Lys Thr Leu Asp Glu Leu
        435                 440                 445

aaa aac gct tta aaa taa 1362
Lys Asn Ala Leu Lys *
    450

<210> 66
<211> 453
<212> PRT

<213> Artificial Sequence

<220>
<223> Chimeric protein

<400> 66
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Asn Lys Lys
            20                  25                  30
Ile Thr Ser Ser Asn Ala Val Leu Leu Ala Val Lys Glu Val Glu Ala
        35                  40                  45
Leu Leu Asp Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Lys Gln Asn Val Ser Ser Leu Asp Glu Lys Asn
        195                 200                 205
Ser Val Ser Val Asp Leu Pro Gly Glu Met Lys Val Leu Val Ser Lys
    210                 215                 220
Glu Lys Asn Lys Asp Gly Lys Tyr Asp Leu Ile Ala Thr Val Asp Lys
225                 230                 235                 240
Leu Glu Leu Lys Gly Thr Ser Asp Lys Asn Asn Gly Ser Gly Val Leu
                245                 250                 255
Glu Gly Val Lys Ala Asp Lys Ser Lys Val Lys Leu Thr Ile Ser Asp
            260                 265                 270
Asp Leu Gly Gln Thr Thr Leu Glu Val Phe Lys Glu Asp Gly Lys Thr
        275                 280                 285
Leu Val Ser Lys Lys Val Thr Ser Lys Asp Lys Ser Ser Thr Glu Glu
    290                 295                 300
Lys Phe Asn Glu Lys Gly Glu Val Ser Glu Lys Ile Ile Thr Arg Ala
305                 310                 315                 320
Asp Gly Thr Arg Leu Glu Tyr Thr Gly Ile Lys Ser Asp Gly Ser Gly
                325                 330                 335
Lys Ala Lys Glu Val Leu Lys Gly Tyr Val Leu Glu Gly Thr Leu Thr
            340                 345                 350
Ala Glu Lys Thr Thr Leu Val Val Lys Glu Gly Thr Val Thr Leu Ser
        355                 360                 365
Lys Asn Ile Ser Lys Ser Gly Glu Val Ser Val Glu Leu Asn Asp Thr
    370                 375                 380
Asp Ser Ser Ala Ala Thr Lys Lys Thr Ala Ala Trp Asn Ser Lys Thr
385                 390                 395                 400
Ser Thr Leu Thr Ile Ser Val Asn Ser Lys Lys Thr Thr Gln Leu Val
                405                 410                 415
Phe Thr Lys Gln Asp Thr Ile Thr Val Gln Lys Tyr Asp Ser Ala Gly
            420                 425                 430
Thr Asn Leu Glu Gly Thr Ala Val Glu Ile Lys Thr Leu Asp Glu Leu
        435                 440                 445
Lys Asn Ala Leu Lys
    450


<210> 67
<211> 1341

<212> DNA
<213> Artificial Sequence

<220>
<223> Chimeric nucleic acid

<221> CDS
<222> (1)...(1341)

<400> 67
atg gct tgt aat aat tca ggg aaa gat ggg aat aca tct gca aat tct     48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata agt aaa aaa     96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg    144
Ile Thr Asp Ser Asn Ala Val Leu Leu Ala Val Lys Glu Val Glu Ala
        35                  40                  45

ttg ctg tca tct ata gat gag ctt gct aaa gct att ggt aaa aaa ata    192
Leu Leu Ser Ser Ile Asp Glu Leu Ala Lys Ala Ile Gly Lys Lys Ile
    50                  55                  60

aaa aac gat ggt agt tta gat aat gaa gca aat cgc aac gag tca ttg    240
Lys Asn Asp Gly Ser Leu Asp Asn Glu Ala Asn Arg Asn Glu Ser Leu
65                  70                  75                  80

tta gca gga gct tat aca ata tca acc tta ata aca caa aaa tta agt    288
Leu Ala Gly Ala Tyr Thr Ile Ser Thr Leu Ile Thr Gln Lys Leu Ser
                85                  90                  95

aaa tta aac gga tca gaa ggt tta aag gaa aag att gcc gca gct aag    336
Lys Leu Asn Gly Ser Glu Gly Leu Lys Glu Lys Ile Ala Ala Ala Lys
            100                 105                 110

aaa tgc tct gaa gag ttt agt act aaa cta aaa gat aat cat gca cag    384
Lys Cys Ser Glu Glu Phe Ser Thr Lys Leu Lys Asp Asn His Ala Gln
        115                 120                 125

ctt ggt ata cag ggc gtt act gat gaa aat gca aaa aaa gct att tta    432
Leu Gly Ile Gln Gly Val Thr Asp Glu Asn Ala Lys Lys Ala Ile Leu
    130                 135                 140

aaa gca aat gca gcg ggt aaa gat aag ggc gtt gaa gaa ctt gaa aag    480
Lys Ala Asn Ala Ala Gly Lys Asp Lys Gly Val Glu Glu Leu Glu Lys
145                 150                 155                 160

ttg tcc gga tca tta gaa agc tta tca aaa gca gct aaa gag atg ctt    528
Leu Ser Gly Ser Leu Glu Ser Leu Ser Lys Ala Ala Lys Glu Met Leu
                165                 170                 175

gct aat tca gtt aaa gag ctt aca agc cct gtt gtc cat ggc aag caa    576
Ala Asn Ser Val Lys Glu Leu Thr Ser Pro Val Val His Gly Lys Gln
            180                 185                 190

aat gtt agc agc ctt gac gag aaa aac agc gtt tca gta gat ttg cct    624
Asn Val Ser Ser Leu Asp Glu Lys Asn Ser Val Ser Val Asp Leu Pro
        195                 200                 205

ggt gaa atg aaa gtt ctt gta agc aaa gaa aaa aac aaa gac ggc aag    672
Gly Glu Met Lys Val Leu Val Ser Lys Glu Lys Asn Lys Asp Gly Lys
    210                 215                 220

tac gat cta att gca aca gta gac aag ctt gag ctt aaa gga act tct    720
Tyr Asp Leu Ile Ala Thr Val Asp Lys Leu Glu Leu Lys Gly Thr Ser

225                 230                 235                 240

gat aaa aac aat gga tct gga gta ctt gaa ggc gta aaa gct gac aaa      768
Asp Lys Asn Asn Gly Ser Gly Val Leu Glu Gly Val Lys Ala Asp Lys
                245                 250                 255

agt aaa gta aaa tta aca att tct gac gat cta ggt caa acc aca ctt      816
Ser Lys Val Lys Leu Thr Ile Ser Asp Asp Leu Gly Gln Thr Thr Leu
            260                 265                 270

gaa gtt ttc aaa gaa gat ggc aaa aca cta gta tca aaa aaa gta act      864
Glu Val Phe Lys Glu Asp Gly Lys Thr Leu Val Ser Lys Lys Val Thr
        275                 280                 285

tcc aaa gac aag tca tca aca gaa gaa aaa ttc aat gaa aaa ggt gaa      912
Ser Lys Asp Lys Ser Ser Thr Glu Glu Lys Phe Asn Glu Lys Gly Glu
    290                 295                 300

gta tct gaa aaa ata ata aca aga gca gac gga acc aga ctt gaa tac      960
Val Ser Glu Lys Ile Ile Thr Arg Ala Asp Gly Thr Arg Leu Glu Tyr
305                 310                 315                 320

aca gga att aaa agc gat gga tct gga aaa gct aaa gag gtt tta aaa      1008
Thr Gly Ile Lys Ser Asp Gly Ser Gly Lys Ala Lys Glu Val Leu Lys
                325                 330                 335

ggc tat gtt ctt gaa gga act cta act gct gaa aaa aca aca ttg gtg      1056
Gly Tyr Val Leu Glu Gly Thr Leu Thr Ala Glu Lys Thr Thr Leu Val
            340                 345                 350

gtt aaa gaa gga act gtt act tta agc aaa aat att tca aaa tct ggg      1104
Val Lys Glu Gly Thr Val Thr Leu Ser Lys Asn Ile Ser Lys Ser Gly
        355                 360                 365

gaa gtt tca gtt gaa ctt aat gac act gac agt agt gct gct act aaa      1152
Glu Val Ser Val Glu Leu Asn Asp Thr Asp Ser Ser Ala Ala Thr Lys
    370                 375                 380

aaa act gca gct tgg aat tca aaa act tct act tta aca att agt gtt      1200
Lys Thr Ala Ala Trp Asn Ser Lys Thr Ser Thr Leu Thr Ile Ser Val
385                 390                 395                 400

aac agc aaa aaa act aca caa ctt gtg ttt act aaa caa tac aca ata      1248
Asn Ser Lys Lys Thr Thr Gln Leu Val Phe Thr Lys Gln Tyr Thr Ile
                405                 410                 415

act gta aaa caa tac gac tcc gca ggt acc aat tta gaa ggc aca gca      1296
Thr Val Lys Gln Tyr Asp Ser Ala Gly Thr Asn Leu Glu Gly Thr Ala
            420                 425                 430

gtc gaa att aaa aca ctt gat gaa ctt aaa aac gct tta aaa taa          1341
Val Glu Ile Lys Thr Leu Asp Glu Leu Lys Asn Ala Leu Lys  *
        435                 440                 445


<210> 68
<211> 446
<212> PRT
<213> Artificial Sequence

<220>
<223> Chimeric protein

<400> 68
Met Ala Cys Asn Asn Ser Gly Lys Asp Gly Asn Thr Ser Ala Asn Ser
 1               5                  10                  15
Ala Asp Glu Ser Val Lys Gly Pro Asn Leu Thr Glu Ile Ser Lys Lys

            20                  25                  30
Ile Thr Asp Ser Asn Ala Val Leu Leu Ala Val Lys Glu Val Glu Ala
        35                  40                  45
Leu Leu Ser Ser Ile Asp Glu Leu Ala Lys Ala Ile Gly Lys Lys Ile
    50                  55                  60
Lys Asn Asp Gly Ser Leu Asp Asn Glu Ala Asn Arg Asn Glu Ser Leu
65                  70                  75                  80
Leu Ala Gly Ala Tyr Thr Ile Ser Thr Leu Ile Thr Gln Lys Leu Ser
                85                  90                  95
Lys Leu Asn Gly Ser Glu Gly Leu Lys Glu Lys Ile Ala Ala Ala Lys
            100                 105                 110
Lys Cys Ser Glu Glu Phe Ser Thr Lys Leu Lys Asp Asn His Ala Gln
        115                 120                 125
Leu Gly Ile Gln Gly Val Thr Asp Glu Asn Ala Lys Lys Ala Ile Leu
    130                 135                 140
Lys Ala Asn Ala Ala Gly Lys Asp Lys Gly Val Glu Glu Leu Glu Lys
145                 150                 155                 160
Leu Ser Gly Ser Leu Glu Ser Leu Ser Lys Ala Ala Lys Glu Met Leu
                165                 170                 175
Ala Asn Ser Val Lys Glu Leu Thr Ser Pro Val Val His Gly Lys Gln
            180                 185                 190
Asn Val Ser Ser Leu Asp Glu Lys Asn Ser Val Ser Val Asp Leu Pro
        195                 200                 205
Gly Glu Met Lys Val Leu Val Ser Lys Glu Lys Asn Lys Asp Gly Lys
    210                 215                 220
Tyr Asp Leu Ile Ala Thr Val Asp Lys Leu Glu Leu Lys Gly Thr Ser
225                 230                 235                 240
Asp Lys Asn Asn Gly Ser Gly Val Leu Glu Gly Val Lys Ala Asp Lys
                245                 250                 255
Ser Lys Val Lys Leu Thr Ile Ser Asp Asp Leu Gly Gln Thr Thr Leu
            260                 265                 270
Glu Val Phe Lys Glu Asp Gly Lys Thr Leu Val Ser Lys Lys Val Thr
        275                 280                 285
Ser Lys Asp Lys Ser Ser Thr Glu Glu Lys Phe Asn Glu Lys Gly Glu
    290                 295                 300
Val Ser Glu Lys Ile Ile Thr Arg Ala Asp Gly Thr Arg Leu Glu Tyr
305                 310                 315                 320
Thr Gly Ile Lys Ser Asp Gly Ser Gly Lys Ala Lys Glu Val Leu Lys
                325                 330                 335
Gly Tyr Val Leu Glu Gly Thr Leu Thr Ala Glu Lys Thr Thr Leu Val
            340                 345                 350
Val Lys Glu Gly Thr Val Thr Leu Ser Lys Asn Ile Ser Lys Ser Gly
        355                 360                 365
Glu Val Ser Val Glu Leu Asn Asp Thr Asp Ser Ser Ala Ala Thr Lys
    370                 375                 380
Lys Thr Ala Ala Trp Asn Ser Lys Thr Ser Thr Leu Thr Ile Ser Val
385                 390                 395                 400
Asn Ser Lys Lys Thr Thr Gln Leu Val Phe Thr Lys Gln Tyr Thr Ile
                405                 410                 415
Thr Val Lys Gln Tyr Asp Ser Ala Gly Thr Asn Leu Glu Gly Thr Ala
            420                 425                 430
Val Glu Ile Lys Thr Leu Asp Glu Leu Lys Asn Ala Leu Lys
        435                 440                 445

<210> 69
<211> 1365
<212> DNA
<213> Artificial Sequence

<220>
<223> Chimeric nucleic acid

<221> CDS
<222> (1)...(1365)

<400> 69
atg gct tgt aat aat tca ggg aaa gat ggg aat aca tct gca aat tct 48

Met Ala Cys Asn Asn Ser Gly Lys Asp Gly Asn Thr Ser Ala Asn Ser
1               5                   10                  15

gct gat gag tct gtt aaa ggg cct aat ctt aca gaa ata agt aaa aaa     96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa     192
Leu Leu Ser Ser Ile Asp Glu Ile Ala Ala Lys Ala Ile Gly Lys Lys
    50                  55                  60

ata cac caa aat aat ggt ttg gat acc gaa tat aat cac aat gga tca     240
Ile His Gln Asn Asn Gly Leu Asp Thr Glu Tyr Asn His Asn Gly Ser
65                  70                  75                  80

ttg tta gcg gga gct tat gca ata tca acc cta ata aaa caa aaa tta     288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag     336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat     384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta     432
Leu Gly Lys Glu Gly Val Thr Asp Ala Asp Ala Lys Glu Ala Ile Leu
    130                 135                 140

aaa aca aat ggt act aaa act aaa ggt gct gaa gaa ctt gga aaa tta     480
Lys Thr Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct     528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa     576
Asn Ser Val Lys Glu Leu Thr Ser Pro Val Val Ala Glu Ser Pro Lys
            180                 185                 190

aaa cct tcc atg gcc aag caa aat gtt agc agc ctt gat gaa aaa aat     624
Lys Pro Ser Met Ala Lys Gln Asn Val Ser Ser Leu Asp Glu Lys Asn
        195                 200                 205

agc gtt tca gta gat tta cct ggt gga atg aca gtt ctt gta agt aaa     672
Ser Val Ser Val Asp Leu Pro Gly Gly Met Thr Val Leu Val Ser Lys
    210                 215                 220

gaa aaa gac aaa gac ggt aaa tac agt cta gag gca aca gta gac aag     720
Glu Lys Asp Lys Asp Gly Lys Tyr Ser Leu Glu Ala Thr Val Asp Lys
225                 230                 235                 240

ctt gag ctt aaa gga act tct gat aaa aac aac ggt tct gga aca ctt     768
Leu Glu Leu Lys Gly Thr Ser Asp Lys Asn Asn Gly Ser Gly Thr Leu
                245                 250                 255

gaa ggt gaa aaa act gac aaa agt aaa gta aaa tta aca att gct gat     816
Glu Gly Glu Lys Thr Asp Lys Ser Lys Val Lys Leu Thr Ile Ala Asp
            260                 265                 270

gac cta agt caa act aaa ttt gaa att ttc aaa gaa gat gcc aaa aca     864

Asp Leu Ser Gln Thr Lys Phe Glu Ile Phe Lys Glu Asp Ala Lys Thr
        275                 280                 285

tta gta tca aaa aaa gta acc ctt aaa gac aag tca tca aca gaa gaa     912
Leu Val Ser Lys Lys Val Thr Leu Lys Asp Lys Ser Ser Thr Glu Glu
    290                 295                 300

aaa ttc aac gaa aag ggt gaa aca tct gaa aaa aca ata gta aga gca     960
Lys Phe Asn Glu Lys Gly Glu Thr Ser Glu Lys Thr Ile Val Arg Ala
305                 310                 315                 320

aat gga acc aga ctt gaa tac aca gac ata aaa agc gat gga tcc gga     1008
Asn Gly Thr Arg Leu Glu Tyr Thr Asp Ile Lys Ser Asp Gly Ser Gly
                325                 330                 335

aaa gct aaa gaa gtt tta aaa gac ttt act ctt gaa gga act cta gct     1056
Lys Ala Lys Glu Val Leu Lys Asp Phe Thr Leu Glu Gly Thr Leu Ala
            340                 345                 350

gct gac ggc aaa aca aca ttg aaa gtt aca gaa ggc act gtt gtt tta     1104
Ala Asp Gly Lys Thr Thr Leu Lys Val Thr Glu Gly Thr Val Val Leu
        355                 360                 365

agc aag aac att tta aaa tcc gga gaa ata aca gtt gca ctt gat gac     1152
Ser Lys Asn Ile Leu Lys Ser Gly Glu Ile Thr Val Ala Leu Asp Asp
    370                 375                 380

tct gac act act cag gct act aaa aaa act gga aaa tgg gat tca aat     1200
Ser Asp Thr Thr Gln Ala Thr Lys Lys Thr Gly Lys Trp Asp Ser Asn
385                 390                 395                 400

act tcc act tta aca att agt gtg aat agc aaa aaa act aaa aac att     1248
Thr Ser Thr Leu Thr Ile Ser Val Asn Ser Lys Lys Thr Lys Asn Ile
                405                 410                 415

gta ttt aca aaa gaa gac aca ata aca gta caa aaa tac gac tca gca     1296
Val Phe Thr Lys Glu Asp Thr Ile Thr Val Gln Lys Tyr Asp Ser Ala
            420                 425                 430

ggc acc aat cta gaa ggc aac gca gtc gaa att aaa aca ctt gat gaa     1344
Gly Thr Asn Leu Glu Gly Asn Ala Val Glu Ile Lys Thr Leu Asp Glu
        435                 440                 445

ctt aaa aac gct tta aaa tag                                         1365
Leu Lys Asn Ala Leu Lys  *
    450


<210> 70
<211> 454
<212> PRT
<213> Artificial Sequence

<220>
<223> Chimeric protein

<400> 70
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Ser Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Tyr Asn His Asn Gly Ser
65                  70                  75                  80
Leu Leu Ala Gly Ala Tyr Ala Ile Ser Thr Leu Ile Lys Gln Lys Leu

85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Thr Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Lys Gln Asn Val Ser Ser Leu Asp Glu Lys Asn
        195                 200                 205
Ser Val Ser Val Asp Leu Pro Gly Gly Met Thr Val Leu Val Ser Lys
    210                 215                 220
Glu Lys Asp Lys Asp Gly Lys Tyr Ser Leu Glu Ala Thr Val Asp Lys
225                 230                 235                 240
Leu Glu Leu Lys Gly Thr Ser Asp Lys Asn Asn Gly Ser Gly Thr Leu
                245                 250                 255
Glu Gly Glu Lys Thr Asp Lys Ser Lys Val Lys Leu Thr Ile Ala Asp
            260                 265                 270
Asp Leu Ser Gln Thr Lys Phe Glu Ile Phe Lys Glu Asp Ala Lys Thr
        275                 280                 285
Leu Val Ser Lys Lys Val Thr Leu Lys Asp Lys Ser Ser Thr Glu Glu
    290                 295                 300
Lys Phe Asn Glu Lys Gly Glu Thr Ser Glu Lys Thr Ile Val Arg Ala
305                 310                 315                 320
Asn Gly Thr Arg Leu Glu Tyr Thr Asp Ile Lys Ser Asp Gly Ser Gly
                325                 330                 335
Lys Ala Lys Glu Val Leu Lys Asp Phe Thr Leu Glu Gly Thr Leu Ala
            340                 345                 350
Ala Asp Gly Lys Thr Thr Leu Lys Val Thr Glu Gly Thr Val Val Leu
        355                 360                 365
Ser Lys Asn Ile Leu Lys Ser Gly Glu Ile Thr Val Ala Leu Asp Asp
    370                 375                 380
Ser Asp Thr Thr Gln Ala Thr Lys Lys Thr Gly Lys Trp Asp Ser Asn
385                 390                 395                 400
Thr Ser Thr Leu Thr Ile Ser Val Asn Ser Lys Lys Thr Lys Asn Ile
                405                 410                 415
Val Phe Thr Lys Glu Asp Thr Ile Thr Val Gln Lys Tyr Asp Ser Ala
            420                 425                 430
Gly Thr Asn Leu Glu Gly Asn Ala Val Glu Ile Lys Thr Leu Asp Glu
        435                 440                 445
Leu Lys Asn Ala Leu Lys
    450


<210> 71
<211> 1344
<212> DNA
<213> Artificial Sequence

<220>
<223> Chimeric nucleic acid

<221> CDS
<222> (1)...(1344)

<400> 71
atg gct tgt aat aat tca ggg aaa gat ggg aat aca tct gca aat tct      48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata agt aaa aaa      96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg 144
Ile Thr Asp Ser Asn Ala Val Leu Leu Ala Val Lys Glu Val Glu Ala
        35                  40                  45

ttg ctg tca tct ata gat gag ctt gct aaa gct att ggt aaa aaa ata 192
Leu Leu Ser Ser Ile Asp Glu Leu Ala Lys Ala Ile Gly Lys Lys Ile
    50                  55                  60

aaa aac gat ggt agt tta gat aat gaa gca aat cgc aac gag tca ttg 240
Lys Asn Asp Gly Ser Leu Asp Asn Glu Ala Asn Arg Asn Glu Ser Leu
65                  70                  75                  80

tta gca gga gct tat aca ata tca acc tta ata aca caa aaa tta agt 288
Leu Ala Gly Ala Tyr Thr Ile Ser Thr Leu Ile Thr Gln Lys Leu Ser
                85                  90                  95

aaa tta aac gga tca gaa ggt tta aag gaa aag att gcc gca gct aag 336
Lys Leu Asn Gly Ser Glu Gly Leu Lys Glu Lys Ile Ala Ala Ala Lys
            100                 105                 110

aaa tgc tct gaa gag ttt agt act aaa cta aaa gat aat cat gca cag 384
Lys Cys Ser Glu Glu Phe Ser Thr Lys Leu Lys Asp Asn His Ala Gln
        115                 120                 125

ctt ggt ata cag ggc gtt act gat gaa aat gca aaa aaa gct att tta 432
Leu Gly Ile Gln Gly Val Thr Asp Glu Asn Ala Lys Lys Ala Ile Leu
    130                 135                 140

aaa gca aat gca gcg ggt aaa gat aag ggc gtt gaa gaa ctt gaa aag 480
Lys Ala Asn Ala Ala Gly Lys Asp Lys Gly Val Glu Glu Leu Glu Lys
145                 150                 155                 160

ttg tcc gga tca tta gaa agc tta tca aaa gca gct aaa gag atg ctt 528
Leu Ser Gly Ser Leu Glu Ser Leu Ser Lys Ala Ala Lys Glu Met Leu
                165                 170                 175

gct aat tca gtt aaa gag ctt aca agc cct gtt gtc cat ggc aag caa 576
Ala Asn Ser Val Lys Glu Leu Thr Ser Pro Val Val His Gly Lys Gln
            180                 185                 190

aat gtt agc agc ctt gat gaa aaa aat agc gtt tca gta gat tta cct 624
Asn Val Ser Ser Leu Asp Glu Lys Asn Ser Val Ser Val Asp Leu Pro
        195                 200                 205

ggt gga atg aca gtt ctt gta agt aaa gaa aaa gac aaa gac ggt aaa 672
Gly Gly Met Thr Val Leu Val Ser Lys Glu Lys Asp Lys Asp Gly Lys
    210                 215                 220

tac agt cta gag gca aca gta gac aag ctt gag ctt aaa gga act tct 720
Tyr Ser Leu Glu Ala Thr Val Asp Lys Leu Glu Leu Lys Gly Thr Ser
225                 230                 235                 240

gat aaa aac aac ggt tct gga aca ctt gaa ggt gaa aaa act gac aaa 768
Asp Lys Asn Asn Gly Ser Gly Thr Leu Glu Gly Glu Lys Thr Asp Lys
                245                 250                 255

agt aaa gta aaa tta aca att gct gat gac cta agt caa act aaa ttt 816
Ser Lys Val Lys Leu Thr Ile Ala Asp Asp Leu Ser Gln Thr Lys Phe
            260                 265                 270

gaa att ttc aaa gaa gat gcc aaa aca tta gta tca aaa aaa gta acc 864
Glu Ile Phe Lys Glu Asp Ala Lys Thr Leu Val Ser Lys Lys Val Thr
        275                 280                 285

ctt aaa gac aag tca tca aca gaa gaa aaa ttc aac gaa aag ggt gaa 912
Leu Lys Asp Lys Ser Ser Thr Glu Glu Lys Phe Asn Glu Lys Gly Glu
    290                 295                 300

aca tct gaa aaa aca ata gta aga gca aat gga acc aga ctt gaa tac     960
Thr Ser Glu Lys Thr Ile Val Arg Ala Asn Gly Thr Arg Leu Glu Tyr
305                 310                 315                 320

aca gac ata aaa agc gat gga tcc gga aaa gct aaa gaa gtt tta aaa     1008
Thr Asp Ile Lys Ser Asp Gly Ser Gly Lys Ala Lys Glu Val Leu Lys
                325                 330                 335

gac ttt act ctt gaa gga act cta gct gct gac ggc aaa aca aca ttg     1056
Asp Phe Thr Leu Glu Gly Thr Leu Ala Ala Asp Gly Lys Thr Thr Leu
            340                 345                 350

aaa gtt aca gaa ggc act gtt gtt tta agc aag aac att tta aaa tcc     1104
Lys Val Thr Glu Gly Thr Val Val Leu Ser Lys Asn Ile Leu Lys Ser
        355                 360                 365

gga gaa ata aca gtt gca ctt gat gac tct gac act act cag gct act     1152
Gly Glu Ile Thr Val Ala Leu Asp Asp Ser Asp Thr Thr Gln Ala Thr
    370                 375                 380

aaa aaa act gga aaa tgg gat tca aat act tcc act tta aca att agt     1200
Lys Lys Thr Gly Lys Trp Asp Ser Asn Thr Ser Thr Leu Thr Ile Ser
385                 390                 395                 400

gtg aat agc aaa aaa act aaa aac att gta ttt aca aaa gaa gac aca     1248
Val Asn Ser Lys Lys Thr Lys Asn Ile Val Phe Thr Lys Glu Asp Thr
                405                 410                 415

ata aca gta caa aaa tac gac tca gca ggc acc aat cta gaa ggc aac     1296
Ile Thr Val Gln Lys Tyr Asp Ser Ala Gly Thr Asn Leu Glu Gly Asn
            420                 425                 430

gca gtc gaa att aaa aca ctt gat gaa ctt aaa aac gct tta aaa tag     1344
Ala Val Glu Ile Lys Thr Leu Asp Glu Leu Lys Asn Ala Leu Lys  *
        435                 440                 445

<210> 72
<211> 447
<212> PRT
<213> Artificial Sequence

<220>
<223> Chimeric protein

<400> 72
Met Ala Cys Asn Asn Ser Gly Lys Asp Gly Asn Thr Ser Ala Asn Ser
 1               5                  10                  15
Ala Asp Glu Ser Val Lys Gly Pro Asn Leu Thr Glu Ile Ser Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Leu Ala Lys Ala Ile Gly Lys Lys Ile
    50                  55                  60
Lys Asn Asp Gly Ser Leu Asp Asn Glu Ala Asn Arg Asn Glu Ser Leu
65                  70                  75                  80
Leu Ala Gly Ala Tyr Thr Ile Ser Thr Leu Ile Thr Gln Lys Leu Ser
                85                  90                  95
Lys Leu Asn Gly Ser Glu Gly Leu Lys Glu Lys Ile Ala Ala Ala Lys
            100                 105                 110
Lys Cys Ser Glu Glu Phe Ser Thr Lys Leu Lys Asp Asn His Ala Gln
        115                 120                 125
Leu Gly Ile Gln Gly Val Thr Asp Glu Asn Ala Lys Lys Ala Ile Leu
    130                 135                 140
Lys Ala Asn Ala Ala Gly Lys Asp Lys Gly Val Glu Glu Leu Glu Lys
145                 150                 155                 160

Leu Ser Gly Ser Leu Glu Ser Leu Ser Lys Ala Ala Lys Glu Met Leu
                165                 170                 175
Ala Asn Ser Val Lys Glu Leu Thr Ser Pro Val Val His Gly Lys Gln
            180                 185                 190
Asn Val Ser Ser Leu Asp Glu Lys Asn Ser Val Ser Val Asp Leu Pro
        195                 200                 205
Gly Gly Met Thr Val Leu Val Ser Lys Glu Lys Asp Lys Asp Gly Lys
    210                 215                 220
Tyr Ser Leu Glu Ala Thr Val Asp Lys Leu Glu Leu Lys Gly Thr Ser
225                 230                 235                 240
Asp Lys Asn Asn Gly Ser Gly Thr Leu Glu Gly Glu Lys Thr Asp Lys
                245                 250                 255
Ser Lys Val Lys Leu Thr Ile Ala Asp Asp Leu Ser Gln Thr Lys Phe
            260                 265                 270
Glu Ile Phe Lys Glu Asp Ala Lys Thr Leu Val Ser Lys Lys Val Thr
        275                 280                 285
Leu Lys Asp Lys Ser Ser Thr Glu Glu Lys Phe Asn Glu Lys Gly Glu
    290                 295                 300
Thr Ser Glu Lys Thr Ile Val Arg Ala Asn Gly Thr Arg Leu Glu Tyr
305                 310                 315                 320
Thr Asp Ile Lys Ser Asp Gly Ser Gly Lys Ala Lys Glu Val Leu Lys
                325                 330                 335
Asp Phe Thr Leu Glu Gly Thr Leu Ala Ala Asp Gly Lys Thr Thr Leu
            340                 345                 350
Lys Val Thr Glu Gly Thr Val Val Leu Ser Lys Asn Ile Leu Lys Ser
        355                 360                 365
Gly Glu Ile Thr Val Ala Leu Asp Asp Ser Asp Thr Thr Gln Ala Thr
    370                 375                 380
Lys Lys Thr Gly Lys Trp Asp Ser Asn Thr Ser Thr Leu Thr Ile Ser
385                 390                 395                 400
Val Asn Ser Lys Lys Thr Lys Asn Ile Val Phe Thr Lys Glu Asp Thr
                405                 410                 415
Ile Thr Val Gln Lys Tyr Asp Ser Ala Gly Thr Asn Leu Glu Gly Asn
            420                 425                 430
Ala Val Glu Ile Lys Thr Leu Asp Glu Leu Lys Asn Ala Leu Lys
        435                 440                 445


<210> 73
<211> 1305
<212> DNA
<213> Artificial Sequence

<220>
<223> Chimeric nucleic acid

<221> CDS
<222> (1)...(1305)

<400> 73
atg gct tgt aat aat tca gga aaa gat ggg aat aca tct gca aat tct     48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata agt aaa aaa     96
Ala Asp Glu Ser Val Lys Gly Pro Asn Leu Thr Glu Ile Ser Lys Lys
            20                  25                  30

att aca gaa tct aac gca gtt gtt ctg gct gtg aaa gaa att gaa act    144
Ile Thr Glu Ser Asn Ala Val Val Leu Ala Val Lys Glu Ile Glu Thr
        35                  40                  45

ttg ctt gca tct ata gat gaa ctt gct act aaa gct att ggt aaa aaa    192
Leu Leu Ala Ser Ile Asp Glu Leu Ala Thr Lys Ala Ile Gly Lys Lys
    50                  55                  60

ata caa caa aat ggt ggt tta gct gtc gaa gcg ggg cat aat gga aca    240
Ile Gln Gln Asn Gly Gly Leu Ala Val Glu Ala Gly His Asn Gly Thr

65                  70                  75                  80

ttg tta gca ggt gct tat aca ata tca aaa cta ata aca caa aaa tta     288
Leu Leu Ala Gly Ala Tyr Thr Ile Ser Lys Leu Ile Thr Gln Lys Leu
                85                  90                  95

gat gga ttg aaa aat tca gaa aaa tta aag gaa aaa att gaa aat gct     336
Asp Gly Leu Lys Asn Ser Glu Lys Leu Lys Glu Lys Ile Glu Asn Ala
            100                 105                 110

aag aaa tgt tct gaa gat ttt act aaa aaa cta gaa gga gaa cat gcg     384
Lys Lys Cys Ser Glu Asp Phe Thr Lys Lys Leu Glu Gly Glu His Ala
        115                 120                 125

caa ctt gga att gaa aat gtt act gat gag aat gca aaa aaa gct att     432
Gln Leu Gly Ile Glu Asn Val Thr Asp Glu Asn Ala Lys Lys Ala Ile
    130                 135                 140

tta ata aca gat gca gct aaa gat aag ggc gct gca gag ctt gaa aag     480
Leu Ile Thr Asp Ala Ala Lys Asp Lys Gly Ala Ala Glu Leu Glu Lys
145                 150                 155                 160

cta ttt aaa gca gta gaa aac ttg gca aaa gca gct aaa gag atg ctt     528
Leu Phe Lys Ala Val Glu Asn Leu Ala Lys Ala Ala Lys Glu Met Leu
                165                 170                 175

gct aat tca gtt aaa gag ctt aca agt cct att gtg cat ggc gtt tca     576
Ala Asn Ser Val Lys Glu Leu Thr Ser Pro Ile Val His Gly Val Ser
            180                 185                 190

gta gat ttg cct ggt gaa atg aaa gtt ctt gta agc aaa gaa aaa aac     624
Val Asp Leu Pro Gly Glu Met Lys Val Leu Val Ser Lys Glu Lys Asn
        195                 200                 205

aaa gac ggc aag tac gat cta att gca aca gta gac aag ctt gag ctt     672
Lys Asp Gly Lys Tyr Asp Leu Ile Ala Thr Val Asp Lys Leu Glu Leu
    210                 215                 220

aaa gga act tct gat aaa aac aat gga tct gga gta ctt gaa ggc gta     720
Lys Gly Thr Ser Asp Lys Asn Asn Gly Ser Gly Val Leu Glu Gly Val
225                 230                 235                 240

aaa gct gac aaa agt aaa gta aaa tta aca att tct gac gat cta ggt     768
Lys Ala Asp Lys Ser Lys Val Lys Leu Thr Ile Ser Asp Asp Leu Gly
                245                 250                 255

caa acc aca ctt gaa gtt ttc aaa gaa gat ggc aaa aca cta gta tca     816
Gln Thr Thr Leu Glu Val Phe Lys Glu Asp Gly Lys Thr Leu Val Ser
            260                 265                 270

aaa aaa gta act tcc aaa gac aag tca tca aca gaa gaa aaa ttc aat     864
Lys Lys Val Thr Ser Lys Asp Lys Ser Ser Thr Glu Glu Lys Phe Asn
        275                 280                 285

gaa aaa ggt gaa gta tct gaa aaa ata ata aca aga gca gac gga acc     912
Glu Lys Gly Glu Val Ser Glu Lys Ile Ile Thr Arg Ala Asp Gly Thr
    290                 295                 300

aga ctt gaa tac aca gga att aaa agc gat gga tct gga aaa gct aaa     960
Arg Leu Glu Tyr Thr Gly Ile Lys Ser Asp Gly Ser Gly Lys Ala Lys
305                 310                 315                 320

gag gtt tta aaa aaa ttt act ctt gaa gga aaa gta gct aat gat aaa     1008
Glu Val Leu Lys Lys Phe Thr Leu Glu Gly Lys Val Ala Asn Asp Lys
                325                 330                 335

gta aca ttg gaa gta aaa gaa gga acc gtt act tta agt aag aac att     1056
Val Thr Leu Glu Val Lys Glu Gly Thr Val Thr Leu Ser Lys Asn Ile

340                 345                 350

tca aaa tct ggg gaa gtt tca gtt gaa ctt aat gac act gac agt agt     1104
Ser Lys Ser Gly Glu Val Ser Val Glu Leu Asn Asp Thr Asp Ser Ser
        355                 360                 365

gct gct act aaa aaa act gca gct tgg aat tca ggc act tca act tta     1152
Ala Ala Thr Lys Lys Thr Ala Ala Trp Asn Ser Gly Thr Ser Thr Leu
    370                 375                 380

aca att act gta aac agt aaa aaa act aaa gac ctt gtg ttt aca aaa     1200
Thr Ile Thr Val Asn Ser Lys Lys Thr Lys Asp Leu Val Phe Thr Lys
385                 390                 395                 400

gaa aac aca att aca gta caa caa tac gac tca aat ggc acc aaa tta     1248
Glu Asn Thr Ile Thr Val Gln Gln Tyr Asp Ser Asn Gly Thr Lys Leu
                405                 410                 415

gag ggg tca gca gtt gaa att aca aaa ctt gat gaa att aaa aac gct     1296
Glu Gly Ser Ala Val Glu Ile Thr Lys Leu Asp Glu Ile Lys Asn Ala
            420                 425                 430

tta aaa taa                                                         1305
Leu Lys  *

<210> 74
<211> 434
<212> PRT
<213> Artificial Sequence

<220>
<223> Chimeric protein

<400> 74
Met Ala Cys Asn Asn Ser Gly Lys Asp Gly Asn Thr Ser Ala Asn Ser
 1               5                  10                  15
Ala Asp Glu Ser Val Lys Gly Pro Asn Leu Thr Glu Ile Ser Lys Lys
            20                  25                  30
Ile Thr Glu Ser Asn Ala Val Val Leu Ala Val Lys Glu Ile Glu Thr
        35                  40                  45
Leu Leu Ala Ser Ile Asp Glu Leu Ala Thr Lys Ala Ile Gly Lys Lys
    50                  55                  60
Ile Gln Gln Asn Gly Gly Leu Ala Val Glu Ala Gly His Asn Gly Thr
65                  70                  75                  80
Leu Leu Ala Gly Ala Tyr Thr Ile Ser Lys Leu Ile Thr Gln Lys Leu
                85                  90                  95
Asp Gly Leu Lys Asn Ser Glu Lys Leu Lys Glu Lys Ile Glu Asn Ala
            100                 105                 110
Lys Lys Cys Ser Glu Asp Phe Thr Lys Lys Leu Glu Gly Glu His Ala
        115                 120                 125
Gln Leu Gly Ile Glu Asn Val Thr Asp Glu Asn Ala Lys Lys Ala Ile
    130                 135                 140
Leu Ile Thr Asp Ala Ala Lys Asp Lys Gly Ala Ala Glu Leu Glu Lys
145                 150                 155                 160
Leu Phe Lys Ala Val Glu Asn Leu Ala Lys Ala Ala Lys Glu Met Leu
                165                 170                 175
Ala Asn Ser Val Lys Glu Leu Thr Ser Pro Ile Val His Gly Val Ser
            180                 185                 190
Val Asp Leu Pro Gly Glu Met Lys Val Leu Val Ser Lys Glu Lys Asn
        195                 200                 205
Lys Asp Gly Lys Tyr Asp Leu Ile Ala Thr Val Asp Lys Leu Glu Leu
    210                 215                 220
Lys Gly Thr Ser Asp Lys Asn Asn Gly Ser Gly Val Leu Glu Gly Val
225                 230                 235                 240
Lys Ala Asp Lys Ser Lys Val Lys Leu Thr Ile Ser Asp Asp Leu Gly
                245                 250                 255

Gln Thr Thr Leu Glu Val Phe Lys Glu Asp Gly Lys Thr Leu Val Ser
            260                 265                 270
Lys Lys Val Thr Ser Lys Asp Lys Ser Ser Thr Glu Glu Lys Phe Asn
        275                 280                 285
Glu Lys Gly Glu Val Ser Glu Lys Ile Ile Thr Arg Ala Asp Gly Thr
    290                 295                 300
Arg Leu Glu Tyr Thr Gly Ile Lys Ser Asp Gly Ser Gly Lys Ala Lys
305                 310                 315                 320
Glu Val Leu Lys Lys Phe Thr Leu Glu Gly Lys Val Ala Asn Asp Lys
                325                 330                 335
Val Thr Leu Glu Val Lys Glu Gly Thr Val Thr Leu Ser Lys Asn Ile
            340                 345                 350
Ser Lys Ser Gly Glu Val Ser Val Glu Leu Asn Asp Thr Asp Ser Ser
        355                 360                 365
Ala Ala Thr Lys Lys Thr Ala Ala Trp Asn Ser Gly Thr Ser Thr Leu
    370                 375                 380
Thr Ile Thr Val Asn Ser Lys Lys Thr Lys Asp Leu Val Phe Thr Lys
385                 390                 395                 400
Glu Asn Thr Ile Thr Val Gln Gln Tyr Asp Ser Asn Gly Thr Lys Leu
                405                 410                 415
Glu Gly Ser Ala Val Glu Ile Thr Lys Leu Asp Glu Ile Lys Asn Ala
            420                 425                 430
Leu Lys

<210> 75
<211> 1332
<212> DNA
<213> Artificial Sequence

<220>
<223> Chimeric nucleic acid

<221> CDS
<222> (1)...(1332)

<400> 75
atg gct tgt agt aat tca ggg aaa ggt ggg gat tct gca tct act aat      48
Met Ala Cys Ser Asn Ser Gly Lys Gly Gly Asp Ser Ala Ser Thr Asn
 1               5                  10                  15

cct gct gac gag tct gcg aaa ggg cct aat ctt aca gaa ata agc aaa      96
Pro Ala Asp Glu Ser Ala Lys Gly Pro Asn Leu Thr Glu Ile Ser Lys
            20                  25                  30

aaa att aca gat tct aat gca ttt gta ctt gct gtt aaa gaa gtt gag     144
Lys Ile Thr Asp Ser Asn Ala Phe Val Leu Ala Val Lys Glu Val Glu
        35                  40                  45

act ttg gtt tta tct ata gat gaa ctt gct aag aaa gct att ggt caa     192
Thr Leu Val Leu Ser Ile Asp Glu Leu Ala Lys Lys Ala Ile Gly Gln
    50                  55                  60

aaa ata gac aat aat aat ggt tta gct gct tta aat aat cag aat gga     240
Lys Ile Asp Asn Asn Asn Gly Leu Ala Ala Leu Asn Asn Gln Asn Gly
65                  70                  75                  80

tcg ttg tta gca gga gcc tat gca ata tca acc cta ata aca gaa aaa     288
Ser Leu Leu Ala Gly Ala Tyr Ala Ile Ser Thr Leu Ile Thr Glu Lys
                85                  90                  95

ttg agt aaa ttg aaa aat tta gaa gaa tta aag aca gaa att gca aag     336
Leu Ser Lys Leu Lys Asn Leu Glu Glu Leu Lys Thr Glu Ile Ala Lys
            100                 105                 110

gct aag aaa tgt tcc gaa gaa ttt act aat aaa cta aaa agt ggt cat     384
Ala Lys Lys Cys Ser Glu Glu Phe Thr Asn Lys Leu Lys Ser Gly His

115                 120                 125

gca gat ctt ggc aaa cag gat gct acc gat gat cat gca aaa gca gct     432
Ala Asp Leu Gly Lys Gln Asp Ala Thr Asp Asp His Ala Lys Ala Ala
    130                 135                 140

att tta aaa aca cat gca act acc gat aaa ggt gct aaa gaa ttt aaa     480
Ile Leu Lys Thr His Ala Thr Thr Asp Lys Gly Ala Lys Glu Phe Lys
145                 150                 155                 160

gat tta ttt gaa tca gta gaa ggt ttg tta aaa gca gct caa gta gca     528
Asp Leu Phe Glu Ser Val Glu Gly Leu Leu Lys Ala Ala Gln Val Ala
                165                 170                 175

cta act aat tca gtt aaa gaa ctt aca agt cct gtt gta gca gaa agt     576
Leu Thr Asn Ser Val Lys Glu Leu Thr Ser Pro Val Val Ala Glu Ser
            180                 185                 190

cca aaa aaa cct tcc atg gcc gtt tca gta gat ttg cct ggt gaa atg     624
Pro Lys Lys Pro Ser Met Ala Val Ser Val Asp Leu Pro Gly Glu Met
        195                 200                 205

aaa gtt ctt gta agc aaa gaa aaa aac aaa gac ggc aag tac gat cta     672
Lys Val Leu Val Ser Lys Glu Lys Asn Lys Asp Gly Lys Tyr Asp Leu
    210                 215                 220

att gca aca gta gac aag ctt gag ctt aaa gga act tct gat aaa aac     720
Ile Ala Thr Val Asp Lys Leu Glu Leu Lys Gly Thr Ser Asp Lys Asn
225                 230                 235                 240

aat gga tct gga gta ctt gaa ggc gta aaa gct gac aaa agt aaa gta     768
Asn Gly Ser Gly Val Leu Glu Gly Val Lys Ala Asp Lys Ser Lys Val
                245                 250                 255

aaa tta aca att tct gac gat cta ggt caa acc aca ctt gaa gtt ttc     816
Lys Leu Thr Ile Ser Asp Asp Leu Gly Gln Thr Thr Leu Glu Val Phe
            260                 265                 270

aaa gaa gat ggc aaa aca cta gta tca aaa aaa gta act tcc aaa gac     864
Lys Glu Asp Gly Lys Thr Leu Val Ser Lys Lys Val Thr Ser Lys Asp
        275                 280                 285

aag tca tca aca gaa gaa aaa ttc aat gaa aaa ggt gaa gta tct gaa     912
Lys Ser Ser Thr Glu Glu Lys Phe Asn Glu Lys Gly Glu Val Ser Glu
    290                 295                 300

aaa ata ata aca aga gca gac gga acc aga ctt gaa tac aca gga att     960
Lys Ile Ile Thr Arg Ala Asp Gly Thr Arg Leu Glu Tyr Thr Gly Ile
305                 310                 315                 320

aaa agc gat gga tct gga aaa gct aaa gag gtt tta aaa aaa ttt act     1008
Lys Ser Asp Gly Ser Gly Lys Ala Lys Glu Val Leu Lys Lys Phe Thr
                325                 330                 335

ctt gaa gga aaa gta gct aat gat aaa gta aca ttg gaa gta aaa gaa     1056
Leu Glu Gly Lys Val Ala Asn Asp Lys Val Thr Leu Glu Val Lys Glu
            340                 345                 350

gga acc gtt act tta agt aag aat att tca aaa tct ggg gaa gtt tca     1104
Gly Thr Val Thr Leu Ser Lys Asn Ile Ser Lys Ser Gly Glu Val Ser
        355                 360                 365

gtt gaa ctt aat gac act gac agt agt gct gct act aaa aaa act gca     1152
Val Glu Leu Asn Asp Thr Asp Ser Ser Ala Ala Thr Lys Lys Thr Ala
    370                 375                 380

gct tgg aat tca aaa act tcc act tta aca att agt gtg aat agc caa     1200
Ala Trp Asn Ser Lys Thr Ser Thr Leu Thr Ile Ser Val Asn Ser Gln

385                 390                 395                 400

aaa acc aaa aac ctt gta ttc aca aaa gaa gac aca ata aca gta caa     1248
Lys Thr Lys Asn Leu Val Phe Thr Lys Glu Asp Thr Ile Thr Val Gln
            405                 410                 415

aaa tac gac tca gca ggc acc aat cta gaa ggc aaa gca gtc gaa att     1296
Lys Tyr Asp Ser Ala Gly Thr Asn Leu Glu Gly Lys Ala Val Glu Ile
            420                 425                 430

aca aca ctt aaa gaa ctt aaa aac gct tta aaa taa                     1332
Thr Thr Leu Lys Glu Leu Lys Asn Ala Leu Lys  *
        435                 440


<210> 76
<211> 443
<212> PRT
<213> Artificial Sequence

<220>
<223> Chimeric protein

<400> 76
Met Ala Cys Ser Asn Ser Gly Lys Gly Gly Asp Ser Ala Ser Thr Asn
1               5                   10                  15
Pro Ala Asp Glu Ser Ala Lys Gly Pro Asn Leu Thr Glu Ile Ser Lys
            20                  25                  30
Lys Ile Thr Asp Ser Asn Ala Phe Val Leu Ala Val Lys Glu Val Glu
        35                  40                  45
Thr Leu Val Leu Ser Ile Asp Glu Leu Ala Lys Lys Ala Ile Gly Gln
    50                  55                  60
Lys Ile Asp Asn Asn Asn Gly Leu Ala Ala Leu Asn Asn Gln Asn Gly
65                  70                  75                  80
Ser Leu Leu Ala Gly Ala Tyr Ala Ile Ser Thr Leu Ile Thr Glu Lys
                85                  90                  95
Leu Ser Lys Leu Lys Asn Leu Glu Glu Leu Lys Thr Glu Ile Ala Lys
            100                 105                 110
Ala Lys Lys Cys Ser Glu Glu Phe Thr Asn Lys Leu Lys Ser Gly His
        115                 120                 125
Ala Asp Leu Gly Lys Gln Asp Ala Thr Asp Asp His Ala Lys Ala Ala
    130                 135                 140
Ile Leu Lys Thr His Ala Thr Thr Asp Lys Gly Ala Lys Glu Phe Lys
145                 150                 155                 160
Asp Leu Phe Glu Ser Val Glu Gly Leu Leu Lys Ala Ala Gln Val Ala
                165                 170                 175
Leu Thr Asn Ser Val Lys Glu Leu Thr Ser Pro Val Val Ala Glu Ser
            180                 185                 190
Pro Lys Lys Pro Ser Met Ala Val Ser Val Asp Leu Pro Gly Glu Met
        195                 200                 205
Lys Val Leu Val Ser Lys Glu Lys Asn Lys Asp Gly Lys Tyr Asp Leu
    210                 215                 220
Ile Ala Thr Val Asp Lys Leu Glu Leu Lys Gly Thr Ser Asp Lys Asn
225                 230                 235                 240
Asn Gly Ser Gly Val Leu Glu Gly Val Lys Ala Asp Lys Ser Lys Val
                245                 250                 255
Lys Leu Thr Ile Ser Asp Asp Leu Gly Gln Thr Thr Leu Glu Val Phe
            260                 265                 270
Lys Glu Asp Gly Lys Thr Leu Val Ser Lys Lys Val Thr Ser Lys Asp
        275                 280                 285
Lys Ser Ser Thr Glu Glu Lys Phe Asn Glu Lys Gly Glu Val Ser Glu
    290                 295                 300
Lys Ile Ile Thr Arg Ala Asp Gly Thr Arg Leu Glu Tyr Thr Gly Ile
305                 310                 315                 320
Lys Ser Asp Gly Ser Gly Lys Ala Lys Glu Val Leu Lys Lys Phe Thr
                325                 330                 335
Leu Glu Gly Lys Val Ala Asn Asp Lys Val Thr Leu Glu Val Lys Glu
            340                 345                 350

Gly Thr Val Thr Leu Ser Lys Asn Ile Ser Lys Ser Gly Glu Val Ser
        355                 360                 365
Val Glu Leu Asn Asp Thr Asp Ser Ser Ala Ala Thr Lys Lys Thr Ala
    370                 375                 380
Ala Trp Asn Ser Lys Thr Ser Thr Leu Thr Ile Ser Val Asn Ser Gln
385                 390                 395                 400
Lys Thr Lys Asn Leu Val Phe Thr Lys Glu Asp Thr Ile Thr Val Gln
                405                 410                 415
Lys Tyr Asp Ser Ala Gly Thr Asn Leu Glu Gly Lys Ala Val Glu Ile
            420                 425                 430
Thr Thr Leu Lys Glu Leu Lys Asn Ala Leu Lys
        435                 440

<210> 77
<211> 1317
<212> DNA
<213> Artificial Sequence

<220>
<223> Chimeric nucleic acid

<221> CDS
<222> (1)...(1317)

<400> 77
atg gct tgt aat aat tca ggt ggg gat tct gca tct act aat cct gat     48
Met Ala Cys Asn Asn Ser Gly Gly Asp Ser Ala Ser Thr Asn Pro Asp
 1               5                  10                  15

gag tct gca aaa gga cct aat ctt acc gta ata agc aaa aaa att aca     96
Glu Ser Ala Lys Gly Pro Asn Leu Thr Val Ile Ser Lys Lys Ile Thr
            20                  25                  30

gat tct aat gca ttt tta ctg gct gtg aaa gaa gtt gag gct ttg ctt    144
Asp Ser Asn Ala Phe Leu Leu Ala Val Lys Glu Val Glu Ala Leu Leu
        35                  40                  45

tca tct ata gat gaa ctt tct aaa gct att ggt aaa aaa ata aaa aat    192
Ser Ser Ile Asp Glu Leu Ser Lys Ala Ile Gly Lys Lys Ile Lys Asn
    50                  55                  60

gat ggt act tta gat aac gaa gca aat cga aac gaa tca ttg ata gca    240
Asp Gly Thr Leu Asp Asn Glu Ala Asn Arg Asn Glu Ser Leu Ile Ala
65                  70                  75                  80

gga gct tat gaa ata tca aaa cta ata aca caa aaa tta agt gta ttg    288
Gly Ala Tyr Glu Ile Ser Lys Leu Ile Thr Gln Lys Leu Ser Val Leu
                85                  90                  95

aat tca gaa gaa tta aag gaa aaa att aaa gag gct aag gat tgt tcc    336
Asn Ser Glu Glu Leu Lys Glu Lys Ile Lys Glu Ala Lys Asp Cys Ser
            100                 105                 110

gaa aaa ttt act act aag cta aaa gat agt cat gca gag ctt ggt ata    384
Glu Lys Phe Thr Thr Lys Leu Lys Asp Ser His Ala Glu Leu Gly Ile
        115                 120                 125

caa agc gtt cag gat gat aat gca aaa aaa gct att tta aaa aca cat    432
Gln Ser Val Gln Asp Asp Asn Ala Lys Lys Ala Ile Leu Lys Thr His
    130                 135                 140

gga act aaa gac aag ggt gct aaa gaa ctt gaa gag tta ttt aaa tca    480
Gly Thr Lys Asp Lys Gly Ala Lys Glu Leu Glu Glu Leu Phe Lys Ser
145                 150                 155                 160

cta gaa agc ttg tca aaa gca gcg caa gca gca tta act aat tca gtt    528
Leu Glu Ser Leu Ser Lys Ala Ala Gln Ala Ala Leu Thr Asn Ser Val

                165                 170                 175

aaa gag ctt aca aat cct gtt gtg gca gaa agt cca aaa aaa cct tcc 576
Lys Glu Leu Thr Asn Pro Val Val Ala Glu Ser Pro Lys Lys Pro Ser
            180                 185                 190

atg gcc gtt tca gta gat ttg cct ggt gaa atg aaa gtt ctt gta agc 624
Met Ala Val Ser Val Asp Leu Pro Gly Glu Met Lys Val Leu Val Ser
        195                 200                 205

aaa gaa aaa aac aaa gac ggc aag tac gat cta att gca aca gta gac 672
Lys Glu Lys Asn Lys Asp Gly Lys Tyr Asp Leu Ile Ala Thr Val Asp
    210                 215                 220

aag ctt gag ctt aaa gga act tct gat aaa aac aat gga tct gga gta 720
Lys Leu Glu Leu Lys Gly Thr Ser Asp Lys Asn Asn Gly Ser Gly Val
225                 230                 235                 240

ctt gaa ggc gta aaa gct gac aaa agt aaa gta aaa tta aca att tct 768
Leu Glu Gly Val Lys Ala Asp Lys Ser Lys Val Lys Leu Thr Ile Ser
                245                 250                 255

gac gat cta ggt caa acc aca ctt gaa gtt ttc aaa gaa gat ggc aaa 816
Asp Asp Leu Gly Gln Thr Thr Leu Glu Val Phe Lys Glu Asp Gly Lys
            260                 265                 270

aca cta gta tca aaa aaa gta act tcc aaa gac aag tca tca aca gaa 864
Thr Leu Val Ser Lys Lys Val Thr Ser Lys Asp Lys Ser Ser Thr Glu
        275                 280                 285

gaa aaa ttc aat gaa aaa ggt gaa gta tct gaa aaa ata ata aca aga 912
Glu Lys Phe Asn Glu Lys Gly Glu Val Ser Glu Lys Ile Ile Thr Arg
    290                 295                 300

gca gac gga acc aga ctt gaa tac aca gga att aaa agc gat gga tct 960
Ala Asp Gly Thr Arg Leu Glu Tyr Thr Gly Ile Lys Ser Asp Gly Ser
305                 310                 315                 320

gga aaa gct aaa gag gtt tta aaa aaa ttt act ctt gaa gga aaa gta 1008
Gly Lys Ala Lys Glu Val Leu Lys Lys Phe Thr Leu Glu Gly Lys Val
                325                 330                 335

gct aat gat aaa gta aca ttg gaa gta aaa gaa gga acc gtt act tta 1056
Ala Asn Asp Lys Val Thr Leu Glu Val Lys Glu Gly Thr Val Thr Leu
            340                 345                 350

agt aag aac att tca aaa tct ggg gaa gtt tca gtt gaa ctt aat gac 1104
Ser Lys Asn Ile Ser Lys Ser Gly Glu Val Ser Val Glu Leu Asn Asp
        355                 360                 365

act gac agt agt gct gct act aaa aaa act gca gct tgg aat tca aaa 1152
Thr Asp Ser Ser Ala Ala Thr Lys Lys Thr Ala Ala Trp Asn Ser Lys
    370                 375                 380

act tct act tta aca att agt gtt aac agc aaa aaa act aca caa ctt 1200
Thr Ser Thr Leu Thr Ile Ser Val Asn Ser Lys Lys Thr Thr Gln Leu
385                 390                 395                 400

gtg ttt act aaa caa gac aca ata act gta caa aaa tac gac tcc gca 1248
Val Phe Thr Lys Gln Asp Thr Ile Thr Val Gln Lys Tyr Asp Ser Ala
                405                 410                 415

ggt acc aat tta gaa ggc aca gca gtc gaa att aaa aca ctt gat gaa 1296
Gly Thr Asn Leu Glu Gly Thr Ala Val Glu Ile Lys Thr Leu Asp Glu
            420                 425                 430

ctt aaa aac gct tta aaa taa 1317
Leu Lys Asn Ala Leu Lys *

        435

<210> 78
<211> 438
<212> PRT
<213> Artificial Sequence

<220>
<223> Chimeric protein

<400> 78
Met Ala Cys Asn Asn Ser Gly Gly Asp Ser Ala Ser Thr Asn Pro Asp
1               5                   10                  15
Glu Ser Ala Lys Gly Pro Asn Leu Thr Val Ile Ser Lys Lys Ile Thr
            20                  25                  30
Asp Ser Asn Ala Phe Leu Leu Ala Val Lys Glu Val Glu Ala Leu Leu
        35                  40                  45
Ser Ser Ile Asp Glu Leu Ser Lys Ala Ile Gly Lys Lys Ile Lys Asn
    50                  55                  60
Asp Gly Thr Leu Asp Asn Glu Ala Asn Arg Asn Glu Ser Leu Ile Ala
65                  70                  75                  80
Gly Ala Tyr Glu Ile Ser Lys Leu Ile Thr Gln Lys Leu Ser Val Leu
                85                  90                  95
Asn Ser Glu Glu Leu Lys Glu Lys Ile Lys Glu Ala Lys Asp Cys Ser
            100                 105                 110
Glu Lys Phe Thr Thr Lys Leu Lys Asp Ser His Ala Glu Leu Gly Ile
        115                 120                 125
Gln Ser Val Gln Asp Asp Asn Ala Lys Lys Ala Ile Leu Lys Thr His
    130                 135                 140
Gly Thr Lys Asp Lys Gly Ala Lys Glu Leu Glu Glu Leu Phe Lys Ser
145                 150                 155                 160
Leu Glu Ser Leu Ser Lys Ala Ala Gln Ala Ala Leu Thr Asn Ser Val
                165                 170                 175
Lys Glu Leu Thr Asn Pro Val Val Ala Glu Ser Pro Lys Lys Pro Ser
            180                 185                 190
Met Ala Val Ser Val Asp Leu Pro Gly Glu Met Lys Val Leu Val Ser
        195                 200                 205
Lys Glu Lys Asn Lys Asp Gly Lys Tyr Asp Leu Ile Ala Thr Val Asp
    210                 215                 220
Lys Leu Glu Leu Lys Gly Thr Ser Asp Lys Asn Asn Gly Ser Gly Val
225                 230                 235                 240
Leu Glu Gly Val Lys Ala Asp Lys Ser Lys Val Lys Leu Thr Ile Ser
                245                 250                 255
Asp Asp Leu Gly Gln Thr Thr Leu Glu Val Phe Lys Glu Asp Gly Lys
            260                 265                 270
Thr Leu Val Ser Lys Lys Val Thr Ser Lys Asp Lys Ser Ser Thr Glu
        275                 280                 285
Glu Lys Phe Asn Glu Lys Gly Glu Val Ser Glu Lys Ile Ile Thr Arg
    290                 295                 300
Ala Asp Gly Thr Arg Leu Glu Tyr Thr Gly Ile Lys Ser Asp Gly Ser
305                 310                 315                 320
Gly Lys Ala Lys Glu Val Leu Lys Lys Phe Thr Leu Glu Gly Lys Val
                325                 330                 335
Ala Asn Asp Lys Val Thr Leu Glu Val Lys Glu Gly Thr Val Thr Leu
            340                 345                 350
Ser Lys Asn Ile Ser Lys Ser Gly Glu Val Ser Val Glu Leu Asn Asp
        355                 360                 365
Thr Asp Ser Ser Ala Ala Thr Lys Lys Thr Ala Ala Trp Asn Ser Lys
    370                 375                 380
Thr Ser Thr Leu Thr Ile Ser Val Asn Ser Lys Lys Thr Thr Gln Leu
385                 390                 395                 400
Val Phe Thr Lys Gln Asp Thr Ile Thr Val Gln Lys Tyr Asp Ser Ala
                405                 410                 415
Gly Thr Asn Leu Glu Gly Thr Ala Val Glu Ile Lys Thr Leu Asp Glu
            420                 425                 430
Leu Lys Asn Ala Leu Lys
        435

<210> 79
<211> 1029
<212> DNA
<213> Artificial Sequence

<220>
<223> Chimeric nucleic acid

<221> CDS
<222> (1)...(1029)

<400> 79
atg gct tgt aat aat tca ggg aaa gat ggg aat aca tct gca aat tct     48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa     96
Ala Asp Glu Ser Val Lys Gly Pro Asn Leu Thr Glu Ile Asn Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa     192
Leu Leu Ser Ser Ile Asp Glu Ile Ala Ala Lys Ala Ile Gly Lys Lys
    50                  55                  60

ata cac caa aat aat ggt ttg gat acc gaa aat aat cac aat gga tca     240
Ile His Gln Asn Asn Gly Leu Asp Thr Glu Asn Asn His Asn Gly Ser
65                  70                  75                  80

ttg tta gcg gga gct tat gca ata tca acc cta ata aaa caa aaa tta     288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag     336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat     384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta     432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta     480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct     528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa     576
Asn Ser Val Lys Glu Leu Thr Ser Pro Val Val Ala Glu Ser Pro Lys
            180                 185                 190

aaa cct tcc atg gcc aag caa aat gtt tct gaa aaa ata ata aca aga     624
Lys Pro Ser Met Ala Lys Gln Asn Val Ser Glu Lys Ile Ile Thr Arg
        195                 200                 205

gca gac gga acc aga ctt gaa tac aca gga att aaa agc gat gga tct     672
Ala Asp Gly Thr Arg Leu Glu Tyr Thr Gly Ile Lys Ser Asp Gly Ser

210                 215                 220

gga aaa gct aaa gag gtt tta aaa ggc tat gtt ctt gaa gga act cta     720
Gly Lys Ala Lys Glu Val Leu Lys Gly Tyr Val Leu Glu Gly Thr Leu
225                 230                 235                 240

act gct gaa aaa aca aca ttg gtg gtt aaa gaa gga act gtt act tta     768
Thr Ala Glu Lys Thr Thr Leu Val Val Lys Glu Gly Thr Val Thr Leu
                245                 250                 255

agc aaa aat att tca aaa tct ggg gaa gtt tca gtt gaa ctt aat gac     816
Ser Lys Asn Ile Ser Lys Ser Gly Glu Val Ser Val Glu Leu Asn Asp
            260                 265                 270

act gac agt agt gct gct act aaa aaa act gca gct tgg aat tca ggc     864
Thr Asp Ser Ser Ala Ala Thr Lys Lys Thr Ala Ala Trp Asn Ser Gly
        275                 280                 285

act tca act tta aca att act gta aac agt aaa aaa act aaa gac ctt     912
Thr Ser Thr Leu Thr Ile Thr Val Asn Ser Lys Lys Thr Lys Asp Leu
    290                 295                 300

gtg ttt aca aaa gaa aac aca att aca gta caa caa tac gac tca aat     960
Val Phe Thr Lys Glu Asn Thr Ile Thr Val Gln Gln Tyr Asp Ser Asn
305                 310                 315                 320

ggc acc aaa tta gag ggg tca gca gtt gaa att aca aaa ctt gat gaa     1008
Gly Thr Lys Leu Glu Gly Ser Ala Val Glu Ile Thr Lys Leu Asp Glu
                325                 330                 335

att aaa aac gct tta aaa taa                                         1029
Ile Lys Asn Ala Leu Lys  *
            340


<210> 80
<211> 342
<212> PRT
<213> Artificial Sequence

<220>
<223> Chimeric protein

<400> 80
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Asn Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190

Lys Pro Ser Met Ala Lys Gln Asn Val Ser Glu Lys Ile Ile Thr Arg
        195                 200                 205
Ala Asp Gly Thr Arg Leu Glu Tyr Thr Gly Ile Lys Ser Asp Gly Ser
    210                 215                 220
Gly Lys Ala Lys Glu Val Leu Lys Gly Tyr Val Leu Glu Gly Thr Leu
225                 230                 235                 240
Thr Ala Glu Lys Thr Thr Leu Val Val Lys Glu Gly Thr Val Thr Leu
                245                 250                 255
Ser Lys Asn Ile Ser Lys Ser Gly Glu Val Ser Val Glu Leu Asn Asp
            260                 265                 270
Thr Asp Ser Ser Ala Ala Thr Lys Lys Thr Ala Ala Trp Asn Ser Gly
        275                 280                 285
Thr Ser Thr Leu Thr Ile Thr Val Asn Ser Lys Lys Thr Lys Asp Leu
    290                 295                 300
Val Phe Thr Lys Glu Asn Thr Ile Thr Val Gln Gln Tyr Asp Ser Asn
305                 310                 315                 320
Gly Thr Lys Leu Glu Gly Ser Ala Val Glu Ile Thr Lys Leu Asp Glu
                325                 330                 335
Ile Lys Asn Ala Leu Lys
            340


<210> 81
<211> 1029
<212> DNA
<213> Artificial Sequence

<220>
<223> Chimeric nucleic acid

<221> CDS
<222> (1)...(1029)

<400> 81
atg gct tgt aat aat tca ggg aaa gat ggg aat aca tct gca aat tct      48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa      96
Ala Asp Glu Ser Val Lys Gly Pro Asn Leu Thr Glu Ile Asn Lys Lys
            20                  25                  30

att acg gat tct aat gcg gtt tta ctt gct gtg aaa gag gtt gaa gcg     144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa     192
Leu Leu Ser Ser Ile Asp Glu Ile Ala Ala Lys Ala Ile Gly Lys Lys
    50                  55                  60

ata cac caa aat aat ggt ttg gat acc gaa aat aat cac aat gga tca     240
Ile His Gln Asn Asn Gly Leu Asp Thr Glu Asn Asn His Asn Gly Ser
 65                  70                  75                  80

ttg tta gcg gga gct tat gca ata tca acc cta ata aaa caa aaa tta     288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag     336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat     384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta     432
Leu Gly Lys Glu Gly Val Thr Asp Ala Asp Ala Lys Glu Ala Ile Leu

130                 135                 140

aaa gca aat ggt act aaa act aaa ggt gct gaa gaa ctt gga aaa tta     480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct     528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa     576
Asn Ser Val Lys Glu Leu Thr Ser Pro Val Val Ala Glu Ser Pro Lys
            180                 185                 190

aaa cct tcc atg gcc aag caa aat gtt tct gaa aaa ata ata aca aga     624
Lys Pro Ser Met Ala Lys Gln Asn Val Ser Glu Lys Ile Ile Thr Arg
        195                 200                 205

gca gac gga acc aga ctt gaa tac aca gga att aaa agc gat gga tct     672
Ala Asp Gly Thr Arg Leu Glu Tyr Thr Gly Ile Lys Ser Asp Gly Ser
    210                 215                 220

gga aaa gct aaa gag gtt tta aaa ggc tat gtt ctt gaa gga act cta     720
Gly Lys Ala Lys Glu Val Leu Lys Gly Tyr Val Leu Glu Gly Thr Leu
225                 230                 235                 240

act gct gaa aaa aca aca ttg gtg gtt aaa gaa gga act gtt act tta     768
Thr Ala Glu Lys Thr Thr Leu Val Val Lys Glu Gly Thr Val Thr Leu
                245                 250                 255

agc aaa aat att tca aaa tct ggg gaa gtt tca gtt gaa ctt aat gac     816
Ser Lys Asn Ile Ser Lys Ser Gly Glu Val Ser Val Glu Leu Asn Asp
            260                 265                 270

act gac agt agt gct gct act aaa aaa act gca gct tgg aat tca aaa     864
Thr Asp Ser Ser Ala Ala Thr Lys Lys Thr Ala Ala Trp Asn Ser Lys
        275                 280                 285

act tcc act tta aca att agt gtg aat agc caa aaa acc aaa aac ctt     912
Thr Ser Thr Leu Thr Ile Ser Val Asn Ser Gln Lys Thr Lys Asn Leu
    290                 295                 300

gta ttc aca aaa gaa gac aca ata aca gta caa aaa tac gac tca gca     960
Val Phe Thr Lys Glu Asp Thr Ile Thr Val Gln Lys Tyr Asp Ser Ala
305                 310                 315                 320

ggc acc aat cta gaa ggc aaa gca gtc gaa att aca aca ctt aaa gaa     1008
Gly Thr Asn Leu Glu Gly Lys Ala Val Glu Ile Thr Thr Leu Lys Glu
                325                 330                 335

ctt aaa aac gct tta aaa taa                                         1029
Leu Lys Asn Ala Leu Lys  *
            340

<210> 82
<211> 342
<212> PRT
<213> Artificial Sequence

<220>
<223> Chimeric protein

<400> 82
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Asn Lys Lys
            20                  25                  30

Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Lys Gln Asn Val Ser Glu Lys Ile Ile Thr Arg
        195                 200                 205
Ala Asp Gly Thr Arg Leu Glu Tyr Thr Gly Ile Lys Ser Asp Gly Ser
    210                 215                 220
Gly Lys Ala Lys Glu Val Leu Lys Gly Tyr Val Leu Glu Gly Thr Leu
225                 230                 235                 240
Thr Ala Glu Lys Thr Thr Leu Val Val Lys Glu Gly Thr Val Thr Leu
                245                 250                 255
Ser Lys Asn Ile Ser Lys Ser Gly Glu Val Ser Val Glu Leu Asn Asp
            260                 265                 270
Thr Asp Ser Ser Ala Ala Thr Lys Lys Thr Ala Ala Trp Asn Ser Lys
        275                 280                 285
Thr Ser Thr Leu Thr Ile Ser Val Asn Ser Gln Lys Thr Lys Asn Leu
    290                 295                 300
Val Phe Thr Lys Glu Asp Thr Ile Thr Val Gln Lys Tyr Asp Ser Ala
305                 310                 315                 320
Gly Thr Asn Leu Glu Gly Lys Ala Val Glu Ile Thr Thr Leu Lys Glu
                325                 330                 335
Leu Lys Asn Ala Leu Lys
            340


<210> 83
<211> 1029
<212> DNA
<213> Artificial Sequence

<220>
<223> Chimeric nucleic acid

<221> CDS
<222> (1)...(1029)

<400> 83
atg gct tgt aat aat tca ggg aaa gat ggg aat aca tct gca aat tct      48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa      96
Ala Asp Glu Ser Val Lys Gly Pro Asn Leu Thr Glu Ile Asn Lys Lys
            20                  25                  30

att acg gat tct aat gcg gtt tta ctt gct gtg aaa gag gtt gaa gcg      144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa      192
Leu Leu Ser Ser Ile Asp Glu Ile Ala Ala Lys Ala Ile Gly Lys Lys

50                  55                  60

ata cac caa aat aat ggt ttg gat acc gaa aat aat cac aat gga tca     240
Ile His Gln Asn Asn Gly Leu Asp Thr Glu Asn Asn His Asn Gly Ser
65                  70                  75                  80

ttg tta gcg gga gct tat gca ata tca acc cta ata aaa caa aaa tta     288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag     336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat     384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta     432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta     480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct     528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa     576
Asn Ser Val Lys Glu Leu Thr Ser Pro Val Val Ala Glu Ser Pro Lys
            180                 185                 190

aaa cct tcc atg gcc aag caa aat gtt tct gaa aaa ata ata aca aga     624
Lys Pro Ser Met Ala Lys Gln Asn Val Ser Glu Lys Ile Ile Thr Arg
        195                 200                 205

gca gac gga acc aga ctt gaa tac aca gga att aaa agc gat gga tct     672
Ala Asp Gly Thr Arg Leu Glu Tyr Thr Gly Ile Lys Ser Asp Gly Ser
    210                 215                 220

gga aaa gct aaa gag gtt tta aaa ggc tat gtt ctt gaa gga act cta     720
Gly Lys Ala Lys Glu Val Leu Lys Gly Tyr Val Leu Glu Gly Thr Leu
225                 230                 235                 240

act gct gaa aaa aca aca ttg gtg gtt aaa gaa gga act gtt act tta     768
Thr Ala Glu Lys Thr Thr Leu Val Val Lys Glu Gly Thr Val Thr Leu
                245                 250                 255

agc aaa aat att tca aaa tct ggg gaa gtt tca gtt gaa ctt aat gac     816
Ser Lys Asn Ile Ser Lys Ser Gly Glu Val Ser Val Glu Leu Asn Asp
            260                 265                 270

act gac agt agt gct gct act aaa aaa act gca gct tgg aat tca aaa     864
Thr Asp Ser Ser Ala Ala Thr Lys Lys Thr Ala Ala Trp Asn Ser Lys
        275                 280                 285

act tct act tta aca att agt gtt aac agc aaa aaa act aca caa ctt     912
Thr Ser Thr Leu Thr Ile Ser Val Asn Ser Lys Lys Thr Thr Gln Leu
    290                 295                 300

gtg ttt act aaa caa gac aca ata act gta caa aaa tac gac tcc gca     960
Val Phe Thr Lys Gln Asp Thr Ile Thr Val Gln Lys Tyr Asp Ser Ala
305                 310                 315                 320

ggt acc aat tta gaa ggc aca gca gtc gaa att aaa aca ctt gat gaa     1008
Gly Thr Asn Leu Glu Gly Thr Ala Val Glu Ile Lys Thr Leu Asp Glu

325                 330                 335

ctt aaa aac gct tta aaa taa                                         1029
Leu Lys Asn Ala Leu Lys  *
            340


<210> 84
<211> 342
<212> PRT
<213> Artificial Sequence

<220>
<223> Chimeric protein

<400> 84
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Asn Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Lys Gln Asn Val Ser Glu Lys Ile Ile Thr Arg
        195                 200                 205
Ala Asp Gly Thr Arg Leu Glu Tyr Thr Gly Ile Lys Ser Asp Gly Ser
    210                 215                 220
Gly Lys Ala Lys Glu Val Leu Lys Gly Tyr Val Leu Glu Gly Thr Leu
225                 230                 235                 240
Thr Ala Glu Lys Thr Thr Leu Val Val Lys Glu Gly Thr Val Thr Leu
                245                 250                 255
Ser Lys Asn Ile Ser Lys Ser Gly Glu Val Ser Val Glu Leu Asn Asp
            260                 265                 270
Thr Asp Ser Ser Ala Ala Thr Lys Lys Thr Ala Ala Trp Asn Ser Lys
        275                 280                 285
Thr Ser Thr Leu Thr Ile Ser Val Asn Ser Lys Lys Thr Thr Gln Leu
    290                 295                 300
Val Phe Thr Lys Gln Asp Thr Ile Thr Val Gln Lys Tyr Asp Ser Ala
305                 310                 315                 320
Gly Thr Asn Leu Glu Gly Thr Ala Val Glu Ile Lys Thr Leu Asp Glu
                325                 330                 335
Leu Lys Asn Ala Leu Lys
            340


<210> 85
<211> 1035
<212> DNA
<213> Artificial Sequence

<220>

<223> Chimeric nucleic acid

<221> CDS
<222> (1)...(1035)

<400> 85
atg gct tgt aat aat tca ggg aaa gat ggg aat aca tct gca aat tct     48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata agt aaa aaa     96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg    144
Ile Thr Asp Ser Asn Ala Val Leu Leu Ala Val Lys Glu Val Glu Ala
        35                  40                  45

ttg ctg tca tct ata gat gaa att gct gct aaa gct att ggt aaa aaa    192
Leu Leu Ser Ser Ile Asp Glu Ile Ala Ala Lys Ala Ile Gly Lys Lys
    50                  55                  60

ata cac caa aat aat ggt ttg gat acc gaa tat aat cac aat gga tca    240
Ile His Gln Asn Asn Gly Leu Asp Thr Glu Tyr Asn His Asn Gly Ser
65                  70                  75                  80

ttg tta gcg gga gct tat gca ata tca acc cta ata aaa caa aaa tta    288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag    336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat    384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta    432
Leu Gly Lys Glu Gly Val Thr Asp Ala Asp Ala Lys Glu Ala Ile Leu
    130                 135                 140

aaa aca aat ggt act aaa act aaa ggt gct gaa gaa ctt gga aaa tta    480
Lys Thr Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct    528
Phe Glu Ser Val Glu Val Leu Ser Lys Ala Ala Lys Glu Met Leu Ala
                165                 170                 175

aat tca gtt aaa gag ctt aca agc cct gtt gtg gca gaa agt cca aaa    576
Asn Ser Val Lys Glu Leu Thr Ser Pro Val Val Ala Glu Ser Pro Lys
            180                 185                 190

aaa cct tcc atg gcc aag caa aat gtt aca tct gaa aaa aca ata gta    624
Lys Pro Ser Met Ala Lys Gln Asn Val Thr Ser Glu Lys Thr Ile Val
        195                 200                 205

aga gca aat gga acc aga ctt gaa tac aca gac ata aaa agc gat gga    672
Arg Ala Asn Gly Thr Arg Leu Glu Tyr Thr Asp Ile Lys Ser Asp Gly
    210                 215                 220

tcc gga aaa gct aaa gaa gtt tta aaa gac ttt act ctt gaa gga act    720
Ser Gly Lys Ala Lys Glu Val Leu Lys Asp Phe Thr Leu Glu Gly Thr
225                 230                 235                 240

cta gct gct gac ggc aaa aca aca ttg aaa gtt aca gaa ggc act gtt    768
Leu Ala Ala Asp Gly Lys Thr Thr Leu Lys Val Thr Glu Gly Thr Val

                245                 250                 255

gtt tta agc aag aac att tta aaa tcc gga gaa ata aca gtt gca ctt 816
Val Leu Ser Lys Asn Ile Leu Lys Ser Gly Glu Ile Thr Val Ala Leu
            260                 265                 270

gat gac tct gac act act cag gct act aaa aaa act gga aaa tgg gat 864
Asp Asp Ser Asp Thr Thr Gln Ala Thr Lys Lys Thr Gly Lys Trp Asp
        275                 280                 285

tca aat act tcc act tta aca att agt gtg aat agc aaa aaa act aaa 912
Ser Asn Thr Ser Thr Leu Thr Ile Ser Val Asn Ser Lys Lys Thr Lys
    290                 295                 300

aac att gta ttt aca aaa gaa gac aca ata aca gta caa aaa tac gac 960
Asn Ile Val Phe Thr Lys Glu Asp Thr Ile Thr Val Gln Lys Tyr Asp
305                 310                 315                 320

tca gca ggc acc aat cta gaa ggc aac gca gtc gaa att aaa aca ctt 1008
Ser Ala Gly Thr Asn Leu Glu Gly Asn Ala Val Glu Ile Lys Thr Leu
                325                 330                 335

gat gaa ctt aaa aac gct tta aaa tag 1035
Asp Glu Leu Lys Asn Ala Leu Lys *
340

<210> 86
<211> 344
<212> PRT
<213> Artificial Sequence

<220>
<223> Chimeric protein

<400> 86
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Ser Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Tyr Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Thr Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Lys Gln Asn Val Thr Ser Glu Lys Thr Ile Val
        195                 200                 205
Arg Ala Asn Gly Thr Arg Leu Glu Tyr Thr Asp Ile Lys Ser Asp Gly
    210                 215                 220
Ser Gly Lys Ala Lys Glu Val Leu Lys Asp Phe Thr Leu Glu Gly Thr
225                 230                 235                 240
Leu Ala Ala Asp Gly Lys Thr Thr Leu Lys Val Thr Glu Gly Thr Val
                245                 250                 255

Val Leu Ser Lys Asn Ile Leu Lys Ser Gly Glu Ile Thr Val Ala Leu
                260                 265                 270
Asp Asp Ser Asp Thr Thr Gln Ala Thr Lys Lys Thr Gly Lys Trp Asp
        275                 280                 285
Ser Asn Thr Ser Thr Leu Thr Ile Ser Val Asn Ser Lys Lys Thr Lys
    290                 295                 300
Asn Ile Val Phe Thr Lys Glu Asp Thr Ile Thr Val Gln Lys Tyr Asp
305                 310                 315                 320
Ser Ala Gly Thr Asn Leu Glu Gly Asn Ala Val Glu Ile Lys Thr Leu
                325                 330                 335
Asp Glu Leu Lys Asn Ala Leu Lys
                340

<210> 87
<211> 1323
<212> DNA
<213> Artificial Sequence

<220>
<223> Chimeric nucleic acid

<221> CDS
<222> (1)...(1323)

<400> 87
atg gct tgt aat aat tca ggg aaa gat ggg aat aca tct gca aat tct    48
Met Ala Cys Asn Asn Ser Gly Lys Asp Gly Asn Thr Ser Ala Asn Ser
 1               5                  10                  15

gct gat gag tct gtt aaa ggg cct aat ctt aca gaa ata aat aaa aaa    96
Ala Asp Glu Ser Val Lys Gly Pro Asn Leu Thr Glu Ile Asn Lys Lys
                20                  25                  30

att acg gat tct aat gcg gtt tta ctt gct gtg aaa gag gtt gaa gcg    144
Ile Thr Asp Ser Asn Ala Val Leu Leu Ala Val Lys Glu Val Glu Ala
            35                  40                  45

ttg ctg tca tct ata gat gaa att gct gct aaa gct att ggt aaa aaa    192
Leu Leu Ser Ser Ile Asp Glu Ile Ala Ala Lys Ala Ile Gly Lys Lys
        50                  55                  60

ata cac caa aat aat ggt ttg gat acc gaa aat aat cac aat gga tca    240
Ile His Gln Asn Asn Gly Leu Asp Thr Glu Asn Asn His Asn Gly Ser
 65                  70                  75                  80

ttg tta gcg gga gct tat gca ata tca acc cta ata aaa caa aaa tta    288
Leu Leu Ala Gly Ala Tyr Ala Ile Ser Thr Leu Ile Lys Gln Lys Leu
                85                  90                  95

gat gga ttg aaa aat gaa gga tta aag gaa aaa att gat gcg gct aag    336
Asp Gly Leu Lys Asn Glu Gly Leu Lys Glu Lys Ile Asp Ala Ala Lys
            100                 105                 110

aaa tgt tct gaa aca ttt act aat aaa tta aaa gaa aaa cac aca gat    384
Lys Cys Ser Glu Thr Phe Thr Asn Lys Leu Lys Glu Lys His Thr Asp
        115                 120                 125

ctt ggt aaa gaa ggt gtt act gat gct gat gca aaa gaa gcc att tta    432
Leu Gly Lys Glu Gly Val Thr Asp Ala Asp Ala Lys Glu Ala Ile Leu
    130                 135                 140

aaa gca aat ggt act aaa act aaa ggt gct gaa gaa ctt gga aaa tta    480
Lys Ala Asn Gly Thr Lys Thr Lys Gly Ala Glu Glu Leu Gly Lys Leu
145                 150                 155                 160

ttt gaa tca gta gag gtc ttg tca aaa gca gct aaa gag atg ctt gct    528
Phe Glu Ser Val Glu Val Leu Ser Lys Ala Ala Lys Glu Met Leu Ala

                165                 170                 175

aat tca gtt aaa gag ctt aca agc cct gtt gtg gca gaa agt cca aaa 576
Asn Ser Val Lys Glu Leu Thr Ser Pro Val Val Ala Glu Ser Pro Lys
            180                 185                 190

aaa cct tcc atg gcc gtt tca gta gat ttg cct ggt gaa atg aaa gtt 624
Lys Pro Ser Met Ala Val Ser Val Asp Leu Pro Gly Glu Met Lys Val
        195                 200                 205

ctt gta agc aaa gaa aaa aac aaa gac ggc aag tac gat cta att gca 672
Leu Val Ser Lys Glu Lys Asn Lys Asp Gly Lys Tyr Asp Leu Ile Ala
    210                 215                 220

aca gta gac aag ctt gag ctt aaa gga act tct gat aaa aac aat gga 720
Thr Val Asp Lys Leu Glu Leu Lys Gly Thr Ser Asp Lys Asn Asn Gly
225                 230                 235                 240

tct gga gta ctt gaa ggc gta aaa gct gac aaa agt aaa gta aaa tta 768
Ser Gly Val Leu Glu Gly Val Lys Ala Asp Lys Ser Lys Val Lys Leu
                245                 250                 255

aca att tct gac gat cta ggt caa acc aca ctt gaa gtt ttc aaa gaa 816
Thr Ile Ser Asp Asp Leu Gly Gln Thr Thr Leu Glu Val Phe Lys Glu
            260                 265                 270

gat ggc aaa aca cta gta tca aaa aaa gta act tcc aaa gac aag tca 864
Asp Gly Lys Thr Leu Val Ser Lys Lys Val Thr Ser Lys Asp Lys Ser
        275                 280                 285

tca aca gaa gaa aaa ttc aat gaa aaa ggt gaa gta tct gaa aaa ata 912
Ser Thr Glu Glu Lys Phe Asn Glu Lys Gly Glu Val Ser Glu Lys Ile
    290                 295                 300

ata aca aga gca gac gga acc aga ctt gaa tac aca gga att aaa agc 960
Ile Thr Arg Ala Asp Gly Thr Arg Leu Glu Tyr Thr Gly Ile Lys Ser
305                 310                 315                 320

gat gga tct gga aaa gct aaa gag gtt tta aaa ggc ttt act ctt gaa 1008
Asp Gly Ser Gly Lys Ala Lys Glu Val Leu Lys Gly Phe Thr Leu Glu
                325                 330                 335

gga aaa gta gct aat gat aaa gta aca ttg gaa gta aaa gaa gga acc 1056
Gly Lys Val Ala Asn Asp Lys Val Thr Leu Glu Val Lys Glu Gly Thr
            340                 345                 350

gtt act tta agt aag att tca aaa tct ggg gaa gtt tca gtt gaa ctt 1104
Val Thr Leu Ser Lys Ile Ser Lys Ser Gly Glu Val Ser Val Glu Leu
        355                 360                 365

aat gac act gac agt agt gct gct act aaa aaa act gca gct tgg aat 1152
Asn Asp Thr Asp Ser Ser Ala Ala Thr Lys Lys Thr Ala Ala Trp Asn
    370                 375                 380

tca aaa act tct act tta aca att agt gtt aac agc aaa aaa act aca 1200
Ser Lys Thr Ser Thr Leu Thr Ile Ser Val Asn Ser Lys Lys Thr Thr
385                 390                 395                 400

caa ctt gtg ttt act aaa caa gac aca ata act gta caa aaa tac gac 1248
Gln Leu Val Phe Thr Lys Gln Asp Thr Ile Thr Val Gln Lys Tyr Asp
                405                 410                 415

tcc gca ggt acc aat tta gaa ggc aca gca gtc gaa att aaa aca ctt 1296
Ser Ala Gly Thr Asn Leu Glu Gly Thr Ala Val Glu Ile Lys Thr Leu
            420                 425                 430

gat gaa ctt aaa aac gct tta aaa taa 1323
Asp Glu Leu Lys Asn Ala Leu Lys *

435                 440

<210> 88
<211> 440
<212> PRT
<213> Artificial Sequence

<220>
<223> Chimeric protein

<400> 88
Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Asn Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Ile Ala Ala Lys Ala Ile Gly Lys Lys
    50                  55                  60
Ile His Gln Asn Asn Gly Leu Asp Thr Glu Asn Asn His Asn Gly Ser
65                  70                  75                  80
Leu Leu Ala Gly Ala Tyr Ala Ile Ser Thr Leu Ile Lys Gln Lys Leu
                85                  90                  95
Asp Gly Leu Lys Asn Glu Gly Leu Lys Glu Lys Ile Asp Ala Ala Lys
            100                 105                 110
Lys Cys Ser Glu Thr Phe Thr Asn Lys Leu Lys Glu Lys His Thr Asp
        115                 120                 125
Leu Gly Lys Glu Gly Val Thr Asp Ala Asp Ala Lys Glu Ala Ile Leu
    130                 135                 140
Lys Ala Asn Gly Thr Lys Thr Lys Gly Ala Glu Glu Leu Gly Lys Leu
145                 150                 155                 160
Phe Glu Ser Val Glu Val Leu Ser Lys Ala Ala Lys Glu Met Leu Ala
                165                 170                 175
Asn Ser Val Lys Glu Leu Thr Ser Pro Val Val Ala Glu Ser Pro Lys
            180                 185                 190
Lys Pro Ser Met Ala Val Ser Val Asp Leu Pro Gly Glu Met Lys Val
        195                 200                 205
Leu Val Ser Lys Glu Lys Asn Lys Asp Gly Lys Tyr Asp Leu Ile Ala
    210                 215                 220
Thr Val Asp Lys Leu Glu Leu Lys Gly Thr Ser Asp Lys Asn Asn Gly
225                 230                 235                 240
Ser Gly Val Leu Glu Gly Val Lys Ala Asp Lys Ser Lys Val Lys Leu
                245                 250                 255
Thr Ile Ser Asp Asp Leu Gly Gln Thr Thr Leu Glu Val Phe Lys Glu
            260                 265                 270
Asp Gly Lys Thr Leu Val Ser Lys Lys Val Thr Ser Lys Asp Lys Ser
        275                 280                 285
Ser Thr Glu Glu Lys Phe Asn Glu Lys Gly Glu Val Ser Glu Lys Ile
    290                 295                 300
Ile Thr Arg Ala Asp Gly Thr Arg Leu Glu Tyr Thr Gly Ile Lys Ser
305                 310                 315                 320
Asp Gly Ser Gly Lys Ala Lys Glu Val Leu Lys Gly Phe Thr Leu Glu
                325                 330                 335
Gly Lys Val Ala Asn Asp Lys Val Thr Leu Glu Val Lys Glu Gly Thr
            340                 345                 350
Val Thr Leu Ser Lys Ile Ser Lys Ser Gly Glu Val Ser Val Glu Leu
        355                 360                 365
Asn Asp Thr Asp Ser Ser Ala Ala Thr Lys Lys Thr Ala Ala Trp Asn
    370                 375                 380
Ser Lys Thr Ser Thr Leu Thr Ile Ser Val Asn Ser Lys Lys Thr Thr
385                 390                 395                 400
Gln Leu Val Phe Thr Lys Gln Asp Thr Ile Thr Val Gln Lys Tyr Asp
                405                 410                 415
Ser Ala Gly Thr Asn Leu Glu Gly Thr Ala Val Glu Ile Lys Thr Leu
            420                 425                 430
Asp Glu Leu Lys Asn Ala Leu Lys
        435                 440

<210> 89
<211> 1302
<212> DNA
<213> Artificial Sequence

<220>
<223> Chimeric nucleic acid

<221> CDS
<222> (1)...(1302)

<400> 89
atg gct tgt aat aat tca ggg aaa gat ggg aat aca tct gca aat tct 48
Met Ala Cys Asn Asn Ser Gly Lys Asp Gly Asn Thr Ser Ala Asn Ser
1               5                   10                  15

gct gat gag tct gtt aaa ggg cct aat ctt aca gaa ata agt aaa aaa 96
Ala Asp Glu Ser Val Lys Gly Pro Asn Leu Thr Glu Ile Ser Lys Lys
            20                  25                  30

att acg gat tct aat gcg gtt tta ctt gct gtg aaa gag gtt gaa gcg 144
Ile Thr Asp Ser Asn Ala Val Leu Leu Ala Val Lys Glu Val Glu Ala
        35                  40                  45

ttg ctg tca tct ata gat gag ctt gct aaa gct att ggt aaa aaa ata 192
Leu Leu Ser Ser Ile Asp Glu Leu Ala Lys Ala Ile Gly Lys Lys Ile
    50                  55                  60

aaa aac gat ggt agt tta gat aat gaa gca aat cgc aac gag tca ttg 240
Lys Asn Asp Gly Ser Leu Asp Asn Glu Ala Asn Arg Asn Glu Ser Leu
65                  70                  75                  80

tta gca gga gct tat aca ata tca acc tta ata aca caa aaa tta agt 288
Leu Ala Gly Ala Tyr Thr Ile Ser Thr Leu Ile Thr Gln Lys Leu Ser
                85                  90                  95

aaa tta aac gga tca gaa ggt tta aag gaa aag att gcc gca gct aag 336
Lys Leu Asn Gly Ser Glu Gly Leu Lys Glu Lys Ile Ala Ala Ala Lys
            100                 105                 110

aaa tgc tct gaa gag ttt agt act aaa cta aaa gat aat cat gca cag 384
Lys Cys Ser Glu Glu Phe Ser Thr Lys Leu Lys Asp Asn His Ala Gln
        115                 120                 125

ctt ggt ata cag ggc gtt act gat gaa aat gca aaa aaa gct att tta 432
Leu Gly Ile Gln Gly Val Thr Asp Glu Asn Ala Lys Lys Ala Ile Leu
    130                 135                 140

aaa gca aat gca gcg ggt aaa gat aag ggc gtt gaa gaa ctt gaa aag 480
Lys Ala Asn Ala Ala Gly Lys Asp Lys Gly Val Glu Glu Leu Glu Lys
145                 150                 155                 160

ttg tcc gga tca tta gaa agc tta tca aaa gca gct aaa gag atg ctt 528
Leu Ser Gly Ser Leu Glu Ser Leu Ser Lys Ala Ala Lys Glu Met Leu
                165                 170                 175

gct aat tca gtt aaa gag ctt aca agc cct gtt gtc cat ggc gtt tca 576
Ala Asn Ser Val Lys Glu Leu Thr Ser Pro Val Val His Gly Val Ser
            180                 185                 190

gta gat ttg cct ggt gaa atg aaa gtt ctt gta agc aaa gaa aaa aac 624
Val Asp Leu Pro Gly Glu Met Lys Val Leu Val Ser Lys Glu Lys Asn
        195                 200                 205

aaa gac ggc aag tac gat cta att gca aca gta gac aag ctt gag ctt 672
Lys Asp Gly Lys Tyr Asp Leu Ile Ala Thr Val Asp Lys Leu Glu Leu

210                 215                 220

aaa gga act tct gat aaa aac aat gga tct gga gta ctt gaa ggc gta     720
Lys Gly Thr Ser Asp Lys Asn Asn Gly Ser Gly Val Leu Glu Gly Val
225                 230                 235                 240

aaa gct gac aaa agt aaa gta aaa tta aca att tct gac gat cta ggt     768
Lys Ala Asp Lys Ser Lys Val Lys Leu Thr Ile Ser Asp Asp Leu Gly
                245                 250                 255

caa acc aca ctt gaa gtt ttc aaa gaa gat ggc aaa aca cta gta tca     816
Gln Thr Thr Leu Glu Val Phe Lys Glu Asp Gly Lys Thr Leu Val Ser
            260                 265                 270

aaa aaa gta act tcc aaa gac aag tca tca aca gaa gaa aaa ttc aat     864
Lys Lys Val Thr Ser Lys Asp Lys Ser Ser Thr Glu Glu Lys Phe Asn
        275                 280                 285

gaa aaa ggt gaa gta tct gaa aaa ata ata aca aga gca gac gga acc     912
Glu Lys Gly Glu Val Ser Glu Lys Ile Ile Thr Arg Ala Asp Gly Thr
    290                 295                 300

aga ctt gaa tac aca gga att aaa agc gat gga tct gga aaa gct aaa     960
Arg Leu Glu Tyr Thr Gly Ile Lys Ser Asp Gly Ser Gly Lys Ala Lys
305                 310                 315                 320

gag gtt tta aaa ggc ttt act ctt gaa gga aaa gta gct aat gat aaa     1008
Glu Val Leu Lys Gly Phe Thr Leu Glu Gly Lys Val Ala Asn Asp Lys
                325                 330                 335

gta aca ttg gaa gta aaa gaa gga acc gtt act tta agt aag att tca     1056
Val Thr Leu Glu Val Lys Glu Gly Thr Val Thr Leu Ser Lys Ile Ser
            340                 345                 350

aaa tct ggg gaa gtt tca gtt gaa ctt aat gac act gac agt agt gct     1104
Lys Ser Gly Glu Val Ser Val Glu Leu Asn Asp Thr Asp Ser Ser Ala
        355                 360                 365

gct act aaa aaa act gca gct tgg aat tca aaa act tct act tta aca     1152
Ala Thr Lys Lys Thr Ala Ala Trp Asn Ser Lys Thr Ser Thr Leu Thr
    370                 375                 380

att agt gtt aac agc aaa aaa act aca caa ctt gtg ttt act aaa caa     1200
Ile Ser Val Asn Ser Lys Lys Thr Thr Gln Leu Val Phe Thr Lys Gln
385                 390                 395                 400

gac aca ata act gta caa aaa tac gac tcc gca ggt acc aat tta gaa     1248
Asp Thr Ile Thr Val Gln Lys Tyr Asp Ser Ala Gly Thr Asn Leu Glu
                405                 410                 415

ggc aca gca gtc gaa att aaa aca ctt gat gaa ctt aaa aac gct tta     1296
Gly Thr Ala Val Glu Ile Lys Thr Leu Asp Glu Leu Lys Asn Ala Leu
            420                 425                 430

aaa taa                                                             1302
Lys  *

<210> 90
<211> 433
<212> PRT
<213> Artificial Sequence

<220>
<223> Chimeric protein

<400> 90

Met Ala Cys Asn Asn Ser Gly Lys Asp Gly Asn Thr Ser Ala Asn Ser
1               5                   10                  15
Ala Asp Glu Ser Val Lys Gly Pro Asn Leu Thr Glu Ile Ser Lys Lys
            20                  25                  30
Ile Thr Asp Ser Asn Ala Val Leu Leu Ala Val Lys Glu Val Glu Ala
        35                  40                  45
Leu Leu Ser Ser Ile Asp Glu Leu Ala Lys Ala Ile Gly Lys Lys Ile
    50                  55                  60
Lys Asn Asp Gly Ser Leu Asp Asn Glu Ala Asn Arg Asn Glu Ser Leu
65                  70                  75                  80
Leu Ala Gly Ala Tyr Thr Ile Ser Thr Leu Ile Thr Gln Lys Leu Ser
                85                  90                  95
Lys Leu Asn Gly Ser Glu Gly Leu Lys Glu Lys Ile Ala Ala Ala Lys
            100                 105                 110
Lys Cys Ser Glu Glu Phe Ser Thr Lys Leu Lys Asp Asn His Ala Gln
        115                 120                 125
Leu Gly Ile Gln Gly Val Thr Asp Glu Asn Ala Lys Lys Ala Ile Leu
    130                 135                 140
Lys Ala Asn Ala Ala Gly Lys Asp Lys Gly Val Glu Glu Leu Glu Lys
145                 150                 155                 160
Leu Ser Gly Ser Leu Glu Ser Leu Ser Lys Ala Ala Lys Glu Met Leu
                165                 170                 175
Ala Asn Ser Val Lys Glu Leu Thr Ser Pro Val Val His Gly Val Ser
            180                 185                 190
Val Asp Leu Pro Gly Glu Met Lys Val Leu Val Ser Lys Glu Lys Asn
        195                 200                 205
Lys Asp Gly Lys Tyr Asp Leu Ile Ala Thr Val Asp Lys Leu Glu Leu
    210                 215                 220
Lys Gly Thr Ser Asp Lys Asn Asn Gly Ser Gly Val Leu Glu Gly Val
225                 230                 235                 240
Lys Ala Asp Lys Ser Lys Val Lys Leu Thr Ile Ser Asp Asp Leu Gly
                245                 250                 255
Gln Thr Thr Leu Glu Val Phe Lys Glu Asp Gly Lys Thr Leu Val Ser
            260                 265                 270
Lys Lys Val Thr Ser Lys Asp Lys Ser Ser Thr Glu Glu Lys Phe Asn
        275                 280                 285
Glu Lys Gly Glu Val Ser Glu Lys Ile Ile Thr Arg Ala Asp Gly Thr
    290                 295                 300
Arg Leu Glu Tyr Thr Gly Ile Lys Ser Asp Gly Ser Gly Lys Ala Lys
305                 310                 315                 320
Glu Val Leu Lys Gly Phe Thr Leu Glu Gly Lys Val Ala Asn Asp Lys
                325                 330                 335
Val Thr Leu Glu Val Lys Glu Gly Thr Val Thr Leu Ser Lys Ile Ser
            340                 345                 350
Lys Ser Gly Glu Val Ser Val Glu Leu Asn Asp Thr Asp Ser Ser Ala
        355                 360                 365
Ala Thr Lys Lys Thr Ala Ala Trp Asn Ser Lys Thr Ser Thr Leu Thr
    370                 375                 380
Ile Ser Val Asn Ser Lys Lys Thr Thr Gln Leu Val Phe Thr Lys Gln
385                 390                 395                 400
Asp Thr Ile Thr Val Gln Lys Tyr Asp Ser Ala Gly Thr Asn Leu Glu
                405                 410                 415
Gly Thr Ala Val Glu Ile Lys Thr Leu Asp Glu Leu Lys Asn Ala Leu
            420                 425                 430
Lys

<210> 91
<211> 31
<212> DNA
<213> Artificial Sequence

<220>
<223> Oligonucleotide Primer

<400> 91
gtcatatggc ttgtaataat tcagggaaag a                                31

<210> 92
<211> 30
<212> DNA
<213> Artificial Sequence

<220>
<223> Oligonucleotide Primer

<400> 92
tttccatgga aggttttttt ggactttctg                                          30

<210> 93
<211> 30
<212> DNA
<213> Artificial Sequence

<220>
<223> Oligonucleotide Primer

<400> 93
tttccatggc caagcaaaat gttagcagcc                                         30

<210> 94
<211> 27
<212> DNA
<213> Artificial Sequence

<220>
<223> Oligonucleotide Primer

<400> 94
taaggatcct tattttaaag cgttttt                                            27

<210> 95
<211> 822
<212> DNA
<213> Artificial Sequence

<220>
<223> Altered OspA Nucleic Acid

<221> CDS
<222> (1)...(822)

<400> 95
atg aaa aaa tat tta ttg gga ata ggt cta ata tta gcc tta ata gca      48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta      96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln

                  85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca atg gca gac gga acc aga     432
Lys Gly Glu Val Ser Glu Lys Ile Ile Thr Met Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 96
<211> 273
<212> PRT
<213> Artificial Sequence

<220>
<223> Altered OspA protein

<400> 96
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60

Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Met Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 97
<211> 822
<212> DNA
<213> Artificial Sequence

<220>
<223> Altered OspA Nucleic Acid

<221> CDS
<222> (1)...(822)

<400> 97
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa    144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa    192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa    240
Gly Thr Ser Asp Lys Asn Asn Gly Ser Gly Val Leu Glu Gly Val Lys
 65                  70                  75                  80

gct gac aaa agt aaa gta aaa tta aca att tct gac gat cta ggt caa    288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa    336

Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa tat     480
Leu Glu Tyr Thr Gly Ile Lys Ser Asp Gly Ser Gly Lys Ala Lys Tyr
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 98
<211> 273
<212> PRT
<213> Artificial Sequence

<220>
<223> Altered OspA protein

<400> 98
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln

                    85                  90                  95
Thr Thr Leu Glu Val Phe Lys Glu Asp Gly Lys Thr Leu Val Ser Lys
                100                 105                 110
Lys Val Thr Ser Lys Asp Lys Ser Ser Thr Glu Glu Lys Phe Asn Glu
            115                 120                 125
Lys Gly Glu Val Ser Glu Lys Ile Ile Thr Arg Ala Asp Gly Thr Arg
        130                 135                 140
Leu Glu Tyr Thr Gly Ile Lys Ser Asp Gly Ser Gly Lys Ala Lys Tyr
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
                180                 185                 190
Lys Ser Gly Glu Val Ser Val Glu Leu Asn Asp Thr Asp Ser Ser Ala
            195                 200                 205
Ala Thr Lys Lys Thr Ala Ala Trp Asn Ser Gly Thr Ser Thr Leu Thr
        210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
                260                 265                 270
Lys

<210> 99
<211> 822
<212> DNA
<213> Artificial Sequence

<220>
<223> Altered OspA Nucleic Acid

<221> CDS
<222> (1)...(822)

<400> 99
atg aaa aaa tat tta ttg gga ata ggt cta ata tta gcc tta ata gca    48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta    96
Cys Lys Gln Asn Val Ser Ser Leu Asp Glu Lys Asn Ser Val Ser Val
                20                  25                  30

gat ttg cct ggt gaa atg aaa gtt ctt gta agc aaa gaa aaa aac aaa    144
Asp Leu Pro Gly Glu Met Lys Val Leu Val Ser Lys Glu Lys Asn Lys
            35                  40                  45

gac ggc aag tac gat cta att gca aca gta gac aag ctt gag ctt aaa    192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa    240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa    288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa    336
Thr Thr Leu Glu Val Phe Lys Glu Asp Gly Lys Thr Leu Val Ser Lys
                100                 105                 110

aaa gta act tcc aaa gac aag tca tca aca gaa gaa aaa ttc aat gaa    384

Lys Val Thr Ser Lys Asp Lys Ser Ser Thr Glu Glu Lys Phe Asn Glu
        115                 120                 125

aaa ggt gaa gta tct gaa aaa ata ata aca atg gca gac gga acc aga     432
Lys Gly Glu Val Ser Glu Lys Ile Ile Thr Met Ala Asp Gly Thr Arg
    130                 135                 140

ctt gaa tac aca gga att aaa agc gat gga tct gga aaa gct aaa tat     480
Leu Glu Tyr Thr Gly Ile Lys Ser Asp Gly Ser Gly Lys Ala Lys Tyr
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 100
<211> 273
<212> PRT
<213> Artificial Sequence

<220>
<223> Altered OspA Protein

<400> 100
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu

        115                     120                     125
Lys Gly Glu Val Ser Glu Lys Ile Ile Thr Met Ala Asp Gly Thr Arg
    130                     135                     140
Leu Glu Tyr Thr Gly Ile Lys Ser Asp Gly Ser Gly Lys Ala Lys Tyr
145                     150                     155                     160
Val Leu Lys Gly Tyr Val Leu Glu Gly Thr Leu Thr Ala Glu Lys Thr
                165                     170                     175
Thr Leu Val Val Lys Glu Gly Thr Val Thr Leu Ser Lys Asn Ile Ser
            180                     185                     190
Lys Ser Gly Glu Val Ser Val Glu Leu Asn Asp Thr Asp Ser Ser Ala
        195                     200                     205
Ala Thr Lys Lys Thr Ala Ala Trp Asn Ser Gly Thr Ser Thr Leu Thr
    210                     215                     220
Ile Thr Val Asn Ser Lys Lys Thr Lys Asp Leu Val Phe Thr Lys Glu
225                     230                     235                     240
Asn Thr Ile Thr Val Gln Gln Tyr Asp Ser Asn Gly Thr Lys Leu Glu
                245                     250                     255
Gly Ser Ala Val Glu Ile Thr Lys Leu Asp Glu Ile Lys Asn Ala Leu
            260                     265                     270
Lys



<210> 101
<211> 822
<212> DNA
<213> Artificial Sequence

<220>
<223> Altered OspA Nucleic Acid

<221> CDS
<222> (1)...(822)

<400> 101
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa    144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa    192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa    240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa    288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa    336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa    384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga    432

Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa tat      480
Leu Glu Tyr Thr Gly Ile Lys Ser Asp Gly Ser Gly Lys Ala Lys Tyr
145                 150                 155                 160

gtt tta aaa ggc tat gtt ctt gaa gga act cta act gct gaa aaa aca      528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca      576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct      624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca      672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa      720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag      768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta      816
Gly Ser Ala Val Glu Ile Thr Lys Leu Asp Glu Ile Lys Asn Ala Leu
            260                 265                 270

aaa taa                                                              822
Lys  *

<210> 102
<211> 273
<212> PRT
<213> Artificial Sequence

<220>
<223> Altered OspA Protein

<400> 102
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Tyr

145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 103
<211> 822
<212> DNA
<213> Artificial Sequence

<220>
<223> Altered OspA Nucleic Acid

<221> CDS
<222> (1)...(822)

<400> 103
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca atg gca gac gga acc aga     432
Lys Gly Glu Val Ser Glu Lys Ile Ile Thr Met Ala Asp Gly Thr Arg
    130                 135                 140

ctt gaa tac aca gga att aaa agc gat gga tct gga aaa gct aaa tat     480

Leu Glu Tyr Thr Gly Ile Lys Ser Asp Gly Ser Gly Lys Ala Lys Tyr
145                 150                 155                 160

gtt tta aaa ggc tat gtt ctt gaa gga act cta act gct gaa aaa aca     528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 104
<211> 273
<212> PRT
<213> Artificial Sequence

<220>
<223> Altered OspA Protein

<400> 104
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Met Ala Asp Gly Thr Arg
    130                 135                 140
Leu Glu Tyr Thr Gly Ile Lys Ser Asp Gly Ser Gly Lys Ala Lys Tyr
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser

                180                 185                 190
Lys Ser Gly Glu Val Ser Val Glu Leu Asn Asp Thr Asp Ser Ser Ala
            195                 200                 205
Ala Thr Lys Lys Thr Ala Ala Trp Asn Ser Gly Thr Ser Thr Leu Thr
        210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 105
<211> 822
<212> DNA
<213> Artificial Sequence

<220>
<223> Altered OspA Nucleic Acid

<221> CDS
<222> (1)...(822)

<400> 105
atg aaa aaa tat tta ttg gga ata ggt cta ata tta gcc tta ata gca      48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta      96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
 65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc ttt gtt ctt gaa gga act cta act gct gaa aaa aca     528

Val Leu Lys Gly Phe Val Leu Glu Gly Thr Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 106
<211> 273
<212> PRT
<213> Artificial Sequence

<220>
<223> Altered OspA protein

<400> 106
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Phe Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr

210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 107
<211> 822
<212> DNA
<213> Artificial Sequence

<220>
<223> Altered OspA Nucleic Acid

<221> CDS
<222> (1)...(822)

<400> 107
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc ttt act ctt gaa gga act cta act gct gaa aaa aca     528
Val Leu Lys Gly Phe Thr Leu Glu Gly Thr Leu Thr Ala Glu Lys Thr
                165                 170                 175

aca ttg gtg gtt aaa gaa gga act gtt act tta agc aaa aat att tca     576

Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 108
<211> 273
<212> PRT
<213> Artificial Sequence

<220>
<223> Altered OspA Protein

<400> 108
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Phe Thr Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu

245                 250                 255
Gly Ser Ala Val Glu Ile Thr Lys Leu Asp Glu Ile Lys Asn Ala Leu
            260                 265                 270
Lys

<210> 109
<211> 822
<212> DNA
<213> Artificial Sequence

<220>
<223> Altered OspA Nucleic Acid

<221> CDS
<222> (1)...(822)

<400> 109
atg aaa aaa tat tta ttg gga ata ggt cta ata tta gcc tta ata gca    48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta    96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa    144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa    192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa    240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa    288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa    336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa    384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga    432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag    480
Leu Glu Tyr Thr Gly Ile Lys Ser Asp Gly Ser Gly Lys Ala Lys Glu
145                 150                 155                 160

gtt tta aaa ggc tat act ctt gaa gga act cta act gct gaa aaa aca    528
Val Leu Lys Gly Tyr Thr Leu Glu Gly Thr Leu Thr Ala Glu Lys Thr
                165                 170                 175

aca ttg gtg gtt aaa gaa gga act gtt act tta agc aaa aat att tca    576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct    624

Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 110
<211> 273
<212> PRT
<213> Artificial Sequence

<220>
<223> Altered OspA Protein

<400> 110
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Thr Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 111
<211> 822
<212> DNA
<213> Artificial Sequence

<220>
<223> Altered OspA Nucleic Acid

<221> CDS
<222> (1)...(822)

<400> 111
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc tat act ctt gaa gga aag cta act gct gaa aaa aca     528
Val Leu Lys Gly Tyr Thr Leu Glu Gly Lys Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672

Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 112
<211> 273
<212> PRT
<213> Artificial Sequence

<220>
<223> Altered OspA Protein

<400> 112
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Thr Leu Glu Gly Lys Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 113

<211> 822
<212> DNA
<213> Artificial Sequence

<220>
<223> Altered OspA Nucleic Acid

<221> CDS
<222> (1)...(822)

<400> 113
atg aaa aaa tat tta ttg gga ata ggt cta ata tta gcc tta ata gca     48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta     96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa     144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa     192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa     240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa     288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa     336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa     384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga     432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag     480
Leu Glu Tyr Thr Gly Ile Lys Ser Asp Gly Ser Gly Lys Ala Lys Glu
145                 150                 155                 160

gtt tta aaa ggc ttt act ctt gaa gga aag cta act gct gaa aaa aca     528
Val Leu Lys Gly Phe Thr Leu Glu Gly Lys Leu Thr Ala Glu Lys Thr
                165                 170                 175

aca ttg gtg gtt aaa gaa gga act gtt act tta agc aaa aat att tca     576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct     624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca     672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa     720

Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag     768
Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 114
<211> 273
<212> PRT
<213> Artificial Sequence

<220>
<223> Altered OspA protein

<400> 114
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Phe Thr Leu Glu Gly Lys Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 115
<211> 822
<212> DNA
<213> Artificial Sequence

<220>
<223> Altered OspA Nucleic Acid

<221> CDS
<222> (1)...(822)

<400> 115
atg aaa aaa tat tta ttg gga ata ggt cta ata tta gcc tta ata gca    48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta    96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa    144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa    192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa    240
Gly Thr Ser Asp Lys Asn Asn Gly Ser Gly Val Leu Glu Gly Val Lys
65                  70                  75                  80

gct gac aaa agt aaa gta aaa tta aca att tct gac gat cta ggt caa    288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa    336
Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa    384
Lys Val Thr Ser Lys Asp Lys Ser Ser Thr Glu Glu Lys Phe Asn Glu
        115                 120                 125

aaa ggt gaa gta tct gaa aaa ata ata aca atg gca gac gga acc aga    432
Lys Gly Glu Val Ser Glu Lys Ile Ile Thr Met Ala Asp Gly Thr Arg
    130                 135                 140

ctt gaa tac aca gga att aaa agc gat gga tct gga aaa gct aaa tat    480
Leu Glu Tyr Thr Gly Ile Lys Ser Asp Gly Ser Gly Lys Ala Lys Tyr
145                 150                 155                 160

gtt tta aaa ggc ttt act ctt gaa gga aag cta act gct gaa aaa aca    528
Val Leu Lys Gly Phe Thr Leu Glu Gly Lys Leu Thr Ala Glu Lys Thr
                165                 170                 175

aca ttg gtg gtt aaa gaa gga act gtt act tta agc atg aat att tca    576
Thr Leu Val Val Lys Glu Gly Thr Val Thr Leu Ser Met Asn Ile Ser
            180                 185                 190

aaa tct ggg gaa gtt tca gtt gaa ctt aat gac act gac agt agt gct    624
Lys Ser Gly Glu Val Ser Val Glu Leu Asn Asp Thr Asp Ser Ser Ala
        195                 200                 205

gct act aaa aaa act gca gct tgg aat tca ggc act tca act tta aca    672
Ala Thr Lys Lys Thr Ala Ala Trp Asn Ser Gly Thr Ser Thr Leu Thr
    210                 215                 220

att act gta aac agt aaa aaa act aaa gac ctt gtg ttt aca aaa gaa    720
Ile Thr Val Asn Ser Lys Lys Thr Lys Asp Leu Val Phe Thr Lys Glu
225                 230                 235                 240

aac aca att aca gta caa caa tac gac tca aat ggc acc aaa tta gag    768

Asn Thr Ile Thr Val Gln Gln Tyr Asp Ser Asn Gly Thr Lys Leu Glu
                245                 250                 255

ggg tca gca gtt gaa att aca aaa ctt gat gaa att aaa aac gct tta     816
Gly Ser Ala Val Glu Ile Thr Lys Leu Asp Glu Ile Lys Asn Ala Leu
            260                 265                 270

aaa taa                                                             822
Lys  *

<210> 116
<211> 273
<212> PRT
<213> Artificial Sequence

<220>
<223> Altered OspA protein

<400> 116
Met Lys Lys Tyr Leu Leu Gly Ile Gly Leu Ile Leu Ala Leu Ile Ala
1               5                   10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110
Lys Val Thr Ser Lys Asp Lys Ser Ser Thr Glu Glu Lys Phe Asn Glu
        115                 120                 125
Lys Gly Glu Val Ser Glu Lys Ile Ile Thr Met Ala Asp Gly Thr Arg
    130                 135                 140
Leu Glu Tyr Thr Gly Ile Lys Ser Asp Gly Ser Gly Lys Ala Lys Tyr
145                 150                 155                 160
Val Leu Lys Gly Phe Thr Leu Glu Gly Lys Leu Thr Ala Glu Lys Thr
                165                 170                 175
Thr Leu Val Val Lys Glu Gly Thr Val Thr Leu Ser Met Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ser Gly Thr Ser Thr Leu Thr
    210                 215                 220
Ile Thr Val Asn Ser Lys Lys Thr Lys Asp Leu Val Phe Thr Lys Glu
225                 230                 235                 240
Asn Thr Ile Thr Val Gln Gln Tyr Asp Ser Asn Gly Thr Lys Leu Glu
                245                 250                 255
Gly Ser Ala Val Glu Ile Thr Lys Leu Asp Glu Ile Lys Asn Ala Leu
            260                 265                 270
Lys

<210> 117
<211> 30
<212> DNA
<213> Artificial Sequence

<220>
<223> Oligonucleotide Primer

<400> 117

gaaaaaataa taacaatggc agacggaacc                                    30

<210> 118
<211> 30
<212> DNA
<213> Artificial Sequence

<220>
<223> Oligonucleotide Primer

<400> 118
ggttccgtct gccattgtta ttattttttc                                    30

<210> 119
<211> 27
<212> DNA
<213> Artificial Sequence

<220>
<223> Oligonucleotide Primer

<400> 119
ggaaaagcta aatatgtttt aaaaggc                                       27

<210> 120
<211> 27
<212> DNA
<213> Artificial Sequence

<220>
<223> Oligonucleotide Primer

<400> 120
gccttttaaa acatatttag cttttcc                                       27

<210> 121
<211> 29
<212> DNA
<213> Artificial Sequence

<220>
<223> Oligonucleotide Primer

<400> 121
gttactttaa gcatgaatat ttcaaaatc                                     29

<210> 122
<211> 29
<212> DNA
<213> Artificial Sequence

<220>
<223> Oligonucleotide Primer

<400> 122
gattttgaaa tattcatgct taaagtaac                                     29

<210> 123
<211> 34
<212> DNA
<213> Artificial Sequence

<220>
<223> Oligonucleotide Primer

<400> 123
gaggttttaa aaggctttac tcttgaagga actc                               34

<210> 124
<211> 34
<212> DNA
<213> Artificial Sequence

<220>
<223> Oligonucleotide Primer

<400> 124
gagttccttc aagagtaaag ccttttaaaa cctg                                34

<210> 125
<211> 23
<212> DNA
<213> Artificial Sequence

<220>
<223> Oligonucleotide Primer

<400> 125
tcttgaagga aagctaactg ctg                                            23

<210> 126
<211> 23
<212> DNA
<213> Artificial Sequence

<220>
<223> Oligonucleotide Primer

<400> 126
cagcagttag ctttccttca aga                                            23

<210> 127
<211> 819
<212> DNA
<213> Borrelia burgdorferi

<220>
<221> CDS
<222> (1)...(819)

<400> 127
atg aaa aaa tat tta ttg gga ata ggt cta ata tta gcc tta ata gca    48
Met Lys Lys Tyr Leu Leu Gly Ile Gly Leu Ile Leu Ala Leu Ile Ala
 1               5                  10                  15

tgt aag caa aat gtt agc agc ctt gac gag aaa aac agc gtt tca gta    96
Cys Lys Gln Asn Val Ser Ser Leu Asp Glu Lys Asn Ser Val Ser Val
            20                  25                  30

gat ttg cct ggt gaa atg aaa gtt ctt gta agc aaa gaa aaa aac aaa    144
Asp Leu Pro Gly Glu Met Lys Val Leu Val Ser Lys Glu Lys Asn Lys
        35                  40                  45

gac ggc aag tac gat cta att gca aca gta gac aag ctt gag ctt aaa    192
Asp Gly Lys Tyr Asp Leu Ile Ala Thr Val Asp Lys Leu Glu Leu Lys
    50                  55                  60

gga act tct gat aaa aac aat gga tct gga gta ctt gaa ggc gta aaa    240
Gly Thr Ser Asp Lys Asn Asn Gly Ser Gly Val Leu Glu Gly Val Lys
 65                  70                  75                  80

gct gac aaa agt aaa gta aaa tta aca att tct gac gat cta ggt caa    288
Ala Asp Lys Ser Lys Val Lys Leu Thr Ile Ser Asp Asp Leu Gly Gln
                85                  90                  95

acc aca ctt gaa gtt ttc aaa gaa gat ggc aaa aca cta gta tca aaa    336

Thr Thr Leu Glu Val Phe Lys Glu Asp Gly Lys Thr Leu Val Ser Lys
            100                 105                 110

aaa gta act tcc aaa gac aag tca tca aca gaa gaa aaa ttc aat gaa      384
Lys Val Thr Ser Lys Asp Lys Ser Ser Thr Glu Glu Lys Phe Asn Glu
        115                 120                 125

aaa ggt gaa gta tct gaa aaa ata ata aca aga gca gac gga acc aga      432
Lys Gly Glu Val Ser Glu Lys Ile Ile Thr Arg Ala Asp Gly Thr Arg
    130                 135                 140

ctt gaa tac aca gga att aaa agc gat gga tct gga aaa gct aaa gag      480
Leu Glu Tyr Thr Gly Ile Lys Ser Asp Gly Ser Gly Lys Ala Lys Glu
145                 150                 155                 160

gtt tta aaa ggc tat gtt ctt gaa gga act cta act gct gaa aaa aca      528
Val Leu Lys Gly Tyr Val Leu Glu Gly Thr Leu Thr Ala Glu Lys Thr
                165                 170                 175

aca ttg gtg gtt aaa gaa gga act gtt act tta agc aaa aat att tca      576
Thr Leu Val Val Lys Glu Gly Thr Val Thr Leu Ser Lys Asn Ile Ser
            180                 185                 190

aaa tct ggg gaa gtt tca gtt gaa ctt aat gac act gac agt agt gct      624
Lys Ser Gly Glu Val Ser Val Glu Leu Asn Asp Thr Asp Ser Ser Ala
        195                 200                 205

gct act aaa aaa act gca gct tgg aat gca ggc act tca act tta aca      672
Ala Thr Lys Lys Thr Ala Ala Trp Asn Ala Gly Thr Ser Thr Leu Thr
    210                 215                 220

att act gta aac aac aaa aaa act aaa gcc ctt gta ttt aca aaa caa      720
Ile Thr Val Asn Asn Lys Lys Thr Lys Ala Leu Val Phe Thr Lys Gln
225                 230                 235                 240

gac aca att aca tca caa aaa tac gac tca gca gga acc aac ttg gaa      768
Asp Thr Ile Thr Ser Gln Lys Tyr Asp Ser Ala Gly Thr Asn Leu Glu
                245                 250                 255

ggc aca gca gtc gaa att aaa aca ctt gat gaa ctt aaa aac gct tta      816
Gly Thr Ala Val Glu Ile Lys Thr Leu Asp Glu Leu Lys Asn Ala Leu
            260                 265                 270

aga                                                                  819
Arg

<210> 128
<211> 273
<212> PRT
<213> Borrelia burgdorferi

<400> 128
Met Lys Lys Tyr Leu Leu Gly Ile Gly Leu Ile Leu Ala Leu Ile Ala
 1               5                  10                  15
Cys Lys Gln Asn Val Ser Ser Leu Asp Glu Lys Asn Ser Val Ser Val
            20                  25                  30
Asp Leu Pro Gly Glu Met Lys Val Leu Val Ser Lys Glu Lys Asn Lys
        35                  40                  45
Asp Gly Lys Tyr Asp Leu Ile Ala Thr Val Asp Lys Leu Glu Leu Lys
    50                  55                  60
Gly Thr Ser Asp Lys Asn Asn Gly Ser Gly Val Leu Glu Gly Val Lys
65                  70                  75                  80
Ala Asp Lys Ser Lys Val Lys Leu Thr Ile Ser Asp Asp Leu Gly Gln
                85                  90                  95
Thr Thr Leu Glu Val Phe Lys Glu Asp Gly Lys Thr Leu Val Ser Lys
            100                 105                 110

Lys Val Thr Ser Lys Asp Lys Ser Ser Thr Glu Glu Lys Phe Asn Glu
        115                 120                 125
Lys Gly Glu Val Ser Glu Lys Ile Ile Thr Arg Ala Asp Gly Thr Arg
    130                 135                 140
Leu Glu Tyr Thr Gly Ile Lys Ser Asp Gly Ser Gly Lys Ala Lys Glu
145                 150                 155                 160
Val Leu Lys Gly Tyr Val Leu Glu Gly Thr Leu Thr Ala Glu Lys Thr
                165                 170                 175
Thr Leu Val Val Lys Glu Gly Thr Val Thr Leu Ser Lys Asn Ile Ser
            180                 185                 190
Lys Ser Gly Glu Val Ser Val Glu Leu Asn Asp Thr Asp Ser Ser Ala
        195                 200                 205
Ala Thr Lys Lys Thr Ala Ala Trp Asn Ala Gly Thr Ser Thr Leu Thr
    210                 215                 220
Ile Thr Val Asn Asn Lys Lys Thr Lys Ala Leu Val Phe Thr Lys Gln
225                 230                 235                 240
Asp Thr Ile Thr Ser Gln Lys Tyr Asp Ser Ala Gly Thr Asn Leu Glu
                245                 250                 255
Gly Thr Ala Val Glu Ile Lys Thr Leu Asp Glu Leu Lys Asn Ala Leu
            260                 265                 270
Arg

<210> 129
<211> 9
<212> PRT
<213> Artificial Sequence

<220>
<223> Residue 165-173 of B31 OspA

<400> 129
Tyr Val Leu Glu Gly Thr Leu Thr Ala
1               5

<210> 130
<211> 9
<212> PRT
<213> Artificial Sequence

<220>
<223> Residue 332-340 of hLFA-1

<400> 130
Tyr Val Ile Glu Gly Thr Ser Lys Gln
1               5